# EUROPÄISCHE PATENTANMELDUNG

(11) **EP 1 790 328 A1**
(43) Veröffentlichungstag der Anmeldung: **30.05.2007**
(21) Anmeldenummer: 06021487.1
(22) Anmeldetag: 13.10.2006
(51) Int. Cl.: A61K 8/49, A61K 8/41, A61K 8/55, A61Q 5/02, A61Q 5/06, A61Q 5/10

(54) **Kosmetische Mittel mit Aniontensid(en) und ionischen Flüssigkeiten II**

(30) Priorität: 23.11.2005 DE 102005056157
(71) Anmelder: HENKEL KOMMANDITGESELLSCHAFT AUF AKTIEN, 40589 Düsseldorf (DE)
(72) Erfinder: Oberkobusch, Doris, 40591 Düsseldorf (DE); Höffkes, Horst, 40595 Düsseldorf (DE)

(57) **Zusammenfassung**

Kosmetische Mittel, die unmittelbar vor der Anwendung durch Vermischen einer fließfähigen Zubereitung A und einer fließfähigen Zubereitung B erhalten werden, wobei die Zubereitung A mindestens ein Aniontensid und die Zubereitung B mindestens eine ionische Flüssigkeit enthalten, weisen vorteilhafte Viskositäts- und Schaumeigenschaften auf.

## Beschreibung

Die vorliegende Erfindung betrifft kosmetische Zusammensetzungen, d.h. kosmetische oder dermatologische Zusammensetzungen zur Anwendung auf der Haut, insbesondere menschlicher Haut, sowie zur Anwendung auf Keratinfasern, insbesondere menschlichen Haaren, und deren Verwendung.

Die erfindungsgemäßen Zusammensetzungen sind kosmetische Mittel. Während man früher zwischen Mitteln zur Pflege des menschlichen Körpers und solchen zur Verschönerung seines Aussehens unterschied nach "Körperpflegemitteln" und "dekorativen Kosmetika", werden diese Produkte heute zusammengefaßt definiert als *"kosmetische Mittel*" oder kosmetische Zusammensetzungen.
Angesichts der Allgemeinzugänglichkeit der kosmetischen Mittel und ihrer Anwendung am menschlichen Körper besteht zum Schutz des Verbrauchers in Deutschland und der Europäischen Union ein umfangreiches Regelwerk. Gesetzliche Grundlage in Deutschland ist das Lebensmittel- und Bedarfsgegenstände-Gesetz (LMBG), das kosmetische Mittel in § 4 wie folgt definiert:
"(1) kosmetische Mittel im Sinne dieses Gesetzes sind Stoffe oder Zubereitungen aus Stoffen, die dazu bestimmt sind, äußerlich am Menschen oder in seiner Mundhöhle zur Reinigung, Pflege oder zur Beeinflussung des Aussehens oder des Körpergeruchs oder zur Vermittlung von Geruchseindrücken angewendet zu werden, es sei denn, daß sie überwiegend dazu bestimmt sind, Krankheiten, Leiden, Körperschäden oder krankhafte Beschwerden zu lindern oder zu beseitigen." [§ 4 LMBG ist bisher nicht an die EU-Kosmetikrichtlinie angepasst, die Kosmetika sechs Funktionen (reinigen, parfümieren, Aussehen verändern, Körpergeruch beeinflussen, schützen und in gutem Zustand halten) zuspricht.]
"(2) Den kosmetischen Mitteln stehen Stoffe oder Zubereitungen aus Stoffen zur Reinigung oder Pflege von Zahnersatz gleich.
(3) Als kosmetische Mittel gelten nicht Stoffe oder Zubereitungen aus Stoffen, die zur Beeinflussung der Körperformen bestimmt sind."

Je nach Anwendungsgebiet unterscheidet man daher ein breite Palette kosmetischer Mittel, beispielsweise zur Hautpflege (Badepräparate, Hautwasch- und -reinigungsmittel, Hautpflegemittel, Augenkosmetika, Lippenpflegemittel, Nagelpflegemittel, Intimpflegemittel, Fußpflegemittel), solche mit spezieller Wirkung (Lichtschutzmittel, Hautbräunungsmittel, Depigmentierungsmittel, Desodorantien, Antihidrotika, Haarentfernungsmittel, Rasiermittel, Duftmittel), solche zur Zahn- u. Mundpflege (Zahn- und Mundpflegemittel, Gebißpflegemittel, Prothesenhaftmittel) und solche zur Haarpflege (Haarwaschmittel, Haarpflegemittel, Haarverfestigungsmittel, Haarverformungsmittel, Mittel zur Farbänderung).

Kosmetische Zusammensetzungen im Sinne der vorliegenden Erfindung sind daher beispielsweise ausgewählt aus der Gruppe der Duschgele, Duschbäder, Zahnreinigungsmittel, Mundwässer, Haarshampoos, Haarkonditionierer, konditionierenden Shampoos, Haarsprays, Haarspülungen, Haarkuren, Haarpackungen, Haar-Tonics, Dauerwell-Fixierlösungen, Haarfärbeshampoos, Haarfärbemittel, Haarfestiger, Haarlegemittel, Haarstyling-Zubereitungen, Fönwell-Lotionen, Schaumfestiger, Haargele, Haarwachse oder deren Kombinationen.

Bei allen diesen Mitteln besteht ein Bedarf nach Inhaltsstoffen, die den Zubereitungen weiteren Nutzen verleihen, sei es aus anwendungstechnischer Sicht, aus herstelltechnischer Sicht oder aus Sicht des Verbrauchers, der beispielsweise aufgrund bestimmter Inhaltstoffe ein verbessertes subjektives Empfinden dem kosmetischen Mittel gegenüber entwickelt. Der Schwerpunkt der Leistung eines Inhaltsstoffes kann dabei je nach kosmetischem Mittel auch unterschiedlich sein. So kann ein und derselbe Stoff in einem Shampoo oder einer Spülung pflegende Eigenschaften für die behandelten Haare bewirken, während er in einem Haarfärbemittel darüber hinaus die Anfärbung der Kopfhaut verringert oder die zum Teil aggressive Wirkung des Färbemittels kompensiert.

Es besteht daher auf dem Gebiet der Kosmetik ständig der Wunsch, neue Einsatzstoffe bereitzustellen, die in einzelnen, vorzugsweise in allen Produktkategorien vorteilhafte Wirkungen entfalten.

In einer Vielzahl von kosmetischen Mitteln werden Aniontenside eingesetzt, beispielsweise um eine Reinigungswirkung bei der Anwendung des Kosmetikums zu erzielen, und/oder um andere Inhaltsstoffe stabil in die Formulierung einarbeiten zu können. Anionische Tenside haben sich hier aufgrund ihres Leistungsspektrums als vorteilhaft erwiesen.

Es hat sich gezeigt, daß der Einsatz von Aniontensiden aber auch zu Problemen führt. So sind Mittel, die Aniontenside enthalten, oft nur schwer zu verdicken bzw. in einer höheren Viskosität stabil einzustellen. Auch der Einsatz kationischer Verbindungen, beispielsweise von kationischen Pflegestoffen, ist of erschwert, da sich unlösliche Ausfällungen bilden, die das Produktaussehen und die Produktleistung beeinträchtigen.

Es wurde nun gefunden, daß sich die genannten Probleme beim Einsatz anionischer Tenside überwinden lassen, wenn ionische Flüssigkeiten in die kosmetischen Mittel eingearbeitet werden. Hierbei werden nicht nur die vorstehend genannten Probleme gelöst, sondern es wird dem jeweiligen Kosmetikum eine Vielzahl von weiteren Vorteilen verliehen.

Gegenstand der vorliegenden Erfindung ist demnach ein kosmetisches Mittel, welches unmittelbar vor der Anwendung durch Vermischen einer fließfähigen Zubereitung A und einer fließfähigen Zubereitung B erhalten wird, wobei
a) die Zubereitung A mindestens ein Aniontensid und
b) die Zubereitung B mindestens eine ionische Flüssigkeit enthält.

Erfindungsgemäß enthält die Zubereitung A mindestens ein Aniontensid.

Als anionische Tenside eignen sich für die erfindungsgemäßen Zusammensetzungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glycol- oder Polyglycolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH. in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Acylglutamate der Formel (I), 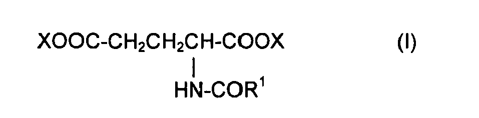 in der R¹CO für einen linearen oder verzweigten Acylrest mit 6 bis 22 Kohlenstoffatomen und 0, 1, 2 oder 3 Doppelbindungen und X für Wasserstoff, ein Alkali- und/oder Erdalkalimetall, Ammonium, Alkylammonium, Alkanolammonium oder Glucammonium steht, beispielsweise Acylglutamate, die sich von Fettsäuren mit 6 bis 22, vorzugsweise 12 bis 18 Kohlenstoffatomen ableiten, wie beispielsweise C_{12/14}- bzw. C_{12/18}-Kokosfettsäure, Laurinsäure, Myristinsäure, Palmitinsäure und/oder Stearinsäure, insbesondere Natrium-N-cocoyl- und Natrium-N-stearoyl-L-glutamat,
- Ester einer hydroxysubstituierten Di- oder Tricarbonsäure der allgemeinen Formel (II), 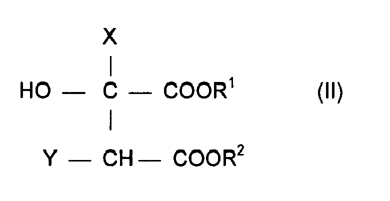 in der X=H oder eine -CH₂COOR-Gruppe ist, Y=H oder -OH ist unter der Bedingung, dass Y=H ist, wenn X=-CH₂COOR ist, R, R¹ und R² unabhängig voneinander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten (C₆-C₁₈)-Alkylpolysaccharide mit 1 bis 6 monomeren Saccharideinheiten und/oder der veretherten aliphatischen (C₆-C₁₆)-Hydroxyalkylpolyole mit 2 bis 16 Hydroxylresten ausgewählt sind, unter der Maßgabe, dass wenigstens eine der Gruppen R, R¹ oder R² ein Rest Z ist,
- Ester der Sulfobernsteinsäure oder der Sulfosuccinate der allgemeinen Formel (III), 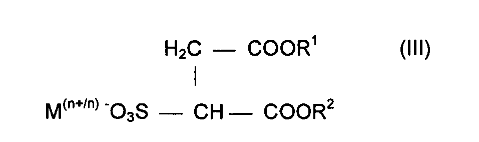 in der M^{(n+/n)} für n = 1 ein Wasserstoffatom, ein Alkalimetallkation, eine Ammoniumgruppe oder das Kation einer ammonium-organischen Base und für n = 2 ein Erdalkalimetallkation darstellt und R¹ und R² unabhängig voneinander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten (C₆-C₁₈)-Alkylpolysaccharide mit 1 bis 6 monomeren Saccharideinheiten und/oder der veretherten aliphatischen (C₆-C₁₆)-Hydro-xyalkylpolyole mit 2 bis 16 Hydroxylresten ausgewählt ist, unter der Maßgabe, dass wenigstens eine der Gruppen R¹ oder R² ein Rest Z ist,
- Sulfobernsteinsäuremono- und -dialkylester mit 8 bis 24 C-Atomen in der Alkylgruppe und Sulfobernsteinsäuremonoalkylpolyoxyethylester mit 8 bis 24 C-Atomen in der Alkylgruppe und 1 bis 6 Oxyethylgruppen,
- Alkylsulfate und Alkylpolyglycolethersulfate der Formel R-(O-CH₂-CH₂)ₓ-OSO₃H, in der R eine bevorzugt lineare Alkylgruppe mit 8 bis 30 C-Atomen und x = 0 oder 1 - 12 ist,
- gemischte oberflächenaktive Hydroxysulfonate gemäß DE-A-37 25 030,
- Ester der Weinsäure und Zitronensäure mit Alkoholen, die Anlagerungsprodukte von etwa 2-15 Molekülen Ethylenoxid und/oder Propylenoxid an C₈₋₂₂-Fettalkohole darstellen,
- Alkyl- und/oder Alkenyletherphosphate,
- sulfatierte Fettsäurealkylenglycolester,
- Monoglyceridsulfate und Monoglyceridethersulfate.

Besonders bevorzugte erfindungsgemäße kosmetische Mittel sind dadurch gekennzeichnet, daß die Zubereitung A als Aniontensid(e) Alkylsulfat(e) und/oder Alkylethersulfat(e), vorzugsweise C₈₋₂₂-Alkylsulfat(e) und/oder C₈₋₂₂-Alkylethersulfat(e) mit 2 bis 20 Ethylenoxideinheiten, besonders bevorzugt C₁₀₋₁₈-Alkylsulfat(e) und/oder C₁₀₋₁₈-Alkylethersulfat(e) mit 3 bis 10 Ethylenoxideinheiten und insbesondere C₁₂₋₁₆-Alkylsulfat(e) und/oder C₁₂₋₁₆-Alkylethersulfat(e) mit 4 bis 8 Ethylenoxideinheiten, vorzugsweise in Mengen von 0,5 bis 60 Gew.-%, besonders bevorzugt von 1 bis 50 Gew.-% und insbesondere von 5 bis 40 Gew.-%, jeweils bezogen auf das Gewicht der Zubereitung A, enthält.

Zusätzlich zu den genannten anionischen Tensiden können die erfindungsgemäßen Mittel - insbesondere in der Zubereitung A - weitere Tenside enthalten.

Bevorzugte weitere anionische Tenside sind Acylglutamate, Acylphosphate, Acylisethionate, Acylsarcosinate und Acyltaurate, jeweils mit einem linearen oder verzweigten Acylrest mit 6 bis 22 Kohlenstoffatomen und 0, 1, 2 oder 3 Doppelbindungen, der in besonders bevorzugten Ausführungsformen aus einem Octanoyl-, Decanoyl-, Lauroyl-, Myristoyl-, Palmitoyl- und Stearoylrest ausgewählt ist, Ester der Weinsäure, Zitronensäure oder Bernsteinsäure bzw. der Salze dieser Säuren mit alkylierter Glucose, insbesondere die Produkte mit der INCI-Bezeichnung Disodium Coco-Glucoside Citrate, Sodium Coco-Glucoside Tartrate und Disodium Coco-Glucoside Sulfosuccinate, Alkylpolyglycolethersulfate und Ethercarbonsäuren mit 8 bis 18 C-Atomen in der Alkylgruppe und bis zu 12 Ethoxygruppen im Molekül, Sulfobernsteinsäuremono- und -dialkylester mit 8 bis 18 C-Atomen in der Alkylgruppe und Sulfobernsteinsäuremonoalkylpolyoxyethylester mit 8 bis 18 C-Atomen in der Alkylgruppe und 1 bis 6 Ethoxygruppen.

Die erfindungsgemäßen Zusammensetzungen können in einer bevorzugten Ausführungsform weitere oberflächenaktive Substanzen enthalten, die, je nach Anwendungsgebiet als Tenside oder als Emulgatoren bezeichnet, aus kationischen, zwitterionischen, ampholytischen und nichtionischen Tensiden und Emulgatoren ausgewählt sind.

Als zwitterionische Tenside und Emulgatoren werden solche oberflächenaktiven Verbindungen bezeichnet, die im Molekül mindestens eine quartäre Ammoniumgruppe und mindestens eine - COO⁽⁻⁾ - oder -SO₃⁽⁻⁾ -Gruppe tragen. Besonders geeignete zwitterionische Tenside und Emulgatoren sind die sogenannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamidderivat.

Unter ampholytischen Tensiden und Emulgatoren werden solche oberflächenaktiven Verbindungen verstanden, die außer einer C₈ - C₂₄ - Alkyl- oder -Acylgruppe mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylaminopropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂- C₁₈ - Acylsarcosin.

Nichtionische Tenside und Emulgatoren enthalten als hydrophile Gruppe z. B. eine Polyolgruppe, eine Polyalkylenglycolethergruppe oder eine Kombination aus Polyol- und Polyglycolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- C₁₂-C₃₀-Fettsäuremono- und -diester von Anlagerungsprodukten von 1 bis 30 Mol Ethylenoxid an Polyole mit 3 bis 6 Kohlenstoffatomen, insbesondere an Glycerin,
- Anlagerungsprodukte von 5 bis 60 Mol Ethylenoxid an Rizinusöl und gehärtetes Rizinusöl,
- Polyolfettsäure(partial)ester, wie Hydagen^{®} HSP (Cognis) oder Sovermol^{®} - Typen (Cognis), insbesondere von gesättigten C₈₋₃₀-Fettsäuren,
- alkoxylierte Triglyceride,
- alkoxylierte Fettsäurealkylester,
- Aminoxide,
- Fettsäurealkanolamide, Fettsäure-N-alkylglucamide und Fettamine sowie deren Ethylenoxid- oder Polyglycerin-Anlagerungsprodukte,
- Sorbitanfettsäureester und Anlagerungsprodukte von Ethylenoxid an Sorbitanfettsäureester wie beispielsweise die Polysorbate,
- Zuckerfettsäureester und Methylglucosid-Fettsäureester sowie deren Ethylenoxid- oder Polyglycerin-Anlagerungsprodukte,
- Alkylpolyglycoside entsprechend der allgemeinen Formel RO-(Z)ₓ wobei R für Alkyl, Z für Zucker sowie x für die Anzahl der Zuckereinheiten steht.
   Besonders bevorzugt sind solche Alkylpolyglycoside, bei denen R
   - im wesentlichen aus C₈- und C₁₀-Alkylgruppen,
   - im wesentlichen aus C₁₂- und C₁₄-Alkylgruppen,
   - im wesentlichen aus C₈- bis C₁₆-Alkylgruppen oder
   - im wesentlichen aus C₁₂- bis C₁₆-Alkylgruppen oder
   - im wesentlichen aus C₁₆ bis C₁₈-Alkylgruppen
   besteht.
Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.
Die erfindungsgemäß verwendbaren Alkylpolyglycoside enthalten im Schnitt 1,1 bis 5 Zuckereinheiten. Alkylpolyglycoside mit x-Werten von 1,1 bis 2,0 sind bevorzugt. Ganz besonders bevorzugt sind Alkylglycoside, bei denen x 1,1 bis 1,8 beträgt.
- Gemische aus Alkyl-(oligo)-glucosiden und Fettalkoholen, z. B. Montanov^{®}68,
- Sterine, z. B. Ergosterin, Stigmasterin, Sitosterin und Mykosterine,
- Phospholipide, z. B. Lecithine bzw. Phosphatidylcholine,
- Polyglycerine und Polyglycerinderivate wie beispielsweise Polyglycerinpoly-12-hydroxystearat (Dehymuls^{®} PGPH) oder Triglycerindiisostearat (Lameform^{®} TGI),
- alkoxylierte Polydialkylsiloxane (INCI-Bezeichnung: Dimethicone Copolyol).

Als bevorzugte nichtionische oberflächenaktive Substanzen haben sich die Alkylpolyglycoside, gegebenenfalls im Gemisch mit Fettalkoholen, alkoxylierte Polydialkylsiloxane, Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen.

Erfindungsgemäß einsetzbar sind weiterhin kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride. Die langen Alkylketten dieser Tenside weisen bevorzugt 10 bis 18 Kohlenstoffatome auf, wie z. B. in Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere bevorzugte kationische Tenside sind die unter den INCI-Bezeichnungen Quaternium-27 und Quaternium-83 bekannten Imidazolium-Verbindungen.

Bevorzugte erfindungsgemäße kosmetische Mittel sind dadurch gekennzeichnet, daß die Zubereitung A zusätzlich Co-Tenside, vorzugsweise Aniontenside aus den Gruppen der Seifen, Ethercarbonsäuren, carboxylierten Alkylglucoside, Sulfosuccinate, Acyllactylate, Fettsäuresarcosinate und/oder Amphotenside, zwitterionische Tenside, Niotenside, beispielsweise Alkylglucoside, Aminoxide, Ethanolamide, enthält.

Als weiteren wesentlichen Inhaltsstoff enthalten die erfindungsgemäßen Mittel mindestens eine ionische Flüssigkeit.

Als wesentlichen Bestandteil enthalten die erfindungsgemäßen Mittel mindestens eine ionische Flüssigkeit. Es ist erfindungsgemäß bevorzugt, die ionische(n) Flüssigkeit(en) innerhalb bestimmter Mengenbereiche einzusetzen. Besonders bevorzugt sind erfindungsgemäße kosmetisches Mittel, bei denen die Zubereitung B bezogen auf ihr Gewicht 0,1 bis 20 Gew.-%, vorzugsweise 0,5 bis 15 Gew.-% und insbesondere 1 bis 12 Gew.-% ionische Flüssigkeit(en) enthält.

Ionische Flüssigkeiten sind Salze mit einem Schmelzpunkt unter 100 °C. Erfindungsgemäß bevorzugt werden ionische Flüssigkeiten mit niedrigen Schmelzpunkten eingesetzt, so daß erfindungsgemäße Mittel, bei denen die ionische Flüssigkeit einen Schmelzpunkt zwischen 5 und 99°C, vorzugsweise zwischen 10 und 95°C, besonders bevorzugt zwischen 15 und 90°C und insbesondere zwischen 20 und 85°C aufweist, bevorzugt sind.

Ionische Flüssigkeiten zeichnen sich durch ihre besonderen Eigenschaften, wie beispielsweise einen praktisch nicht vorhandenem Dampfdruck und eine geringe Viskosität aus.

Ionische Flüssigkeiten sind Salze und umfassen demnach mindestens ein Kation und mindestens ein Anion. In bevorzugten Ausführungsformen der vorliegenden Erfindung ist das Kation der ionischen Flüssigkeit(en) ein organisches Kation. Als Gegenion kommen organische und/oder anorganische Anionen in Frage. Insgesamt sind erfindungsgemäße Mittel bevorzugt, bei denen die ionische Flüssigkeit ein organisches Kation und ein organisches und/oder anorganisches Anion umfaßt.

Ionische Flüssigkeiten sind Salze und umfassen demnach mindestens ein Kation und mindestens ein Anion. In bevorzugten Ausführungsformen der vorliegenden Erfindung ist das Kation der ionischen Flüssigkeit(en) ein organisches Kation. Als Gegenion kommen organische und/oder anorganische Anionen in Frage. Insgesamt sind erfindungsgemäße Mittel bevorzugt, bei denen die ionische Flüssigkeit ein organisches Kation und ein organisches und/oder anorganisches Anion umfaßt.

Als Kationen kommen vorzugsweise solche aus den Gruppen Imidazolium, Pyridinium, Pyrrolidinium, Phosphonium, Ammonium, Guanidinium und Isoronium in Frage. Besonders bevorzugte Kationen für ionische Flüssigkeiten stammen aus den Gruppen der
- monosubstituierten Imidazoliumionen:

- disubstituierten Imidazoliumionen:
- trisubstituierten Imidazoliumionen:
- Pyridiniumionen:
- Pyrolidiniumionen:
- Phosphoniumionen:
- Ammoniumionen:
- Guanidiniumionen:
- Isouroniumionen:

Als Anionen kommen vorzugsweise solche aus den Gruppen der Halogenide, der Sulfate und Sulfonate, der Amide, Imide und Methane, der Borate, der Phosphate und Antimonate, der Anionen von Fettsäuren sowie Trifluoracetate und Cobalttetracarbonyl in Frage.

Bevorzugte Anionen sind Chlorid, Bromid, Iodid, Methylsulfat, Ethylsulfat, Propylsulfat, Butylsulfat, Hexylsulfat, Octylsulfat, Trifluormethansulfonat, Tosylat, Dicyanamid, bis(trifluoromethyl)imid, bis(trifluoromethylsulfonyl)imid, bis(trifluoromethylsulfonyl)methan, tris(trifluoromethylsulfonyl)methid, Tetrafluoroborat, Tetracyanoborat, bis[1,2-benzeneDiolato(2-)-O,O']-borat, [salicylato(2-)]-borat, bis[malonato(2-)]-borat, bis[oxalato(2-)]-borat, bis[2,2'-biphenylDiolato(2-)-O,O']-borat, hexafluorophosphat, bis(pentafluoroethyl)phosphinat, tris-(pentafluoroethyl)trifluorophosphat, tris(heptafluoropropyl)trifluorophosphat, tris(nonafluorobutyl)trifluorophosphat, bis(2,4,4-trimethylpentyl)phosphinat, hexafluoroantimonat, Diethylphosphat.

Bestimmte ionische Flüssigkeiten sind im Rahmen der vorliegenden Erfindung besonders bevorzugt. Hier sind erfindungsgemäße kosmetisches Mittel bevorzugt, bei denen die Zubereitung B mindestens eine ionische Flüssigkeit enthält, die ausgewählt ist aus 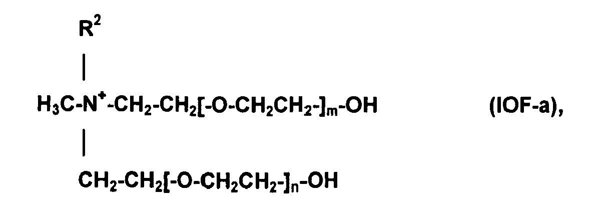 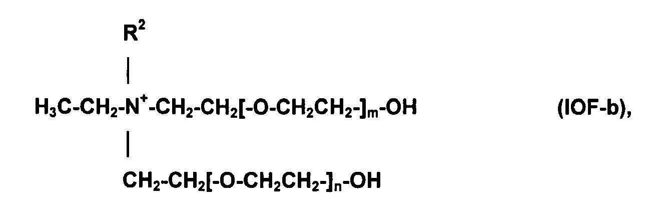 in denen die Indices m und n unabhängig voneinander jeweils für Werte von 1 bis 15, vorzugsweise von 1, 2, 3, 4, 5, 6, 7, 8, 9, 10, stehen, das Gegenion ausgewählt ist aus Chlorid, Bromid, Methosulfat, Ethosulfat und der Rest R² ein gesättigter oder ungesättigter langkettiger Fettrest ist.

Besonders bevorzugte ionische Flüssigkeiten werden durch die Formeln IOF-2a bzw. IOF 2-b beschrieben und sind in den nachstehenden Tabellen aufgeführt. Dabei können selbstverständlich auch Mischungen der genannten ionischen Flüssigkeiten in den erfindungsgemäßen Mitteln enthalten sein. Dies ist beispielsweise dann der Fall, wenn langkettige Alky- bzw. Alkeny-Reste aus nativen Quellen eingesetzt werden, da der Anteil der verschiedenen Fettsäuren in den Fetten je nach Herkunft variiert.

**Tabelle 1: ionische Flüssigkeiten der Formeln IOF-a und IOF-b:**

| **Nr.** | **R²** | **m** | **n** | **Gegenion** |
|---|---|---|---|---|
| 1 | H₃C-(CH₂)₅- | 1 | 1 | Chlorid |
| 2 | H₃C-(CH₂)₅- | 2 | 1 | Chlorid |
| 3 | H₃C-(CH₂)₅- | 3 | 1 | Chlorid |
| 4 | H₃C-(CH₂)₅- | 4 | 1 | Chlorid |
| 5 | H₃C-(CH₂)₅- | 5 | 1 | Chlorid |
| 6 | H₃C-(CH₂)₅- | 6 | 1 | Chlorid |
| 7 | H₃C-(CH₂)₅- | 7 | 1 | Chlorid |
| 8 | H₃C-(CH₂)₅- | 8 | 1 | Chlorid |
| 9 | H₃C-(CH₂)₅- | 9 | 1 | Chlorid |
| 10 | H₃C-(CH₂)₅- | 10 | 1 | Chlorid |
| 11 | H₃C-(CH₂)₅- | 2 | 2 | Chlorid |
| 12 | H₃C-(CH₂)₅- | 3 | 2 | Chlorid |
| 13 | H₃C-(CH₂)₅- | 4 | 2 | Chlorid |
| 14 | H₃C-(CH₂)₅- | 5 | 2 | Chlorid |
| 15 | H₃C-(CH₂)₅- | 6 | 2 | Chlorid |
| 16 | H₃C-(CH₂)₅- | 7 | 2 | Chlorid |
| 17 | H₃C-(CH₂)₅- | 8 | 2 | Chlorid |
| 18 | H₃C-(CH₂)₅- | 9 | 2 | Chlorid |
| 19 | H₃C-(CH₂)₅- | 10 | 2 | Chlorid |
| 20 | H₃C-(CH₂)₅- | 3 | 3 | Chlorid |
| 21 | H₃C-(CH₂)₅- | 4 | 3 | Chlorid |
| 22 | H₃C-(CH₂)₅- | 5 | 3 | Chlorid |
| 23 | H₃C-(CH₂)₅- | 6 | 3 | Chlorid |
| 24 | H₃C-(CH₂)₅- | 7 | 3 | Chlorid |
| 25 | H₃C-(CH₂)₅- | 8 | 3 | Chlorid |
| 26 | H₃C-(CH₂)₅- | 9 | 3 | Chlorid |
| 27 | H₃C-(CH₂)₅- | 10 | 3 | Chlorid |
| 28 | H₃C-(CH₂)₅- | 4 | 4 | Chlorid |
| 29 | H₃C-(CH₂)₅- | 5 | 4 | Chlorid |
| 30 | H₃C-(CH₂)₅- | 6 | 4 | Chlorid |
| 31 | H₃C-(CH₂)₅- | 7 | 4 | Chlorid |
| 32 | H₃C-(CH₂)₅- | 8 | 4 | Chlorid |
| 33 | H₃C-(CH₂)₅- | 9 | 4 | Chlorid |
| 34 | H₃C-(CH₂)₅- | 10 | 4 | Chlorid |
| 35 | H₃C-(CH₂)₅- | 5 | 5 | Chlorid |
| 36 | H₃C-(CH₂)₅- | 6 | 5 | Chlorid |
| 37 | H₃C-(CH₂)₅- | 7 | 5 | Chlorid |
| 38 | H₃C-(CH₂)₅- | 8 | 5 | Chlorid |
| 39 | H₃C-(CH₂)₅- | 9 | 5 | Chlorid |
| 40 | H₃C-(CH₂)₅- | 10 | 5 | Chlorid |
| 41 | H₃C-(CH₂)₅- | 6 | 6 | Chlorid |
| 42 | H₃C-(CH₂)₅- | 7 | 6 | Chlorid |
| 43 | H₃C-(CH₂)₅- | 8 | 6 | Chlorid |
| 44 | H₃C-(CH₂)₅- | 9 | 6 | Chlorid |
| 45 | H₃C-(CH₂)₅- | 10 | 6 | Chlorid |
| 46 | H₃C-(CH₂)₅- | 7 | 7 | Chlorid |
| 47 | H₃C-(CH₂)₅- | 8 | 7 | Chlorid |
| 48 | H₃C-(CH₂)₅- | 9 | 7 | Chlorid |
| 49 | H₃C-(CH₂)₅- | 10 | 7 | Chlorid |
| 50 | H₃C-(CH₂)₅- | 8 | 8 | Chlorid |
| 51 | H₃C-(CH₂)₅- | 9 | 8 | Chlorid |
| 52 | H₃C-(CH₂)₅- | 10 | 8 | Chlorid |
| 53 | H₃C-(CH₂)₅- | 9 | 9 | Chlorid |
| 54 | H₃C-(CH₂)₅- | 10 | 9 | Chlorid |
| 55 | H₃C-(CH₂)₅- | 10 | 10 | Chlorid |
| 56 | H₃C-(CH₂)₅- | 1 | 1 | Bromid |
| 57 | H₃C-(CH₂)₅- | 2 | 1 | Bromid |
| 58 | H₃C-(CH₂)₅- | 3 | 1 | Bromid |
| 59 | H₃C-(CH₂)₅- | 4 | 1 | Bromid |
| 60 | H₃C-(CH₂)₅- | 5 | 1 | Bromid |
| 61 | H₃C-(CH₂)₅- | 6 | 1 | Bromid |
| 62 | H₃C-(CH₂)₅- | 7 | 1 | Bromid |
| 63 | H₃C-(CH₂)₅- | 8 | 1 | Bromid |
| 64 | H₃C-(CH₂)₅- | 9 | 1 | Bromid |
| 65 | H₃C-(CH₂)₅- | 10 | 1 | Bromid |
| 66 | H₃C-(CH₂)₅- | 2 | 2 | Bromid |
| 67 | H₃C-(CH₂)₅- | 3 | 2 | Bromid |
| 68 | H₃C-(CH₂)₅- | 4 | 2 | Bromid |
| 69 | H₃C-(CH₂)₅- | 5 | 2 | Bromid |
| 70 | H₃C-(CH₂)₅- | 6 | 2 | Bromid |
| 71 | H₃C-(CH₂)₅- | 7 | 2 | Bromid |
| 72 | H₃C-(CH₂)₅- | 8 | 2 | Bromid |
| 73 | H₃C-(CH₂)₅- | 9 | 2 | Bromid |
| 74 | H₃C-(CH₂)₅- | 10 | 2 | Bromid |
| 75 | H₃C-(CH₂)₅- | 3 | 3 | Bromid |
| 76 | H₃C-(CH₂)₅- | 4 | 3 | Bromid |
| 77 | H₃C-(CH₂)₅- | 5 | 3 | Bromid |
| 78 | H₃C-(CH₂)₅- | 6 | 3 | Bromid |
| 79 | H₃C-(CH₂)₅- | 7 | 3 | Bromid |
| 80 | H₃C-(CH₂)₅- | 8 | 3 | Bromid |
| 81 | H₃C-(CH₂)₅- | 9 | 3 | Bromid |
| 82 | H₃C-(CH₂)₅- | 10 | 3 | Bromid |
| 83 | H₃C-(CH₂)₅- | 4 | 4 | Bromid |
| 84 | H₃C-(CH₂)₅- | 5 | 4 | Bromid |
| 85 | H₃C-(CH₂)₅- | 6 | 4 | Bromid |
| 86 | H₃C-(CH₂)₅- | 7 | 4 | Bromid |
| 87 | H₃C-(CH₂)₅- | 8 | 4 | Bromid |
| 88 | H₃C-(CH₂)₅- | 9 | 4 | Bromid |
| 89 | H₃C-(CH₂)₅- | 10 | 4 | Bromid |
| 90 | H₃C-(CH₂)₅- | 5 | 5 | Bromid |
| 91 | H₃C-(CH₂)₅- | 6 | 5 | Bromid |
| 92 | H₃C-(CH₂)₅- | 7 | 5 | Bromid |
| 93 | H₃C-(CH₂)₅- | 8 | 5 | Bromid |
| 94 | H₃C-(CH₂)₅- | 9 | 5 | Bromid |
| 95 | H₃C-(CH₂)₅- | 10 | 5 | Bromid |
| 96 | H₃C-(CH₂)₅- | 6 | 6 | Bromid |
| 97 | H₃C-(CH₂)₅- | 7 | 6 | Bromid |
| 98 | H₃C-(CH₂)₅- | 8 | 6 | Bromid |
| 99 | H₃C-(CH₂)₅- | 9 | 6 | Bromid |
| 100 | H₃C-(CH₂)₅- | 10 | 6 | Bromid |
| 101 | H₃C-(CH₂)₅- | 7 | 7 | Bromid |
| 102 | H₃C-(CH₂)₅- | 8 | 7 | Bromid |
| 103 | H₃C-(CH₂)₅- | 9 | 7 | Bromid |
| 104 | H₃C-(CH₂)₅- | 10 | 7 | Bromid |
| 105 | H₃C-(CH₂)₅- | 8 | 8 | Bromid |
| 106 | H₃C-(CH₂)₅- | 9 | 8 | Bromid |
| 107 | H₃C-(CH₂)₅- | 10 | 8 | Bromid |
| 108 | H₃C-(CH₂)₅- | 9 | 9 | Bromid |
| 109 | H₃C-(CH₂)₅- | 10 | 9 | Bromid |
| 110 | H₃C-(CH₂)₅- | 10 | 10 | Bromid |
| 111 | H₃C-(CH₂)₅- | 1 | 1 | H₃C-OSO₃⁻ |
| 112 | H₃C-(CH₂)₅- | 2 | 1 | H₃C-OSO₃⁻ |
| 113 | H₃C-(CH₂)₅- | 3 | 1 | H₃C-OSO₃⁻ |
| 114 | H₃C-(CH₂)₅- | 4 | 1 | H₃C-OSO₃⁻ |
| 115 | H₃C-(CH₂)₅- | 5 | 1 | H₃C-OSO₃⁻ |
| 116 | H₃C-(CH₂)₅- | 6 | 1 | H₃C-OSO₃⁻ |
| 117 | H₃C-(CH₂)₅- | 7 | 1 | H₃C-OSO₃⁻ |
| 118 | H₃C-(CH₂)₅- | 8 | 1 | H₃C-OSO₃⁻ |
| 119 | H₃C-(CH₂)₅- | 9 | 1 | H₃C-OSO₃⁻ |
| 120 | H₃C-(CH₂)₅- | 10 | 1 | H₃C-OSO₃⁻ |
| 121 | H₃C-(CH₂)₅- | 2 | 2 | H₃C-OSO₃⁻ |
| 122 | H₃C-(CH₂)₅- | 3 | 2 | H₃C-OSO₃⁻ |
| 123 | H₃C-(CH₂)₅- | 4 | 2 | H₃C-OSO₃⁻ |
| 124 | H₃C-(CH₂)₅- | 5 | 2 | H₃C-OSO₃⁻ |
| 125 | H₃C-(CH₂)₅- | 6 | 2 | H₃C-OSO₃⁻ |
| 126 | H₃C-(CH₂)₅- | 7 | 2 | H₃C-OSO₃⁻ |
| 127 | H₃C-(CH₂)₅- | 8 | 2 | H₃C-OSO₃⁻ |
| 128 | H₃C-(CH₂)₅- | 9 | 2 | H₃C-OSO₃⁻ |
| 129 | H₃C-(CH₂)₅- | 10 | 2 | H₃C-OSO₃⁻ |
| 130 | H₃C-(CH₂)₅- | 3 | 3 | H₃C-OSO₃⁻ |
| 131 | H₃C-(CH₂)₅- | 4 | 3 | H₃C-OSO₃⁻ |
| 132 | H₃C-(CH₂)₅- | 5 | 3 | H₃C-OSO₃⁻ |
| 133 | H₃C-(CH₂)₅- | 6 | 3 | H₃C-OSO₃⁻ |
| 134 | H₃C-(CH₂)₅- | 7 | 3 | H₃C-OSO₃⁻ |
| 135 | H₃C-(CH₂)₅- | 8 | 3 | H₃C-OSO₃⁻ |
| 136 | H₃C-(CH₂)₅- | 9 | 3 | H₃C-OSO₃⁻ |
| 137 | H₃C-(CH₂)₅- | 10 | 3 | H₃C-OSO₃⁻ |
| 138 | H₃C-(CH₂)₅- | 4 | 4 | H₃C-OSO₃⁻ |
| 139 | H₃C-(CH₂)₅- | 5 | 4 | H₃C-OSO₃⁻ |
| 140 | H₃C-(CH₂)₅- | 6 | 4 | H₃C-OSO₃⁻ |
| 141 | H₃C-(CH₂)₅- | 7 | 4 | H₃C-OSO₃⁻ |
| 142 | H₃C-(CH₂)₅- | 8 | 4 | H₃C-OSO₃⁻ |
| 143 | H₃C-(CH₂)₅- | 9 | 4 | H₃C-OSO₃⁻ |
| 144 | H₃C-(CH₂)₅- | 10 | 4 | H₃C-OSO₃⁻ |
| 145 | H₃C-(CH₂)₅- | 5 | 5 | H₃C-OSO₃⁻ |
| 146 | H₃C-(CH₂)₅- | 6 | 5 | H₃C-OSO₃⁻ |
| 147 | H₃C-(CH₂)₅- | 7 | 5 | H₃C-OSO₃⁻ |
| 148 | H₃C-(CH₂)₅- | 8 | 5 | H₃C-OSO₃⁻ |
| 149 | H₃C-(CH₂)₅- | 9 | 5 | H₃C-OSO₃⁻ |
| 150 | H₃C-(CH₂)₅- | 10 | 5 | H₃C-OSO₃⁻ |
| 151 | H₃C-(CH₂)₅- | 6 | 6 | H₃C-OSO₃⁻ |
| 152 | H₃C-(CH₂)₅- | 7 | 6 | H₃C-OSO₃⁻ |
| 153 | H₃C-(CH₂)₅- | 8 | 6 | H₃C-OSO₃⁻ |
| 154 | H₃C-(CH₂)₅- | 9 | 6 | H₃C-OSO₃⁻ |
| 155 | H₃C-(CH₂)₅- | 10 | 6 | H₃C-OSO₃⁻ |
| 156 | H₃C-(CH₂)₅- | 7 | 7 | H₃C-OSO₃⁻ |
| 157 | H₃C-(CH₂)₅- | 8 | 7 | H₃C-OSO₃⁻ |
| 158 | H₃C-(CH₂)₅- | 9 | 7 | H₃C-OSO₃⁻ |
| 159 | H₃C-(CH₂)₅- | 10 | 7 | H₃C-OSO₃⁻ |
| 160 | H₃C-(CH₂)₅- | 8 | 8 | H₃C-OSO₃⁻ |
| 161 | H₃C-(CH₂)₅- | 9 | 8 | H₃C-OSO₃⁻ |
| 162 | H₃C-(CH₂)₅- | 10 | 8 | H₃C-OSO₃⁻ |
| 163 | H₃C-(CH₂)₅- | 9 | 9 | H₃C-OSO₃⁻ |
| 164 | H₃C-(CH₂)₅- | 10 | 9 | H₃C-OSO₃⁻ |
| 165 | H₃C-(CH₂)₅- | 10 | 10 | H₃C-OSO₃⁻ |
| 166 | H₃C-(CH₂)₅- | 1 | 1 | H₃C-CH₂-OSO₃⁻ |
| 167 | H₃C-(CH₂)₅- | 2 | 1 | H₃C-CH₂-OSO₃⁻ |
| 168 | H₃C-(CH₂)₅- | 3 | 1 | H₃C-CH₂-OSO₃⁻ |
| 169 | H₃C-(CH₂)₅- | 4 | 1 | H₃C-CH₂-OSO₃⁻ |
| 170 | H₃C-(CH₂)₅- | 5 | 1 | H₃C-CH₂-OSO₃⁻ |
| 171 | H₃C-(CH₂)₅- | 6 | 1 | H₃C-CH₂-OSO₃⁻ |
| 172 | H₃C-(CH₂)₅- | 7 | 1 | H₃C-CH₂-OSO₃⁻ |
| 173 | H₃C-(CH₂)₅- | 8 | 1 | H₃C-CH₂-OSO₃⁻ |
| 174 | H₃C-(CH₂)₅- | 9 | 1 | H₃C-CH₂-OSO₃⁻ |
| 175 | H₃C-(CH₂)₅- | 10 | 1 | H₃C-CH₂-OSO₃⁻ |
| 176 | H₃C-(CH₂)₅- | 2 | 2 | H₃C-CH₂-OSO₃⁻ |
| 177 | H₃C-(CH₂)₅- | 3 | 2 | H₃C-CH₂-OSO₃⁻ |
| 178 | H₃C-(CH₂)₅- | 4 | 2 | H₃C-CH₂-OSO₃⁻ |
| 179 | H₃C-(CH₂)₅- | 5 | 2 | H₃C-CH₂-OSO₃⁻ |
| 180 | H₃C-(CH₂)₅- | 6 | 2 | H₃C-CH₂-OSO₃⁻ |
| 181 | H₃C-(CH₂)₅- | 7 | 2 | H₃C-CH₂-OSO₃⁻ |
| 182 | H₃C-(CH₂)₅- | 8 | 2 | H₃C-CH₂-OSO₃⁻ |
| 183 | H₃C-(CH₂)₅- | 9 | 2 | H₃C-CH₂-OSO₃⁻ |
| 184 | H₃C-(CH₂)₅- | 10 | 2 | H₃C-CH₂-OSO₃⁻ |
| 185 | H₃C-(CH₂)₅- | 3 | 3 | H₃C-CH₂-OSO₃⁻ |
| 186 | H₃C-(CH₂)₅- | 4 | 3 | H₃C-CH₂-OSO₃⁻ |
| 187 | H₃C-(CH₂)₅- | 5 | 3 | H₃C-CH₂-OSO₃⁻ |
| 188 | H₃C-(CH₂)₅- | 6 | 3 | H₃C-CH₂-OSO₃⁻ |
| 189 | H₃C-(CH₂)₅- | 7 | 3 | H₃C-CH₂-OSO₃⁻ |
| 190 | H₃C-(CH₂)₅- | 8 | 3 | H₃C-CH₂-OSO₃⁻ |
| 191 | H₃C-(CH₂)₅- | 9 | 3 | H₃C-CH₂-OSO₃⁻ |
| 192 | H₃C-(CH₂)₅- | 10 | 3 | H₃C-CH₂-OSO₃⁻ |
| 193 | H₃C-(CH₂)₅- | 4 | 4 | H₃C-CH₂-OSO₃⁻ |
| 194 | H₃C-(CH₂)₅- | 5 | 4 | H₃C-CH₂-OSO₃⁻ |
| 195 | H₃C-(CH₂)₅- | 6 | 4 | H₃C-CH₂-OSO₃⁻ |
| 196 | H₃C-(CH₂)₅- | 7 | 4 | H₃C-CH₂-OSO₃⁻ |
| 197 | H₃C-(CH₂)₅- | 8 | 4 | H₃C-CH₂-OSO₃⁻ |
| 198 | H₃C-(CH₂)₅- | 9 | 4 | H₃C-CH₂-OSO₃⁻ |
| 199 | H₃C-(CH₂)₅- | 10 | 4 | H₃C-CH₂-OSO₃⁻ |
| 200 | H₃C-(CH₂)₅- | 5 | 5 | H₃C-CH₂-OSO₃⁻ |
| 201 | H₃C-(CH₂)₅- | 6 | 5 | H₃C-CH₂-OSO₃⁻ |
| 202 | H₃C-(CH₂)₅- | 7 | 5 | H₃C-CH₂-OSO₃⁻ |
| 203 | H₃C-(CH₂)₅- | 8 | 5 | H₃C-CH₂-OSO₃⁻ |
| 204 | H₃C-(CH₂)₅- | 9 | 5 | H₃C-CH₂-OSO₃⁻ |
| 205 | H₃C-(CH₂)₅- | 10 | 5 | H₃C-CH₂-OSO₃⁻ |
| 206 | H₃C-(CH₂)₅- | 6 | 6 | H₃C-CH₂-OSO₃⁻ |
| 207 | H₃C-(CH₂)₅- | 7 | 6 | H₃C-CH₂-OSO₃⁻ |
| 208 | H₃C-(CH₂)₅- | 8 | 6 | H₃C-CH₂-OSO₃⁻ |
| 209 | H₃C-(CH₂)₅- | 9 | 6 | H₃C-CH₂-OSO₃⁻ |
| 210 | H₃C-(CH₂)₅- | 10 | 6 | H₃C-CH₂-OSO₃⁻ |
| 211 | H₃C-(CH₂)₅- | 7 | 7 | H₃C-CH₂-OSO₃⁻ |
| 212 | H₃C-(CH₂)₅- | 8 | 7 | H₃C-CH₂-OSO₃⁻ |
| 213 | H₃C-(CH₂)₅- | 9 | 7 | H₃C-CH₂-OSO₃⁻ |
| 214 | H₃C-(CH₂)₅- | 10 | 7 | H₃C-CH₂-OSO₃⁻ |
| 215 | H₃C-(CH₂)₅- | 8 | 8 | H₃C-CH₂-OSO₃⁻ |
| 216 | H₃C-(CH₂)₅- | 9 | 8 | H₃C-CH₂-OSO₃⁻ |
| 217 | H₃C-(CH₂)₅- | 10 | 8 | H₃C-CH₂-OSO₃⁻ |
| 218 | H₃C-(CH₂)₅- | 9 | 9 | H₃C-CH₂-OSO₃⁻ |
| 219 | H₃C-(CH₂)₅- | 10 | 9 | H₃C-CH₂-OSO₃⁻ |
| 220 | H₃C-(CH₂)₅- | 10 | 10 | H₃C-CH₂-OSO₃⁻ |
| 221 | H₃C-(CH₂)₇- | 1 | 1 | Chlorid |
| 222 | H₃C-(CH₂)₇- | 2 | 1 | Chlorid |
| 223 | H₃C-(CH₂)₇- | 3 | 1 | Chlorid |
| 224 | H₃C-(CH₂)₇- | 4 | 1 | Chlorid |
| 225 | H₃C-(CH₂)₇- | 5 | 1 | Chlorid |
| 226 | H₃C-(CH₂)₇- | 6 | 1 | Chlorid |
| 227 | H₃C-(CH₂)₇- | 7 | 1 | Chlorid |
| 228 | H₃C-(CH₂)₇- | 8 | 1 | Chlorid |
| 229 | H₃C-(CH₂)₇- | 9 | 1 | Chlorid |
| 230 | H₃C-(CH₂)₇- | 10 | 1 | Chlorid |
| 231 | H₃C-(CH₂)₇- | 2 | 2 | Chlorid |
| 232 | H₃C-(CH₂)₇- | 3 | 2 | Chlorid |
| 233 | H₃C-(CH₂)₇- | 4 | 2 | Chlorid |
| 234 | H₃C-(CH₂)₇- | 5 | 2 | Chlorid |
| 235 | H₃C-(CH₂)₇- | 6 | 2 | Chlorid |
| 236 | H₃C-(CH₂)₇- | 7 | 2 | Chlorid |
| 237 | H₃C-(CH₂)₇- | 8 | 2 | Chlorid |
| 238 | H₃C-(CH₂)₇- | 9 | 2 | Chlorid |
| 239 | H₃C-(CH₂)₇- | 10 | 2 | Chlorid |
| 240 | H₃C-(CH₂)₇- | 3 | 3 | Chlorid |
| 241 | H₃C-(CH₂)₇- | 4 | 3 | Chlorid |
| 242 | H₃C-(CH₂)₇- | 5 | 3 | Chlorid |
| 243 | H₃C-(CH₂)₇- | 6 | 3 | Chlorid |
| 244 | H₃C-(CH₂)₇- | 7 | 3 | Chlorid |
| 245 | H₃C-(CH₂)₇- | 8 | 3 | Chlorid |
| 246 | H₃C-(CH₂)₇- | 9 | 3 | Chlorid |
| 247 | H₃C-(CH₂)₇- | 10 | 3 | Chlorid |
| 248 | H₃C-(CH₂)₇- | 4 | 4 | Chlorid |
| 249 | H₃C-(CH₂)₇- | 5 | 4 | Chlorid |
| 250 | H₃C-(CH₂)₇- | 6 | 4 | Chlorid |
| 251 | H₃C-(CH₂)₇- | 7 | 4 | Chlorid |
| 252 | H₃C-(CH₂)₇- | 8 | 4 | Chlorid |
| 253 | H₃C-(CH₂)₇- | 9 | 4 | Chlorid |
| 254 | H₃C-(CH₂)₇- | 10 | 4 | Chlorid |
| 255 | H₃C-(CH₂)₇- | 5 | 5 | Chlorid |
| 256 | H₃C-(CH₂)₇- | 6 | 5 | Chlorid |
| 257 | H₃C-(CH₂)₇- | 7 | 5 | Chlorid |
| 258 | H₃C-(CH₂)₇- | 8 | 5 | Chlorid |
| 259 | H₃C-(CH₂)₇- | 9 | 5 | Chlorid |
| 260 | H₃C-(CH₂)₇- | 10 | 5 | Chlorid |
| 261 | H₃C-(CH₂)₇- | 6 | 6 | Chlorid |
| 262 | H₃C-(CH₂)₇- | 7 | 6 | Chlorid |
| 263 | H₃C-(CH₂)₇- | 8 | 6 | Chlorid |
| 264 | H₃C-(CH₂)₇- | 9 | 6 | Chlorid |
| 265 | H₃C-(CH₂)₇- | 10 | 6 | Chlorid |
| 266 | H₃C-(CH₂)₇- | 7 | 7 | Chlorid |
| 267 | H₃C-(CH₂)₇- | 8 | 7 | Chlorid |
| 268 | H₃C-(CH₂)₇- | 9 | 7 | Chlorid |
| 269 | H₃C-(CH₂)₇- | 10 | 7 | Chlorid |
| 270 | H₃C-(CH₂)₇- | 8 | 8 | Chlorid |
| 271 | H₃C-(CH₂)₇- | 9 | 8 | Chlorid |
| 272 | H₃C-(CH₂)₇- | 10 | 8 | Chlorid |
| 273 | H₃C-(CH₂)₇- | 9 | 9 | Chlorid |
| 274 | H₃C-(CH₂)₇- | 10 | 9 | Chlorid |
| 275 | H₃C-(CH₂)₇- | 10 | 10 | Chlorid |
| 276 | H₃C-(CH₂)₇- | 1 | 1 | Bromid |
| 277 | H₃C-(CH₂)₇- | 2 | 1 | Bromid |
| 278 | H₃C-(CH₂)₇- | 3 | 1 | Bromid |
| 279 | H₃C-(CH₂)₇- | 4 | 1 | Bromid |
| 280 | H₃C-(CH₂)₇- | 5 | 1 | Bromid |
| 281 | H₃C-(CH₂)₇- | 6 | 1 | Bromid |
| 282 | H₃C-(CH₂)₇- | 7 | 1 | Bromid |
| 283 | H₃C-(CH₂)₇- | 8 | 1 | Bromid |
| 284 | H₃C-(CH₂)₇- | 9 | 1 | Bromid |
| 285 | H₃C-(CH₂)₇- | 10 | 1 | Bromid |
| 286 | H₃C-(CH₂)₇- | 2 | 2 | Bromid |
| 287 | H₃C-(CH₂)₇- | 3 | 2 | Bromid |
| 288 | H₃C-(CH₂)₇- | 4 | 2 | Bromid |
| 289 | H₃C-(CH₂)₇- | 5 | 2 | Bromid |
| 290 | H₃C-(CH₂)₇- | 6 | 2 | Bromid |
| 291 | H₃C-(CH₂)₇- | 7 | 2 | Bromid |
| 292 | H₃C-(CH₂)₇- | 8 | 2 | Bromid |
| 293 | H₃C-(CH₂)₇- | 9 | 2 | Bromid |
| 294 | H₃C-(CH₂)₇- | 10 | 2 | Bromid |
| 295 | H₃C-(CH₂)₇- | 3 | 3 | Bromid |
| 296 | H₃C-(CH₂)₇- | 4 | 3 | Bromid |
| 297 | H₃C-(CH₂)₇- | 5 | 3 | Bromid |
| 298 | H₃C-(CH₂)₇- | 6 | 3 | Bromid |
| 299 | H₃C-(CH₂)₇- | 7 | 3 | Bromid |
| 300 | H₃C-(CH₂)₇- | 8 | 3 | Bromid |
| 301 | H₃C-(CH₂)₇- | 9 | 3 | Bromid |
| 302 | H₃C-(CH₂)₇- | 10 | 3 | Bromid |
| 303 | H₃C-(CH₂)₇- | 4 | 4 | Bromid |
| 304 | H₃C-(CH₂)₇- | 5 | 4 | Bromid |
| 305 | H₃C-(CH₂)₇- | 6 | 4 | Bromid |
| 306 | H₃C-(CH₂)₇- | 7 | 4 | Bromid |
| 307 | H₃C-(CH₂)₇- | 8 | 4 | Bromid |
| 308 | H₃C-(CH₂)₇- | 9 | 4 | Bromid |
| 309 | H₃C-(CH₂)₇- | 10 | 4 | Bromid |
| 310 | H₃C-(CH₂)₇- | 5 | 5 | Bromid |
| 311 | H₃C-(CH₂)₇- | 6 | 5 | Bromid |
| 312 | H₃C-(CH₂)₇- | 7 | 5 | Bromid |
| 313 | H₃C-(CH₂)₇- | 8 | 5 | Bromid |
| 314 | H₃C-(CH₂)₇- | 9 | 5 | Bromid |
| 315 | H₃C-(CH₂)₇- | 10 | 5 | Bromid |
| 316 | H₃C-(CH₂)₇- | 6 | 6 | Bromid |
| 317 | H₃C-(CH₂)₇- | 7 | 6 | Bromid |
| 318 | H₃C-(CH₂)₇- | 8 | 6 | Bromid |
| 319 | H₃C-(CH₂)₇- | 9 | 6 | Bromid |
| 320 | H₃C-(CH₂)₇- | 10 | 6 | Bromid |
| 321 | H₃C-(CH₂)₇- | 7 | 7 | Bromid |
| 322 | H₃C-(CH₂)₇- | 8 | 7 | Bromid |
| 323 | H₃C-(CH₂)₇- | 9 | 7 | Bromid |
| 324 | H₃C-(CH₂)₇- | 10 | 7 | Bromid |
| 325 | H₃C-(CH₂)₇- | 8 | 8 | Bromid |
| 326 | H₃C-(CH₂)₇- | 9 | 8 | Bromid |
| 327 | H₃C-(CH₂)₇- | 10 | 8 | Bromid |
| 328 | H₃C-(CH₂)₇- | 9 | 9 | Bromid |
| 329 | H₃C-(CH₂)₇- | 10 | 9 | Bromid |
| 330 | H₃C-(CH₂)₇- | 10 | 10 | Bromid |
| 331 | H₃C-(CH₂)₇- | 1 | 1 | H₃C-OSO₃⁻ |
| 332 | H₃C-(CH₂)₇- | 2 | 1 | H₃C-OSO₃⁻ |
| 333 | H₃C-(CH₂)₇- | 3 | 1 | H₃C-OSO₃⁻ |
| 334 | H₃C-(CH₂)₇- | 4 | 1 | H₃C-OSO₃⁻ |
| 335 | H₃C-(CH₂)₇- | 5 | 1 | H₃C-OSO₃⁻ |
| 336 | H₃C-(CH₂)₇- | 6 | 1 | H₃C-OSO₃⁻ |
| 337 | H₃C-(CH₂)₇- | 7 | 1 | H₃C-OSO₃⁻ |
| 338 | H₃C-(CH₂)₇- | 8 | 1 | H₃C-OSO₃⁻ |
| 339 | H₃C-(CH₂)₇- | 9 | 1 | H₃C-OSO₃⁻ |
| 340 | H₃C-(CH₂)₇- | 10 | 1 | H₃C-OSO₃⁻ |
| 341 | H₃C-(CH₂)₇- | 2 | 2 | H₃C-OSO₃⁻ |
| 342 | H₃C-(CH₂)₇- | 3 | 2 | H₃C-OSO₃⁻ |
| 343 | H₃C-(CH₂)₇- | 4 | 2 | H₃C-OSO₃⁻ |
| 344 | H₃C-(CH₂)₇- | 5 | 2 | H₃C-OSO₃⁻ |
| 345 | H₃C-(CH₂)₇- | 6 | 2 | H₃C-OSO₃⁻ |
| 346 | H₃C-(CH₂)₇- | 7 | 2 | H₃C-OSO₃⁻ |
| 347 | H₃C-(CH₂)₇- | 8 | 2 | H₃C-OSO₃⁻ |
| 348 | H₃C-(CH₂)₇- | 9 | 2 | H₃C-OSO₃⁻ |
| 349 | H₃C-(CH₂)₇- | 10 | 2 | H₃C-OSO₃⁻ |
| 350 | H₃C-(CH₂)₇- | 3 | 3 | H₃C-OSO₃⁻ |
| 351 | H₃C-(CH₂)₇- | 4 | 3 | H₃C-OSO₃⁻ |
| 352 | H₃C-(CH₂)₇- | 5 | 3 | H₃C-OSO₃⁻ |
| 353 | H₃C-(CH₂)₇- | 6 | 3 | H₃C-OSO₃⁻ |
| 354 | H₃C-(CH₂)₇- | 7 | 3 | H₃C-OSO₃⁻ |
| 355 | H₃C-(CH₂)₇- | 8 | 3 | H₃C-OSO₃⁻ |
| 356 | H₃C-(CH₂)₇- | 9 | 3 | H₃C-OSO₃⁻ |
| 357 | H₃C-(CH₂)₇- | 10 | 3 | H₃C-OSO₃⁻ |
| 358 | H₃C-(CH₂)₇- | 4 | 4 | H₃C-OSO₃⁻ |
| 359 | H₃C-(CH₂)₇- | 5 | 4 | H₃C-OSO₃⁻ |
| 360 | H₃C-(CH₂)₇- | 6 | 4 | H₃C-OSO₃⁻ |
| 361 | H₃C-(CH₂)₇- | 7 | 4 | H₃C-OSO₃⁻ |
| 362 | H₃C-(CH₂)₇- | 8 | 4 | H₃C-OSO₃⁻ |
| 363 | H₃C-(CH₂)₇- | 9 | 4 | H₃C-OSO₃⁻ |
| 364 | H₃C-(CH₂)₇- | 10 | 4 | H₃C-OSO₃⁻ |
| 365 | H₃C-(CH₂)₇- | 5 | 5 | H₃C-OSO₃⁻ |
| 366 | H₃C-(CH₂)₇- | 6 | 5 | H₃C-OSO₃⁻ |
| 367 | H₃C-(CH₂)₇- | 7 | 5 | H₃C-OSO₃⁻ |
| 368 | H₃C-(CH₂)₇- | 8 | 5 | H₃C-OSO₃⁻ |
| 369 | H₃C-(CH₂)₇- | 9 | 5 | H₃C-OSO₃⁻ |
| 370 | H₃C-(CH₂)₇- | 10 | 5 | H₃C-OSO₃⁻ |
| 371 | H₃C-(CH₂)₇- | 6 | 6 | H₃C-OSO₃⁻ |
| 372 | H₃C-(CH₂)₇- | 7 | 6 | H₃C-OSO₃⁻ |
| 373 | H₃C-(CH₂)₇- | 8 | 6 | H₃C-OSO₃⁻ |
| 374 | H₃C-(CH₂)₇- | 9 | 6 | H₃C-OSO₃⁻ |
| 375 | H₃C-(CH₂)₇- | 10 | 6 | H₃C-OSO₃⁻ |
| 376 | H₃C-(CH₂)₇- | 7 | 7 | H₃C-OSO₃⁻ |
| 377 | H₃C-(CH₂)₇- | 8 | 7 | H₃C-OSO₃⁻ |
| 378 | H₃C-(CH₂)₇- | 9 | 7 | H₃C-OSO₃⁻ |
| 379 | H₃C-(CH₂)₇- | 10 | 7 | H₃C-OSO₃⁻ |
| 380 | H₃C-(CH₂)₇- | 8 | 8 | H₃C-OSO₃⁻ |
| 381 | H₃C-(CH₂)₇- | 9 | 8 | H₃C-OSO₃⁻ |
| 382 | H₃C-(CH₂)₇- | 10 | 8 | H₃C-OSO₃⁻ |
| 383 | H₃C-(CH₂)₇- | 9 | 9 | H₃C-OSO₃⁻ |
| 384 | H₃C-(CH₂)₇- | 10 | 9 | H₃C-OSO₃⁻ |
| 385 | H₃C-(CH₂)₇- | 10 | 10 | H₃C-OSO₃⁻ |
| 386 | H₃C-(CH₂)₇- | 1 | 1 | H₃C-CH₂-OSO₃⁻ |
| 387 | H₃C-(CH₂)₇- | 2 | 1 | H₃C-CH₂-OSO₃⁻ |
| 388 | H₃C-(CH₂)₇- | 3 | 1 | H₃C-CH₂-OSO₃⁻ |
| 389 | H₃C-(CH₂)₇- | 4 | 1 | H₃C-CH₂-OSO₃⁻ |
| 390 | H₃C-(CH₂)₇- | 5 | 1 | H₃C-CH₂-OSO₃⁻ |
| 391 | H₃C-(CH₂)₇- | 6 | 1 | H₃C-CH₂-OSO₃⁻ |
| 392 | H₃C-(CH₂)₇- | 7 | 1 | H₃C-CH₂-OSO₃⁻ |
| 393 | H₃C-(CH₂)₇- | 8 | 1 | H₃C-CH₂-OSO₃⁻ |
| 394 | H₃C-(CH₂)₇- | 9 | 1 | H₃C-CH₂-OSO₃⁻ |
| 395 | H₃C-(CH₂)₇- | 10 | 1 | H₃C-CH₂-OSO₃⁻ |
| 396 | H₃C-(CH₂)₇- | 2 | 2 | H₃C-CH₂-OSO₃⁻ |
| 397 | H₃C-(CH₂)₇- | 3 | 2 | H₃C-CH₂-OSO₃⁻ |
| 398 | H₃C-(CH₂)₇- | 4 | 2 | H₃C-CH₂-OSO₃⁻ |
| 399 | H₃C-(CH₂)₇- | 5 | 2 | H₃C-CH₂-OSO₃⁻ |
| 400 | H₃C-(CH₂)₇- | 6 | 2 | H₃C-CH₂-OSO₃⁻ |
| 401 | H₃C-(CH₂)₇- | 7 | 2 | H₃C-CH₂-OSO₃⁻ |
| 402 | H₃C-(CH₂)₇- | 8 | 2 | H₃C-CH₂-OSO₃⁻ |
| 403 | H₃C-(CH₂)₇- | 9 | 2 | H₃C-CH₂-OSO₃⁻ |
| 404 | H₃C-(CH₂)₇- | 10 | 2 | H₃C-CH₂-OSO₃⁻ |
| 405 | H₃C-(CH₂)₇- | 3 | 3 | H₃C-CH₂-OSO₃⁻ |
| 406 | H₃C-(CH₂)₇- | 4 | 3 | H₃C-CH₂-OSO₃⁻ |
| 407 | H₃C-(CH₂)₇- | 5 | 3 | H₃C-CH₂-OSO₃⁻ |
| 408 | H₃C-(CH₂)₇- | 6 | 3 | H₃C-CH₂-OSO₃⁻ |
| 409 | H₃C-(CH₂)₇- | 7 | 3 | H₃C-CH₂-OSO₃⁻ |
| 410 | H₃C-(CH₂)₇- | 8 | 3 | H₃C-CH₂-OSO₃⁻ |
| 411 | H₃C-(CH₂)₇- | 9 | 3 | H₃C-CH₂-OSO₃⁻ |
| 412 | H₃C-(CH₂)₇- | 10 | 3 | H₃C-CH₂-OSO₃⁻ |
| 413 | H₃C-(CH₂)₇- | 4 | 4 | H₃C-CH₂-OSO₃⁻ |
| 414 | H₃C-(CH₂)₇- | 5 | 4 | H₃C-CH₂-OSO₃⁻ |
| 415 | H₃C-(CH₂)₇- | 6 | 4 | H₃C-CH₂-OSO₃⁻ |
| 416 | H₃C-(CH₂)₇- | 7 | 4 | H₃C-CH₂-OSO₃⁻ |
| 417 | H₃C-(CH₂)₇- | 8 | 4 | H₃C-CH₂-OSO₃⁻ |
| 418 | H₃C-(CH₂)₇- | 9 | 4 | H₃C-CH₂-OSO₃⁻ |
| 419 | H₃C-(CH₂)₇- | 10 | 4 | H₃C-CH₂-OSO₃⁻ |
| 420 | H₃C-(CH₂)₇- | 5 | 5 | H₃C-CH₂-OSO₃⁻ |
| 421 | H₃C-(CH₂)₇- | 6 | 5 | H₃C-CH₂-OSO₃⁻ |
| 422 | H₃C-(CH₂)₇- | 7 | 5 | H₃C-CH₂-OSO₃⁻ |
| 423 | H₃C-(CH₂)₇- | 8 | 5 | H₃C-CH₂-OSO₃⁻ |
| 424 | H₃C-(CH₂)₇- | 9 | 5 | H₃C-CH₂-OSO₃⁻ |
| 425 | H₃C-(CH₂)₇- | 10 | 5 | H₃C-CH₂-OSO₃⁻ |
| 426 | H₃C-(CH₂)₇- | 6 | 6 | H₃C-CH₂-OSO₃⁻ |
| 427 | H₃C-(CH₂)₇- | 7 | 6 | H₃C-CH₂-OSO₃⁻ |
| 428 | H₃C-(CH₂)₇- | 8 | 6 | H₃C-CH₂-OSO₃⁻ |
| 429 | H₃C-(CH₂)₇- | 9 | 6 | H₃C-CH₂-OSO₃⁻ |
| 430 | H₃C-(CH₂)₇- | 10 | 6 | H₃C-CH₂-OSO₃⁻ |
| 431 | H₃C-(CH₂)₇- | 7 | 7 | H₃C-CH₂-OSO₃⁻ |
| 432 | H₃C-(CH₂)₇- | 8 | 7 | H₃C-CH₂-OSO₃⁻ |
| 433 | H₃C-(CH₂)₇- | 9 | 7 | H₃C-CH₂-OSO₃⁻ |
| 434 | H₃C-(CH₂)₇- | 10 | 7 | H₃C-CH₂-OSO₃⁻ |
| 435 | H₃C-(CH₂)₇- | 8 | 8 | H₃C-CH₂-OSO₃⁻ |
| 436 | H₃C-(CH₂)₇- | 9 | 8 | H₃C-CH₂-OSO₃⁻ |
| 437 | H₃C-(CH₂)₇- | 10 | 8 | H₃C-CH₂-OSO₃⁻ |
| 438 | H₃C-(CH₂)₇- | 9 | 9 | H₃C-CH₂-OSO₃⁻ |
| 439 | H₃C-(CH₂)₇- | 10 | 9 | H₃C-CH₂-OSO₃⁻ |
| 440 | H₃C-(CH₂)₇- | 10 | 10 | H₃C-CH₂-OSO₃⁻ |
| 441 | H₃C-(CH₂)₉- | 1 | 1 | Chlorid |
| 442 | H₃C-(CH₂)₉- | 2 | 1 | Chlorid |
| 443 | H₃C-(CH₂)₉- | 3 | 1 | Chlorid |
| 444 | H₃C-(CH₂)₉- | 4 | 1 | Chlorid |
| 445 | H₃C-(CH₂)₉- | 5 | 1 | Chlorid |
| 446 | H₃C-(CH₂)₉- | 6 | 1 | Chlorid |
| 447 | H₃C-(CH₂)₉- | 7 | 1 | Chlorid |
| 448 | H₃C-(CH₂)₉- | 8 | 1 | Chlorid |
| 449 | H₃C-(CH₂)₉- | 9 | 1 | Chlorid |
| 450 | H₃C-(CH₂)₉- | 10 | 1 | Chlorid |
| 451 | H₃C-(CH₂)₉- | 2 | 2 | Chlorid |
| 452 | H₃C-(CH₂)₉- | 3 | 2 | Chlorid |
| 453 | H₃C-(CH₂)₉- | 4 | 2 | Chlorid |
| 454 | H₃C-(CH₂)₉- | 5 | 2 | Chlorid |
| 455 | H₃C-(CH₂)₉- | 6 | 2 | Chlorid |
| 456 | H₃C-(CH₂)₉- | 7 | 2 | Chlorid |
| 457 | H₃C-(CH₂)₉- | 8 | 2 | Chlorid |
| 458 | H₃C-(CH₂)₉- | 9 | 2 | Chlorid |
| 459 | H₃C-(CH₂)₉- | 10 | 2 | Chlorid |
| 460 | H₃C-(CH₂)₉- | 3 | 3 | Chlorid |
| 461 | H₃C-(CH₂)₉- | 4 | 3 | Chlorid |
| 462 | H₃C-(CH₂)₉- | 5 | 3 | Chlorid |
| 463 | H₃C-(CH₂)₉- | 6 | 3 | Chlorid |
| 464 | H₃C-(CH₂)₉- | 7 | 3 | Chlorid |
| 465 | H₃C-(CH₂)₉- | 8 | 3 | Chlorid |
| 466 | H₃C-(CH₂)₉- | 9 | 3 | Chlorid |
| 467 | H₃C-(CH₂)₉- | 10 | 3 | Chlorid |
| 468 | H₃C-(CH₂)₉- | 4 | 4 | Chlorid |
| 469 | H₃C-(CH₂)₉- | 5 | 4 | Chlorid |
| 470 | H₃C-(CH₂)₉- | 6 | 4 | Chlorid |
| 471 | H₃C-(CH₂)₉- | 7 | 4 | Chlorid |
| 472 | H₃C-(CH₂)₉- | 8 | 4 | Chlorid |
| 473 | H₃C-(CH₂)₉- | 9 | 4 | Chlorid |
| 474 | H₃C-(CH₂)₉- | 10 | 4 | Chlorid |
| 475 | H₃C-(CH₂)₉- | 5 | 5 | Chlorid |
| 476 | H₃C-(CH₂)₉- | 6 | 5 | Chlorid |
| 477 | H₃C-(CH₂)₉- | 7 | 5 | Chlorid |
| 478 | H₃C-(CH₂)₉- | 8 | 5 | Chlorid |
| 479 | H₃C-(CH₂)₉- | 9 | 5 | Chlorid |
| 480 | H₃C-(CH₂)₉- | 10 | 5 | Chlorid |
| 481 | H₃C-(CH₂)₉- | 6 | 6 | Chlorid |
| 482 | H₃C-(CH₂)₉- | 7 | 6 | Chlorid |
| 483 | H₃C-(CH₂)₉- | 8 | 6 | Chlorid |
| 484 | H₃C-(CH₂)₉- | 9 | 6 | Chlorid |
| 485 | H₃C-(CH₂)₉- | 10 | 6 | Chlorid |
| 486 | H₃C-(CH₂)₉- | 7 | 7 | Chlorid |
| 487 | H₃C-(CH₂)₉- | 8 | 7 | Chlorid |
| 488 | H₃C-(CH₂)₉- | 9 | 7 | Chlorid |
| 489 | H₃C-(CH₂)₉- | 10 | 7 | Chlorid |
| 490 | H₃C-(CH₂)₉- | 8 | 8 | Chlorid |
| 491 | H₃C-(CH₂)₉- | 9 | 8 | Chlorid |
| 492 | H₃C-(CH₂)₉- | 10 | 8 | Chlorid |
| 493 | H₃C-(CH₂)₉- | 9 | 9 | Chlorid |
| 494 | H₃C-(CH₂)₉- | 10 | 9 | Chlorid |
| 495 | H₃C-(CH₂)₉- | 10 | 10 | Chlorid |
| 496 | H₃C-(CH₂)₉- | 1 | 1 | Bromid |
| 497 | H₃C-(CH₂)₉- | 2 | 1 | Bromid |
| 498 | H₃C-(CH₂)₉- | 3 | 1 | Bromid |
| 499 | H₃C-(CH₂)₉- | 4 | 1 | Bromid |
| 500 | H₃C-(CH₂)₉- | 5 | 1 | Bromid |
| 501 | H₃C-(CH₂)₉- | 6 | 1 | Bromid |
| 502 | H₃C-(CH₂)₉- | 7 | 1 | Bromid |
| 503 | H₃C-(CH₂)₉- | 8 | 1 | Bromid |
| 504 | H₃C-(CH₂)₉- | 9 | 1 | Bromid |
| 505 | H₃C-(CH₂)₉- | 10 | 1 | Bromid |
| 506 | H₃C-(CH₂)₉- | 2 | 2 | Bromid |
| 507 | H₃C-(CH₂)₉- | 3 | 2 | Bromid |
| 508 | H₃C-(CH₂)₉- | 4 | 2 | Bromid |
| 509 | H₃C-(CH₂)₉- | 5 | 2 | Bromid |
| 510 | H₃C-(CH₂)₉- | 6 | 2 | Bromid |
| 511 | H₃C-(CH₂)₉- | 7 | 2 | Bromid |
| 512 | H₃C-(CH₂)₉- | 8 | 2 | Bromid |
| 513 | H₃C-(CH₂)₉- | 9 | 2 | Bromid |
| 514 | H₃C-(CH₂)₉- | 10 | 2 | Bromid |
| 515 | H₃C-(CH₂)₉- | 3 | 3 | Bromid |
| 516 | H₃C-(CH₂)₉- | 4 | 3 | Bromid |
| 517 | H₃C-(CH₂)₉- | 5 | 3 | Bromid |
| 518 | H₃C-(CH₂)₉- | 6 | 3 | Bromid |
| 519 | H₃C-(CH₂)₉- | 7 | 3 | Bromid . |
| 520 | H₃C-(CH₂)₉- | 8 | 3 | Bromid |
| 521 | H₃C-(CH₂)₉- | 9 | 3 | Bromid |
| 522 | H₃C-(CH₂)₉- | 10 | 3 | Bromid |
| 523 | H₃C-(CH₂)₉- | 4 | 4 | Bromid |
| 524 | H₃C-(CH₂)₉- | 5 | 4 | Bromid |
| 525 | H₃C-(CH₂)₉- | 6 | 4 | Bromid |
| 526 | H₃C-(CH₂)₉- | 7 | 4 | Bromid |
| 527 | H₃C-(CH₂)₉- | 8 | 4 | Bromid |
| 528 | H₃C-(CH₂)₉- | 9 | 4 | Bromid |
| 529 | H₃C-(CH₂)₉- | 10 | 4 | Bromid |
| 530 | H₃C-(CH₂)₉- | 5 | 5 | Bromid |
| 531 | H₃C-(CH₂)₉- | 6 | 5 | Bromid |
| 532 | H₃C-(CH₂)₉- | 7 | 5 | Bromid |
| 533 | H₃C-(CH₂)₉- | 8 | 5 | Bromid |
| 534 | H₃C-(CH₂)₉- | 9 | 5 | Bromid |
| 535 | H₃C-(CH₂)₉- | 10 | 5 | Bromid |
| 536 | H₃C-(CH₂)₉- | 6 | 6 | Bromid |
| 537 | H₃C-(CH₂)₉- | 7 | 6 | Bromid |
| 538 | H₃C-(CH₂)₉- | 8 | 6 | Bromid |
| 539 | H₃C-(CH₂)₉- | 9 | 6 | Bromid |
| 540 | H₃C-(CH₂)₉- | 10 | 6 | Bromid |
| 541 | H₃C-(CH₂)₉- | 7 | 7 | Bromid |
| 542 | H₃C-(CH₂)₉- | 8 | 7 | Bromid |
| 543 | H₃C-(CH₂)₉- | 9 | 7 | Bromid |
| 544 | H₃C-(CH₂)₉- | 10 | 7 | Bromid |
| 545 | H₃C-(CH₂)₉- | 8 | 8 | Bromid |
| 546 | H₃C-(CH₂)₉- | 9 | 8 | Bromid |
| 547 | H₃C-(CH₂)₉- | 10 | 8 | Bromid |
| 548 | H₃C-(CH₂)₉- | 9 | 9 | Bromid |
| 549 | H₃C-(CH₂)₉- | 10 | 9 | Bromid |
| 550 | H₃C-(CH₂)₉- | 10 | 10 | Bromid |
| 551 | H₃C-(CH₂)₉- | 1 | 1 | H₃C-OSO₃⁻ |
| 552 | H₃C-(CH₂)₉- | 2 | 1 | H₃C-OSO₃⁻ |
| 553 | H₃C-(CH₂)₉- | 3 | 1 | H₃C-OSO₃⁻ |
| 554 | H₃C-(CH₂)₉- | 4 | 1 | H₃C-OSO₃⁻ |
| 555 | H₃C-(CH₂)₉- | 5 | 1 | H₃C-OSO₃⁻ |
| 556 | H₃C-(CH₂)₉- | 6 | 1 | H₃C-OSO₃⁻ |
| 557 | H₃C-(CH₂)₉- | 7 | 1 | H₃C-OSO₃⁻ |
| 558 | H₃C-(CH₂)₉- | 8 | 1 | H₃C-OSO₃⁻ |
| 559 | H₃C-(CH₂)₉- | 9 | 1 | H₃C-OSO₃⁻ |
| 560 | H₃C-(CH₂)₉- | 10 | 1 | H₃C-OSO₃⁻ |
| 561 | H₃C-(CH₂)₉- | 2 | 2 | H₃C-OSO₃⁻ |
| 562 | H₃C-(CH₂)₉- | 3 | 2 | H₃C-OSO₃⁻ |
| 563 | H₃C-(CH₂)₉- | 4 | 2 | H₃C-OSO₃⁻ |
| 564 | H₃C-(CH₂)₉- | 5 | 2 | H₃C-OSO₃⁻ |
| 565 | H₃C-(CH₂)₉- | 6 | 2 | H₃C-OSO₃⁻ |
| 566 | H₃C-(CH₂)₉- | 7 | 2 | H₃C-OSO₃⁻ |
| 567 | H₃C-(CH₂)₉- | 8 | 2 | H₃C-OSO₃⁻ |
| 568 | H₃C-(CH₂)₉- | 9 | 2 | H₃C-OSO₃⁻ |
| 569 | H₃C-(CH₂)₉- | 10 | 2 | H₃C-OSO₃⁻ |
| 570 | H₃C-(CH₂)₉- | 3 | 3 | H₃C-OSO₃⁻ |
| 571 | H₃C-(CH₂)₉- | 4 | 3 | H₃C-OSO₃⁻ |
| 572 | H₃C-(CH₂)₉- | 5 | 3 | H₃C-OSO₃⁻ |
| 573 | H₃C-(CH₂)₉- | 6 | 3 | H₃C-OSO₃⁻ |
| 574 | H₃C-(CH₂)₉- | 7 | 3 | H₃C-OSO₃⁻ |
| 575 | H₃C-(CH₂)₉- | 8 | 3 | H₃C-OSO₃⁻ |
| 576 | H₃C-(CH₂)₉- | 9 | 3 | H₃C-OSO₃⁻ |
| 577 | H₃C-(CH₂)₉- | 10 | 3 | H₃C-OSO₃⁻ |
| 578 | H₃C-(CH₂)₉- | 4 | 4 | H₃C-OSO₃⁻ |
| 579 | H₃C-(CH₂)₉- | 5 | 4 | H₃C-OSO₃⁻ |
| 580 | H₃C-(CH₂)₉- | 6 | 4 | H₃C-OSO₃⁻ |
| 581 | H₃C-(CH₂)₉- | 7 | 4 | H₃C-OSO₃⁻ |
| 582 | H₃C-(CH₂)₉- | 8 | 4 | H₃C-OSO₃⁻ |
| 583 | H₃C-(CH₂)₉- | 9 | 4 | H₃C-OSO₃⁻ |
| 584 | H₃C-(CH₂)₉- | 10 | 4 | H₃C-OSO₃⁻ |
| 585 | H₃C-(CH₂)₉- | 5 | 5 | H₃C-OSO₃⁻ |
| 586 | H₃C-(CH₂)₉- | 6 | 5 | H₃C-OSO₃⁻ |
| 587 | H₃C-(CH₂)₉- | 7 | 5 | H₃C-OSO₃⁻ |
| 588 | H₃C-(CH₂)₉- | 8 | 5 | H₃C-OSO₃⁻ |
| 589 | H₃C-(CH₂)₉- | 9 | 5 | H₃C-OSO₃⁻ |
| 590 | H₃C-(CH₂)₉- | 10 | 5 | H₃C-OSO₃⁻ |
| 591 | H₃C-(CH₂)₉- | 6 | 6 | H₃C-OSO₃⁻ |
| 592 | H₃C-(CH₂)₉- | 7 | 6 | H₃C-OSO₃⁻ |
| 593 | H₃C-(CH₂)₉- | 8 | 6 | H₃C-OSO₃⁻ |
| 594 | H₃C-(CH₂)₉- | 9 | 6 | H₃C-OSO₃⁻ |
| 595 | H₃C-(CH₂)₉- | 10 | 6 | H₃C-OSO₃⁻ |
| 596 | H₃C-(CH₂)₉- | 7 | 7 | H₃C-OSO₃⁻ |
| 597 | H₃C-(CH₂)₉- | 8 | 7 | H₃C-OSO₃⁻ |
| 598 | H₃C-(CH₂)₉- | 9 | 7 | H₃C-OSO₃⁻ |
| 599 | H₃C-(CH₂)₉- | 10 | 7 | H₃C-OSO₃⁻ |
| 600 | H₃C-(CH₂)₉- | 8 | 8 | H₃C-OSO₃⁻ |
| 601 | H₃C-(CH₂)₉- | 9 | 8 | H₃C-OSO₃⁻ |
| 602 | H₃C-(CH₂)₉- | 10 | 8 | H₃C-OSO₃⁻ |
| 603 | H₃C-(CH₂)₉- | 9 | 9 | H₃C-OSO₃⁻ |
| 604 | H₃C-(CH₂)₉- | 10 | 9 | H₃C-OSO₃⁻ |
| 605 | H₃C-(CH₂)₉- | 10 | 10 | H₃C-OSO₃⁻ |
| 606 | H₃C-(CH₂)₉- | 1 | 1 | H₃C-CH₂-OSO₃⁻ |
| 607 | H₃C-(CH₂)₉- | 2 | 1 | H₃C-CH₂-OSO₃⁻ |
| 608 | H₃C-(CH₂)₉- | 3 | 1 | H₃C-CH₂-OSO₃⁻ |
| 609 | H₃C-(CH₂)₉- | 4 | 1 | H₃C-CH₂-OSO₃⁻ |
| 610 | H₃C-(CH₂)₉- | 5 | 1 | H₃C-CH₂-OSO₃⁻ |
| 611 | H₃C-(CH₂)₉- | 6 | 1 | H₃C-CH₂-OSO₃⁻ |
| 612 | H₃C-(CH₂)₉- | 7 | 1 | H₃C-CH₂-OSO₃⁻ |
| 613 | H₃C-(CH₂)₉- | 8 | 1 | H₃C-CH₂-OSO₃⁻ |
| 614 | H₃C-(CH₂)₉- | 9 | 1 | H₃C-CH₂-OSO₃⁻ |
| 615 | H₃C-(CH₂)₉- | 10 | 1 | H₃C-CH₂-OSO₃⁻ |
| 616 | H₃C-(CH₂)₉- | 2 | 2 | H₃C-CH₂-OSO₃⁻ |
| 617 | H₃C-(CH₂)₉- | 3 | 2 | H₃C-CH₂-OSO₃⁻ |
| 618 | H₃C-(CH₂)₉- | 4 | 2 | H₃C-CH₂-OSO₃⁻ |
| 619 | H₃C-(CH₂)₉- | 5 | 2 | H₃C-CH₂-OSO₃⁻ |
| 620 | H₃C-(CH₂)₉- | 6 | 2 | H₃C-CH₂-OSO₃⁻ |
| 621 | H₃C-(CH₂)₉- | 7 | 2 | H₃C-CH₂-OSO₃⁻ |
| 622 | H₃C-(CH₂)₉- | 8 | 2 | H₃C-CH₂-OSO₃⁻ |
| 623 | H₃C-(CH₂)₉- | 9 | 2 | H₃C-CH₂-OSO₃⁻ |
| 624 | H₃C-(CH₂)₉- | 10 | 2 | H₃C-CH₂-OSO₃⁻ |
| 625 | H₃C-(CH₂)₉- | 3 | 3 | H₃C-CH₂-OSO₃⁻ |
| 626 | H_{3C}-(CH₂)₉- | 4 | 3 | H₃C-CH₂-OSO₃⁻ |
| 627 | H₃C-(CH₂)₉- | 5 | 3 | H₃C-CH₂-OSO₃⁻ |
| 628 | H₃C-(CH₂)₉- | 6 | 3 | H₃C-CH₂-OSO₃⁻ |
| 629 | H₃C-(CH₂)₉- | 7 | 3 | H₃C-CH₂-OSO₃⁻ |
| 630 | H₃C-(CH₂)₉- | 8 | 3 | H₃C-CH₂-OSO₃⁻ |
| 631 | H₃C-(CH₂)₉- | 9 | 3 | H₃C-CH₂-OSO₃⁻ |
| 632 | H₃C-(CH₂)₉- | 10 | 3 | H₃C-CH₂-OSO₃⁻ |
| 633 | H₃C-(CH₂)₉- | 4 | 4 | H₃C-CH₂-OSO₃⁻ |
| 634 | H₃C-(CH₂)₉- | 5 | 4 | H₃C-CH₂-OSO₃⁻ |
| 635 | H₃C-(CH₂)₉- | 6 | 4 | H₃C-CH₂-OSO₃⁻ |
| 636 | H₃C-(CH₂)₉- | 7 | 4 | H₃C-CH₂-OSO₃⁻ |
| 637 | H₃C-(CH₂)₉- | 8 | 4 | H₃C-CH₂-OSO₃⁻ |
| 638 | H₃C-(CH₂)₉- | 9 | 4 | H₃C-CH₂-OSO₃⁻ |
| 639 | H₃C-(CH₂)₉- | 10 | 4 | H₃C-CH₂-OSO₃⁻ |
| 640 | H₃C-(CH₂)₉- | 5 | 5 | H₃C-CH₂-OSO₃⁻ |
| 641 | H₃C-(CH₂)₉- | 6 | 5 | H₃C-CH₂-OSO₃⁻ |
| 642 | H₃C-(CH₂)₉- | 7 | 5 | H₃C-CH₂-OSO₃⁻ |
| 643 | H₃C-(CH₂)₉- | 8 | 5 | H₃C-CH₂-OSO₃⁻ |
| 644 | H₃C-(CH₂)₉- | 9 | 5 | H₃C-CH₂-OSO₃⁻ |
| 645 | H₃C-(CH₂)₉- | 10 | 5 | H₃C-CH₂-OSO₃⁻ |
| 646 | H₃C-(CH₂)₉- | 6 | 6 | H₃C-CH₂-OSO₃⁻ |
| 647 | H₃C-(CH₂)₉- | 7 | 6 | H₃C-CH₂-OSO₃⁻ |
| 648 | H₃C-(CH₂)₉- | 8 | 6 | H₃C-CH₂-OSO₃⁻ |
| 649 | H₃C-(CH₂)₉- | 9 | 6 | H₃C-CH₂-OSO₃⁻ |
| 650 | H₃C-(CH₂)₉- | 10 | 6 | H₃C-CH₂-OSO₃⁻ |
| 651 | H₃C-(CH₂)₉- | 7 | 7 | H₃C-CH₂-OSO₃⁻ |
| 652 | H₃C-(CH₂)₉- | 8 | 7 | H₃C-CH₂-OSO₃⁻ |
| 653 | H₃C-(CH₂)₉- | 9 | 7 | H₃C-CH₂-OSO₃⁻ |
| 654 | H₃C-(CH₂)₉- | 10 | 7 | H₃C-CH₂-OSO₃⁻ |
| 655 | H₃C-(CH₂)₉- | 8 | 8 | H₃C-CH₂-OSO₃⁻ |
| 656 | H₃C-(CH₂)₉- | 9 | 8 | H₃C-CH₂-OSO₃⁻ |
| 657 | H₃C-(CH₂)₉- | 10 | 8 | H₃C-CH₂-OSO₃⁻ |
| 658 | H₃C-(CH₂)₉- | 9 | 9 | H₃C-CH₂-OSO₃⁻ |
| 659 | H₃C-(CH₂)₉- | 10 | 9 | H₃C-CH₂-OSO₃⁻ |
| 660 | H₃C-(CH₂)₉- | 10 | 10 | H₃C-CH₂-OSO₃⁻ |
| 661 | H₃C-(CH₂)₁₁- | 1 | 1 | Chlorid |
| 662 | H₃C-(CH₂)₁₁- | 2 | 1 | Chlorid |
| 663 | H₃C-(CH₂)₁₁- | 3 | 1 | Chlorid |
| 664 | H₃C-(CH₂)₁₁- | 4 | 1 | Chlorid |
| 665 | H₃C-(CH₂)₁₁- | 5 | 1 | Chlorid |
| 666 | H₃C-(CH₂)₁₁- | 6 | 1 | Chlorid |
| 667 | H₃C-(CH₂)₁₁- | 7 | 1 | Chlorid |
| 668 | H₃C-(CH₂)₁₁- | 8 | 1 | Chlorid |
| 669 | H₃C-(CH₂)₁₁- | 9 | 1 | Chlorid |
| 670 | H₃C-(CH₂)₁₁- | 10 | 1 | Chlorid |
| 671 | H₃C-(CH₂)₁₁- | 2 | 2 | Chlorid |
| 672 | H₃C-(CH₂)₁₁- | 3 | 2 | Chlorid |
| 673 | H₃C-(CH₂)₁₁- | 4 | 2 | Chlorid |
| 674 | H₃C-(CH₂)₁₁- | 5 | 2 | Chlorid |
| 675 | H₃C-(CH₂)₁₁- | 6 | 2 | Chlorid |
| 676 | H₃C-(CH₂)₁₁- | 7 | 2 | Chlorid |
| 677 | H₃C-(CH₂)₁₁- | 8 | 2 | Chlorid |
| 678 | H₃C-(CH₂)₁₁- | 9 | 2 | Chlorid |
| 679 | H₃C-(CH₂)₁₁- | 10 | 2 | Chlorid |
| 680 | H₃C-(CH₂)₁₁- | 3 | 3 | Chlorid |
| 681 | H₃C-(CH₂)₁₁- | 4 | 3 | Chlorid |
| 682 | H₃C-(CH₂)₁₁- | 5 | 3 | Chlorid |
| 683 | H₃C-(CH₂)₁₁- | 6 | 3 | Chlorid |
| 684 | H₃C-(CH₂)₁₁- | 7 | 3 | Chlorid |
| 685 | H₃C-(CH₂)₁₁- | 8 | 3 | Chlorid |
| 686 | H₃C-(CH₂)₁₁- | 9 | 3 | Chlorid |
| 687 | H₃C-(CH₂)₁₁- | 10 | 3 | Chlorid |
| 688 | H₃C-(CH₂)₁₁- | 4 | 4 | Chlorid |
| 689 | H₃C-(CH₂)₁₁- | 5 | 4 | Chlorid |
| 690 | H₃C-(CH₂)₁₁- | 6 | 4 | Chlorid |
| 691 | H₃C-(CH₂)₁₁- | 7 | 4 | Chlorid |
| 692 | H₃C-(CH₂)₁₁- | 8 | 4 | Chlorid |
| 693 | H₃C-(CH₂)₁₁- | 9 | 4 | Chlorid |
| 694 | H₃C-(CH₂)₁₁- | 10 | 4 | Chlorid |
| 695 | H₃C-(CH₂)₁₁- | 5 | 5 | Chlorid |
| 696 | H₃C-(CH₂)₁₁- | 6 | 5 | Chlorid |
| 697 | H₃C-(CH₂)₁₁- | 7 | 5 | Chlorid |
| 698 | H₃C-(CH₂)₁₁- | 8 | 5 | Chlorid |
| 699 | H₃C-(CH₂)₁₁- | 9 | 5 | Chlorid |
| 700 | H₃C-(CH₂)₁₁- | 10 | 5 | Chlorid |
| 701 | H₃C-(CH₂)₁₁- | 6 | 6 | Chlorid |
| 702 | H₃C-(CH₂)₁₁- | 7 | 6 | Chlorid |
| 703 | H₃C-(CH₂)₁₁- | 8 | 6 | Chlorid |
| 704 | H₃C-(CH₂)₁₁- | 9 | 6 | Chlorid |
| 705 | H₃C-(CH₂)₁₁- | 10 | 6 | Chlorid |
| 706 | H₃C-(CH₂)₁₁- | 7 | 7 | Chlorid |
| 707 | H₃C-(CH₂)₁₁- | 8 | 7 | Chlorid |
| 708 | H₃C-(CH₂)₁₁- | 9 | 7 | Chlorid |
| 709 | H₃C-(CH₂)₁₁- | 10 | 7 | Chlorid |
| 710 | H₃C-(CH₂)₁₁- | 8 | 8 | Chlorid |
| 711 | H₃C-(CH₂)₁₁- | 9 | 8 | Chlorid |
| 712 | H₃C-(CH₂)₁₁- | 10 | 8 | Chlorid |
| 713 | H₃C-(CH₂)₁₁- | 9 | 9 | Chlorid |
| 714 | H₃C-(CH₂)₁₁- | 10 | 9 | Chlorid |
| 715 | H₃C-(CH₂)₁₁- | 10 | 10 | Chlorid |
| 716 | H₃C-(CH₂)₁₁- | 1 | 1 | Bromid |
| 717 | H₃C-(CH₂)₁₁- | 2 | 1 | Bromid |
| 718 | H₃C-(CH₂)₁₁- | 3 | 1 | Bromid |
| 719 | H₃C-(CH₂)₁₁- | 4 | 1 | Bromid |
| 720 | H₃C-(CH₂)₁₁- | 5 | 1 | Bromid |
| 721 | H₃C-(CH₂)₁₁- | 6 | 1 | Bromid |
| 722 | H₃C-(CH₂)₁₁- | 7 | 1 | Bromid |
| 723 | H₃C-(CH₂)₁₁- | 8 | 1 | Bromid |
| 724 | H₃C-(CH₂)₁₁- | 9 | 1 | Bromid |
| 725 | H₃C-(CH₂)₁₁- | 10 | 1 | Bromid |
| 726 | H₃C-(CH₂)₁₁- | 2 | 2 | Bromid |
| 727 | H₃C-(CH₂)₁₁- | 3 | 2 | Bromid |
| 728 | H₃C-(CH₂)₁₁- | 4 | 2 | Bromid |
| 729 | H₃C-(CH₂)₁₁- | 5 | 2 | Bromid |
| 730 | H₃C-(CH₂)₁₁- | 6 | 2 | Bromid |
| 731 | H₃C-(CH₂)₁₁- | 7 | 2 | Bromid |
| 732 | H₃C-(CH₂)₁₁- | 8 | 2 | Bromid |
| 733 | H₃C-(CH₂)₁₁- | 9 | 2 | Bromid |
| 734 | H₃C-(CH₂)₁₁- | 10 | 2 | Bromid |
| 735 | H₃C-(CH₂)₁₁- | 3 | 3 | Bromid |
| 736 | H₃C-(CH₂)₁₁- | 4 | 3 | Bromid |
| 737 | H₃C-(CH₂)₁₁- | 5 | 3 | Bromid |
| 738 | H₃C-(CH₂)₁₁- | 6 | 3 | Bromid |
| 739 | H₃C-(CH₂)₁₁- | 7 | 3 | Bromid |
| 740 | H₃C-(CH₂)₁₁- | 8 | 3 | Bromid |
| 741 | H₃C-(CH₂)₁₁- | 9 | 3 | Bromid |
| 742 | H₃C-(CH₂)₁₁- | 10 | 3 | Bromid |
| 743 | H₃C-(CH₂)₁₁- | 4 | 4 | Bromid |
| 744 | H₃C-(CH₂)₁₁- | 5 | 4 | Bromid |
| 745 | H₃C-(CH₂)₁₁- | 6 | 4 | Bromid |
| 746 | H₃C-(CH₂)₁₁- | 7 | 4 | Bromid |
| 747 | H₃C-(CH₂)₁₁- | 8 | 4 | Bromid |
| 748 | H₃C-(CH₂)₁₁- | 9 | 4 | Bromid |
| 749 | H₃C-(CH₂)₁₁- | 10 | 4 | Bromid |
| 750 | H₃C-(CH₂)₁₁- | 5 | 5 | Bromid |
| 751 | H₃C-(CH₂)₁₁- | 6 | 5 | Bromid |
| 752 | H₃C-(CH₂)₁₁- | 7 | 5 | Bromid |
| 753 | H₃C-(CH₂)₁₁- | 8 | 5 | Bromid |
| 754 | H₃C-(CH₂)₁₁- | 9 | 5 | Bromid |
| 755 | H₃C-(CH₂)₁₁- | 10 | 5 | Bromid |
| 756 | H₃C-(CH₂)₁₁- | 6 | 6 | Bromid |
| 757 | H₃C-(CH₂)₁₁- | 7 | 6 | Bromid |
| 758 | H₃C-(CH₂)₁₁- | 8 | 6 | Bromid |
| 759 | H₃C-(CH₂)₁₁- | 9 | 6 | Bromid |
| 760 | H₃C-(CH₂)₁₁- | 10 | 6 | Bromid |
| 761 | H₃C-(CH₂)₁₁- | 7 | 7 | Bromid |
| 762 | H₃C-(CH₂)₁₁- | 8 | 7 | Bromid |
| 763 | H₃C-(CH₂)₁₁- | 9 | 7 | Bromid |
| 764 | H₃C-(CH₂)₁₁- | 10 | 7 | Bromid |
| 765 | H₃C-(CH₂)₁₁- | 8 | 8 | Bromid |
| 766 | H₃C-(CH₂)₁₁- | 9 | 8 | Bromid |
| 767 | H₃C-(CH₂)₁₁- | 10 | 8 | Bromid |
| 768 | H₃C-(CH₂)₁₁- | 9 | 9 | Bromid |
| 769 | H₃C-(CH₂)₁₁- | 10 | 9 | Bromid |
| 770 | H₃C-(CH₂)₁₁- | 10 | 10 | Bromid |
| 771 | H₃C-(CH₂)₁₁- | 1 | 1 | H₃C-OSO₃⁻ |
| 772 | H₃C-(CH₂)₁₁- | 2 | 1 | H₃C-OSO₃⁻ |
| 773 | H₃C-(CH₂)₁₁- | 3 | 1 | H₃C-OSO₃⁻ |
| 774 | H₃C-(CH₂)₁₁- | 4 | 1 | H₃C-OSO₃⁻ |
| 775 | H₃C-(CH₂)₁₁- | 5 | 1 | H₃C-OSO₃⁻ |
| 776 | H₃C-(CH₂)₁₁- | 6 | 1 | H₃C-OSO₃⁻ |
| 777 | H₃C-(CH₂)₁₁- | 7 | 1 | H₃C-OSO₃⁻ |
| 778 | H₃C-(CH₂)₁₁- | 8 | 1 | H₃C-OSO₃⁻ |
| 779 | H₃C-(CH₂)₁₁- | 9 | 1 | H₃C-OSO₃⁻ |
| 780 | H₃C-(CH₂)₁₁- | 10 | 1 | H₃C-OSO₃⁻ |
| 781 | H₃C-(CH₂)₁₁- | 2 | 2 | H₃C-OSO₃⁻ |
| 782 | H₃C-(CH₂)₁₁- | 3 | 2 | H₃C-OSO₃⁻ |
| 783 | H₃C-(CH₂)₁₁- | 4 | 2 | H₃C-OSO₃⁻ |
| 784 | H₃C-(CH₂)₁₁- | 5 | 2 | H₃C-OSO₃⁻ |
| 785 | H₃C-(CH₂)₁₁- | 6 | 2 | H₃C-OSO₃⁻ |
| 786 | H₃C-(CH₂)₁₁- | 7 | 2 | H₃C-OSO₃⁻ |
| 787 | H₃C-(CH₂)₁₁- | 8 | 2 | H₃C-OSO₃⁻ |
| 788 | H₃C-(CH₂)₁₁- | 9 | 2 | H₃C-OSO₃⁻ |
| 789 | H₃C-(CH₂)₁₁- | 10 | 2 | H₃C-OSO₃⁻ |
| 790 | H₃C-(CH₂)₁₁- | 3 | 3 | H₃C-OSO₃⁻ |
| 791 | H₃C-(CH₂)₁₁- | 4 | 3 | H₃C-OSO₃⁻ |
| 792 | H₃C-(CH₂)₁₁- | 5 | 3 | H₃C-OSO₃⁻ |
| 793 | H₃C-(CH₂)₁₁- | 6 | 3 | H₃C-OSO₃⁻ |
| 794 | H₃C-(CH₂)₁₁- | 7 | 3 | H₃C-OSO₃⁻ |
| 795 | H₃C-(CH₂)₁₁- | 8 | 3 | H₃C-OSO₃⁻ |
| 796 | H₃C-(CH₂)₁₁- | 9 | 3 | H₃C-OSO₃⁻ |
| 797 | H₃C-(CH₂)₁₁- | 10 | 3 | H₃C-OSO₃⁻ |
| 798 | H₃C-(CH₂)₁₁- | 4 | 4 | H₃C-OSO₃⁻ |
| 799 | H₃C-(CH₂)₁₁- | 5 | 4 | H₃C-OSO₃⁻ |
| 800 | H₃C-(CH₂)₁₁- | 6 | 4 | H₃C-OSO₃⁻ |
| 801 | H₃C-(CH₂)₁₁- | 7 | 4 | H₃C-OSO₃⁻ |
| 802 | H₃C-(CH₂)₁₁- | 8 | 4 | H₃C-OSO₃⁻ |
| 803 | H₃C-(CH₂)₁₁- | 9 | 4 | H₃C-OSO₃⁻ |
| 804 | H₃C-(CH₂)₁₁- | 10 | 4 | H₃C-OSO₃⁻ |
| 805 | H₃C-(CH₂)₁₁- | 5 | 5 | H₃C-OSO₃⁻ |
| 806 | H₃C-(CH₂)₁₁- | 6 | 5 | H₃C-OSO₃⁻ |
| 807 | H₃C-(CH₂)₁₁- | 7 | 5 | H₃C-OSO₃⁻ |
| 808 | H₃C-(CH₂)₁₁- | 8 | 5 | H₃C-OSO₃⁻ |
| 809 | H₃C-(CH₂)₁₁- | 9 | 5 | H₃C-OSO₃⁻ |
| 810 | H₃C-(CH₂)₁₁- | 10 | 5 | H₃C-OSO₃⁻ |
| 811 | H₃C-(CH₂)₁₁- | 6 | 6 | H₃C-OSO₃⁻ |
| 812 | H₃C-(CH₂)₁₁- | 7 | 6 | H₃C-OSO₃⁻ |
| 813 | H₃C-(CH₂)₁₁- | 8 | 6 | H₃C-OSO₃⁻ |
| 814 | H₃C-(CH₂)₁₁- | 9 | 6 | H₃C-OSO₃⁻ |
| 815 | H₃C-(CH₂)₁₁- | 10 | 6 | H₃C-OSO₃⁻ |
| 816 | H₃C-(CH₂)₁₁- | 7 | 7 | H₃C-OSO₃⁻ |
| 817 | H₃C-(CH₂)₁₁- | 8 | 7 | H₃C-OSO₃⁻ |
| 818 | H₃C-(CH₂)₁₁- | 9 | 7 | H₃C-OSO₃⁻ |
| 819 | H₃C-(CH₂)₁₁- | 10 | 7 | H₃C-OSO₃⁻ |
| 820 | H₃C-(CH₂)₁₁- | 8 | 8 | H₃C-OSO₃⁻ |
| 821 | H₃C-(CH₂)₁₁- | 9 | 8 | H₃C-OSO₃⁻ |
| 822 | H₃C-(CH₂)₁₁- | 10 | 8 | H₃C-OSO₃⁻ |
| 823 | H₃C-(CH₂)₁₁- | 9 | 9 | H₃C-OSO₃⁻ |
| 824 | H₃C-(CH₂)₁₁- | 10 | 9 | H₃C-OSO₃⁻ |
| 825 | H₃C-(CH₂)₁₁- | 10 | 10 | H₃C-OSO₃⁻ |
| 826 | H₃C-(CH₂)₁₁- | 1 | 1 | H₃C-CH₂-OSO₃⁻ |
| 827 | H₃C-(CH₂)₁₁- | 2 | 1 | H₃C-CH₂-OSO₃⁻ |
| 828 | H₃C-(CH₂)₁₁- | 3 | 1 | H₃C-CH₂-OSO₃⁻ |
| 829 | H₃C-(CH₂)₁₁- | 4 | 1 | H₃C-CH₂-OSO₃⁻ |
| 830 | H₃C-(CH₂)₁₁- | 5 | 1 | H₃C-CH₂-OSO₃⁻ |
| 831 | H₃C-(CH₂)₁₁- | 6 | 1 | H₃C-CH₂-OSO₃⁻ |
| 832 | H₃C-(CH₂)₁₁- | 7 | 1 | H₃C-CH₂-OSO₃⁻ |
| 833 | H₃C-(CH₂)₁₁- | 8 | 1 | H₃C-CH₂-OSO₃⁻ |
| 834 | H₃C-(CH₂)₁₁- | 9 | 1 | H₃C-CH₂-OSO₃⁻ |
| 835 | H₃C-(CH₂)₁₁- | 10 | 1 | H₃C-CH₂-OSO₃⁻ |
| 836 | H₃C-(CH₂)₁₁- | 2 | 2 | H₃C-CH₂-OSO₃⁻ |
| 837 | H₃C-(CH₂)₁₁- | 3 | 2 | H₃C-CH₂-OSO₃⁻ |
| 838 | H₃C-(CH₂)₁₁- | 4 | 2 | H₃C-CH₂-OSO₃⁻ |
| 839 | H₃C-(CH₂)₁₁- | 5 | 2 | H₃C-CH₂-OSO₃⁻ |
| 840 | H₃C-(CH₂)₁₁- | 6 | 2 | H₃C-CH₂-OSO₃⁻ |
| 841 | H₃C-(CH₂)₁₁- | 7 | 2 | H₃C-CH₂-OSO₃⁻ |
| 842 | H₃C-(CH₂)₁₁- | 8 | 2 | H₃C-CH₂-OSO₃⁻ |
| 843 | H₃C-(CH₂)₁₁- | 9 | 2 | H₃C-CH₂-OSO₃⁻ |
| 844 | H₃C-(CH₂)₁₁- | 10 | 2 | H₃C-CH₂-OSO₃⁻ |
| 845 | H₃C-(CH₂)₁₁- | 3 | 3 | H₃C-CH₂-OSO₃⁻ |
| 846 | H₃C-(CH₂)₁₁- | 4 | 3 | H₃C-CH₂-OSO₃⁻ |
| 847 | H₃C-(CH₂)₁₁- | 5 | 3 | H₃C-CH₂-OSO₃⁻ |
| 848 | H₃C-(CH₂)₁₁- | 6 | 3 | H₃C-CH₂-OSO₃⁻ |
| 849 | H₃C-(CH₂)₁₁- | 7 | 3 | H₃C-CH₂-OSO₃⁻ |
| 850 | H₃C-(CH₂)₁₁- | 8 | 3 | H₃C-CH₂-OSO₃⁻ |
| 851 | H₃C-(CH₂)₁₁- | 9 | 3 | H₃C-CH₂-OSO₃⁻ |
| 852 | H₃C-(CH₂)₁₁- | 10 | 3 | H₃C-CH₂-OSO₃⁻ |
| 853 | H₃C-(CH₂)₁₁- | 4 | 4 | H₃C-CH₂-OSO₃⁻ |
| 854 | H₃C-(CH₂)₁₁- | 5 | 4 | H₃C-CH₂-OSO₃⁻ |
| 855 | H₃C-(CH₂)₁₁- | 6 | 4 | H₃C-CH₂-OSO₃⁻ |
| 856 | H₃C-(CH₂)₁₁- | 7 | 4 | H₃C-CH₂-OSO₃⁻ |
| 857 | H₃C-(CH₂)₁₁- | 8 | 4 | H₃C-CH₂-OSO₃⁻ |
| 858 | H₃C-(CH₂)₁₁- | 9 | 4 | H₃C-CH₂-OSO₃⁻ |
| 859 | H₃C-(CH₂)₁₁- | 10 | 4 | H₃C-CH₂-OSO₃⁻ |
| 860 | H₃C-(CH₂)₁₁- | 5 | 5 | H₃C-CH₂-OSO₃⁻ |
| 861 | H₃C-(CH₂)₁₁- | 6 | 5 | H₃C-CH₂-OSO₃⁻ |
| 862 | H₃C-(CH₂)₁₁- | 7 | 5 | H₃C-CH₂-OSO₃⁻ |
| 863 | H₃C-(CH₂)₁₁- | 8 | 5 | H₃C-CH₂-OSO₃⁻ |
| 864 | H₃C-(CH₂)₁₁- | 9 | 5 | H₃C-CH₂-OSO₃⁻ |
| 865 | H₃C-(CH₂)₁₁- | 10 | 5 | H₃C-CH₂-OSO₃⁻ |
| 866 | H₃C-(CH₂)₁₁- | 6 | 6 | H₃C-CH₂-OSO₃⁻ |
| 867 | H₃C-(CH₂)₁₁- | 7 | 6 | H₃C-CH₂-OSO₃⁻ |
| 868 | H₃C-(CH₂)₁₁- | 8 | 6 | H₃C-CH₂-OSO₃⁻ |
| 869 | H₃C-(CH₂)₁₁- | 9 | 6 | H₃C-CH₂-OSO₃⁻ |
| 870 | H₃C-(CH₂)₁₁- | 10 | 6 | H₃C-CH₂-OSO₃⁻ |
| 871 | H₃C-(CH₂)₁₁- | 7 | 7 | H₃C-CH₂-OSO₃⁻ |
| 872 | H₃C-(CH₂)₁₁- | 8 | 7 | H₃C-CH₂-OSO₃⁻ |
| 873 | H₃C-(CH₂)₁₁- | 9 | 7 | H₃C-CH₂-OSO₃⁻ |
| 874 | H₃C-(CH₂)₁₁- | 10 | 7 | H₃C-CH₂-OSO₃⁻ |
| 875 | H₃C-(CH₂)₁₁- | 8 | 8 | H₃C-CH₂-OSO₃⁻ |
| 876 | H₃C-(CH₂)₁₁- | 9 | 8 | H₃C-CH₂-OSO₃⁻ |
| 877 | H₃C-(CH₂)₁₁- | 10 | 8 | H₃C-CH₂-OSO₃⁻ |
| 878 | H₃C-(CH₂)₁₁- | 9 | 9 | H₃C-CH₂-OSO₃⁻ |
| 879 | H₃C-(CH₂)₁₁- | 10 | 9 | H₃C-CH₂-OSO₃⁻ |
| 880 | H₃C-(CH₂)₁₁- | 10 | 10 | H₃C-CH₂-OSO₃⁻ |
| 881 | H₃C-(CH₂)₁₃- | 1 | 1 | Chlorid |
| 882 | H₃C-(CH₂)₁₃- | 2 | 1 | Chlorid |
| 883 | H₃C-(CH₂)₁₃- | 3 | 1 | Chlorid |
| 884 | H₃C-(CH₂)₁₃- | 4 | 1 | Chlorid |
| 885 | H₃C-(CH₂)₁₃- | 5 | 1 | Chlorid |
| 886 | H₃C-(CH₂)₁₃- | 6 | 1 | Chlorid |
| 887 | H₃C-(CH₂)₁₃- | 7 | 1 | Chlorid |
| 888 | H₃C-(CH₂)₁₃- | 8 | 1 | Chlorid |
| 889 | H₃C-(CH₂)₁₃- | 9 | 1 | Chlorid |
| 890 | H₃C-(CH₂)₁₃- | 10 | 1 | Chlorid |
| 891 | H₃C-(CH₂)₁₃- | 2 | 2 | Chlorid |
| 892 | H₃C-(CH₂)₁₃- | 3 | 2 | Chlorid |
| 893 | H₃C-(CH₂)₁₃- | 4 | 2 | Chlorid |
| 894 | H₃C-(CH₂)₁₃- | 5 | 2 | Chlorid |
| 895 | H₃C-(CH₂)₁₃- | 6 | 2 | Chlorid |
| 896 | H₃C-(CH₂)₁₃- | 7 | 2 | Chlorid |
| 897 | H₃C-(CH₂)₁₃- | 8 | 2 | Chlorid |
| 898 | H₃C-(CH₂)₁₃- | 9 | 2 | Chlorid |
| 899 | H₃C-(CH₂)₁₃- | 10 | 2 | Chlorid |
| 900 | H₃C-(CH₂)₁₃- | 3 | 3 | Chlorid |
| 901 | H₃C-(CH₂)₁₃- | 4 | 3 | Chlorid |
| 902 | H₃C-(CH₂)₁₃- | 5 | 3 | Chlorid |
| 903 | H₃C-(CH₂)₁₃- | 6 | 3 | Chlorid |
| 904 | H₃C-(CH₂)₁₃- | 7 | 3 | Chlorid |
| 905 | H₃C-(CH₂)₁₃- | 8 | 3 | Chlorid |
| 906 | H₃C-(CH₂)₁₃- | 9 | 3 | Chlorid |
| 907 | H₃C-(CH₂)₁₃- | 10 | 3 | Chlorid |
| 908 | H₃C-(CH₂)₁₃- | 4 | 4 | Chlorid |
| 909 | H₃C-(CH₂)₁₃- | 5 | 4 | Chlorid |
| 910 | H₃C-(CH₂)₁₃- | 6 | 4 | Chlorid |
| 911 | H₃C-(CH₂)₁₃- | 7 | 4 | Chlorid |
| 912 | H₃C-(CH₂)₁₃- | 8 | 4 | Chlorid |
| 913 | H₃C-(CH₂)₁₃- | 9 | 4 | Chlorid |
| 914 | H₃C-(CH₂)₁₃- | 10 | 4 | Chlorid |
| 915 | H₃C-(CH₂)₁₃- | 5 | 5 | Chlorid |
| 916 | H₃C-(CH₂)₁₃- | 6 | 5 | Chlorid |
| 917 | H₃C-(CH₂)₁₃- | 7 | 5 | Chlorid |
| 918 | H₃C-(CH₂)₁₃- | 8 | 5 | Chlorid |
| 919 | H₃C-(CH₂)₁₃- | 9 | 5 | Chlorid |
| 920 | H₃C-(CH₂)₁₃- | 10 | 5 | Chlorid |
| 921 | H₃C-(CH₂)₁₃- | 6 | 6 | Chlorid |
| 922 | H₃C-(CH₂)₁₃- | 7 | 6 | Chlorid |
| 923 | H₃C-(CH₂)₁₃- | 8 | 6 | Chlorid |
| 924 | H₃C-(CH₂)₁₃- | 9 | 6 | Chlorid |
| 925 | H₃C-(CH₂)₁₃- | 10 | 6 | Chlorid |
| 926 | H₃C-(CH₂)₁₃- | 7 | 7 | Chlorid |
| 927 | H₃C-(CH₂)₁₃- | 8 | 7 | Chlorid |
| 928 | H₃C-(CH₂)₁₃- | 9 | 7 | Chlorid |
| 929 | H₃C-(CH₂)₁₃- | 10 | 7 | Chlorid |
| 930 | H₃C-(CH₂)₁₃- | 8 | 8 | Chlorid |
| 931 | H₃C-(CH₂)₁₃- | 9 | 8 | Chlorid |
| 932 | H₃C-(CH₂)₁₃- | 10 | 8 | Chlorid |
| 933 | H₃C-(CH₂)₁₃- | 9 | 9 | Chlorid |
| 934 | H₃C-(CH₂)₁₃- | 10 | 9 | Chlorid |
| 935 | H₃C-(CH₂)₁₃- | 10 | 10 | Chlorid |
| 936 | H₃C-(CH₂)₁₃- | 1 | 1 | Bromid |
| 937 | H₃C-(CH₂)₁₃- | 2 | 1 | Bromid |
| 938 | H₃C-(CH₂)₁₃- | 3 | 1 | Bromid |
| 939 | H₃C-(CH₂)₁₃- | 4 | 1 | Bromid |
| 940 | H₃C-(CH₂)₁₃- | 5 | 1 | Bromid |
| 941 | H₃C-(CH₂)₁₃- | 6 | 1 | Bromid |
| 942 | H₃C-(CH₂)₁₃- | 7 | 1 | Bromid |
| 943 | H₃C-(CH₂)₁₃- | 8 | 1 | Bromid |
| 944 | H₃C-(CH₂)₁₃- | 9 | 1 | Bromid |
| 945 | H₃C-(CH₂)₁₃- | 10 | 1 | Bromid |
| 946 | H₃C-(CH₂)₁₃- | 2 | 2 | Bromid |
| 947 | H₃C-(CH₂)₁₃- | 3 | 2 | Bromid |
| 948 | H₃C-(CH₂)₁₃- | 4 | 2 | Bromid |
| 949 | H₃C-(CH₂)₁₃- | 5 | 2 | Bromid |
| 950 | H₃C-(CH₂)₁₃- | 6 | 2 | Bromid |
| 951 | H₃C-(CH₂)₁₃- | 7 | 2 | Bromid |
| 952 | H₃C-(CH₂)₁₃- | 8 | 2 | Bromid |
| 953 | H₃C-(CH₂)₁₃- | 9 | 2 | Bromid |
| 954 | H₃C-(CH₂)₁₃- | 10 | 2 | Bromid |
| 955 | H₃C-(CH₂)₁₃- | 3 | 3 | Bromid |
| 956 | H₃C-(CH₂)₁₃- | 4 | 3 | Bromid |
| 957 | H₃C-(CH₂)₁₃- | 5 | 3 | Bromid |
| 958 | H₃C-(CH₂)₁₃- | 6 | 3 | Bromid |
| 959 | H₃C-(CH₂)₁₃- | 7 | 3 | Bromid |
| 960 | H₃C-(CH₂)₁₃- | 8 | 3 | Bromid |
| 961 | H₃C-(CH₂)₁₃- | 9 | 3 | Bromid |
| 962 | H₃C-(CH₂)₁₃- | 10 | 3 | Bromid |
| 963 | H₃C-(CH₂)₁₃- | 4 | 4 | Bromid |
| 964 | H₃C-(CH₂)₁₃- | 5 | 4 | Bromid |
| 965 | H₃C-(CH₂)₁₃- | 6 | 4 | Bromid |
| 966 | H₃C-(CH₂)₁₃- | 7 | 4 | Bromid |
| 967 | H₃C-(CH₂)₁₃- | 8 | 4 | Bromid |
| 968 | H₃C-(CH₂)₁₃- | 9 | 4 | Bromid |
| 969 | H₃C-(CH₂)₁₃- | 10 | 4 | Bromid |
| 970 | H₃C-(CH₂)₁₃- | 5 | 5 | Bromid |
| 971 | H₃C-(CH₂)₁₃- | 6 | 5 | Bromid |
| 972 | H₃C-(CH₂)₁₃- | 7 | 5 | Bromid |
| 973 | H₃C-(CH₂)₁₃- | 8 | 5 | Bromid |
| 974 | H₃C-(CH₂)₁₃- | 9 | 5 | Bromid |
| 975 | H₃C-(CH₂)₁₃- | 10 | 5 | Bromid |
| 976 | H₃C-(CH₂)₁₃- | 6 | 6 | Bromid |
| 977 | H₃C-(CH₂)₁₃- | 7 | 6 | Bromid |
| 978 | H₃C-(CH₂)₁₃- | 8 | 6 | Bromid |
| 979 | H₃C-(CH₂)₁₃- | 9 | 6 | Bromid |
| 980 | H₃C-(CH₂)₁₃- | 10 | 6 | Bromid |
| 981 | H₃C-(CH₂)₁₃- | 7 | 7 | Bromid |
| 982 | H₃C-(CH₂)₁₃- | 8 | 7 | Bromid |
| 983 | H₃C-(CH₂)₁₃- | 9 | 7 | Bromid |
| 984 | H₃C-(CH₂)₁₃- | 10 | 7 | Bromid |
| 985 | H₃C-(CH₂)₁₃- | 8 | 8 | Bromid |
| 986 | H₃C-(CH₂)₁₃- | 9 | 8 | Bromid |
| 987 | H₃C-(CH₂)₁₃- | 10 | 8 | Bromid |
| 988 | H₃C-(CH₂)₁₃- | 9 | 9 | Bromid |
| 989 | H₃C-(CH₂)₁₃- | 10 | 9 | Bromid |
| 990 | H₃C-(CH₂)₁₃- | 10 | 10 | Bromid |
| 991 | H₃C-(CH₂)₁₃- | 1 | 1 | H₃C-OSO₃⁻ |
| 992 | H₃C-(CH₂)₁₃- | 2 | 1 | H₃C-OSO₃⁻ |
| 993 | H₃C-(CH₂)₁₃- | 3 | 1 | H₃C-OSO₃⁻ |
| 994 | H₃C-(CH₂)₁₃- | 4 | 1 | H₃C-OSO₃⁻ |
| 995 | H₃C-(CH₂)₁₃- | 5 | 1 | H₃C-OSO₃⁻ |
| 996 | H₃C-(CH₂)₁₃- | 6 | 1 | H₃C-OSO₃⁻ |
| 997 | H₃C-(CH₂)₁₃- | 7 | 1 | H₃C-OSO₃⁻ |
| 998 | H₃C-(CH₂)₁₃- | 8 | 1 | H₃C-OSO₃⁻ |
| 999 | H₃C-(CH₂)₁₃- | 9 | 1 | H₃C-OSO₃⁻ |
| 1000 | H₃C-(CH₂)₁₃- | 10 | 1 | H₃C-OSO₃⁻ |
| 1001 | H₃C-(CH₂)₁₃- | 2 | 2 | H₃C-OSO₃⁻ |
| 1002 | H₃C-(CH₂)₁₃- | 3 | 2 | H₃C-OSO₃⁻ |
| 1003 | H₃C-(CH₂)₁₃- | 4 | 2 | H₃C-OSO₃⁻ |
| 1004 | H₃C-(CH₂)₁₃- | 5 | 2 | H₃C-OSO₃⁻ |
| 1005 | H₃C-(CH₂)₁₃- | 6 | 2 | H₃C-OSO₃⁻ |
| 1006 | H₃C-(CH₂)₁₃- | 7 | 2 | H₃C-OSO₃⁻ |
| 1007 | H₃C-(CH₂)₁₃- | 8 | 2 | H₃C-OSO₃⁻ |
| 1008 | H₃C-(CH₂)₁₃- | 9 | 2 | H₃C-OSO₃⁻ |
| 1009 | H₃C-(CH₂)₁₃- | 10 | 2 | H₃C-OSO₃⁻ |
| 1010 | H₃C-(CH₂)₁₃- | 3 | 3 | H₃C-OSO₃⁻ |
| 1011 | H₃C-(CH₂)₁₃- | 4 | 3 | H₃C-OSO₃⁻ |
| 1012 | H₃C-(CH₂)₁₃- | 5 | 3 | H₃C-OSO₃⁻ |
| 1013 | H₃C-(CH₂)₁₃- | 6 | 3 | H₃C-OSO₃⁻ |
| 1014 | H₃C-(CH₂)₁₃- | 7 | 3 | H₃C-OSO₃⁻ |
| 1015 | H₃C-(CH₂)₁₃- | 8 | 3 | H₃C-OSO₃⁻ |
| 1016 | H₃C-(CH₂)₁₃- | 9 | 3 | H₃C-OSO₃⁻ |
| 1017 | H₃C-(CH₂)₁₃- | 10 | 3 | H₃C-OSO₃⁻ |
| 1018 | H₃C-(CH₂)₁₃- | 4 | 4 | H₃C-OSO₃⁻ |
| 1019 | H₃C-(CH₂)₁₃- | 5 | 4 | H₃C-OSO₃⁻ |
| 1020 | H₃C-(CH₂)₁₃- | 6 | 4 | H₃C-OSO₃⁻ |
| 1021 | H₃C-(CH₂)₁₃- | 7 | 4 | H₃C-OSO₃⁻ |
| 1022 | H₃C-(CH₂)₁₃- | 8 | 4 | H₃C-OSO₃⁻ |
| 1023 | H₃C-(CH₂)₁₃- | 9 | 4 | H₃C-OSO₃⁻ |
| 1024 | H₃C-(CH₂)₁₃- | 10 | 4 | H₃C-OSO₃⁻ |
| 1025 | H₃C-(CH₂)₁₃- | 5 | 5 | H₃C-OSO₃⁻ |
| 1026 | H₃C-(CH₂)₁₃- | 6 | 5 | H₃C-OSO₃⁻ |
| 1027 | H₃C-(CH₂)₁₃- | 7 | 5 | H₃C-OSO₃⁻ |
| 1028 | H₃C-(CH₂)₁₃- | 8 | 5 | H₃C-OSO₃⁻ |
| 1029 | H₃C-(CH₂)₁₃- | 9 | 5 | H₃C-OSO₃⁻ |
| 1030 | H₃C-(CH₂)₁₃- | 10 | 5 | H₃C-OSO₃⁻ |
| 1031 | H₃C-(CH₂)₁₃- | 6 | 6 | H₃C-OSO₃⁻ |
| 1032 | H₃C-(CH₂)₁₃- | 7 | 6 | H₃C-OSO₃⁻ |
| 1033 | H₃C-(CH₂)₁₃- | 8 | 6 | H₃C-OSO₃⁻ |
| 1034 | H₃C-(CH₂)₁₃- | 9 | 6 | H₃C-OSO₃⁻ |
| 1035 | H₃C-(CH₂)₁₃- | 10 | 6 | H₃C-OSO₃⁻ |
| 1036 | H₃C-(CH₂)₁₃- | 7 | 7 | H₃C-OSO₃⁻ |
| 1037 | H₃C-(CH₂)₁₃- | 8 | 7 | H₃C-OSO₃⁻ |
| 1038 | H₃C-(CH₂)₁₃- | 9 | 7 | H₃C-OSO₃⁻ |
| 1039 | H₃C-(CH₂)₁₃- | 10 | 7 | H₃C-OSO₃⁻ |
| 1040 | H₃C-(CH₂)₁₃- | 8 | 8 | H₃C-OSO₃⁻ |
| 1041 | H₃C-(CH₂)₁₃- | 9 | 8 | H₃C-OSO₃⁻ |
| 1042 | H₃C-(CH₂)₁₃- | 10 | 8 | H₃C-OSO₃⁻ |
| 1043 | H₃C-(CH₂)₁₃- | 9 | 9 | H₃C-OSO₃⁻ |
| 1044 | H₃C-(CH₂)₁₃- | 10 | 9 | H₃C-OSO₃⁻ |
| 1045 | H₃C-(CH₂)₁₃- | 10 | 10 | H₃C-OSO₃⁻ |
| 1046 | H₃C-(CH₂)₁₃- | 1 | 1 | H₃C-CH₂-OSO₃⁻ |
| 1047 | H₃C-(CH₂)₁₃- | 2 | 1 | H₃C-CH₂-OSO₃⁻ |
| 1048 | H₃C-(CH₂)₁₃- | 3 | 1 | H₃C-CH₂-OSO₃⁻ |
| 1049 | H₃C-(CH₂)₁₃- | 4 | 1 | H₃C-CH₂-OSO₃⁻ |
| 1050 | H₃C-(CH₂)₁₃- | 5 | 1 | H₃C-CH₂-OSO₃⁻ |
| 1051 | H₃C-(CH₂)₁₃- | 6 | 1 | H₃C-CH₂-OSO₃⁻ |
| 1052 | H₃C-(CH₂)₁₃- | 7 | 1 | H₃C-CH₂-OSO₃⁻ |
| 1053 | H₃C-(CH₂)₁₃- | 8 | 1 | H₃C-CH₂-OSO₃⁻ |
| 1054 | H₃C-(CH₂)₁₃- | 9 | 1 | H₃C-CH₂-OSO₃⁻ |
| 1055 | H₃C-(CH₂)₁₃- | 10 | 1 | H₃C-CH₂-OSO₃⁻ |
| 1056 | H₃C-(CH₂)₁₃- | 2 | 2 | H₃C-CH₂-OSO₃⁻ |
| 1057 | H₃C-(CH₂)₁₃- | 3 | 2 | H₃C-CH₂-OSO₃⁻ |
| 1058 | H₃C-(CH₂)₁₃- | 4 | 2 | H₃C-CH₂-OSO₃⁻ |
| 1059 | H₃C-(CH₂)₁₃- | 5 | 2 | H₃C-CH₂-OSO₃⁻ |
| 1060 | H₃C-(CH₂)₁₃- | 6 | 2 | H₃C-CH₂-OSO₃⁻ |
| 1061 | H₃C-(CH₂)₁₃- | 7 | 2 | H₃C-CH₂-OSO₃⁻ |
| 1062 | H₃C-(CH₂)₁₃- | 8 | 2 | H₃C-CH₂-OSO₃⁻ |
| 1063 | H₃C-(CH₂)₁₃- | 9 | 2 | H₃C-CH₂-OSO₃⁻ |
| 1064 | H₃C-(CH₂)₁₃- | 10 | 2 | H₃C-CH₂-OSO₃⁻ |
| 1065 | H₃C-(CH₂)₁₃- | 3 | 3 | H₃C-CH₂-OSO₃⁻ |
| 1066 | H₃C-(CH₂)₁₃- | 4 | 3 | H₃C-CH₂-OSO₃⁻ |
| 1067 | H₃C-(CH₂)₁₃- | 5 | 3 | H₃C-CH₂-OSO₃⁻ |
| 1068 | H₃C-(CH₂)₁₃- | 6 | 3 | H₃C-CH₂-OSO₃⁻ |
| 1069 | H₃C-(CH₂)₁₃- | 7 | 3 | H₃C-CH₂-OSO₃⁻ |
| 1070 | H₃C-(CH₂)₁₃- | 8 | 3 | H₃C-CH₂-OSO₃⁻ |
| 1071 | H₃C-(CH₂)₁₃- | 9 | 3 | H₃C-CH₂-OSO₃⁻ |
| 1072 | H₃C-(CH₂)₁₃- | 10 | 3 | H₃C-CH₂-OSO₃⁻ |
| 1073 | H₃C-(CH₂)₁₃- | 4 | 4 | H₃C-CH₂-OSO₃⁻ |
| 1074 | H₃C-(CH₂)₁₃- | 5 | 4 | H₃C-CH₂-OSO₃⁻ |
| 1075 | H₃C-(CH₂)₁₃- | 6 | 4 | H₃C-CH₂-OSO₃⁻ |
| 1076 | H₃C-(CH₂)₁₃- | 7 | 4 | H₃C-CH₂-OSO₃⁻ |
| 1077 | H₃C-(CH₂)₁₃- | 8 | 4 | H₃C-CH₂-OSO₃⁻ |
| 1078 | H₃C-(CH₂)₁₃- | 9 | 4 | H₃C-CH₂-OSO₃⁻ |
| 1079 | H₃C-(CH₂)₁₃- | 10 | 4 | H₃C-CH₂-OSO₃⁻ |
| 1080 | H₃C-(CH₂)₁₃- | 5 | 5 | H₃C-CH₂-OSO₃⁻ |
| 1081 | H₃C-(CH₂)₁₃- | 6 | 5 | H₃C-CH₂-OSO₃⁻ |
| 1082 | H₃C-(CH₂)₁₃- | 7 | 5 | H₃C-CH₂-OSO₃⁻ |
| 1083 | H₃C-(CH₂)₁₃- | 8 | 5 | H₃C-CH₂-OSO₃⁻ |
| 1084 | H₃C-(CH₂)₁₃- | 9 | 5 | H₃C-CH₂-OSO₃⁻ |
| 1085 | H₃C-(CH₂)₁₃- | 10 | 5 | H₃C-CH₂-OSO₃⁻ |
| 1086 | H₃C-(CH₂)₁₃- | 6 | 6 | H₃C-CH₂-OSO₃⁻ |
| 1087 | H₃C-(CH₂)₁₃- | 7 | 6 | H₃C-CH₂-OSO₃⁻ |
| 1088 | H₃C-(CH₂)₁₃- | 8 | 6 | H₃C-CH₂-OSO₃⁻ |
| 1089 | H₃C-(CH₂)₁₃- | 9 | 6 | H₃C-CH₂-OSO₃⁻ |
| 1090 | H₃C-(CH₂)₁₃- | 10 | 6 | H₃C-CH₂-OSO₃⁻ |
| 1091 | H₃C-(CH₂)₁₃- | 7 | 7 | H₃C-CH₂-OSO₃⁻ |
| 1092 | H₃C-(CH₂)₁₃- | 8 | 7 | H₃C-CH₂-OSO₃⁻ |
| 1093 | H₃C-(CH₂)₁₃- | 9 | 7 | H₃C-CH₂-OSO₃⁻ |
| 1094 | H₃C-(CH₂)₁₃- | 10 | 7 | H₃C-CH₂-OSO₃⁻ |
| 1095 | H₃C-(CH₂)₁₃- | 8 | 8 | H₃C-CH₂-OSO₃⁻ |
| 1096 | H₃C-(CH₂)₁₃- | 9 | 8 | H₃C-CH₂-OSO₃⁻ |
| 1097 | H₃C-(CH₂)₁₃- | 10 | 8 | H₃C-CH₂-OSO₃⁻ |
| 1098 | H₃C-(CH₂)₁₃- | 9 | 9 | H₃C-CH₂-OSO₃⁻ |
| 1099 | H₃C-(CH₂)₁₃- | 10 | 9 | H₃C-CH₂-OSO₃⁻ |
| 1100 | H₃C-(CH₂)₁₃- | 10 | 10 | H₃C-CH₂-OSO₃⁻ |
| 1101 | H₃C-(CH₂)₁₅- | 1 | 1 | Chlorid |
| 1102 | H₃C-(CH₂)₁₅- | 2 | 1 | Chlorid |
| 1103 | H₃C-(CH₂)₁₅- | 3 | 1 | Chlorid |
| 1104 | H₃C-(CH₂)₁₅- | 4 | 1 | Chlorid |
| 1105 | H₃C-(CH₂)₁₅- | 5 | 1 | Chlorid |
| 1106 | H₃C-(CH₂)₁₅- | 6 | 1 | Chlorid |
| 1107 | H₃C-(CH₂)₁₅- | 7 | 1 | Chlorid |
| 1108 | H₃C-(CH₂)₁₅- | 8 | 1 | Chlorid |
| 1109 | H₃C-(CH₂)₁₅- | 9 | 1 | Chlorid |
| 1110 | H₃C-(CH₂)₁₅- | 10 | 1 | Chlorid |
| 1111 | H₃C-(CH₂)₁₅- | 2 | 2 | Chlorid |
| 1112 | H₃C-(CH₂)₁₅- | 3 | 2 | Chlorid |
| 1113 | H₃C-(CH₂)₁₅- | 4 | 2 | Chlorid |
| 1114 | H₃C-(CH₂)₁₅- | 5 | 2 | Chlorid |
| 1115 | H₃C-(CH₂)₁₅- | 6 | 2 | Chlorid |
| 1116 | H₃C-(CH₂)₁₅- | 7 | 2 | Chlorid |
| 1117 | H₃C-(CH₂)₁₅- | 8 | 2 | Chlorid |
| 1118 | H₃C-(CH₂)₁₅- | 9 | 2 | Chlorid |
| 1119 | H₃C-(CH₂)₁₅- | 10 | 2 | Chlorid |
| 1120 | H₃C-(CH₂)₁₅- | 3 | 3 | Chlorid |
| 1121 | H₃C-(CH₂)₁₅- | 4 | 3 | Chlorid |
| 1122 | H₃C-(CH₂)₁₅- | 5 | 3 | Chlorid |
| 1123 | H₃C-(CH₂)₁₅- | 6 | 3 | Chlorid |
| 1124 | H₃C-(CH₂)₁₅- | 7 | 3 | Chlorid |
| 1125 | H₃C-(CH₂)₁₅- | 8 | 3 | Chlorid |
| 1126 | H₃C-(CH₂)₁₅- | 9 | 3 | Chlorid |
| 1127 | H₃C-(CH₂)₁₅- | 10 | 3 | Chlorid |
| 1128 | H₃C-(CH₂)₁₅- | 4 | 4 | Chlorid |
| 1129 | H₃C-(CH₂)₁₅- | 5 | 4 | Chlorid |
| 1130 | H₃C-(CH₂)₁₅- | 6 | 4 | Chlorid |
| 1131 | H₃C-(CH₂)₁₅- | 7 | 4 | Chlorid |
| 1132 | H₃C-(CH₂)₁₅- | 8 | 4 | Chlorid |
| 1133 | H₃C-(CH₂)₁₅- | 9 | 4 | Chlorid |
| 1134 | H₃C-(CH₂)₁₅- | 10 | 4 | Chlorid |
| 1135 | H₃C-(CH₂)₁₅- | 5 | 5 | Chlorid |
| 1136 | H₃C-(CH₂)₁₅- | 6 | 5 | Chlorid |
| 1137 | H₃C-(CH₂)₁₅- | 7 | 5 | Chlorid |
| 1138 | H₃C-(CH₂)₁₅- | 8 | 5 | Chlorid |
| 1139 | H₃C-(CH₂)₁₅- | 9 | 5 | Chlorid |
| 1140 | H₃C-(CH₂)₁₅- | 10 | 5 | Chlorid |
| 1141 | H₃C-(CH₂)₁₅- | 6 | 6 | Chlorid |
| 1142 | H₃C-(CH₂)₁₅- | 7 | 6 | Chlorid |
| 1143 | H₃C-(CH₂)₁₅- | 8 | 6 | Chlorid |
| 1144 | H₃C-(CH₂)₁₅- | 9 | 6 | Chlorid |
| 1145 | H₃C-(CH₂)₁₅- | 10 | 6 | Chlorid |
| 1146 | H₃C-(CH₂)₁₅- | 7 | 7 | Chlorid |
| 1147 | H₃C-(CH₂)₁₅- | 8 | 7 | Chlorid |
| 1148 | H₃C-(CH₂)₁₅- | 9 | 7 | Chlorid |
| 1149 | H₃C-(CH₂)₁₅- | 10 | 7 | Chlorid |
| 1150 | H₃C-(CH₂)₁₅- | 8 | 8 | Chlorid |
| 1151 | H₃C-(CH₂)₁₅- | 9 | 8 | Chlorid |
| 1152 | H₃C-(CH₂)₁₅- | 10 | 8 | Chlorid |
| 1153 | H₃C-(CH₂)₁₅- | 9 | 9 | Chlorid |
| 1154 | H₃C-(CH₂)₁₅- | 10 | 9 | Chlorid |
| 1155 | H₃C-(CH₂)₁₅- | 10 | 10 | Chlorid |
| 1156 | H₃C-(CH₂)₁₅- | 1 | 1 | Bromid |
| 1157 | H₃C-(CH₂)₁₅- | 2 | 1 | Bromid |
| 1158 | H₃C-(CH₂)₁₅- | 3 | 1 | Bromid |
| 1159 | H₃C-(CH₂)₁₅- | 4 | 1 | Bromid |
| 1160 | H₃C-(CH₂)₁₅- | 5 | 1 | Bromid |
| 1161 | H₃C-(CH₂)₁₅- | 6 | 1 | Bromid |
| 1162 | H₃C-(CH₂)₁₅- | 7 | 1 | Bromid |
| 1163 | H₃C-(CH₂)₁₅- | 8 | 1 | Bromid |
| 1164 | H₃C-(CH₂)₁₅- | 9 | 1 | Bromid |
| 1165 | H₃C-(CH₂)₁₅- | 10 | 1 | Bromid |
| 1166 | H₃C-(CH₂)₁₅- | 2 | 2 | Bromid |
| 1167 | H₃C-(CH₂)₁₅- | 3 | 2 | Bromid |
| 1168 | H₃C-(CH₂)₁₅- | 4 | 2 | Bromid |
| 1169 | H₃C-(CH₂)₁₅- | 5 | 2 | Bromid |
| 1170 | H₃C-(CH₂)₁₅- | 6 | 2 | Bromid |
| 1171 | H₃C-(CH₂)₁₅- | 7 | 2 | Bromid |
| 1172 | H₃C-(CH₂)₁₅- | 8 | 2 | Bromid |
| 1173 | H₃C-(CH₂)₁₅- | 9 | 2 | Bromid |
| 1174 | H₃C-(CH₂)₁₅- | 10 | 2 | Bromid |
| 1175 | H₃C-(CH₂)₁₅- | 3 | 3 | Bromid |
| 1176 | H₃C-(CH₂)₁₅- | 4 | 3 | Bromid |
| 1177 | H₃C-(CH₂)₁₅- | 5 | 3 | Bromid |
| 1178 | H₃C-(CH₂)₁₅- | 6 | 3 | Bromid |
| 1179 | H₃C-(CH₂)₁₅- | 7 | 3 | Bromid |
| 1180 | H₃C-(CH₂)₁₅- | 8 | 3 | Bromid |
| 1181 | H₃C-(CH₂)₁₅- | 9 | 3 | Bromid |
| 1182 | H₃C-(CH₂)₁₅- | 10 | 3 | Bromid |
| 1183 | H₃C-(CH₂)₁₅- | 4 | 4 | Bromid |
| 1184 | H₃C-(CH₂)₁₅- | 5 | 4 | Bromid |
| 1185 | H₃C-(CH₂)₁₅- | 6 | 4 | Bromid |
| 1186 | H₃C-(CH₂)₁₅- | 7 | 4 | Bromid |
| 1187 | H₃C-(CH₂)₁₅- | 8 | 4 | Bromid |
| 1188 | H₃C-(CH₂)₁₅- | 9 | 4 | Bromid |
| 1189 | H₃C-(CH₂)₁₅- | 10 | 4 | Bromid |
| 1190 | H₃C-(CH₂)₁₅- | 5 | 5 | Bromid |
| 1191 | H₃C-(CH₂)₁₅- | 6 | 5 | Bromid |
| 1192 | H₃C-(CH₂)₁₅- | 7 | 5 | Bromid |
| 1193 | H₃C-(CH₂)₁₅- | 8 | 5 | Bromid |
| 1194 | H₃C-(CH₂)₁₅- | 9 | 5 | Bromid |
| 1195 | H₃C-(CH₂)₁₅- | 10 | 5 | Bromid |
| 1196 | H₃C-(CH₂)₁₅- | 6 | 6 | Bromid |
| 1197 | H₃C-(CH₂)₁₅- | 7 | 6 | Bromid |
| 1198 | H₃C-(CH₂)₁₅- | 8 | 6 | Bromid |
| 1199 | H₃C-(CH₂)₁₅- | 9 | 6 | Bromid |
| 1200 | H₃C-(CH₂)₁₅- | 10 | 6 | Bromid |
| 1201 | H₃C-(CH₂)₁₅- | 7 | 7 | Bromid |
| 1202 | H₃C-(CH₂)₁₅- | 8 | 7 | Bromid |
| 1203 | H₃C-(CH₂)₁₅- | 9 | 7 | Bromid |
| 1204 | H₃C-(CH₂)₁₅- | 10 | 7 | Bromid |
| 1205 | H₃C-(CH₂)₁₅- | 8 | 8 | Bromid |
| 1206 | H₃C-(CH₂)₁₅- | 9 | 8 | Bromid |
| 1207 | H₃C-(CH₂)₁₅- | 10 | 8 | Bromid |
| 1208 | H₃C-(CH₂)₁₅- | 9 | 9 | Bromid |
| 1209 | H₃C-(CH₂)₁₅- | 10 | 9 | Bromid |
| 1210 | H₃C-(CH₂)₁₅- | 10 | 10 | Bromid |
| 1211 | H₃C-(CH₂)₁₅- | 1 | 1 | H₃C-OSO₃⁻ |
| 1212 | H₃C-(CH₂)₁₅- | 2 | 1 | H₃C-OSO₃⁻ |
| 1213 | H₃C-(CH₂)₁₅- | 3 | 1 | H₃C-OSO₃⁻ |
| 1214 | H₃C-(CH₂)₁₅- | 4 | 1 | H₃C-OSO₃⁻ |
| 1215 | H₃C-(CH₂)₁₅- | 5 | 1 | H₃C-OSO₃⁻ |
| 1216 | H₃C-(CH₂)₁₅- | 6 | 1 | H₃C-OSO₃⁻ |
| 1217 | H₃C-(CH₂)₁₅- | 7 | 1 | H₃C-OSO₃⁻ |
| 1218 | H₃C-(CH₂)₁₅- | 8 | 1 | H₃C-OSO₃⁻ |
| 1219 | H₃C-(CH₂)₁₅- | 9 | 1 | H₃C-OSO₃⁻ |
| 1220 | H₃C-(CH₂)₁₅- | 10 | 1 | H₃C-OSO₃⁻ |
| 1221 | H₃C-(CH₂)₁₅- | 2 | 2 | H₃C-OSO₃⁻ |
| 1222 | H₃C-(CH₂)₁₅- | 3 | 2 | H₃C-OSO₃⁻ |
| 1223 | H₃C-(CH₂)₁₅- | 4 | 2 | H₃C-OSO₃⁻ |
| 1224 | H₃C-(CH₂)₁₅- | 5 | 2 | H₃C-OSO₃⁻ |
| 1225 | H₃C-(CH₂)₁₅- | 6 | 2 | H₃C-OSO₃⁻ |
| 1226 | H₃C-(CH₂)₁₅- | 7 | 2 | H₃C-OSO₃⁻ |
| 1227 | H₃C-(CH₂)₁₅- | 8 | 2 | H₃C-OSO₃⁻ |
| 1228 | H₃C-(CH₂)₁₅- | 9 | 2 | H₃C-OSO₃⁻ |
| 1229 | H₃C-(CH₂)₁₅- | 10 | 2 | H₃C-OSO₃⁻ |
| 1230 | H₃C-(CH₂)₁₅- | 3 | 3 | H₃C-OSO₃⁻ |
| 1231 | H₃C-(CH₂)₁₅- | 4 | 3 | H₃C-OSO₃⁻ |
| 1232 | H₃C-(CH₂)₁₅- | 5 | 3 | H₃C-OSO₃⁻ |
| 1233 | H₃C-(CH₂)₁₅- | 6 | 3 | H₃C-OSO₃⁻ |
| 1234 | H₃C-(CH₂)₁₅- | 7 | 3 | H₃C-OSO₃⁻ |
| 1235 | H₃C-(CH₂)₁₅- | 8 | 3 | H₃C-OSO₃⁻ |
| 1236 | H₃C-(CH₂)₁₅- | 9 | 3 | H₃C-OSO₃⁻ |
| 1237 | H₃C-(CH₂)₁₅- | 10 | 3 | H₃C-OSO₃⁻ |
| 1238 | H₃C-(CH₂)₁₅- | 4 | 4 | H₃C-OSO₃⁻ |
| 1239 | H₃C-(CH₂)₁₅- | 5 | 4 | H₃C-OSO₃⁻ |
| 1240 | H₃C-(CH₂)₁₅- | 6 | 4 | H₃C-OSO₃⁻ |
| 1241 | H₃C-(CH₂)₁₅- | 7 | 4 | H₃C-OSO₃⁻ |
| 1242 | H₃C-(CH₂)₁₅- | 8 | 4 | H₃C-OSO₃⁻ |
| 1243 | H₃C-(CH₂)₁₅- | 9 | 4 | H₃C-OSO₃⁻ |
| 1244 | H₃C-(CH₂)₁₅- | 10 | 4 | H₃C-OSO₃⁻ |
| 1245 | H₃C-(CH₂)₁₅- | 5 | 5 | H₃C-OSO₃⁻ |
| 1246 | H₃C-(CH₂)₁₅- | 6 | 5 | H₃C-OSO₃⁻ |
| 1247 | H₃C-(CH₂)₁₅- | 7 | 5 | H₃C-OSO₃⁻ |
| 1248 | H₃C-(CH₂)₁₅- | 8 | 5 | H₃C-OSO₃⁻ |
| 1249 | H₃C-(CH₂)₁₅- | 9 | 5 | H₃C-OSO₃⁻ |
| 1250 | H₃C-(CH₂)₁₅- | 10 | 5 | H₃C-OSO₃⁻ |
| 1251 | H₃C-(CH₂)₁₅- | 6 | 6 | H₃C-OSO₃⁻ |
| 1252 | H₃C-(CH₂)₁₅- | 7 | 6 | H₃C-OSO₃⁻ |
| 1253 | H₃C-(CH₂)₁₅- | 8 | 6 | H₃C-OSO₃⁻ |
| 1254 | H₃C-(CH₂)₁₅- | 9 | 6 | H₃C-OSO₃⁻ |
| 1255 | H₃C-(CH₂)₁₅- | 10 | 6 | H₃C-OSO₃⁻ |
| 1256 | H₃C-(CH₂)₁₅- | 7 | 7 | H₃C-OSO₃⁻ |
| 1257 | H₃C-(CH₂)₁₅- | 8 | 7 | H₃C-OSO₃⁻ |
| 1258 | H₃C-(CH₂)₁₅- | 9 | 7 | H₃C-OSO₃⁻ |
| 1259 | H₃C-(CH₂)₁₅- | 10 | 7 | H₃C-OSO₃⁻ |
| 1260 | H₃C-(CH₂)₁₅- | 8 | 8 | H₃C-OSO₃⁻ |
| 1261 | H₃C-(CH₂)₁₅- | 9 | 8 | H₃C-OSO₃⁻ |
| 1262 | H₃C-(CH₂)₁₅- | 10 | 8 | H₃C-OSO₃⁻ |
| 1263 | H3_{C}-(CH₂)₁₅- | 9 | 9 | H₃C-OSO₃⁻ |
| 1264 | H₃C-(CH₂)₁₅- | 10 | 9 | H₃C-OSO₃⁻ |
| 1265 | H₃C-(CH₂)₁₅- | 10 | 10 | H₃C-OSO₃⁻ |
| 1266 | H₃C-(CH₂)₁₅- | 1 | 1 | H₃C-CH₂-OSO₃⁻ |
| 1267 | H₃C-(CH₂)₁₅- | 2 | 1 | H₃C-CH₂-OSO₃⁻ |
| 1268 | H₃C-(CH₂)₁₅- | 3 | 1 | H₃C-CH₂-OSO₃⁻ |
| 1269 | H₃C-(CH₂)₁₅- | 4 | 1 | H₃C-CH₂-OSO₃⁻ |
| 1270 | H₃C-(CH₂)₁₅- | 5 | 1 | H₃C-CH₂-OSO₃⁻ |
| 1271 | H₃C-(CH₂)₁₅- | 6 | 1 | H₃C-CH₂-OSO₃⁻ |
| 1272 | H₃C-(CH₂)₁₅- | 7 | 1 | H₃C-CH₂-OSO₃⁻ |
| 1273 | H₃C-(CH₂)₁₅- | 8 | 1 | H₃C-CH₂-OSO₃⁻ |
| 1274 | H₃C-(CH₂)₁₅- | 9 | 1 | H₃C-CH₂-OSO₃⁻ |
| 1275 | H₃C-(CH₂)₁₅- | 10 | 1 | H₃C-CH₂-OSO₃⁻ |
| 1276 | H₃C-(CH₂)₁₅- | 2 | 2 | H₃C-CH₂-OSO₃⁻ |
| 1277 | H₃C-(CH₂)₁₅- | 3 | 2 | H₃C-CH₂-OSO₃⁻ |
| 1278 | H₃C-(CH₂)₁₅- | 4 | 2 | H₃C-CH₂-OSO₃⁻ |
| 1279 | H₃C-(CH₂)₁₅- | 5 | 2 | H₃C-CH₂-OSO₃⁻ |
| 1280 | H₃C-(CH₂)₁₅- | 6 | 2 | H₃C-CH₂-OSO₃⁻ |
| 1281 | H₃C-(CH₂)₁₅- | 7 | 2 | H₃C-CH₂-OSO₃⁻ |
| 1282 | H₃C-(CH₂)₁₅- | 8 | 2 | H₃C-CH₂-OSO₃⁻ |
| 1283 | H₃C-(CH₂)₁₅- | 9 | 2 | H₃C-CH₂-OSO₃⁻ |
| 1284 | H₃C-(CH₂)₁₅- | 10 | 2 | H₃C-CH₂-OSO₃⁻ |
| 1285 | H₃C-(CH₂)₁₅- | 3 | 3 | H₃C-CH₂-OSO₃⁻ |
| 1286 | H₃C-(CH₂)₁₅- | 4 | 3 | H₃C-CH₂-OSO₃⁻ |
| 1287 | H₃C-(CH₂)₁₅- | 5 | 3 | H₃C-CH₂-OSO₃⁻ |
| 1288 | H₃C-(CH₂)₁₅- | 6 | 3 | H₃C-CH₂-OSO₃⁻ |
| 1289 | H₃C-(CH₂)₁₅- | 7 | 3 | H₃C-CH₂-OSO₃⁻ |
| 1290 | H₃C-(CH₂)₁₅- | 8 | 3 | H₃C-CH₂-OSO₃⁻ |
| 1291 | H₃C-(CH₂)₁₅- | 9 | 3 | H₃C-CH₂-OSO₃⁻ |
| 1292 | H₃C-(CH₂)₁₅- | 10 | 3 | H₃C-CH₂-OSO₃⁻ |
| 1293 | H₃C-(CH₂)₁₅- | 4 | 4 | H₃C-CH₂-OSO₃⁻ |
| 1294 | H₃C-(CH₂)₁₅- | 5 | 4 | H₃C-CH₂-OSO₃⁻ |
| 1295 | H₃C-(CH₂)₁₅- | 6 | 4 | H₃C-CH₂-OSO₃⁻ |
| 1296 | H₃C-(CH₂)₁₅- | 7 | 4 | H₃C-CH₂-OSO₃⁻ |
| 1297 | H₃C-(CH₂)₁₅- | 8 | 4 | H₃C-CH₂-OSO₃⁻ |
| 1298 | H₃C-(CH₂)₁₅- | 9 | 4 | H₃C-CH₂-OSO₃⁻ |
| 1299 | H₃C-(CH₂)₁₅- | 10 | 4 | H₃C-CH₂-OSO₃⁻ |
| 1300 | H₃C-(CH₂)₁₅- | 5 | 5 | H₃C-CH₂-OSO₃⁻ |
| 1301 | H₃C-(CH₂)₁₅- | 6 | 5 | H₃C-CH₂-OSO₃⁻ |
| 1302 | H₃C-(CH₂)₁₅- | 7 | 5 | H₃C-CH₂-OSO₃⁻ |
| 1303 | H₃C-(CH₂)₁₅- | 8 | 5 | H₃C-CH₂-OSO₃⁻ |
| 1304 | H₃C-(CH₂)₁₅- | 9 | 5 | H₃C-CH₂-OSO₃⁻ |
| 1305 | H₃C-(CH₂)₁₅- | 10 | 5 | H₃C-CH₂-OSO₃⁻ |
| 1306 | H₃C-(CH₂)₁₅- | 6 | 6 | H₃C-CH₂-OSO₃⁻ |
| 1307 | H₃C-(CH₂)₁₅- | 7 | 6 | H₃C-CH₂-OSO₃⁻ |
| 1308 | H₃C-(CH₂)₁₅- | 8 | 6 | H₃C-CH₂-OSO₃⁻ |
| 1309 | H₃C-(CH₂)₁₅- | 9 | 6 | H₃C-CH₂-OSO₃⁻ |
| 1310 | H₃C-(CH₂)₁₅- | 10 | 6 | H₃C-CH₂-OSO₃⁻ |
| 1311 | H₃C-(CH₂)₁₅- | 7 | 7 | H₃C-CH₂-OSO₃⁻ |
| 1312 | H₃C-(CH₂)₁₅- | 8 | 7 | H₃C-CH₂-OSO₃⁻ |
| 1313 | H₃C-(CH₂)₁₅- | 9 | 7 | H₃C-CH₂-OSO₃⁻ |
| 1314 | H₃C-(CH₂)₁₅- | 10 | 7 | H₃C-CH₂-OSO₃⁻ |
| 1315 | H₃C-(CH₂)₁₅- | 8 | 8 | H₃C-CH₂-OSO₃⁻ |
| 1316 | H₃C-(CH₂)₁₅- | 9 | 8 | H₃C-CH₂-OSO₃⁻ |
| 1317 | H₃C-(CH₂)₁₅- | 10 | 8 | H₃C-CH₂-OSO₃⁻ |
| 1318 | H₃C-(CH₂)₁₅- | 9 | 9 | H₃C-CH₂-OSO₃⁻ |
| 1319 | H₃C-(CH₂)₁₅- | 10 | 9 | H₃C-CH₂-OSO₃⁻ |
| 1320 | H₃C-(CH₂)₁₅- | 10 | 10 | H₃C-CH₂-OSO₃⁻ |
| 1321 | H₃C-(CH₂)₁₇- | 1 | 1 | Chlorid |
| 1322 | H₃C-(CH₂)₁₇- | 2 | 1 | Chlorid |
| 1323 | H₃C-(CH₂)₁₇- | 3 | 1 | Chlorid |
| 1324 | H₃C-(CH₂)₁₇- | 4 | 1 | Chlorid |
| 1325 | H₃C-(CH₂)₁₇- | 5 | 1 | Chlorid |
| 1326 | H₃C-(CH₂)₁₇- | 6 | 1 | Chlorid |
| 1327 | H₃C-(CH₂)₁₇- | 7 | 1 | Chlorid |
| 1328 | H₃C-(CH₂)₁₇- | 8 | 1 | Chlorid |
| 1329 | H₃C-(CH₂)₁₇- | 9 | 1 | Chlorid |
| 1330 | H₃C-(CH₂)₁₇- | 10 | 1 | Chlorid |
| 1331 | H₃C-(CH₂)₁₇- | 2 | 2 | Chlorid |
| 1332 | H₃C-(CH₂)₁₇- | 3 | 2 | Chlorid |
| 1333 | H₃C-(CH₂)₁₇- | 4 | 2 | Chlorid |
| 1334 | H₃C-(CH₂)₁₇- | 5 | 2 | Chlorid |
| 1335 | H₃C-(CH₂)₁₇- | 6 | 2 | Chlorid |
| 1336 | H₃C-(CH₂)₁₇- | 7 | 2 | Chlorid |
| 1337 | H₃C-(CH₂)₁₇- | 8 | 2 | Chlorid |
| 1338 | H₃C-(CH₂)₁₇- | 9 | 2 | Chlorid |
| 1339 | H₃C-(CH₂)₁₇- | 10 | 2 | Chlorid |
| 1340 | H₃C-(CH₂)₁₇- | 3 | 3 | Chlorid |
| 1341 | H₃C-(CH₂)₁₇- | 4 | 3 | Chlorid |
| 1342 | H₃C-(CH₂)₁₇- | 5 | 3 | Chlorid |
| 1343 | H₃C-(CH₂)₁₇- | 6 | 3 | Chlorid |
| 1344 | H₃C-(CH₂)₁₇- | 7 | 3 | Chlorid |
| 1345 | H₃C-(CH₂)₁₇- | 8 | 3 | Chlorid |
| 1346 | H₃C-(CH₂)₁₇- | 9 | 3 | Chlorid |
| 1347 | H₃C-(CH₂)₁₇- | 10 | 3 | Chlorid |
| 1348 | H₃C-(CH₂)₁₇- | 4 | 4 | Chlorid |
| 1349 | H₃C-(CH₂)₁₇- | 5 | 4 | Chlorid |
| 1350 | H₃C-(CH₂)₁₇- | 6 | 4 | Chlorid |
| 1351 | H₃C-(CH₂)₁₇- | 7 | 4 | Chlorid |
| 1352 | H₃C-(CH₂)₁₇- | 8 | 4 | Chlorid |
| 1353 | H₃C-(CH₂)₁₇- | 9 | 4 | Chlorid |
| 1354 | H₃C-(CH₂)₁₇- | 10 | 4 | Chlorid |
| 1355 | H₃C-(CH₂)₁₇- | 5 | 5 | Chlorid |
| 1356 | H₃C-(CH₂)₁₇- | 6 | 5 | Chlorid |
| 1357 | H₃C-(CH₂)₁₇- | 7 | 5 | Chlorid |
| 1358 | H₃C-(CH₂)₁₇- | 8 | 5 | Chlorid |
| 1359 | H₃C-(CH₂)₁₇- | 9 | 5 | Chlorid |
| 1360 | H₃C-(CH₂)₁₇- | 10 | 5 | Chlorid |
| 1361 | H₃C-(CH₂)₁₇- | 6 | 6 | Chlorid |
| 1362 | H₃C-(CH₂)₁₇- | 7 | 6 | Chlorid |
| 1363 | H₃C-(CH₂)₁₇- | 8 | 6 | Chlorid |
| 1364 | H₃C-(CH₂)₁₇- | 9 | 6 | Chlorid |
| 1365 | H₃C-(CH₂)₁₇- | 10 | 6 | Chlorid |
| 1366 | H₃C-(CH₂)₁₇- | 7 | 7 | Chlorid |
| 1367 | H₃C-(CH₂)₁₇- | 8 | 7 | Chlorid |
| 1368 | H₃C-(CH₂)₁₇- | 9 | 7 | Chlorid |
| 1369 | H₃C-(CH₂)₁₇- | 10 | 7 | Chlorid |
| 1370 | H₃C-(CH₂)₁₇- | 8 | 8 | Chlorid |
| 1371 | H₃C-(CH₂)₁₇- | 9 | 8 | Chlorid |
| 1372 | H₃C-(CH₂)₁₇- | 10 | 8 | Chlorid |
| 1373 | H₃C-(CH₂)₁₇- | 9 | 9 | Chlorid |
| 1374 | H₃C-(CH₂)₁₇- | 10 | 9 | Chlorid |
| 1375 | H₃C-(CH₂)₁₇- | 10 | 10 | Chlorid |
| 1376 | H₃C-(CH₂)₁₇- | 1 | 1 | Bromid |
| 1377 | H₃C-(CH₂)₁₇- | 2 | 1 | Bromid |
| 1378 | H₃C-(CH₂)₁₇- | 3 | 1 | Bromid |
| 1379 | H₃C-(CH₂)₁₇- | 4 | 1 | Bromid |
| 1380 | H₃C-(CH₂)₁₇- | 5 | 1 | Bromid |
| 1381 | H₃C-(CH₂)₁₇- | 6 | 1 | Bromid |
| 1382 | H₃C-(CH₂)₁₇- | 7 | 1 | Bromid |
| 1383 | H₃C-(CH₂)₁₇- | 8 | 1 | Bromid |
| 1384 | H₃C-(CH₂)₁₇- | 9 | 1 | Bromid |
| 1385 | H₃C-(CH₂)₁₇- | 10 | 1 | Bromid |
| 1386 | H₃C-(CH₂)₁₇- | 2 | 2 | Bromid |
| 1387 | H₃C-(CH₂)₁₇- | 3 | 2 | Bromid |
| 1388 | H₃C-(CH₂)₁₇- | 4 | 2 | Bromid |
| 1389 | H₃C-(CH₂)₁₇- | 5 | 2 | Bromid |
| 1390 | H₃C-(CH₂)₁₇- | 6 | 2 | Bromid |
| 1391 | H₃C-(CH₂)₁₇- | 7 | 2 | Bromid |
| 1392 | H₃C-(CH₂)₁₇ | 8 | 2 | Bromid |
| 1393 | H₃C-(CH₂)₁₇- | 9 | 2 | Bromid |
| 1394 | H₃C-(CH₂)₁₇- | 10 | 2 | Bromid |
| 1395 | H₃C-(CH₂)₁₇- | 3 | 3 | Bromid |
| 1396 | H₃C-(CH₂)₁₇- | 4 | 3 | Bromid |
| 1397 | H₃C-(CH₂)₁₇- | 5 | 3 | Bromid |
| 1398 | H₃C-(CH₂)₁₇- | 6 | 3 | Bromid |
| 1399 | H₃C-(CH₂)₁₇- | 7 | 3 | Bromid |
| 1400 | H₃C-(CH₂)₁₇- | 8 | 3 | Bromid |
| 1401 | H₃C-(CH₂)₁₇- | 9 | 3 | Bromid |
| 1402 | H₃C-(CH₂)₁₇- | 10 | 3 | Bromid |
| 1403 | H₃C-(CH₂)₁₇- | 4 | 4 | Bromid |
| 1404 | H₃C-(CH₂)₁₇- | 5 | 4 | Bromid |
| 1405 | H₃C-(CH₂)₁₇- | 6 | 4 | Bromid |
| 1406 | H₃C-(CH₂)₁₇- | 7 | 4 | Bromid |
| 1407 | H₃C-(CH₂)₁₇- | 8 | 4 | Bromid |
| 1408 | H₃C-(CH₂)₁₇- | 9 | 4 | Bromid |
| 1409 | H₃C-(CH₂)₁₇- | 10 | 4 | Bromid |
| 1410 | H₃C-(CH₂)₁₇- | 5 | 5 | Bromid |
| 1411 | H₃C-(CH₂)₁₇- | 6 | 5 | Bromid |
| 1412 | H₃C-(CH₂)₁₇- | 7 | 5 | Bromid |
| 1413 | H₃C-(CH₂)₁₇- | 8 | 5 | Bromid |
| 1414 | H₃C-(CH₂)₁₇- | 9 | 5 | Bromid |
| 1415 | H₃C-(CH₂)₁₇- | 10 | 5 | Bromid |
| 1416 | H₃C-(CH₂)₁₇- | 6 | 6 | Bromid |
| 1417 | H₃C-(CH₂)₁₇- | 7 | 6 | Bromid |
| 1418 | H₃C-(CH₂)₁₇- | 8 | 6 | Bromid |
| 1419 | H₃C-(CH₂)₁₇- | 9 | 6 | Bromid |
| 1420 | H₃C-(CH₂)₁₇- | 10 | 6 | Bromid |
| 1421 | H₃C-(CH₂)₁₇- | 7 | 7 | Bromid |
| 1422 | H₃C-(CH₂)₁₇- | 8 | 7 | Bromid |
| 1423 | H₃C-(CH₂)₁₇- | 9 | 7 | Bromid |
| 1424 | H₃C-(CH₂)₁₇- | 10 | 7 | Bromid |
| 1425 | H₃C-(CH₂)₁₇- | 8 | 8 | Bromid |
| 1426 | H₃C-(CH₂)₁₇- | 9 | 8 | Bromid |
| 1427 | H₃C-(CH₂)₁₇- | 10 | 8 | Bromid |
| 1428 | H₃C-(CH₂)₁₇- | 9 | 9 | Bromid |
| 1429 | H₃C-(CH₂)₁₇- | 10 | 9 | Bromid |
| 1430 | H₃C-(CH₂)₁₇- | 10 | 10 | Bromid |
| 1431 | H₃C-(CH₂)₁₇- | 1 | 1 | H₃C-OSO₃⁻ |
| 1432 | H₃C-(CH₂)₁₇- | 2 | 1 | H₃C-OSO₃⁻ |
| 1433 | H₃C-(CH₂)₁₇- | 3 | 1 | H₃C-OSO₃⁻ |
| 1434 | H₃C-(CH₂)₁₇- | 4 | 1 | H₃C-OSO₃⁻ |
| 1435 | H₃C-(CH₂)₁₇- | 5 | 1 | H₃C-OSO₃⁻ |
| 1436 | H₃C-(CH₂)₁₇- | 6 | 1 | H₃C-OSO₃⁻ |
| 1437 | H₃C-(CH₂)₁₇- | 7 | 1 | H₃C-OSO₃⁻ |
| 1438 | H₃C-(CH₂)₁₇- | 8 | 1 | H₃C-OSO₃⁻ |
| 1439 | H₃C-(CH₂)₁₇- | 9 | 1 | H₃C-OSO₃⁻ |
| 1440 | H₃C-(CH₂)₁₇- | 10 | 1 | H₃C-OSO₃⁻ |
| 1441 | H₃C-(CH₂)₁₇- | 2 | 2 | H₃C-OSO₃⁻ |
| 1442 | H₃C-(CH₂)₁₇- | 3 | 2 | H₃C-OSO₃⁻ |
| 1443 | H₃C-(CH₂)₁₇- | 4 | 2 | H₃C-OSO₃⁻ |
| 1444 | H₃C-(CH₂)₁₇- | 5 | 2 | H₃C-OSO₃⁻ |
| 1445 | H₃C-(CH₂)₁₇- | 6 | 2 | H₃C-OSO₃⁻ |
| 1446 | H₃C-(CH₂)₁₇- | 7 | 2 | H₃C-OSO₃⁻ |
| 1447 | H₃C-(CH₂)₁₇- | 8 | 2 | H₃C-OSO₃⁻ |
| 1448 | H₃C-(CH₂)₁₇- | 9 | 2 | H₃C-OSO₃⁻ |
| 1449 | H₃C-(CH₂)₁₇- | 10 | 2 | H₃C-OSO₃⁻ |
| 1450 | H₃C-(CH₂)₁₇- | 3 | 3 | H₃C-OSO₃⁻ |
| 1451 | H₃C-(CH₂)₁₇- | 4 | 3 | H₃C-OSO₃⁻ |
| 1452 | H₃C-(CH₂)₁₇- | 5 | 3 | H₃C-OSO₃⁻ |
| 1453 | H₃C-(CH₂)₁₇- | 6 | 3 | H₃C-OSO₃⁻ |
| 1454 | H₃C-(CH₂)₁₇- | 7 | 3 | H₃C-OSO₃⁻ |
| 1455 | H₃C-(CH₂)₁₇- | 8 | 3 | H₃C-OSO₃⁻ |
| 1456 | H₃C-(CH₂)₁₇- | 9 | 3 | H₃C-OSO₃⁻ |
| 1457 | H₃C-(CH₂)₁₇- | 10 | 3 | H₃C-OSO₃⁻ |
| 1458 | H₃C-(CH₂)₁₇- | 4 | 4 | H₃C-OSO₃⁻ |
| 1459 | H₃C-(CH₂)₁₇- | 5 | 4 | H₃C-OSO₃⁻ |
| 1460 | H₃C-(CH₂)₁₇- | 6 | 4 | H₃C-OSO₃⁻ |
| 1461 | H₃C-(CH₂)₁₇- | 7 | 4 | H₃C-OSO₃⁻ |
| 1462 | H₃C-(CH₂)₁₇- | 8 | 4 | H₃C-OSO₃⁻ |
| 1463 | H₃C-(CH₂)₁₇- | 9 | 4 | H₃C-OSO₃⁻ |
| 1464 | H₃C-(CH₂)₁₇- | 10 | 4 | H₃C-OSO₃⁻ |
| 1465 | H₃C-(CH₂)₁₇- | 5 | 5 | H₃C-OSO₃⁻ |
| 1466 | H₃C-(CH₂)₁₇- | 6 | 5 | H₃C-OSO₃⁻ |
| 1467 | H₃C-(CH₂)₁₇- | 7 | 5 | H₃C-OSO₃⁻ |
| 1468 | H₃C-(CH₂)₁₇- | 8 | 5 | H₃C-OSO₃⁻ |
| 1469 | H₃C-(CH₂)₁₇- | 9 | 5 | H₃C-OSO₃⁻ |
| 1470 | H₃C-(CH₂)₁₇- | 10 | 5 | H₃C-OSO₃⁻ |
| 1471 | H₃C-(CH₂)₁₇- | 6 | 6 | H₃C-OSO₃⁻ |
| 1472 | H₃C-(CH₂)₁₇- | 7 | 6 | H₃C-OSO₃⁻ |
| 1473 | H₃C-(CH₂)₁₇- | 8 | 6 | H₃C-OSO₃⁻ |
| 1474 | H₃C-(CH₂)₁₇- | 9 | 6 | H₃C-OSO₃⁻ |
| 1475 | H₃C-(CH₂)₁₇- | 10 | 6 | H₃C-OSO₃⁻ |
| 1476 | H₃C-(CH₂)₁₇- | 7 | 7 | H₃C-OSO₃⁻ |
| 1477 | H₃C-(CH₂)₁₇- | 8 | 7 | H₃C-OSO₃⁻ |
| 1478 | H₃C-(CH₂)₁₇- | 9 | 7 | H₃C-OSO₃⁻ |
| 1479 | H₃C-(CH₂)₁₇- | 10 | 7 | H₃C-OSO₃⁻ |
| 1480 | H₃C-(CH₂)₁₇- | 8 | 8 | H₃C-OSO₃⁻ |
| 1481 | H₃C-(CH₂)₁₇- | 9 | 8 | H₃C-OSO₃⁻ |
| 1482 | H₃C-(CH₂)₁₇- | 10 | 8 | H₃C-OSO₃⁻ |
| 1483 | H₃C-(CH₂)₁₇- | 9 | 9 | H₃C-OSO₃⁻ |
| 1484 | H₃C-(CH₂)₁₇- | 10 | 9 | H₃C-OSO₃⁻ |
| 1485 | H₃C-(CH₂)₁₇- | 10 | 10 | H₃C-OSO₃⁻ |
| 1486 | H₃C-(CH₂)₁₇- | 1 | 1 | H₃C-CH₂-OSO₃⁻ |
| 1487 | H₃C-(CH₂)₁₇- | 2 | 1 | H₃C-CH₂-OSO₃⁻ |
| 1488 | H₃C-(CH₂)₁₇- | 3 | 1 | H₃C-CH₂-OSO₃⁻ |
| 1489 | H₃C-(CH₂)₁₇- | 4 | 1 | H₃C-CH₂-OSO₃⁻ |
| 1490 | H₃C-(CH₂)₁₇- | 5 | 1 | H₃C-CH₂-OSO₃⁻ |
| 1491 | H₃C-(CH₂)₁₇- | 6 | 1 | H₃C-CH₂-OSO₃⁻ |
| 1492 | H₃C-(CH₂)₁₇- | 7 | 1 | H₃C-CH₂-OSO₃⁻ |
| 1493 | H₃C-(CH₂)₁₇- | 8 | 1 | H₃C-CH₂-OSO₃⁻ |
| 1494 | H₃C-(CH₂)₁₇- | 9 | 1 | H₃C-CH₂-OSO₃⁻ |
| 1495 | H₃C-(CH₂)₁₇- | 10 | 1 | H₃C-CH₂-OSO₃⁻ |
| 1496 | H₃C-(CH₂)₁₇- | 2 | 2 | H₃C-CH₂-OSO₃⁻ |
| 1497 | H₃C-(CH₂)₁₇- | 3 | 2 | H₃C-CH₂-OSO₃⁻ |
| 1498 | H₃C-(CH₂)₁₇- | 4 | 2 | H₃C-CH₂-OSO₃⁻ |
| 1499 | H₃C-(CH₂)₁₇- | 5 | 2 | H₃C-CH₂-OSO₃⁻ |
| 1500 | H₃C-(CH₂)₁₇- | 6 | 2 | H₃C-CH₂-OSO₃⁻ |
| 1501 | H₃C-(CH₂)₁₇- | 7 | 2 | H₃C-CH₂-OSO₃⁻ |
| 1502 | H₃C-(CH₂)₁₇- | 8 | 2 | H₃C-CH₂-OSO₃⁻ |
| 1503 | H₃C-(CH₂)₁₇- | 9 | 2 | H₃C-CH₂-OSO₃⁻ |
| 1504 | H₃C-(CH₂)₁₇- | 10 | 2 | H₃C-CH₂-OSO₃⁻ |
| 1505 | H₃C-(CH₂)₁₇- | 3 | 3 | H₃C-CH₂-OSO₃⁻ |
| 1506 | H₃C-(CH₂)₁₇- | 4 | 3 | H₃C-CH₂-OSO₃⁻ |
| 1507 | H₃C-(CH₂)₁₇- | 5 | 3 | H₃C-CH₂-OSO₃⁻ |
| 1508 | H₃C-(CH₂)₁₇- | 6 | 3 | H₃C-CH₂-OSO₃⁻ |
| 1509 | H₃C-(CH₂)₁₇- | 7 | 3 | H₃C-CH₂-OSO₃⁻ |
| 1510 | H₃C-(CH₂)₁₇- | 8 | 3 | H₃C-CH₂-OSO₃⁻ |
| 1511 | H₃C-(CH₂)₁₇- | 9 | 3 | H₃C-CH₂-OSO₃⁻ |
| 1512 | H₃C-(CH₂)₁₇- | 10 | 3 | H₃C-CH₂-OSO₃⁻ |
| 1513 | H₃C-(CH₂)₁₇- | 4 | 4 | H₃C-CH₂-OSO₃⁻ |
| 1514 | H₃C-(CH₂)₁₇- | 5 | 4 | H₃C-CH₂-OSO₃⁻ |
| 1515 | H₃C-(CH₂)₁₇- | 6 | 4 | H₃C-CH₂-OSO₃⁻ |
| 1516 | H₃C-(CH₂)₁₇- | 7 | 4 | H₃C-CH₂-OSO₃⁻ |
| 1517 | H₃C-(CH₂)₁₇- | 8 | 4 | H₃C-CH₂-OSO₃⁻ |
| 1518 | H₃C-(CH₂)₁₇- | 9 | 4 | H₃C-CH₂-OSO₃⁻ |
| 1519 | H₃C-(CH₂)₁₇- | 10 | 4 | H₃C-CH₂-OSO₃⁻ |
| 1520 | H₃C-(CH₂)₁₇- | 5 | 5 | H₃C-CH₂-OSO₃⁻ |
| 1521 | H₃C-(CH₂)₁₇- | 6 | 5 | H₃C-CH₂-OSO₃⁻ |
| 1522 | H₃C-(CH₂)₁₇- | 7 | 5 | H₃C-CH₂-OSO₃⁻ |
| 1523 | H₃C-(CH₂)₁₇- | 8 | 5 | H₃C-CH₂-OSO₃⁻ |
| 1524 | H₃C-(CH₂)₁₇- | 9 | 5 | H₃C-CH₂-OSO₃⁻ |
| 1525 | H₃C-(CH₂)₁₇- | 10 | 5 | H₃C-CH₂-OSO₃⁻ |
| 1526 | H₃C-(CH₂)₁₇- | 6 | 6 | H₃C-CH₂-OSO₃⁻ |
| 1527 | H₃C-(CH₂)₁₇- | 7 | 6 | H₃C-CH₂-OSO₃⁻ |
| 1528 | H₃C-(CH₂)₁₇- | 8 | 6 | H₃C-CH₂-OSO₃⁻ |
| 1529 | H₃C-(CH₂)₁₇- | 9 | 6 | H₃C-CH₂-OSO₃⁻ |
| 1530 | H₃C-(CH₂)₁₇- | 10 | 6 | H₃C-CH₂-OSO₃⁻ |
| 1531 | H₃C-(CH₂)₁₇- | 7 | 7 | H₃C-CH₂-OSO₃⁻ |
| 1532 | H₃C-(CH₂)₁₇- | 8 | 7 | H₃C-CH₂-OSO₃⁻ |
| 1533 | H₃C-(CH₂)₁₇- | 9 | 7 | H₃C-CH₂-OSO₃⁻ |
| 1534 | H₃C-(CH₂)₁₇- | 10 | 7 | H₃C-CH₂-OSO₃⁻ |
| 1535 | H₃C-(CH₂)₁₇- | 8 | 8 | H₃C-CH₂-OSO₃⁻ |
| 1536 | H₃C-(CH₂)₁₇- | 9 | 8 | H₃C-CH₂-OSO₃⁻ |
| 1537 | H₃C-(CH₂)₁₇- | 10 | 8 | H₃C-CH₂-OSO₃⁻ |
| 1538 | H₃C-(CH₂)₁₇- | 9 | 9 | H₃C-CH₂-OSO₃⁻ |
| 1539 | H₃C-(CH₂)₁₇- | 10 | 9 | H₃C-CH₂-OSO₃⁻ |
| 1540 | H₃C-(CH₂)₁₇- | 10 | 10 | H₃C-CH₂-OSO₃⁻ |
| 1541 | H₃C-(CH₂)₄-CH=CH-(CH₂)₈- | 1 | 1 | Chlorid |
| 1542 | H₃C-(CH₂)₄-CH=CH-(CH₂)₈- | 2 | 1 | Chlorid |
| 1543 | H₃C-(CH₂)₄-CH=CH-(CH₂)₈- | 3 | 1 | Chlorid |
| 1544 | H₃C-(CH₂)₄-CH=CH-(CH₂)₈- | 4 | 1 | Chlorid |
| 1545 | H₃C-(CH₂)₄-CH=CH-(CH₂)₈- | 5 | 1 | Chlorid |
| 1546 | H₃C-(CH₂)₄-CH=CH-(CH₂)₈- | 6 | 1 | Chlorid |
| 1547 | H₃C-(CH₂)₄-CH=CH-(CH₂)₈- | 7 | 1 | Chlorid |
| 1548 | H₃C-(CH₂)₄-CH=CH-(CH₂)₈- | 8 | 1 | Chlorid |
| 1549 | H₃C-(CH₂)₄-CH=CH-(CH₂)₈- | 9 | 1 | Chlorid |
| 1550 | H₃C-(CH₂)₄-CH=CH-(CH₂)₈- | 10 | 1 | Chlorid |
| 1551 | H₃C-(CH₂)₄-CH=CH-(CH₂)₈- | 2 | 2 | Chlorid |
| 1552 | H₃C-(CH₂)₄-CH=CH-(CH₂)₈- | 3 | 2 | Chlorid |
| 1553 | H₃C-(CH₂)₄-CH=CH-(CH₂)₈- | 4 | 2 | Chlorid |
| 1554 | H₃C-(CH₂)₄-CH=CH-(CH₂)₈- | 5 | 2 | Chlorid |
| 1555 | H₃C-(CH₂)₄-CH=CH-(CH₂)₈- | 6 | 2 | Chlorid |
| 1556 | H₃C-(CH₂)₄-CH=CH-(CH₂)₈- | 7 | 2 | Chlorid |
| 1557 | H₃C-(CH₂)₄-CH=CH-(CH₂)₈- | 8 | 2 | Chlorid |
| 1558 | H₃C-(CH₂)₄-CH=CH-(CH₂)₈- | 9 | 2 | Chlorid |
| 1559 | H₃C-(CH₂)₄-CH=CH-(CH₂)₈- | 10 | 2 | Chlorid |
| 1560 | H₃C-(CH₂)₄-CH=CH-(CH₂)₈- | 3 | 3 | Chlorid |
| 1561 | H₃C-(CH₂)₄-CH=CH-(CH₂)₈- | 4 | 3 | Chlorid |
| 1562 | H₃C-(CH₂)₄-CH=CH-(CH₂)₈- | 5 | 3 | Chlorid |
| 1563 | H₃C-(CH₂)₄-CH=CH-(CH₂)₈- | 6 | 3 | Chlorid |
| 1564 | H₃C-(CH₂)₄-CH=CH-(CH₂)₈- | 7 | 3 | Chlorid |
| 1565 | H₃C-(CH₂)₄-CH=CH-(CH₂)₈- | 8 | 3 | Chlorid |
| 1566 | H₃C-(CH₂)₄-CH=CH-(CH₂)₈- | 9 | 3 | Chlorid |
| 1567 | H₃C-(CH₂)₄-CH=CH-(CH₂)₈- | 10 | 3 | Chlorid |
| 1568 | H₃C-(CH₂)₄-CH=CH-(CH₂)₈- | 4 | 4 | Chlorid |
| 1569 | H₃C-(CH₂)₄-CH=CH-(CH₂)₈- | 5 | 4 | Chlorid |
| 1570 | H₃C-(CH₂)₄-CH=CH-(CH₂)₈- | 6 | 4 | Chlorid |
| 1571 | H₃C-(CH₂)₄-CH=CH-(CH₂)₈- | 7 | 4 | Chlorid |
| 1572 | H₃C-(CH₂)₄-CH=CH-(CH₂)₈- | 8 | 4 | Chlorid |
| 1573 | H₃C-(CH₂)₄-CH=CH-(CH₂)₈- | 9 | 4 | Chlorid |
| 1574 | H₃C-(CH₂)₄-CH=CH-(CH₂)₈- | 10 | 4 | Chlorid |
| 1575 | H₃C-(CH₂)₄-CH=CH-(CH₂)₈- | 5 | 5 | Chlorid |
| 1576 | H₃C-(CH₂)₄-CH=CH-(CH₂)₈- | 6 | 5 | Chlorid |
| 1577 | H₃C-(CH₂)₄-CH=CH-(CH₂)₈- | 7 | 5 | Chlorid |
| 1578 | H₃C-(CH₂)₄-CH=CH-(CH₂)₈- | 8 | 5 | Chlorid |
| 1579 | H₃C-(CH₂)₄-CH=CH-(CH₂)₈- | 9 | 5 | Chlorid |
| 1580 | H₃C-(CH₂)₄-CH=CH-(CH₂)₈- | 10 | 5 | Chlorid |
| 1581 | H₃C-(CH₂)₄-CH=CH-(CH₂)₈- | 6 | 6 | Chlorid |
| 1582 | H₃C-(CH₂)₄-CH=CH-(CH₂)₈- | 7 | 6 | Chlorid |
| 1583 | H₃C-(CH₂)₄-CH=CH-(CH₂)₈- | 8 | 6 | Chlorid |
| 1584 | H₃C-(CH₂)₄-CH=CH-(CH₂)₈- | 9 | 6 | Chlorid |
| 1585 | H₃C-(CH₂)₄-CH=CH-(CH₂)₈- | 10 | 6 | Chlorid |
| 1586 | H₃C-(CH₂)₄-CH=CH-(CH₂)₈- | 7 | 7 | Chlorid |
| 1587 | H₃C-(CH₂)₄-CH=CH-(CH₂)₈- | 8 | 7 | Chlorid |
| 1588 | H₃C-(CH₂)₄-CH=CH-(CH₂)₈- | 9 | 7 | Chlorid |
| 1589 | H₃C-(CH₂)₄-CH=CH-(CH₂)₈- | 10 | 7 | Chlorid |
| 1590 | H₃C-(CH₂)₄-CH=CH-(CH₂)₈- | 8 | 8 | Chlorid |
| 1591 | H₃C-(CH₂)₄-CH=CH-(CH₂)₈- | 9 | 8 | Chlorid |
| 1592 | H₃C-(CH₂)₄-CH=CH-(CH₂)₈- | 10 | 8 | Chlorid |
| 1593 | H₃C-(CH₂)₄-CH=CH-(CH₂)₈- | 9 | 9 | Chlorid |
| 1594 | H₃C-(CH₂)₄-CH=CH-(CH₂)₈- | 10 | 9 | Chlorid |
| 1595 | H₃C-(CH₂)₄-CH=CH-(CH₂)₈- | 10 | 10 | Chlorid |
| 1596 | H₃C-(CH₂)₄-CH=CH-(CH₂)₈- | 1 | 1 | Bromid |
| 1597 | H₃C-(CH₂)₄-CH=CH-(CH₂)₈- | 2 | 1 | Bromid |
| 1598 | H₃C-(CH₂)₄-CH=CH-(CH₂)₈- | 3 | 1 | Bromid |
| 1599 | H₃C-(CH₂)₄-CH=CH-(CH₂)₈- | 4 | 1 | Bromid |
| 1600 | H₃C-(CH₂)₄-CH=CH-(CH₂)₈- | 5 | 1 | Bromid |
| 1601 | H₃C-(CH₂)₄-CH=CH-(CH₂)₈- | 6 | 1 | Bromid |
| 1602 | H₃C-(CH₂)₄-CH=CH-(CH₂)₈- | 7 | 1 | Bromid |
| 1603 | H₃C-(CH₂)₄-CH=CH-(CH₂)₈- | 8 | 1 | Bromid |
| 1604 | H₃C-(CH₂)₄-CH=CH-(CH₂)₈- | 9 | 1 | Bromid |
| 1605 | H₃C-(CH₂)₄-CH=CH-(CH₂)₈- | 10 | 1 | Bromid |
| 1606 | H₃C-(CH₂)₄-CH=CH-(CH₂)₈- | 2 | 2 | Bromid |
| 1607 | H₃C-(CH₂)₄-CH=CH-(CH₂)₈- | 3 | 2 | Bromid |
| 1608 | H₃C-(CH₂)₄-CH=CH-(CH₂)₈- | 4 | 2 | Bromid |
| 1609 | H₃C-(CH₂)₄-CH=CH-(CH₂)₈- | 5 | 2 | Bromid |
| 1610 | H₃C-(CH₂)₄-CH=CH-(CH₂)₈- | 6 | 2 | Bromid |
| 1611 | H₃C-(CH₂)₄-CH=CH-(CH₂)₈- | 7 | 2 | Bromid |
| 1612 | H₃C-(CH₂)₄-CH=CH-(CH₂)₈- | 8 | 2 | Bromid |
| 1613 | H₃C-(CH₂)₄-CH=CH-(CH₂)₈- | 9 | 2 | Bromid |
| 1614 | H₃C-(CH₂)₄-CH=CH-(CH₂)₈- | 10 | 2 | Bromid |
| 1615 | H₃C-(CH₂)₄-CH=CH-(CH₂)₈- | 3 | 3 | Bromid |
| 1616 | H₃C-(CH₂)₄-CH=CH-(CH₂)₈- | 4 | 3 | Bromid |
| 1617 | H₃C-(CH₂)₄-CH=CH-(CH₂)₈- | 5 | 3 | Bromid |
| 1618 | H₃C-(CH₂)₄-CH=CH-(CH₂)₈- | 6 | 3 | Bromid |
| 1619 | H₃C-(CH₂)₄-CH=CH-(CH₂)₈- | 7 | 3 | Bromid |
| 1620 | H₃C-(CH₂)₄-CH=CH-(CH₂)₈- | 8 | 3 | Bromid |
| 1621 | H₃C-(CH₂)₄-CH=CH-(CH₂)₈- | 9 | 3 | Bromid |
| 1622 | H₃C-(CH₂)₄-CH=CH-(CH₂)₈- | 10 | 3 | Bromid |
| 1623 | H₃C-(CH₂)₄-CH=CH-(CH₂)₈- | 4 | 4 | Bromid |
| 1624 | H₃C-(CH₂)₄-CH=CH-(CH₂)₈- | 5 | 4 | Bromid |
| 1625 | H₃C-(CH₂)₄-CH=CH-(CH₂)₈- | 6 | 4 | Bromid |
| 1626 | H₃C-(CH₂)₄-CH=CH-(CH₂)₈- | 7 | 4 | Bromid |
| 1627 | H₃C-(CH₂)₄-CH=CH-(CH₂)₈- | 8 | 4 | Bromid |
| 1628 | H₃C-(CH₂)₄-CH=CH-(CH₂)₈- | 9 | 4 | Bromid |
| 1629 | H₃C-(CH₂)₄-CH=CH-(CH₂)₈- | 10 | 4 | Bromid |
| 1630 | H₃C-(CH₂)₄-CH=CH-(CH₂)₈- | 5 | 5 | Bromid |
| 1631 | H₃C-(CH₂)₄-CH=CH-(CH₂)₈- | 6 | 5 | Bromid |
| 1632 | H₃C-(CH₂)₄-CH=CH-(CH₂)₈- | 7 | 5 | Bromid |
| 1633 | H₃C-(CH₂)₄-CH=CH-(CH₂)₈- | 8 | 5 | Bromid |
| 1634 | H₃C-(CH₂)₄-CH=CH-(CH₂)₈- | 9 | 5 | Bromid |
| 1635 | H₃C-(CH₂)₄-CH=CH-(CH₂)₈- | 10 | 5 | Bromid |
| 1636 | H₃C-(CH₂)₄-CH=CH-(CH₂)₈- | 6 | 6 | Bromid |
| 1637 | H₃C-(CH₂)₄-CH=CH-(CH₂)₈- | 7 | 6 | Bromid |
| 1638 | H₃C-(CH₂)₄-CH=CH-(CH₂)₈- | 8 | 6 | Bromid |
| 1639 | H₃C-(CH₂)₄-CH=CH-(CH₂)₈- | 9 | 6 | Bromid |
| 1640 | H₃C-(CH₂)₄-CH=CH-(CH₂)₈- | 10 | 6 | Bromid |
| 1641 | H₃C-(CH₂)₄-CH=CH-(CH₂)₈- | 7 | 7 | Bromid |
| 1642 | H₃C-(CH₂)₄-CH=CH-(CH₂)₈- | 8 | 7 | Bromid |
| 1643 | H₃C-(CH₂)₄-CH=CH-(CH₂)₈- | 9 | 7 | Bromid |
| 1644 | H₃C-(CH₂)₄-CH=CH-(CH₂)₈- | 10 | 7 | Bromid |
| 1645 | H₃C-(CH₂)₄-CH=CH-(CH₂)₈- | 8 | 8 | Bromid |
| 1646 | H₃C-(CH₂)₄-CH=CH-(CH₂)₈- | 9 | 8 | Bromid |
| 1647 | H₃C-(CH₂)₄-CH=CH-(CH₂)₈- | 10 | 8 | Bromid |
| 1648 | H₃C-(CH₂)₄-CH=CH-(CH₂)₈- | 9 | 9 | Bromid |
| 1649 | H₃C-(CH₂)₄-CH=CH-(CH₂)₈- | 10 | 9 | Bromid |
| 1650 | H₃C-(CH₂)₄-CH=CH-(CH₂)₈- | 10 | 10 | Bromid |
| 1651 | H₃C-(CH₂)₄-CH=CH-(CH₂)₈- | 1 | 1 | H₃C-OSO₃⁻ |
| 1652 | H₃C-(CH₂)₄-CH=CH-(CH₂)₈- | 2 | 1 | H₃C-OSO₃⁻ |
| 1653 | H₃C-(CH₂)₄-CH=CH-(CH₂)₈- | 3 | 1 | H₃C-OSO₃⁻ |
| 1654 | H₃C-(CH₂)₄-CH=CH-(CH₂)₈- | 4 | 1 | H₃C-OSO₃⁻ |
| 1655 | H₃C-(CH₂)₄-CH=CH-(CH₂)₈- | 5 | 1 | H₃C-OSO₃⁻ |
| 1656 | H₃C-(CH₂)₄-CH=CH-(CH₂)₈- | 6 | 1 | H₃C-OSO₃⁻ |
| 1657 | H₃C-(CH₂)₄-CH=CH-(CH₂)₈- | 7 | 1 | H₃C-OSO₃⁻ |
| 1658 | H₃C-(CH₂)₄-CH=CH-(CH₂)₈- | 8 | 1 | H₃C-OSO₃⁻ |
| 1659 | H₃C-(CH₂)₄-CH=CH-(CH₂)₈- | 9 | 1 | H₃C-OSO₃⁻ |
| 1660 | H₃C-(CH₂)₄-CH=CH-(CH₂)₈- | 10 | 1 | H₃C-OSO₃⁻ |
| 1661 | H₃C-(CH₂)₄-CH=CH-(CH₂)₈- | 2 | 2 | H₃C-OSO₃⁻ |
| 1662 | H₃C-(CH₂)₄-CH=CH-(CH₂)₈- | 3 | 2 | H₃C-OSO₃⁻ |
| 1663 | H₃C-(CH₂)₄-CH=CH-(CH₂)₈- | 4 | 2 | H₃C-OSO₃⁻ |
| 1664 | H₃C-(CH₂)₄-CH=CH-(CH₂)₈- | 5 | 2 | H₃C-OSO₃⁻ |
| 1665 | H₃C-(CH₂)₄-CH=CH-(CH₂)₈- | 6 | 2 | H₃C-OSO₃⁻ |
| 1666 | H₃C-(CH₂)₄-CH=CH-(CH₂)₈- | 7 | 2 | H₃C-OSO₃⁻ |
| 1667 | H₃C-(CH₂)₄-CH=CH-(CH₂)₈- | 8 | 2 | H₃C-OSO₃⁻ |
| 1668 | H₃C-(CH₂)₄-CH=CH-(CH₂)₈- | 9 | 2 | H₃C-OSO₃⁻ |
| 1669 | H₃C-(CH₂)₄-CH=CH-(CH₂)₈- | 10 | 2 | H₃C-OSO₃⁻ |
| 1670 | H₃C-(CH₂)₄-CH=CH-(CH₂)₈- | 3 | 3 | H₃C-OSO₃⁻ |
| 1671 | H₃C-(CH₂)₄-CH=CH-(CH₂)₈- | 4 | 3 | H₃C-OSO₃⁻ |
| 1672 | H₃C-(CH₂)₄-CH=CH-(CH₂)₈- | 5 | 3 | H₃C-OSO₃⁻ |
| 1673 | H₃C-(CH₂)₄-CH=CH-(CH₂)₈- | 6 | 3 | H₃C-OSO₃⁻ |
| 1674 | H₃C-(CH₂)₄-CH=CH-(CH₂)₈- | 7 | 3 | H₃C-OSO₃⁻ |
| 1675 | H₃C-(CH₂)₄-CH=CH-(CH₂)₈- | 8 | 3 | H₃C-OSO₃⁻ |
| 1676 | H₃C-(CH₂)₄-CH=CH-(CH₂)₈- | 9 | 3 | H₃C-OSO₃⁻ |
| 1677 | H₃C-(CH₂)₄-CH=CH-(CH₂)₈- | 10 | 3 | H₃C-OSO₃⁻ |
| 1678 | H₃C-(CH₂)₄-CH=CH-(CH₂)₈- | 4 | 4 | H₃C-OSO₃⁻ |
| 1679 | H₃C-(CH₂)₄-CH=CH-(CH₂)₈- | 5 | 4 | H₃C-OSO₃⁻ |
| 1680 | H₃C-(CH₂)₄-CH=CH-(CH₂)₈- | 6 | 4 | H₃C-OSO₃⁻ |
| 1681 | H₃C-(CH₂)₄-CH=CH-(CH₂)₈- | 7 | 4 | H₃C-OSO₃⁻ |
| 1682 | H₃C-(CH₂)₄-CH=CH-(CH₂)₈- | 8 | 4 | H₃C-OSO₃⁻ |
| 1683 | H₃C-(CH₂)₄-CH=CH-(CH₂)₈- | 9 | 4 | H₃C-OSO₃⁻ |
| 1684 | H₃C-(CH₂)₄-CH=CH-(CH₂)₈- | 10 | 4 | H₃C-OSO₃⁻ |
| 1685 | H₃C-(CH₂)₄-CH=CH-(CH₂)₈- | 5 | 5 | H₃C-OSO₃⁻ |
| 1686 | H₃C-(CH₂)₄-CH=CH-(CH₂)₈- | 6 | 5 | H₃C-OSO₃⁻ |
| 1687 | H₃C-(CH₂)₄-CH=CH-(CH₂)₈- | 7 | 5 | H₃C-OSO₃⁻ |
| 1688 | H₃C-(CH₂)₄-CH=CH-(CH₂)₈- | 8 | 5 | H₃C-OSO₃⁻ |
| 1689 | H₃C-(CH₂)₄-CH=CH-(CH₂)₈- | 9 | 5 | H₃C-OSO₃⁻ |
| 1690 | H₃C-(CH₂)₄-CH=CH-(CH₂)₈- | 10 | 5 | H₃C-OSO₃⁻ |
| 1691 | H₃C-(CH₂)₄-CH=CH-(CH₂)₈- | 6 | 6 | H₃C-OSO₃⁻ |
| 1692 | H₃C-(CH₂)₄-CH=CH-(CH₂)₈- | 7 | 6 | H₃C-OSO₃⁻ |
| 1693 | H₃C-(CH₂)₄-CH=CH-(CH₂)₈- | 8 | 6 | H₃C-OSO₃⁻ |
| 1694 | H₃C-(CH₂)₄-CH=CH-(CH₂)₈- | 9 | 6 | H₃C-OSO₃⁻ |
| 1695 | H₃C-(CH₂)₄-CH=CH-(CH₂)₈- | 10 | 6 | H₃C-OSO₃⁻ |
| 1696 | H₃C-(CH₂)₄-CH=CH-(CH₂)₈- | 7 | 7 | H₃C-OSO₃⁻ |
| 1697 | H₃C-(CH₂)₄-CH=CH-(CH₂)₈- | 8 | 7 | H₃C-OSO₃⁻ |
| 1698 | H₃C-(CH₂)₄-CH=CH-(CH₂)₈- | 9 | 7 | H₃C-OSO₃⁻ |
| 1699 | H₃C-(CH₂)₄-CH=CH-(CH₂)₈- | 10 | 7 | H₃C-OSO₃⁻ |
| 1700 | H₃C-(CH₂)₄-CH=CH-(CH₂)₈- | 8 | 8 | H₃C-OSO₃⁻ |
| 1701 | H₃C-(CH₂)₄-CH=CH-(CH₂)₈- | 9 | 8 | H₃C-OSO₃⁻ |
| 1702 | H₃C-(CH₂)₄-CH=CH-(CH₂)₈- | 10 | 8 | H₃C-OSO₃⁻ |
| 1703 | H₃C-(CH₂)₄-CH=CH-(CH₂)₈- | 9 | 9 | H₃C-OSO₃⁻ |
| 1704 | H₃C-(CH₂)₄-CH=CH-(CH₂)₈- | 10 | 9 | H₃C-OSO₃⁻ |
| 1705 | H₃C-(CH₂)₄-CH=CH-(CH₂)₈- | 10 | 10 | H₃C-OSO₃⁻ |
| 1706 | H₃C-(CH₂)₄-CH=CH-(CH₂)₈- | 1 | 1 | H₃C-CH₂-OSO₃⁻ |
| 1707 | H₃C-(CH₂)₄-CH=CH-(CH₂)₈- | 2 | 1 | H₃C-CH₂-OSO₃⁻ |
| 1708 | H₃C-(CH₂)₄-CH=CH-(CH₂)₈- | 3 | 1 | H₃C-CH₂-OSO₃⁻ |
| 1709 | H₃C-(CH₂)₄-CH=CH-(CH₂)₈- | 4 | 1 | H₃C-CH₂-OSO₃⁻ |
| 1710 | H₃C-(CH₂)₄-CH=CH-(CH₂)₈- | 5 | 1 | H₃C-CH₂-OSO₃⁻ |
| 1711 | H₃C-(CH₂)₄-CH=CH-(CH₂)₈- | 6 | 1 | H₃C-CH₂-OSO₃⁻ |
| 1712 | H₃C-(CH₂)₄-CH=CH-(CH₂)₈- | 7 | 1 | H₃C-CH₂-OSO₃⁻ |
| 1713 | H₃C-(CH₂)₄-CH=CH-(CH₂)₈- | 8 | 1 | H₃C-CH₂-OSO₃⁻ |
| 1714 | H₃C-(CH₂)₄-CH=CH-(CH₂)₈- | 9 | 1 | H₃C-CH₂-OSO₃⁻ |
| 1715 | H₃C-(CH₂)₄-CH=CH-(CH₂)₈- | 10 | 1 | H₃C-CH₂-OSO₃⁻ |
| 1716 | H₃C-(CH₂)₄-CH=CH-(CH₂)₈- | 2 | 2 | H₃C-CH₂-OSO₃⁻ |
| 1717 | H₃C-(CH₂)₄-CH=C_{H}-(CH₂)₈- | 3 | 2 | H₃C-CH₂-OSO₃⁻ |
| 1718 | H₃C-(CH₂)₄-CH=CH-(CH₂)₈- | 4 | 2 | H₃C-CH₂-OSO₃⁻ |
| 1719 | H₃C-(CH₂)₄-CH=CH-(CH₂)₈- | 5 | 2 | H₃C-CH₂-OSO₃⁻ |
| 1720 | H₃C-(CH₂)₄-CH=CH-(CH₂)₈- | 6 | 2 | H₃C-CH₂-OSO₃⁻ |
| 1721 | H₃C-(CH₂)₄-CH=CH-(CH₂)₈- | 7 | 2 | H₃C-CH₂-OSO₃⁻ |
| 1722 | H₃C-(CH₂)₄-CH=CH-(CH₂)₈- | 8 | 2 | H₃C-CH₂-OSO₃⁻ |
| 1723 | H₃C-(CH₂)₄-CH=CH-(CH₂)₈- | 9 | 2 | H₃C-CH₂-OSO₃⁻ |
| 1724 | H₃C-(CH₂)₄-CH=CH-(CH₂)₈- | 10 | 2 | H₃C-CH₂-OSO₃⁻ |
| 1725 | H₃C-(CH₂)₄-CH=CH-(CH₂)₈- | 3 | 3 | H₃C-CH₂-OSO₃⁻ |
| 1726 | H₃C-(CH₂)₄-CH=CH-(CH₂)₈- | 4 | 3 | H₃C-CH₂-OSO₃⁻ |
| 1727 | H₃C-(CH₂)₄-CH=CH-(CH₂)₈- | 5 | 3 | H₃C-CH₂-OSO₃⁻ |
| 1728 | H₃C-(CH₂)₄-CH=CH-(CH₂)₈- | 6 | 3 | H₃C-CH₂-OSO₃⁻ |
| 1729 | H₃C-(CH₂)₄-CH=CH-(CH₂)₈- | 7 | 3 | H₃C-CH₂-OSO₃⁻ |
| 1730 | H₃C-(CH₂)₄-CH=CH-(CH₂)₈- | 8 | 3 | H₃C-CH₂-OSO₃⁻ |
| 1731 | H₃C-(CH₂)₄-CH=CH-(CH₂)₈- | 9 | 3 | H₃C-CH₂-OSO₃⁻ |
| 1732 | H₃C-(CH₂)₄-CH=CH-(CH₂)₈- | 10 | 3 | H₃C-CH₂-OSO₃⁻ |
| 1733 | H₃C-(CH₂)₄-CH=CH-(CH₂)₈- | 4 | 4 | H₃C-CH₂-OSO₃⁻ |
| 1734 | H₃C-(CH₂)₄-CH=CH-(CH₂)₈- | 5 | 4 | H₃C-CH₂-OSO₃⁻ |
| 1735 | H₃C-(CH₂)₄-CH=CH-(CH₂)₈- | 6 | 4 | H₃C-CH₂-OSO₃⁻ |
| 1736 | H₃C-(CH₂)₄-CH=CH-(CH₂)₈- | 7 | 4 | H₃C-CH₂-OSO₃⁻ |
| 1737 | H₃C-(CH₂)₄-CH=CH-(CH₂)₈- | 8 | 4 | H₃C-CH₂-OSO₃⁻ |
| 1738 | H₃C-(CH₂)₄-CH=CH-(CH₂)₈- | 9 | 4 | H₃C-CH₂-OSO₃⁻ |
| 1739 | H₃C-(CH₂)₄-CH=CH-(CH₂)₈- | 10 | 4 | H₃C-CH₂-OSO₃⁻ |
| 1740 | H₃C-(CH₂)₄-CH=CH-(CH₂)₈- | 5 | 5 | H₃C-CH₂-OSO₃⁻ |
| 1741 | H₃C-(CH₂)₄-CH=CH-(CH₂)₈- | 6 | 5 | H₃C-CH₂-OSO₃⁻ |
| 1742 | H₃C-(CH₂)₄-CH=CH-(CH₂)₈- | 7 | 5 | H₃C-CH₂-OSO₃⁻ |
| 1743 | H₃C-(CH₂)₄-CH=CH-(CH₂)₈- | 8 | 5 | H₃C-CH₂-OSO₃⁻ |
| 1744 | H₃C-(CH₂)₄-CH=CH-(CH₂)₈- | 9 | 5 | H₃C-CH₂-OSO₃⁻ |
| 1745 | H₃C-(CH₂)₄-CH=CH-(CH₂)₈- | 10 | 5 | H₃C-CH₂-OSO₃⁻ |
| 1746 | H₃C-(CH₂)₄-CH=CH-(CH₂)₈- | 6 | 6 | H₃C-CH₂-OSO₃⁻ |
| 1747 | H₃C-(CH₂)₄-CH=CH-(CH₂)₈- | 7 | 6 | H₃C-CH₂-OSO₃⁻ |
| 1748 | H₃C-(CH₂)₄-CH=CH-(CH₂)₈- | 8 | 6 | H₃C-CH₂-OSO₃⁻ |
| 1749 | H₃C-(CH₂)₄-CH=CH-(CH₂)₈- | 9 | 6 | H₃C-CH₂-OSO₃⁻ |
| 1750 | H₃C-(CH₂)₄-CH=CH-(CH₂)₈- | 10 | 6 | H₃C-CH₂-OSO₃⁻ |
| 1751 | H₃C-(CH₂)₄-CH=CH-(CH₂)₈- | 7 | 7 | H₃C-CH₂-OSO₃⁻ |
| 1752 | H₃C-(CH₂)₄-CH=CH-(CH₂)₈- | 8 | 7 | H₃C-CH₂-OSO₃⁻ |
| 1753 | H₃C-(CH₂)₄-CH=CH-(CH₂)₈- | 9 | 7 | H₃C-CH₂-OSO₃⁻ |
| 1754 | H_{3C}-(CH₂)₄-CH=CH-(CH₂)₈- | 10 | 7 | H₃C-CH₂-OSO₃⁻ |
| 1755 | H₃C-(CH₂)₄-CH=CH-(CH₂)₈- | 8 | 8 | H₃C-CH₂-OSO₃⁻ |
| 1756 | H₃C-(CH₂)₄-CH=CH-(CH₂)₈- | 9 | 8 | H₃C-CH₂-OSO₃⁻ |
| 1757 | H₃C-(CH₂)₄-CH=CH-(CH₂)₈- | 10 | 8 | H₃C-CH₂-OSO₃⁻ |
| 1758 | H₃C-(CH₂)₄-CH=CH-(CH₂)₈- | 9 | 9 | H₃C-CH₂-OSO₃⁻ |
| 1759 | H₃C-(CH₂)₄-CH=CH-(CH₂)₈- | 10 | 9 | H₃C-CH₂-OSO₃⁻ |
| 1760 | H₃C-(CH₂)₄-CH=CH-(CH₂)₈- | 10 | 10 | H₃C-CH₂-OSO₃⁻ |
| 1761 | H₃C-(CH₂)₆-CH=CH-(CH₂)₈- | 1 | 1 | Chlorid |
| 1762 | H₃C-(CH₂)₆-CH=CH-(CH₂)₈- | 2 | 1 | Chlorid |
| 1763 | H₃C-(CH₂)₆-CH=CH-(CH₂)₈- | 3 | 1 | Chlorid |
| 1764 | H₃C-(CH₂)₆-CH=CH-(CH₂)₈- | 4 | 1 | Chlorid |
| 1765 | H₃C-(CH₂)₆-CH=CH-(CH₂)₈- | 5 | 1 | Chlorid |
| 1766 | H₃C-(CH₂)₆-CH=CH-(CH₂)₈- | 6 | 1 | Chlorid |
| 1767 | H₃C-(CH₂)₆-CH=CH-(CH₂)₈- | 7 | 1 | Chlorid |
| 1768 | H₃C-(CH₂)₆-CH=CH-(CH₂)₈- | 8 | 1 | Chlorid |
| 1769 | H₃C-(CH₂)₆-CH=CH-(CH₂)₈- | 9 | 1 | Chlorid |
| 1770 | H₃C-(CH₂)₆-CH=CH-(CH₂)₈- | 10 | 1 | Chlorid |
| 1771 | H₃C-(CH₂)₆-CH=CH-(CH₂)₈- | 2 | 2 | Chlorid |
| 1772 | H₃C-(CH₂)₆-CH=CH-(CH₂)₈- | 3 | 2 | Chlorid |
| 1773 | H₃C-(CH₂)₆-CH=CH-(CH₂)₈- | 4 | 2 | Chlorid |
| 1774 | H₃C-(CH₂)₆-CH=CH-(CH₂)₈- | 5 | 2 | Chlorid |
| 1775 | H₃C-(CH₂)₆-CH=CH-(CH₂)₈- | 6 | 2 | Chlorid |
| 1776 | H₃C-(CH₂)₆-CH=CH-(CH₂)₈- | 7 | 2 | Chlorid |
| 1777 | H₃C-(CH₂)₆-CH=CH-(CH₂)₈- | 8 | 2 | Chlorid |
| 1778 | H₃C-(CH₂)₆-CH=CH-(CH₂)₈- | 9 | 2 | Chlorid |
| 1779 | H₃C-(CH₂)₆-CH=CH-(CH₂)₈- | 10 | 2 | Chlorid |
| 1780 | H₃C-(CH₂)₆-CH=CH-(CH₂)₈- | 3 | 3 | Chlorid |
| 1781 | H₃C-(CH₂)₆-CH=CH-(CH₂)₈- | 4 | 3 | Chlorid |
| 1782 | H₃C-(CH₂)₆-CH=CH-(CH₂)₈- | 5 | 3 | Chlorid |
| 1783 | H₃C-(CH₂)₆-CH=CH-(CH₂)₈- | 6 | 3 | Chlorid |
| 1784 | H₃C-(CH₂)₆-CH=CH-(CH₂)₈- | 7 | 3 | Chlorid |
| 1785 | H₃C-(CH₂)₆-CH=CH-(CH₂)₈- | 8 | 3 | Chlorid |
| 1786 | H₃C-(CH₂)₆-CH=CH-(CH₂)₈- | 9 | 3 | Chlorid |
| 1787 | H₃C-(CH₂)₆-CH=CH-(CH₂)₈- | 10 | 3 | Chlorid |
| 1788 | H₃C-(CH₂)₆-CH=CH-(CH₂)₈- | 4 | 4 | Chlorid |
| 1789 | H₃C-(CH₂)₆-CH=CH-(CH₂)₈- | 5 | 4 | Chlorid |
| 1790 | H₃C-(CH₂)₆-CH=CH-(CH₂)₈- | 6 | 4 | Chlorid |
| 1791 | H₃C-(CH₂)₆-CH=CH-(CH₂)₈- | 7 | 4 | Chlorid |
| 1792 | H₃C-(CH₂)₆-CH=CH-(CH₂)₈- | 8 | 4 | Chlorid |
| 1793 | H₃C-(CH₂)₆-CH=CH-(CH₂)₈- | 9 | 4 | Chlorid |
| 1794 | H₃C-(CH₂)₆-CH=CH-(CH₂)₈- | 10 | 4 | Chlorid |
| 1795 | H₃C-(CH₂)₆-CH=CH-(CH₂)₈- | 5 | 5 | Chlorid |
| 1796 | H₃C-(CH₂)₆-CH=CH-(CH₂)₈- | 6 | 5 | Chlorid |
| 1797 | H₃C-(CH₂)₆-CH=CH-(CH₂)₈- | 7 | 5 | Chlorid |
| 1798 | H₃C-(CH₂)₆-CH=CH-(CH₂)₈- | 8 | 5 | Chlorid |
| 1799 | H₃C-(CH₂)₆-CH=CH-(CH₂)₈- | 9 | 5 | Chlorid |
| 1800 | H₃C-(CH₂)₆-CH=CH-(CH₂)₈- | 10 | 5 | Chlorid |
| 1801 | H₃C-(CH₂)₆-CH=CH-(CH₂)₈- | 6 | 6 | Chlorid |
| 1802 | H₃C-(CH₂)₆-CH=CH-(CH₂)₈- | 7 | 6 | Chlorid |
| 1803 | H₃C-(CH₂)₆-CH=CH-(CH₂)₈- | 8 | 6 | Chlorid |
| 1804 | H₃C-(CH₂)₆-CH=CH-(CH₂)₈- | 9 | 6 | Chlorid |
| 1805 | H₃C-(CH₂)₆-CH=CH-(CH₂)₈- | 10 | 6 | Chlorid |
| 1806 | H₃C-(CH₂)₆-CH=CH-(CH₂)₈- | 7 | 7 | Chlorid |
| 1807 | H₃C-(CH₂)₆-CH=CH-(CH₂)₈- | 8 | 7 | Chlorid |
| 1808 | H₃C-(CH₂)₆-CH=CH-(CH₂)₈- | 9 | 7 | Chlorid |
| 1809 | H₃C-(CH₂)₆-CH=CH-(CH₂)₈- | 10 | 7 | Chlorid |
| 1810 | H₃C-(CH₂)₆-CH=CH-(CH₂)₈- | 8 | 8 | Chlorid |
| 1811 | H₃C-(CH₂)₆-CH=CH-(CH₂)₈- | 9 | 8 | Chlorid |
| 1812 | H₃C-(CH₂)₆-CH=CH-(CH₂)₈- | 10 | 8 | Chlorid |
| 1813 | H₃C-(CH₂)₆-CH=CH-(CH₂)₈- | 9 | 9 | Chlorid |
| 1814 | H₃C-(CH₂)₆-CH=CH-(CH₂)₈- | 10 | 9 | Chlorid |
| 1815 | H₃C-(CH₂)₆-CH=CH-(CH₂)₈- | 10 | 10 | Chlorid |
| 1816 | H₃C-(CH₂)₆-CH=CH-(CH₂)₈- | 1 | 1 | Bromid |
| 1817 | H₃C-(CH₂)₆-CH=CH-(CH₂)₈- | 2 | 1 | Bromid |
| 1818 | H₃C-(CH₂)₆-CH=CH-(CH₂)₈- | 3 | 1 | Bromid |
| 1819 | H₃C-(CH₂)₆-CH=CH-(CH₂)₈- | 4 | 1 | Bromid |
| 1820 | H₃C-(CH₂)₆-CH=CH-(CH₂)₈- | 5 | 1 | Bromid |
| 1821 | H₃C-(CH₂)₆-CH=CH-(CH₂)₈- | 6 | 1 | Bromid |
| 1822 | H₃C-(CH₂)₆-CH=CH-(CH₂)₈- | 7 | 1 | Bromid |
| 1823 | H₃C-(CH₂)₆-CH=CH-(CH₂)₈- | 8 | 1 | Bromid |
| 1824 | H₃C-(CH₂)₆-CH=CH-(CH₂)₈- | 9 | 1 | Bromid |
| 1825 | H₃C-(CH₂)₆-CH=CH-(CH₂)₈- | 10 | 1 | Bromid |
| 1826 | H₃C-(CH₂)₆-CH=CH-(CH₂)₈- | 2 | 2 | Bromid |
| 1827 | H₃C-(CH₂)₆-CH=CH-(CH₂)₈- | 3 | 2 | Bromid |
| 1828 | H₃C-(CH₂)₆-CH=CH-(CH₂)₈- | 4 | 2 | Bromid |
| 1829 | H₃C-(CH₂)₆-CH=CH-(CH₂)₈- | 5 | 2 | Bromid |
| 1830 | H₃C-(CH₂)₆-CH=CH-(CH₂)₈- | 6 | 2 | Bromid |
| 1831 | H₃C-(CH₂)₆-CH=CH-(CH₂)₈- | 7 | 2 | Bromid |
| 1832 | H₃C-(CH₂)₆-CH=CH-(CH₂)₈- | 8 | 2 | Bromid |
| 1833 | H₃C-(CH₂)₆-CH=CH-(CH₂)₈- | 9 | 2 | Bromid |
| 1834 | H₃C-(CH₂)₆-CH=CH-(CH₂)₈- | 10 | 2 | Bromid |
| 1835 | H₃C-(CH₂)₆-CH=CH-(CH₂)₈- | 3 | 3 | Bromid |
| 1836 | H₃C-(CH₂)₆-CH=CH-(CH₂)₈- | 4 | 3 | Bromid |
| 1837 | H₃C-(CH₂)₆-CH=CH-(CH₂)₈- | 5 | 3 | Bromid |
| 1838 | H₃C-(CH₂)₆-CH=CH-(CH₂)₈- | 6 | 3 | Bromid |
| 1839 | H₃C-(CH₂)₆-CH=CH-(CH₂)₈- | 7 | 3 | Bromid |
| 1840 | H₃C-(CH₂)₆-CH=CH-(CH₂)₈- | 8 | 3 | Bromid |
| 1841 | H₃C-(CH₂)₆-CH=CH-(CH₂)₈- | 9 | 3 | Bromid |
| 1842 | H₃C-(CH₂)₆-CH=CH-(CH₂)₈- | 10 | 3 | Bromid |
| 1843 | H₃C-(CH₂)₆-CH=CH-(CH₂)₈- | 4 | 4 | Bromid |
| 1844 | H₃C-(CH₂)₆-CH=CH-(CH₂)₈- | 5 | 4 | Bromid |
| 1845 | H₃C-(CH₂)₆-CH=CH-(CH₂)₈- | 6 | 4 | Bromid |
| 1846 | H₃C-(CH₂)₆-CH=CH-(CH₂)₈- | 7 | 4 | Bromid |
| 1847 | H₃C-(CH₂)₆-CH=CH-(CH₂)₈- | 8 | 4 | Bromid |
| 1848 | H₃C-(CH₂)₆-CH=CH-(CH₂)₈- | 9 | 4 | Bromid |
| 1849 | H₃C-(CH₂)₆-CH=CH-(CH₂)₈- | 10 | 4 | Bromid |
| 1850 | H₃C-(CH₂)₆-CH=CH-(CH₂)₈- | 5 | 5 | Bromid |
| 1851 | H₃C-(CH₂)₆-CH=CH-(CH₂)₈- | 6 | 5 | Bromid |
| 1852 | H₃C-(CH₂)₆-CH=CH-(CH₂)₈- | 7 | 5 | Bromid |
| 1853 | H₃C-(CH₂)₆-CH=CH-(CH₂)₈- | 8 | 5 | Bromid |
| 1854 | H₃C-(CH₂)₆-CH=CH-(CH₂)₈- | 9 | 5 | Bromid |
| 1855 | H₃C-(CH₂)₆-CH=CH-(CH₂)₈- | 10 | 5 | Bromid |
| 1856 | H₃C-(CH₂)₆-CH=CH-(CH₂)₈- | 6 | 6 | Bromid |
| 1857 | H₃C-(CH₂)₆-CH=CH-(CH₂)₈- | 7 | 6 | Bromid |
| 1858 | H₃C-(CH₂)₆-CH=CH-(CH₂)₈- | 8 | 6 | Bromid |
| 1859 | H₃C-(CH₂)₆-CH=CH-(CH₂)₈- | 9 | 6 | Bromid |
| 1860 | H₃C-(CH₂)₆-CH=CH-(CH₂)₈- | 10 | 6 | Bromid |
| 1861 | H₃C-(CH₂)₆-CH=CH-(CH₂)₈- | 7 | 7 | Bromid |
| 1862 | H₃C-(CH₂)₆-CH=CH-(CH₂)₈- | 8 | 7 | Bromid |
| 1863 | H₃C-(CH₂)₆-CH=CH-(CH₂)₈- | 9 | 7 | Bromid |
| 1864 | H₃C-(CH₂)₆-CH=CH-(CH₂)₈- | 10 | 7 | Bromid |
| 1865 | H₃C-(CH₂)₆-CH=CH-(CH₂)₈- | 8 | 8 | Bromid |
| 1866 | H₃C-(CH₂)₆-CH=CH-(CH₂)₈- | 9 | 8 | Bromid |
| 1867 | H₃C-(CH₂)₆-CH=CH-(CH₂)₈- | 10 | 8 | Bromid |
| 1868 | H₃C-(CH₂)₆-CH=CH-(CH₂)₈- | 9 | 9 | Bromid |
| 1869 | H₃C-(CH₂)₆-CH=CH-(CH₂)₈- | 10 | 9 | Bromid |
| 1870 | H₃C-(CH₂)₆-CH=CH-(CH₂)₈- | 10 | 10 | Bromid |
| 1871 | H₃C-(CH₂)₆-CH=CH-(CH₂)₈- | 1 | 1 | H₃C-OSO₃⁻ |
| 1872 | H₃C-(CH₂)₆-CH=CH-(CH₂)₈- | 2 | 1 | H₃C-OSO₃⁻ |
| 1873 | H₃C-(CH₂)₆-CH=CH-(CH₂)₈- | 3 | 1 | H₃C-OSO₃⁻ |
| 1874 | H₃C-(CH₂)₆-CH=CH-(CH₂)₈- | 4 | 1 | H₃C-OSO₃⁻ |
| 1875 | H₃C-(CH₂)₆-CH=CH-(CH₂)₈- | 5 | 1 | H₃C-OSO₃⁻ |
| 1876 | H₃C-(CH₂)₆-CH=CH-(CH₂)₈- | 6 | 1 | H₃C-OSO₃⁻ |
| 1877 | H₃C-(CH₂)₆-CH=CH-(CH₂)₈- | 7 | 1 | H₃C-OSO₃⁻ |
| 1878 | H₃C-(CH₂)₆-CH=CH-(CH₂)₈- | 8 | 1 | H₃C-OSO₃⁻ |
| 1879 | H₃C-(CH₂)₆-CH=CH-(CH₂)₈- | 9 | 1 | H₃C-OSO₃⁻ |
| 1880 | H₃C-(CH₂)₆-CH=CH-(CH₂)₈- | 10 | 1 | H₃C-OSO₃⁻ |
| 1881 | H₃C-(CH₂)₆-CH=CH-(CH₂)₈- | 2 | 2 | H₃C-OSO₃⁻ |
| 1882 | H₃C-(CH₂)₆-CH=CH-(CH₂)₈- | 3 | 2 | H₃C-OSO₃⁻ |
| 1883 | H_{3C}-(CH₂)₆-CH=CH-(CH₂)₈- | 4 | 2 | H₃C-OSO₃⁻ |
| 1884 | H₃C-(CH₂)₆-CH=CH-(CH₂)₈- | 5 | 2 | H₃C-OSO₃⁻ |
| 1885 | H₃C-(CH₂)₆-CH=CH-(CH₂)₈- | 6 | 2 | H₃C-OSO₃⁻ |
| 1886 | H₃C-(CH₂)₆-CH=CH-(CH₂)₈- | 7 | 2 | H₃C-OSO₃⁻ |
| 1887 | H₃C-(CH₂)₆-CH=CH-(CH₂)₈- | 8 | 2 | H₃C-OSO₃⁻ |
| 1888 | H₃C-(CH₂)₆-CH=CH-(CH₂)₈- | 9 | 2 | H₃C-OSO₃⁻ |
| 1889 | H₃C-(CH₂)₆-CH=CH-(CH₂)₈- | 10 | 2 | H₃C-OSO₃⁻ |
| 1890 | H₃C-(CH₂)₆-CH=CH-(CH₂)₈- | 3 | 3 | H₃C-OSO₃⁻ |
| 1891 | H₃C-(CH₂)₆-CH=CH-(CH₂)₈- | 4 | 3 | H₃C-OSO₃⁻ |
| 1892 | H₃C-(CH₂)₆-CH=CH-(CH₂)₈- | 5 | 3 | H₃C-OSO₃⁻ |
| 1893 | H₃C-(CH₂)₆-CH=CH-(CH₂)₈- | 6 | 3 | H₃C-OSO₃⁻ |
| 1894 | H₃C-(CH₂)₆-CH=CH-(CH₂)₈- | 7 | 3 | H₃C-OSO₃⁻ |
| 1895 | H₃C-(CH₂)₆-CH=CH-(CH₂)₈- | 8 | 3 | H₃C-OSO₃⁻ |
| 1896 | H₃C-(CH₂)₆-CH=CH-(CH₂)₈- | 9 | 3 | H₃C-OSO₃⁻ |
| 1897 | H₃C-(CH₂)₆-CH=CH-(CH₂)₈- | 10 | 3 | H₃C-OSO₃⁻ |
| 1898 | H₃C-(CH₂)₆-CH=CH-(CH₂)₈- | 4 | 4 | H₃C-OSO₃⁻ |
| 1899 | H₃C-(CH₂)₆-CH=CH-(CH₂)₈- | 5 | 4 | H₃C-OSO₃⁻ |
| 1900 | H₃C-(CH₂)₆-CH=CH-(CH₂)₈- | 6 | 4 | H₃C-OSO₃⁻ |
| 1901 | H₃C-(CH₂)₆-CH=CH-(CH₂)₈- | 7 | 4 | H₃C-OSO₃⁻ |
| 1902 | H₃C-(CH₂)₆-CH=CH-(CH₂)₈- | 8 | 4 | H₃C-OSO₃⁻ |
| 1903 | H₃C-(CH₂)₆-CH=CH-(CH₂)₈- | 9 | 4 | H₃C-OSO₃⁻ |
| 1904 | H₃C-(CH₂)₆-CH=CH-(CH₂)₈- | 10 | 4 | H₃C-OSO₃⁻ |
| 1905 | H₃C-(CH₂)₆-CH=CH-(CH₂)₈- | 5 | 5 | H₃C-OSO₃⁻ |
| 1906 | H₃C-(CH₂)₆-CH=CH-(CH₂)₈- | 6 | 5 | H₃C-OSO₃⁻ |
| 1907 | H₃C-(CH₂)₆-CH=CH-(CH₂)₈- | 7 | 5 | H₃C-OSO₃⁻ |
| 1908 | H₃C-(CH₂)₆-CH=CH-(CH₂)₈- | 8 | 5 | H₃C-OSO₃⁻ |
| 1909 | H₃C-(CH₂)₆-CH=CH-(CH₂)₈- | 9 | 5 | H₃C-OSO₃⁻ |
| 1910 | H₃C-(CH₂)₆-CH=CH-(CH₂)₈- | 10 | 5 | H₃C-OSO₃⁻ |
| 1911 | H₃C-(CH₂)₆-CH=CH-(CH₂)₈- | 6 | 6 | H₃C-OSO₃⁻ |
| 1912 | H₃C-(CH₂)₆-CH=CH-(CH₂)₈- | 7 | 6 | H₃C-OSO₃⁻ |
| 1913 | H₃C-(CH₂)₆-CH=CH-(CH₂)₈- | 8 | 6 | H₃C-OSO₃⁻ |
| 1914 | H₃C-(CH₂)₆-CH=CH-(CH₂)₈- | 9 | 6 | H₃C-OSO₃⁻ |
| 1915 | H₃C-(CH₂)₆-CH=CH-(CH₂)₈- | 10 | 6 | H₃C-OSO₃⁻ |
| 1916 | H₃C-(CH₂)₆-CH=CH-(CH₂)₈- | 7 | 7 | H₃C-OSO₃⁻ |
| 1917 | H₃C-(CH₂)₆-CH=CH-(CH₂)₈- | 8 | 7 | H₃C-OSO₃⁻ |
| 1918 | H₃C-(CH₂)₆-CH=CH-(CH₂)₈- | 9 | 7 | H₃C-OSO₃⁻ |
| 1919 | H₃C-(CH₂)₆-CH=CH-(CH₂)₈- | 10 | 7 | H₃C-OSO₃⁻ |
| 1920 | H₃C-(CH₂)₆-CH=CH-(CH₂)₈- | 8 | 8 | H₃C-OSO₃⁻ |
| 1921 | H₃C-(CH₂)₆-CH=CH-(CH₂)₈- | 9 | 8 | H₃C-OSO₃⁻ |
| 1922 | H₃C-(CH₂)₆-CH=CH-(CH₂)₈- | 10 | 8 | H₃C-OSO₃⁻ |
| 1923 | H₃C-(CH₂)₆-CH=CH-(CH₂)₈- | 9 | 9 | H₃C-OSO₃⁻ |
| 1924 | H₃C-(CH₂)₆-CH=CH-(CH₂)₈- | 10 | 9 | H₃C-OSO₃⁻ |
| 1925 | H₃C-(CH₂)₆-CH=CH-(CH₂)₈- | 10 | 10 | H₃C-OSO₃⁻ |
| 1926 | H₃C-(CH₂)₆-CH=CH-(CH₂)₈- | 1 | 1 | H₃C-CH₂-OSO₃⁻ |
| 1927 | H₃C-(CH₂)₆-CH=CH-(CH₂)₈- | 2 | 1 | H₃C-CH₂-OSO₃⁻ |
| 1928 | H₃C-(CH₂)₆-CH=CH-(CH₂)₈- | 3 | 1 | H₃C-CH₂-OSO₃⁻ |
| 1929 | H₃C-(CH₂)₆-CH=CH-(CH₂)₈- | 4 | 1 | H₃C-CH₂-OSO₃⁻ |
| 1930 | H₃C-(CH₂)₆-CH=CH-(CH₂)₈- | 5 | 1 | H₃C-CH₂-OSO₃⁻ |
| 1931 | H₃C-(CH₂)₆-CH=CH-(CH₂)₈- | 6 | 1 | H₃C-CH₂-OSO₃⁻ |
| 1932 | H₃C-(CH₂)₆-CH=CH-(CH₂)₈- | 7 | 1 | H₃C-CH₂-OSO₃⁻ |
| 1933 | H₃C-(CH₂)₆-CH=CH-(CH₂)₈- | 8 | 1 | H₃C-CH₂-OSO₃⁻ |
| 1934 | H₃C-(CH₂)₆-CH=CH-(CH₂)₈- | 9 | 1 | H₃C-CH₂-OSO₃⁻ |
| 1935 | H₃C-(CH₂)₆-CH=CH-(CH₂)₈- | 10 | 1 | H₃C-CH₂-OSO₃⁻ |
| 1936 | H₃C-(CH₂)₆-CH=CH-(CH₂)₈- | 2 | 2 | H₃C-CH₂-OSO₃⁻ |
| 1937 | H₃C-(CH₂)₆-CH=CH-(CH₂)₈- | 3 | 2 | H₃C-CH₂-OSO₃⁻ |
| 1938 | H₃C-(CH₂)₆-CH=CH-(CH₂)₈- | 4 | 2 | H₃C-CH₂-OSO₃⁻ |
| 1939 | H₃C-(CH₂)₆-CH=CH-(CH₂)₈- | 5 | 2 | H₃C-CH₂-OSO₃⁻ |
| 1940 | H₃C-(CH₂)₆-CH=CH-(CH₂)₈- | 6 | 2 | H₃C-CH₂-OSO₃⁻ |
| 1941 | H₃C-(CH₂)₆-CH=CH-(CH₂)₈- | 7 | 2 | H₃C-CH₂-OSO₃⁻ |
| 1942 | H₃C-(CH₂)₆-CH=CH-(CH₂)₈- | 8 | 2 | H₃C-CH₂-OSO₃⁻ |
| 1943 | H₃C-(CH₂)₆-CH=CH-(CH₂)₈- | 9 | 2 | H₃C-CH₂-OSO₃⁻ |
| 1944 | H₃C-(CH₂)₆-CH=CH-(CH₂)₈- | 10 | 2 | H₃C-CH₂-OSO₃⁻ |
| 1945 | H₃C-(CH₂)₆-CH=CH-(CH₂)₈- | 3 | 3 | H₃C-CH₂-OSO₃⁻ |
| 1946 | H₃C-(CH₂)₆-CH=CH-(CH₂)₈- | 4 | 3 | H₃C-CH₂-OSO₃⁻ |
| 1947 | H₃C-(CH₂)₆-CH=CH-(CH₂)₈- | 5 | 3 | H₃C-CH₂-OSO₃⁻ |
| 1948 | H₃C-(CH₂)₆-CH=CH-(CH₂)₈- | 6 | 3 | H₃C-CH₂-OSO₃⁻ |
| 1949 | H₃C-(CH₂)₆-CH=CH-(CH₂)₈- | 7 | 3 | H₃C-CH₂-OSO₃⁻ |
| 1950 | H₃C-(CH₂)₆-CH=CH-(CH₂)₈- | 8 | 3 | H₃C-CH₂-OSO₃⁻ |
| 1951 | H₃C-(CH₂)₆-CH=CH-(CH₂)₈- | 9 | 3 | H₃C-CH₂-OSO₃⁻ |
| 1952 | H₃C-(CH₂)₆-CH=CH-(CH₂)₈- | 10 | 3 | H₃C-CH₂-OSO₃⁻ |
| 1953 | H₃C-(CH₂)₆-CH=CH-(CH₂)₈- | 4 | 4 | H₃C-CH₂-OSO₃⁻ |
| 1954 | H₃C-(CH₂)₆-CH=CH-(CH₂)₈- | 5 | 4 | H₃C-CH₂-OSO₃⁻ |
| 1955 | H₃C-(CH₂)₆-CH=CH-(CH₂)₈- | 6 | 4 | H₃C-CH₂-OSO₃⁻ |
| 1956 | H₃C-(CH₂)₆-CH=CH-(CH₂)₈- | 7 | 4 | H₃C-CH₂-OSO₃⁻ |
| 1957 | H₃C-(CH₂)₆-CH=CH-(CH₂)₈- | 8 | 4 | H₃C-CH₂-OSO₃⁻ |
| 1958 | H₃C-(CH₂)₆-CH=CH-(CH₂)₈- | 9 | 4 | H₃C-CH₂-OSO₃⁻ |
| 1959 | H₃C-(CH₂)₆-CH=CH-(CH₂)₈- | 10 | 4 | H₃C-CH₂-OSO₃⁻ |
| 1960 | H₃C-(CH₂)₆-CH=CH-(CH₂)₈- | 5 | 5 | H₃C-CH₂-OSO₃⁻ |
| 1961 | H₃C-(CH₂)₆-CH=CH-(CH₂)₈- | 6 | 5 | H₃C-CH₂-OSO₃⁻ |
| 1962 | H₃C-(CH₂)₆-CH=CH-(CH₂)₈- | 7 | 5 | H₃C-CH₂-OSO₃⁻ |
| 1963 | H₃C-(CH₂)₆-CH=CH-(CH₂)₈- | 8 | 5 | H₃C-CH₂-OSO₃⁻ |
| 1964 | H₃C-(CH₂)₆-CH=CH-(CH₂)₈- | 9 | 5 | H₃C-CH₂-OSO₃⁻ |
| 1965 | H₃C-(CH₂)₆-CH=CH-(CH₂)₈- | 10 | 5 | H₃C-CH₂-OSO₃⁻ |
| 1966 | H₃C-(CH₂)₆-CH=CH-(CH₂)₈- | 6 | 6 | H₃C-CH₂-OSO₃⁻ |
| 1967 | H₃C-(CH₂)₆-CH=CH-(CH₂)₈- | 7 | 6 | H₃C-CH₂-OSO₃⁻ |
| 1968 | H₃C-(CH₂)₆-CH=CH-(CH₂)₈- | 8 | 6 | H₃C-CH₂-OSO₃⁻ |
| 1969 | H₃C-(CH₂)₆-CH=CH-(CH₂)₈- | 9 | 6 | H₃C-CH₂-OSO₃⁻ |
| 1970 | H₃C-(CH₂)₆-CH=CH-(CH₂)₈- | 10 | 6 | H₃C-CH₂-OSO₃⁻ |
| 1971 | H₃C-(CH₂)₆-CH=CH-(CH₂)₈- | 7 | 7 | H₃C-CH₂-OSO₃⁻ |
| 1972 | H₃C-(CH₂)₆-CH=CH-(CH₂)₈- | 8 | 7 | H₃C-CH₂-OSO₃⁻ |
| 1973 | H₃C-(CH₂)₆-CH=CH-(CH₂)₈- | 9 | 7 | H₃C-CH₂-OSO₃⁻ |
| 1974 | H₃C-(CH₂)₆-CH=CH-(CH₂)₈- | 10 | 7 | H₃C-CH₂-OSO₃⁻ |
| 1975 | H₃C-(CH₂)₆-CH=CH-(CH₂)₈- | 8 | 8 | H₃C-CH₂-OSO₃⁻ |
| 1976 | H₃C-(CH₂)₆-CH=CH-(CH₂)₈- | 9 | 8 | H₃C-CH₂-OSO₃⁻ |
| 1977 | H₃C-(CH₂)₆-CH=CH-(CH₂)₈- | 10 | 8 | H₃C-CH₂-OSO₃⁻ |
| 1978 | H₃C-(CH₂)₆-CH=CH-(CH₂)₈- | 9 | 9 | H₃C-CH₂-OSO₃⁻ |
| 1979 | H₃C-(CH₂)₆-CH=CH-(CH₂)₈- | 10 | 9 | H₃C-CH₂-OSO₃⁻ |
| 1980 | H₃C-(CH₂)₆-CH=CH-(CH₂)₈- | 10 | 10 | H₃C-CH₂-OSO₃⁻ |
| 1981 | H₃C-(CH₂)₄-CH=CH-CH₂-CH=CH-(CH₂)₈- | 1 | 1 | Chlorid |
| 1982 | H₃C-(CH₂)₄-CH=CH-CH₂-CH=CH-(CH₂)₈- | 2 | 1 | Chlorid |
| 1983 | H₃C-(CH₂)₄-CH=CH-CH₂-CH=CH-(CH₂)₈- | 3 | 1 | Chlorid |
| 1984 | H₃C-(CH₂)₄-CH=CH-CH₂-CH=CH-(CH₂)₈- | 4 | 1 | Chlorid |
| 1985 | H₃C-(CH₂)₄-CH=CH-CH₂-CH=CH-(CH₂)₈- | 5 | 1 | Chlorid |
| 1986 | H₃C-(CH₂)₄-CH=CH-CH₂-CH=CH-(CH₂)₈- | 6 | 1 | Chlorid |
| 1987 | H₃C-(CH₂)₄-CH=CH-CH₂-CH=CH-(CH₂)₈- | 7 | 1 | Chlorid |
| 1988 | H₃C-(CH₂)₄-CH=CH-CH₂-CH=CH-(CH₂)₈- | 8 | 1 | Chlorid |
| 1989 | H₃C-(CH₂)₄-CH=CH-CH₂-CH=CH-(CH₂)₈- | 9 | 1 | Chlorid |
| 1990 | H₃C-(CH₂)₄-CH=CH-CH₂-CH=CH-(CH₂)₈- | 10 | 1 | Chlorid |
| 1991 | H₃C-(CH₂)₄-CH=CH-CH₂-CH=CH-(CH₂)₈- | 2 | 2 | Chlorid |
| 1992 | H₃C-(CH₂)₄-CH=CH-CH₂-CH=CH-(CH₂)₈- | 3 | 2 | Chlorid |
| 1993 | H₃C-(CH₂)₄-CH=CH-CH₂-CH=CH-(CH₂)₈- | 4 | 2 | Chlorid |
| 1994 | H₃C-(CH₂)₄-CH=CH-CH₂-CH=CH-(CH₂)₈- | 5 | 2 | Chlorid |
| 1995 | H₃C-(CH₂)₄-CH=CH-CH₂-CH=CH-(CH₂)₈- | 6 | 2 | Chlorid |
| 1996 | H₃C-(CH₂)₄-CH=CH-CH₂-CH=CH-(CH₂)₈- | 7 | 2 | Chlorid |
| 1997 | H₃C-(CH₂)₄-CH=CH-CH₂-CH=CH-(CH₂)₈- | 8 | 2 | Chlorid |
| 1998 | H₃C-(CH₂)₄-CH=CH-CH₂-CH=CH-(CH₂)₈- | 9 | 2 | Chlorid |
| 1999 | H₃C-(CH₂)₄-CH=CH-CH₂-CH=CH-(CH₂)₈- | 10 | 2 | Chlorid |
| 2000 | H₃C-(CH₂)₄-CH=CH-CH₂-CH=CH-(CH₂)₈- | 3 | 3 | Chlorid |
| 2001 | H₃C-(CH₂)₄-CH=CH-CH₂-CH=CH-(CH₂)₈- | 4 | 3 | Chlorid |
| 2002 | H₃C-(CH₂)₄-CH=CH-CH₂-CH=CH-(CH₂)₈- | 5 | 3 | Chlorid |
| 2003 | H₃C-(CH₂)₄-CH=CH-CH₂-CH=CH-(CH₂)₈- | 6 | 3 | Chlorid |
| 2004 | H₃C-(CH₂)₄-CH=CH-CH₂-CH=CH-(CH₂)₈- | 7 | 3 | Chlorid |
| 2005 | H₃C-(CH₂)₄-CH=CH-CH₂-CH=CH-(CH₂)₈- | 8 | 3 | Chlorid |
| 2006 | H₃C-(CH₂)₄-CH=CH-CH₂-CH=CH-(CH₂)₈- | 9 | 3 | Chlorid |
| 2007 | H₃C-(CH₂)₄-CH=CH-CH₂-CH=CH-(CH₂)₈- | 10 | 3 | Chlorid |
| 2008 | H₃C-(CH₂)₄-CH=CH-CH₂-CH=CH-(CH₂)₈- | 4 | 4 | Chlorid |
| 2009 | H₃C-(CH₂)₄-CH=CH-CH₂-CH=CH-(CH₂)₈- | 5 | 4 | Chlorid |
| 2010 | H₃C-(CH₂)₄-CH=CH-CH₂-CH=CH-(CH₂)₈- | 6 | 4 | Chlorid |
| 2011 | H₃C-(CH₂)₄-CH=CH-CH₂-CH=CH-(CH₂)₈- | 7 | 4 | Chlorid |
| 2012 | H₃C-(CH₂)₄-CH=CH-CH₂-CH=CH-(CH₂)₈- | 8 | 4 | Chlorid |
| 2013 | H₃C-(CH₂)₄-CH=CH-CH₂-CH=CH-(CH₂)₈- | 9 | 4 | Chlorid |
| 2014 | H₃C-(CH₂)₄-CH=CH-CH₂-CH=CH-(CH₂)₈- | 10 | 4 | Chlorid |
| 2015 | H₃C-(CH₂)₄-CH=CH-CH₂-CH=CH-(CH₂)₈- | 5 | 5 | Chlorid |
| 2016 | H₃C-(CH₂)₄-CH=CH-CH₂-CH=CH-(CH₂)₈- | 6 | 5 | Chlorid |
| 2017 | H₃C-(CH₂)₄-CH=CH-CH₂-CH=CH-(CH₂)₈- | 7 | 5 | Chlorid |
| 2018 | H₃C-(CH₂)₄-CH=CH-CH₂-CH=CH-(CH₂)₈- | 8 | 5 | Chlorid |
| 2019 | H₃C-(CH₂)₄-CH=CH-CH₂-CH=CH-(CH₂)₈- | 9 | 5 | Chlorid |
| 2020 | H₃C-(CH₂)₄-CH=CH-CH₂-CH=CH-(CH₂)₈- | 10 | 5 | Chlorid |
| 2021 | H₃C-(CH₂)₄-CH=CH-CH₂-CH=CH-(CH₂)₈- | 6 | 6 | Chlorid |
| 2022 | H₃C-(CH₂)₄-CH=CH-CH₂-CH=CH-(CH₂)₈- | 7 | 6 | Chlorid |
| 2023 | H₃C-(CH₂)₄-CH=CH-CH₂-CH=CH-(CH₂)₈- | 8 | 6 | Chlorid |
| 2024 | H₃C-(CH₂)₄-CH=CH-CH₂-CH=CH-(CH₂)₈- | 9 | 6 | Chlorid |
| 2025 | H₃C-(CH₂)₄-CH=CH-CH₂-CH=CH-(CH₂)₈- | 10 | 6 | Chlorid |
| 2026 | H₃C-(CH₂)₄-CH=CH-CH₂-CH=CH-(CH₂)₈- | 7 | 7 | Chlorid |
| 2027 | H₃C-(CH₂)₄-CH=CH-CH₂-CH=CH-(CH₂)₈- | 8 | 7 | Chlorid |
| 2028 | H₃C-(CH₂)₄-CH=CH-CH₂-CH=CH-(CH₂)₈- | 9 | 7 | Chlorid |
| 2029 | H₃C-(CH₂)₄-CH=CH-CH₂-CH=CH-(CH₂)₈- | 10 | 7 | Chlorid |
| 2030 | H₃C-(CH₂)₄-CH=CH-CH₂-CH=CH-(CH₂)₈- | 8 | 8 | Chlorid |
| 2031 | H₃C-(CH₂)₄-CH=CH-CH₂-CH=CH-(CH₂)₈- | 9 | 8 | Chlorid |
| 2032 | H₃C-(CH₂)₄-CH=CH-CH₂-CH=CH-(CH₂)₈- | 10 | 8 | Chlorid |
| 2033 | H₃C-(CH₂)₄-CH=CH-CH₂-CH=CH-(CH₂)₈- | 9 | 9 | Chlorid |
| 2034 | H₃C-(CH₂)₄-CH=CH-CH₂-CH=CH-(CH₂)₈- | 10 | 9 | Chlorid |
| 2035 | H₃C-(CH₂)₄-CH=CH-CH₂-CH=CH-(CH₂)₈- | 10 | 10 | Chlorid |
| 2036 | H₃C-(CH₂)₄-CH=CH-CH₂-CH=CH-(CH₂)₈- | 1 | 1 | Bromid |
| 2037 | H₃C-(CH₂)₄-CH=CH-CH₂-CH=CH-(CH₂)₈- | 2 | 1 | Bromid |
| 2038 | H₃C-(CH₂)₄-CH=CH-CH₂-CH=CH-(CH₂)₈- | 3 | 1 | Bromid |
| 2039 | H₃C-(CH₂)₄-CH=CH-CH₂-CH=CH-(CH₂)₈- | 4 | 1 | Bromid |
| 2040 | H₃C-(CH₂)₄-CH=CH-CH₂-CH=CH-(CH₂)₈- | 5 | 1 | Bromid |
| 2041 | H₃C-(CH₂)₄-CH=CH-CH₂-CH=CH-(CH₂)₈- | 6 | 1 | Bromid |
| 2042 | H₃C-(CH₂)₄-CH=CH-CH₂-CH=CH-(CH₂)₈- | 7 | 1 | Bromid |
| 2043 | H₃C-(CH₂)₄-CH=CH-CH₂-CH=CH-(CH₂)₈- | 8 | 1 | Bromid |
| 2044 | H₃C-(CH₂)₄-CH=CH-CH₂-CH=CH-(CH₂)₈- | 9 | 1 | Bromid |
| 2045 | H₃C-(CH₂)₄-CH=CH-CH₂-CH=CH-(CH₂)₈- | 10 | 1 | Bromid |
| 2046 | H₃C-(CH₂)₄-CH=CH-CH₂-CH=CH-(CH₂)₈- | 2 | 2 | Bromid |
| 2047 | H₃C-(CH₂)₄-CH=CH-CH₂-CH=CH-(CH₂)₈- | 3 | 2 | Bromid |
| 2048 | H₃C-(CH₂)₄-CH=CH-CH₂-CH=CH-(CH₂)₈- | 4 | 2 | Bromid |
| 2049 | H₃C-(CH₂)₄-CH=CH-CH₂-CH=CH-(CH₂)₈- | 5 | 2 | Bromid |
| 2050 | H₃C-(CH₂)₄-CH=CH-CH₂-CH=CH-(CH₂)₈- | 6 | 2 | Bromid |
| 2051 | H₃C-(CH₂)₄-CH=CH-CH₂-CH=CH-(CH₂)₈- | 7 | 2 | Bromid |
| 2052 | H₃C-(CH₂)₄-CH=CH-CH₂-CH=CH-(CH₂)₈- | 8 | 2 | Bromid |
| 2053 | H₃C-(CH₂)₄-CH=CH-CH₂-CH=CH-(CH₂)₈- | 9 | 2 | Bromid |
| 2054 | H₃C-(CH₂)₄-CH=CH-CH₂-CH=CH-(CH₂)₈- | 10 | 2 | Bromid |
| 2055 | H₃C-(CH₂)₄-CH=CH-CH₂-CH=CH-(CH₂)₈- | 3 | 3 | Bromid |
| 2056 | H₃C-(CH₂)₄-CH=CH-CH₂-CH=CH-(CH₂)₈- | 4 | 3 | Bromid |
| 2057 | H₃C-(CH₂)₄-CH=CH-CH₂-CH=CH-(CH₂)₈- | 5 | 3 | Bromid |
| 2058 | H₃C-(CH₂)₄-CH=CH-CH₂-CH=CH-(CH₂)₈- | 6 | 3 | Bromid |
| 2059 | H₃C-(CH₂)₄-CH=CH-CH₂-CH=CH-(CH₂)₈- | 7 | 3 | Bromid |
| 2060 | H₃C-(CH₂)₄-CH=CH-CH₂-CH=CH-(CH₂)₈- | 8 | 3 | Bromid |
| 2061 | H₃C-(CH₂)₄-CH=CH-CH₂-CH=CH-(CH₂)₈- | 9 | 3 | Bromid |
| 2062 | H₃C-(CH₂)₄-CH=CH-CH₂-CH=CH-(CH₂)₈- | 10 | 3 | Bromid |
| 2063 | H₃C-(CH₂)₄-CH=CH-CH₂-CH=CH-(CH₂)₈- | 4 | 4 | Bromid |
| 2064 | H₃C-(CH₂)₄-CH=CH-CH₂-CH=CH-(CH₂)₈- | 5 | 4 | Bromid |
| 2065 | H₃C-(CH₂)₄-CH=CH-CH₂-CH=CH-(CH₂)₈- | 6 | 4 | Bromid |
| 2066 | H₃C-(CH₂)₄-CH=CH-CH₂-CH=CH-(CH₂)₈- | 7 | 4 | Bromid |
| 2067 | H₃C-(CH₂)₄-CH=CH-CH₂-CH=CH-(CH₂)₈- | 8 | 4 | Bromid |
| 2068 | H₃C-(CH₂)₄-CH=CH-CH₂-CH=CH-(CH₂)₈- | 9 | 4 | Bromid |
| 2069 | H₃C-(CH₂)₄-CH=CH-CH₂-CH=CH-(CH₂)₈- | 10 | 4 | Bromid |
| 2070 | H₃C-(CH₂)₄-CH=CH-CH₂-CH=CH-(CH₂)₈- | 5 | 5 | Bromid |
| 2071 | H₃C-(CH₂)₄-CH=CH-CH₂-CH=CH-(CH₂)₈- | 6 | 5 | Bromid |
| 2072 | H₃C-(CH₂)₄-CH=CH-CH₂-CH=CH-(CH₂)₈- | 7 | 5 | Bromid |
| 2073 | H₃C-(CH₂)₄-CH=CH-CH₂-CH=CH-(CH₂)₈- | 8 | 5 | Bromid |
| 2074 | H₃C-(CH₂)₄-CH=CH-CH₂-CH=CH-(CH₂)₈- | 9 | 5 | Bromid |
| 2075 | H₃C-(CH₂)₄-CH=CH-CH₂-CH=CH-(CH₂)₈- | 10 | 5 | Bromid |
| 2076 | H₃C-(CH₂)₄-CH=CH-CH₂-CH=CH-(CH₂)₈- | 6 | 6 | Bromid |
| 2077 | H₃C-(CH₂)₄-CH=CH-CH₂-CH=CH-(CH₂)₈- | 7 | 6 | Bromid |
| 2078 | H₃C-(CH₂)₄-CH=CH-CH₂-CH=CH-(CH₂)₈- | 8 | 6 | Bromid |
| 2079 | H₃C-(CH₂)₄-CH=CH-CH₂-CH=CH-(CH₂)₈- | 9 | 6 | Bromid |
| 2080 | H₃C-(CH₂)₄-CH=CH-CH₂-CH=CH-(CH₂)₈- | 10 | 6 | Bromid |
| 2081 | H₃C-(CH₂)₄-CH=CH-CH₂-CH=CH-(CH₂)₈- | 7 | 7 | Bromid |
| 2082 | H₃C-(CH₂)₄-CH=CH-CH₂-CH=CH-(CH₂)₈- | 8 | 7 | Bromid |
| 2083 | H₃C-(CH₂)₄-CH=CH-CH₂-CH=CH-(CH₂)₈- | 9 | 7 | Bromid |
| 2084 | H₃C-(CH₂)₄-CH=CH-CH₂-CH=CH-(CH₂)₈- | 10 | 7 | Bromid |
| 2085 | H₃C-(CH₂)₄-CH=CH-CH₂-CH=CH-(CH₂)₈- | 8 | 8 | Bromid |
| 2086 | H₃C-(CH₂)₄-CH=CH-CH₂-CH=CH-(CH₂)₈- | 9 | 8 | Bromid |
| 2087 | H₃C-(CH₂)₄-CH=CH-CH₂-CH=CH-(CH₂)₈- | 10 | 8 | Bromid |
| 2088 | H₃C-(CH₂)₄-CH=CH-CH₂-CH=CH-(CH₂)₈- | 9 | 9 | Bromid |
| 2089 | H₃C-(CH₂)₄-CH=CH-CH₂-CH=CH-(CH₂)₈- | 10 | 9 | Bromid |
| 2090 | H₃C-(CH₂)₄-CH=CH-CH₂-CH=CH-(CH₂)₈- | 10 | 10 | Bromid |
| 2091 | H₃C-(CH₂)₄-CH=CH-CH₂-CH=CH-(CH₂)₈- | 1 | 1 | H₃C-OSO₃⁻ |
| 2092 | H₃C-(CH₂)₄-CH=CH-CH₂-CH=CH-(CH₂)₈- | 2 | 1 | H₃C-OSO₃⁻ |
| 2093 | H₃C-(CH₂)₄-CH=CH-CH₂-CH=CH-(CH₂)₈- | 3 | 1 | H₃C-OSO₃⁻ |
| 2094 | H₃C-(CH₂)₄-CH=CH-CH₂-CH=CH-(CH₂)₈- | 4 | 1 | H₃C-OSO₃⁻ |
| 2095 | H₃C-(CH₂)₄-CH=CH-CH₂-CH=CH-(CH₂)₈- | 5 | 1 | H₃C-OSO₃⁻ |
| 2096 | H₃C-(CH₂)₄-CH=CH-CH₂-CH=CH-(CH₂)₈- | 6 | 1 | H₃C-OSO₃⁻ |
| 2097 | H₃C-(CH₂)₄-CH=CH-CH₂-CH=CH-(CH₂)₈- | 7 | 1 | H₃C-OSO₃⁻ |
| 2098 | H₃C-(CH₂)₄-CH=CH-CH₂-CH=CH-(CH₂)₈- | 8 | 1 | H₃C-OSO₃⁻ |
| 2099 | H₃C-(CH₂)₄-CH=CH-CH₂-CH=CH-(CH₂)₈- | 9 | 1 | H₃C-OSO₃⁻ |
| 2100 | H₃C-(CH₂)₄-CH=CH-CH₂-CH=CH-(CH₂)₈- | 10 | 1 | H₃C-OSO₃⁻ |
| 2101 | H₃C-(CH₂)₄-CH=CH-CH₂-CH=CH-(CH₂)₈- | 2 | 2 | H₃C-OSO₃⁻ |
| 2102 | H₃C-(CH₂)₄-CH=CH-CH₂-CH=CH-(CH₂)₈- | 3 | 2 | H₃C-OSO₃⁻ |
| 2103 | H₃C-(CH₂)₄-CH=CH-CH₂-CH=CH-(CH₂)₈- | 4 | 2 | H₃C-OSO₃⁻ |
| 2104 | H₃C-(CH₂)₄-CH=CH-CH₂-CH=CH-(CH₂)₈- | 5 | 2 | H₃C-OSO₃⁻ |
| 2105 | H₃C-(CH₂)₄-CH=CH-CH₂-CH=CH-(CH₂)₈- | 6 | 2 | H₃C-OSO₃⁻ |
| 2106 | H₃C-(CH₂)₄-CH=CH-CH₂-CH=CH-(CH₂)₈- | 7 | 2 | H₃C-OSO₃⁻ |
| 2107 | H₃C-(CH₂)₄-CH=CH-CH₂-CH=CH-(CH₂)₈- | 8 | 2 | H₃C-OSO₃⁻ |
| 2108 | H₃C-(CH₂)₄-CH=CH-CH₂-CH=CH-(CH₂)₈- | 9 | 2 | H₃C-OSO₃⁻ |
| 2109 | H₃C-(CH₂)₄-CH=CH-CH₂-CH=CH-(CH₂)₈- | 10 | 2 | H₃C-OSO₃⁻ |
| 2110 | H₃C-(CH₂)₄-CH=CH-CH₂-CH=CH-(CH₂)₈- | 3 | 3 | H₃C-OSO₃⁻ |
| 2111 | H₃C-(CH₂)₄-CH=CH-CH₂-CH=CH-(CH₂)₈- | 4 | 3 | H₃C-OSO₃⁻ |
| 2112 | H₃C-(CH₂)₄-CH=CH-CH₂-CH=CH-(CH₂)₈- | 5 | 3 | H₃C-OSO₃⁻ |
| 2113 | H₃C-(CH₂)₄-CH=CH-CH₂-CH=CH-(CH₂)₈- | 6 | 3 | H₃C-OSO₃⁻ |
| 2114 | H₃C-(CH₂)₄-CH=CH-CH₂-CH=CH-(CH₂)₈- | 7 | 3 | H₃C-OSO₃⁻ |
| 2115 | H₃C-(CH₂)₄-CH=CH-CH₂-CH=CH-(CH₂)₈- | 8 | 3 | H₃C-OSO₃⁻ |
| 2116 | H₃C-(CH₂)₄-CH=CH-CH₂-CH=CH-(CH₂)₈- | 9 | 3 | H₃C-OSO₃⁻ |
| 2117 | H₃C-(CH₂)₄-CH=CH-CH₂-CH=CH-(CH₂)₈- | 10 | 3 | H₃C-OSO₃⁻ |
| 2118 | H₃C-(CH₂)₄-CH=CH-CH₂-CH=CH-(CH₂)₈- | 4 | 4 | H₃C-OSO₃⁻ |
| 2119 | H₃C-(CH₂)₄-CH=CH-CH₂-CH=CH-(CH₂)₈- | 5 | 4 | H₃C-OSO₃⁻ |
| 2120 | H₃C-(CH₂)₄-CH=CH-CH₂-CH=CH-(CH₂)₈- | 6 | 4 | H₃C-OSO₃⁻ |
| 2121 | H₃C-(CH₂)₄-CH=CH-CH₂-CH=CH-(CH₂)₈- | 7 | 4 | H₃C-OSO₃⁻ |
| 2122 | H₃C-(CH₂)₄-CH=CH-CH₂-CH=CH-(CH₂)₈- | 8 | 4 | H₃C-OSO₃⁻ |
| 2123 | H₃C-(CH₂)₄-CH=CH-CH₂-CH=CH-(CH₂)₈- | 9 | 4 | H₃C-OSO₃⁻ |
| 2124 | H₃C-(CH₂)₄-CH=CH-CH₂-CH=CH-(CH₂)₈- | 10 | 4 | H₃C-OSO₃⁻ |
| 2125 | H₃C-(CH₂)₄-CH=CH-CH₂-CH=CH-(CH₂)₈- | 5 | 5 | H₃C-OSO₃⁻ |
| 2126 | H₃C-(CH₂)₄-CH=CH-CH₂-CH=CH-(CH₂)₈- | 6 | 5 | H₃C-OSO₃⁻ |
| 2127 | H₃C-(CH₂)₄-CH=CH-CH₂-CH=CH-(CH₂)₈- | 7 | 5 | H₃C-OSO₃⁻ |
| 2128 | H₃C-(CH₂)₄-CH=CH-CH₂-CH=CH-(CH₂)₈- | 8 | 5 | H₃C-OSO₃⁻ |
| 2129 | H₃C-(CH₂)₄-CH=CH-CH₂-CH=CH-(CH₂)₈- | 9 | 5 | H₃C-OSO₃⁻ |
| 2130 | H₃C-(CH₂)₄-CH=CH-CH₂-CH=CH-(CH₂)₈- | 10 | 5 | H₃C-OSO₃⁻ |
| 2131 | H₃C-(CH₂)₄-CH=CH-CH₂-CH=CH-(CH₂)₈- | 6 | 6 | H₃C-OSO₃⁻ |
| 2132 | H₃C-(CH₂)₄-CH=CH-CH₂-CH=CH-(CH₂)₈- | 7 | 6 | H₃C-OSO₃⁻ |
| 2133 | H₃C-(CH₂)₄-CH=CH-CH₂-CH=CH-(CH₂)₈- | 8 | 6 | H₃C-OSO₃⁻ |
| 2134 | H₃C-(CH₂)₄-CH=CH-CH₂-CH=CH-(CH₂)₈- | 9 | 6 | H₃C-OSO₃⁻ |
| 2135 | H₃C-(CH₂)₄-CH=CH-CH₂-CH=CH-(CH₂)₈- | 10 | 6 | H₃C-OSO₃⁻ |
| 2136 | H₃C-(CH₂)₄-CH=CH-CH₂-CH=CH-(CH₂)₈- | 7 | 7 | H₃C-OSO₃⁻ |
| 2137 | H₃C-(CH₂)₄-CH=CH-CH₂-CH=CH-(CH₂)₈- | 8 | 7 | H₃C-OSO₃⁻ |
| 2138 | H₃C-(CH₂)₄-CH=CH-CH₂-CH=CH-(CH₂)₈- | 9 | 7 | H₃C-OSO₃⁻ |
| 2139 | H₃C-(CH₂)₄-CH=CH-CH₂-CH=CH-(CH₂)₈- | 10 | 7 | H₃C-OSO₃⁻ |
| 2140 | H₃C-(CH₂)₄-CH=CH-CH₂-CH=CH-(CH₂)₈- | 8 | 8 | H₃C-OSO₃⁻ |
| 2141 | H₃C-(CH₂)₄-CH=CH-CH₂-CH=CH-(CH₂)₈- | 9 | 8 | H₃C-OSO₃⁻ |
| 2142 | H₃C-(CH₂)₄-CH=CH-CH₂-CH=CH-(CH₂)₈- | 10 | 8 | H₃C-OSO₃⁻ |
| 2143 | H₃C-(CH₂)₄-CH=CH-CH₂-CH=CH-(CH₂)₈- | 9 | 9 | H₃C-OSO₃⁻ |
| 2144 | H₃C-(CH₂)₄-CH=CH-CH₂-CH=CH-(CH₂)₈- | 10 | 9 | H₃C-OSO₃⁻ |
| 2145 | H₃C-(CH₂)₄-CH=CH-CH₂-CH=CH-(CH₂)₈- | 10 | 10 | H₃C-OSO₃⁻ |
| 2146 | H₃C-(CH₂)₄-CH=CH-CH₂-CH=CH-(CH₂)₈- | 1 | 1 | H₃C-CH₂-OSO₃⁻ |
| 2147 | H₃C-(CH₂)₄-CH=CH-CH₂-CH=CH-(CH₂)₈- | 2 | 1 | H₃C-CH₂-OSO₃⁻ |
| 2148 | H₃C-(CH₂)₄-CH=CH-CH₂-CH=CH-(CH₂)₈- | 3 | 1 | H₃C-CH₂-OSO₃⁻ |
| 2149 | H₃C-(CH₂)₄-CH=CH-CH₂-CH=CH-(CH₂)₈- | 4 | 1 | H₃C-CH₂-OSO₃⁻ |
| 2150 | H₃C-(CH₂)₄-CH=CH-CH₂-CH=CH-(CH₂)₈- | 5 | 1 | H₃C-CH₂-OSO₃⁻ |
| 2151 | H₃C-(CH₂)₄-CH=CH-CH₂-CH=CH-(CH₂)₈- | 6 | 1 | H₃C-CH₂-OSO₃⁻ |
| 2152 | H₃C-(CH₂)₄-CH=CH-CH₂-CH=CH-(CH₂)₈- | 7 | 1 | H₃C-CH₂-OSO₃⁻ |
| 2153 | H₃C-(CH₂)₄-CH=CH-CH₂-CH=CH-(CH₂)₈- | 8 | 1 | H₃C-CH₂-OSO₃⁻ |
| 2154 | H₃C-(CH₂)₄-CH=CH-CH₂-CH=CH-(CH₂)₈- | 9 | 1 | H₃C-CH₂-OSO₃⁻ |
| 2155 | H₃C-(CH₂)₄-CH=CH-CH₂-CH=CH-(CH₂)₈- | 10 | 1 | H₃C-CH₂-OSO₃⁻ |
| 2156 | H₃C-(CH₂)₄-CH=CH-CH₂-CH=CH-(CH₂)₈- | 2 | 2 | H₃C-CH₂-OSO₃⁻ |
| 2157 | H₃C-(CH₂)₄-CH=CH-CH₂-CH=CH-(CH₂)₈- | 3 | 2 | H₃C-CH₂-OSO₃⁻ |
| 2158 | H₃C-(CH₂)₄-CH=CH-CH₂-CH=CH-(CH₂)₈- | 4 | 2 | H₃C-CH₂-OSO₃⁻ |
| 2159 | H₃C-(CH₂)₄-CH=CH-CH₂-CH=CH-(CH₂)₈- | 5 | 2 | H₃C-CH₂-OSO₃⁻ |
| 2160 | H_{3C}-(CH₂)₄-CH=CH-CH₂-CH=CH-(CH₂)₈- | 6 | 2 | H₃C-CH₂-OSO₃⁻ |
| 2161 | H₃C-(CH₂)₄-CH=CH-CH₂-CH=CH-(CH₂)₈- | 7 | 2 | H₃C-CH₂-OSO₃⁻ |
| 2162 | H₃C-(CH₂)₄-CH=CH-CH₂-CH=CH-(CH₂)₈- | 8 | 2 | H₃C-CH₂-OSO₃⁻ |
| 2163 | H₃C-(CH₂)₄-CH=CH-CH₂-CH=CH-(CH₂)₈- | 9 | 2 | H₃C-CH₂-OSO₃⁻ |
| 2164 | H₃C-(CH₂)₄-CH=CH-CH₂-CH=CH-(CH₂)₈- | 10 | 2 | H₃C-CH₂-OSO₃⁻ |
| 2165 | H₃C-(CH₂)₄-CH=CH-CH₂-CH=CH-(CH₂)₈- | 3 | 3 | H₃C-CH₂-OSO₃⁻ |
| 2166 | H₃C-(CH₂)₄-CH=CH-CH₂-CH=CH-(CH₂)₈- | 4 | 3 | H₃C-CH₂-OSO₃⁻ |
| 2167 | H₃C-(CH₂)₄-CH=CH-CH₂-CH=CH-(CH₂)₈- | 5 | 3 | H₃C-CH₂-OSO₃⁻ |
| 2168 | H₃C-(CH₂)₄-CH=CH-CH₂-CH=CH-(CH₂)₈- | 6 | 3 | H₃C-CH₂-OSO₃⁻ |
| 2169 | H₃C-(CH₂)₄-CH=CH-CH₂-CH=CH-(CH₂)₈- | 7 | 3 | H₃C-CH₂-OSO₃⁻ |
| 2170 | H₃C-(CH₂)₄-CH=CH-CH₂-CH=CH-(CH₂)₈- | 8 | 3 | H₃C-CH₂-OSO₃⁻ |
| 2171 | H₃C-(CH₂)₄-CH=CH-CH₂-CH=CH-(CH₂)₈- | 9 | 3 | H₃C-CH₂-OSO₃⁻ |
| 2172 | H₃C-(CH₂)₄-CH=CH-CH₂-CH=CH-(CH₂)₈- | 10 | 3 | H₃C-CH₂-OSO₃⁻ |
| 2173 | H₃C-(CH₂)₄-CH=CH-CH₂-CH=CH-(CH₂)₈- | 4 | 4 | H₃C-CH₂-OSO₃⁻ |
| 2174 | H₃C-(CH₂)₄-CH=CH-CH₂-CH=CH-(CH₂)₈- | 5 | 4 | H₃C-CH₂-OSO₃⁻ |
| 2175 | H₃C-(CH₂)₄-CH=CH-CH₂-CH=CH-(CH₂)₈- | 6 | 4 | H₃C-CH₂-OSO₃⁻ |
| 2176 | H₃C-(CH₂)₄-CH=CH-CH₂-CH=CH-(CH₂)₈- | 7 | 4 | H₃C-CH₂-OSO₃⁻ |
| 2177 | H₃C-(CH₂)₄-CH=CH-CH₂-CH=CH-(CH₂)₈- | 8 | 4 | H₃C-CH₂-OSO₃⁻ |
| 2178 | H₃C-(CH₂)₄-CH=CH-CH₂-CH=CH-(CH₂)₈- | 9 | 4 | H₃C-CH₂-OSO₃⁻ |
| 2179 | H₃C-(CH₂)₄-CH=CH-CH₂-CH=CH-(CH₂)₈- | 10 | 4 | H₃C-CH₂-OSO₃⁻ |
| 2180 | H₃C-(CH₂)₄-CH=CH-CH₂-CH=CH-(CH₂)₈- | 5 | 5 | H₃C-CH₂-OSO₃⁻ |
| 2181 | H₃C-(CH₂)₄-CH=CH-CH₂-CH=CH-(CH₂)₈- | 6 | 5 | H₃C-CH₂-OSO₃⁻ |
| 2182 | H₃C-(CH₂)₄-CH=CH-CH₂-CH=CH-(CH₂)₈- | 7 | 5 | H₃C-CH₂-OSO₃⁻ |
| 2183 | H₃C-(CH₂)₄-CH=CH-CH₂-CH=CH-(CH₂)₈- | 8 | 5 | H₃C-CH₂-OSO₃⁻ |
| 2184 | H₃C-(CH₂)₄-CH=CH-CH₂-CH=CH-(CH₂)₈- | 9 | 5 | H₃C-CH₂-OSO₃⁻ |
| 2185 | H₃C-(CH₂)₄-CH=CH-CH₂-CH=CH-(CH₂)₈- | 10 | 5 | H₃C-CH₂-OSO₃⁻ |
| 2186 | H₃C-(CH₂)₄-CH=CH-CH₂-CH=CH-(CH₂)₈- | 6 | 6 | H₃C-CH₂-OSO₃⁻ |
| 2187 | H₃C-(CH₂)₄-CH=CH-CH₂-CH=CH-(CH₂)₈- | 7 | 6 | H₃C-CH₂-OSO₃⁻ |
| 2188 | H₃C-(CH₂)₄-CH=CH-CH₂-CH=CH-(CH₂)₈- | 8 | 6 | H₃C-CH₂-OSO₃⁻ |
| 2189 | H₃C-(CH₂)₄-CH=CH-CH₂-CH=CH-(CH₂)₈- | 9 | 6 | H₃C-CH₂-OSO₃⁻ |
| 2190 | H₃C-(CH₂)₄-CH=CH-CH₂-CH=CH-(CH₂)₈- | 10 | 6 | H₃C-CH₂-OSO₃⁻ |
| 2191 | H₃C-(CH₂)₄-CH=CH-CH₂-CH=CH-(CH₂)₈- | 7 | 7 | H₃C-CH₂-OSO₃⁻ |
| 2192 | H₃C-(CH₂)₄-CH=CH-CH₂-CH=CH-(CH₂)₈- | 8 | 7 | H₃C-CH₂-OSO₃⁻ |
| 2193 | H₃C-(CH₂)₄-CH=CH-CH₂-CH=CH-(CH₂)₈- | 9 | 7 | H₃C-CH₂-OSO₃⁻ |
| 2194 | H₃C-(CH₂)₄-CH=CH-CH₂-CH=CH-(CH₂)₈- | 10 | 7 | H₃C-CH₂-OSO₃⁻ |
| 2195 | H₃C-(CH₂)₄-CH=CH-CH₂-CH=CH-(CH₂)₈- | 8 | 8 | H₃C-CH₂-OSO₃⁻ |
| 2196 | H₃C-(CH₂)₄-CH=CH-CH₂-CH=CH-(CH₂)₈- | 9 | 8 | H₃C-CH₂-OSO₃⁻ |
| 2197 | H₃C-(CH₂)₄-CH=CH-CH₂-CH=CH-(CH₂)₈- | 10 | 8 | H₃C-CH₂-OSO₃⁻ |
| 2198 | H₃C-(CH₂)₄-CH=CH-CH₂-CH=CH-(CH₂)₈- | 9 | 9 | H₃C-CH₂-OSO₃⁻ |
| 2199 | H₃C-(CH₂)₄-CH=CH-CH₂-CH=CH-(CH₂)₈- | 10 | 9 | H₃C-CH₂-OSO₃⁻ |
| 2200 | H₃C-(CH₂)₄-CH=CH-CH₂-CH=CH-(CH₂)₈- | 10 | 10 | H₃C-CH₂-OSO₃⁻ |
| 2201 | H₃C-CH₂-CH=CH-CH₂-CH=CH-CH₂-CH=CH-(CH₂)₈- | 1 | 1 | Chlorid |
| 2202 | H₃C-CH₂-CH=CH-CH₂-CH=CH-CH₂-CH=CH-(CH₂)₈- | 2 | 1 | Chlorid |
| 2203 | H₃C-CH₂-CH=CH-CH₂-CH=CH-CH₂-CH=CH-(CH₂)₈- | 3 | 1 | Chlorid |
| 2204 | H₃C-CH₂-CH=CH-CH₂-CH=CH-CH₂-CH=CH-(CH₂)₈- | 4 | 1 | Chlorid |
| 2205 | H₃C-CH₂-CH=CH-CH₂-CH=CH-CH₂-CH=CH-(CH₂)₈- | 5 | 1 | Chlorid |
| 2206 | H₃C-CH₂-CH=CH-CH₂-CH=CH-CH₂-CH=CH-(CH₂)₈- | 6 | 1 | Chlorid |
| 2207 | H₃C-CH₂-CH=CH-CH₂-CH=CH-CH₂-CH=CH-(CH₂)₈- | 7 | 1 | Chlorid |
| 2208 | H₃C-CH₂-CH=CH-CH₂-CH=CH-CH₂-CH=CH-(CH₂)₈- | 8 | 1 | Chlorid |
| 2209 | H₃C-CH₂-CH=CH-CH₂-CH=CH-CH₂-CH=CH-(CH₂)₈- | 9 | 1 | Chlorid |
| 2210 | H₃C-CH₂-CH=CH-CH₂-CH=CH-CH₂-CH=CH-(CH₂)₈- | 10 | 1 | Chlorid |
| 2211 | H₃C-CH₂-CH=CH-CH₂-CH=CH-CH₂-CH=CH-(CH₂)₈- | 2 | 2 | Chlorid |
| 2212 | H₃C-CH₂-CH=CH-CH₂-CH=CH-CH₂-CH=CH-(CH₂)₈- | 3 | 2 | Chlorid |
| 2213 | H₃C-CH₂-CH=CH-CH₂-CH=CH-CH₂-CH=CH-(CH₂)₈- | 4 | 2 | Chlorid |
| 2214 | H₃C-CH₂-CH=CH-CH₂-CH=CH-CH₂-CH=CH-(CH₂)₈- | 5 | 2 | Chlorid |
| 2215 | H₃C-CH₂-CH=CH-CH₂-CH=CH-CH₂-CH=CH-(CH₂)₈- | 6 | 2 | Chlorid |
| 2216 | H₃C-CH₂-CH=CH-CH₂-CH=CH-CH₂-CH=CH-(CH₂)₈- | 7 | 2 | Chlorid |
| 2217 | H₃C-CH₂-CH=CH-CH₂-CH=CH-CH₂-CH=CH-(CH₂)₈- | 8 | 2 | Chlorid |
| 2218 | H₃C-CH₂-CH=CH-CH₂-CH=CH-CH₂-CH=CH-(CH₂)₈- | 9 | 2 | Chlorid |
| 2219 | H₃C-CH₂-CH=CH-CH₂-CH=CH-CH₂-CH=CH-(CH₂)₈- | 10 | 2 | Chlorid |
| 2220 | H₃C-CH₂-CH=CH-CH₂-CH=CH-CH₂-CH=CH-(CH₂)₈- | 3 | 3 | Chlorid |
| 2221 | H₃C-CH₂-CH=CH-CH₂-CH=CH-CH₂-CH=CH-(CH₂)₈- | 4 | 3 | Chlorid |
| 2222 | H₃C-CH₂-CH=CH-CH₂-CH=CH-CH₂-CH=CH-(CH₂)₈- | 5 | 3 | Chlorid |
| 2223 | H₃C-CH₂-CH=CH-CH₂-CH=CH-CH₂-CH=CH-(CH₂)₈- | 6 | 3 | Chlorid |
| 2224 | H₃C-CH₂-CH=CH-CH₂-CH=CH-CH₂-CH=CH-(CH₂)₈- | 7 | 3 | Chlorid |
| 2225 | H₃C-CH₂-CH=CH-CH₂-CH=CH-CH₂-CH=CH-(CH₂)₈- | 8 | 3 | Chlorid |
| 2226 | H₃C-CH₂-CH=CH-CH₂-CH=CH-CH₂-CH=CH-(CH₂)₈- | 9 | 3 | Chlorid |
| 2227 | H₃C-CH₂-CH=CH-CH₂-CH=CH-CH₂-CH=CH-(CH₂)₈- | 10 | 3 | Chlorid |
| 2228 | H₃C-CH₂-CH=CH-CH₂-CH=CH-CH₂-CH=CH-(CH₂)₈- | 4 | 4 | Chlorid |
| 2229 | H₃C-CH₂-CH=CH-CH₂-CH=CH-CH₂-CH=CH-(CH₂)₈- | 5 | 4 | Chlorid |
| 2230 | H₃C-CH₂-CH=CH-CH₂-CH=CH-CH₂-CH=CH-(CH₂)₈- | 6 | 4 | Chlorid |
| 2231 | H₃C-CH₂-CH=CH-CH₂-CH=CH-CH₂-CH=CH-(CH₂)₈- | 7 | 4 | Chlorid |
| 2232 | H₃C-CH₂-CH=CH-CH₂-CH=CH-CH₂-CH=CH-(CH₂)₈- | 8 | 4 | Chlorid |
| 2233 | H₃C-CH₂-CH=CH-CH₂-CH=CH-CH₂-CH=CH-(CH₂)₈- | 9 | 4 | Chlorid |
| 2234 | H₃C-CH₂-CH=CH-CH₂-CH=CH-CH₂-CH=CH-(CH₂)₈- | 10 | 4 | Chlorid |
| 2235 | H₃C-CH₂-CH=CH-CH₂-CH=CH-CH₂-CH=CH-(CH₂)₈- | 5 | 5 | Chlorid |
| 2236 | H₃C-CH₂-CH=CH-CH₂-CH=CH-CH₂-CH=CH-(CH₂)₈- | 6 | 5 | Chlorid |
| 2237 | H₃C-CH₂-CH=CH-CH₂-CH=CH-CH₂-CH=CH-(CH₂)₈- | 7 | 5 | Chlorid |
| 2238 | H₃C-CH₂-CH=CH-CH₂-CH=CH-CH₂-CH=CH-(CH₂)₈- | 8 | 5 | Chlorid |
| 2239 | H₃C-CH₂-CH=CH-CH₂-CH=CH-CH₂-CH=CH-(CH₂)₈- | 9 | 5 | Chlorid |
| 2240 | H₃C-CH₂-CH=CH-CH₂-CH=CH-CH₂-CH=CH-(CH₂)₈- | 10 | 5 | Chlorid |
| 2241 | H₃C-CH₂-CH=CH-CH₂-CH=CH-CH₂-CH=CH-(CH₂)₈- | 6 | 6 | Chlorid |
| 2242 | H₃C-CH₂-CH=CH-CH₂-CH=CH-CH₂-CH=CH-(CH₂)₈- | 7 | 6 | Chlorid |
| 2243 | H₃C-CH₂-CH=CH-CH₂-CH=CH-CH₂-CH=CH-(CH₂)₈- | 8 | 6 | Chlorid |
| 2244 | H₃C-CH₂-CH=CH-CH₂-CH=CH-CH₂-CH=CH-(CH₂)₈- | 9 | 6 | Chlorid |
| 2245 | H₃C-CH₂-CH=CH-CH₂-CH=CH-CH₂-CH=CH-(CH₂)₈- | 10 | 6 | Chlorid |
| 2246 | H₃C-CH₂-CH=CH-CH₂-CH=CH-CH₂-CH=CH-(CH₂)₈- | 7 | 7 | Chlorid |
| 2247 | H₃C-CH₂-CH=CH-CH₂-CH=CH-CH₂-CH=CH-(CH₂)₈- | 8 | 7 | Chlorid |
| 2248 | H₃C-CH₂-CH=CH-CH₂-CH=CH-CH₂-CH=CH-(CH₂)₈- | 9 | 7 | Chlorid |
| 2249 | H₃C-CH₂-CH=CH-CH₂-CH=CH-CH₂-CH=CH-(CH₂)₈- | 10 | 7 | Chlorid |
| 2250 | H₃C-CH₂-CH=CH-CH₂-CH=CH-CH₂-CH=CH-(CH₂)₈- | 8 | 8 | Chlorid |
| 2251 | H₃C-CH₂-CH=CH-CH₂-CH=CH-CH₂-CH=CH-(CH₂)₈- | 9 | 8 | Chlorid |
| 2252 | H₃C-CH₂-CH=CH-CH₂-CH=CH-CH₂-CH=CH-(CH₂)₈- | 10 | 8 | Chlorid |
| 2253 | H₃C-CH₂-CH=CH-CH₂-CH=CH-CH₂-CH=CH-(CH₂)₈- | 9 | 9 | Chlorid |
| 2254 | H₃C-CH₂-CH=CH-CH₂-CH=CH-CH₂-CH=CH-(CH₂)₈- | 10 | 9 | Chlorid |
| 2255 | H₃C-CH₂-CH=CH-CH₂-CH=CH-CH₂-CH=CH-(CH₂)₈- | 10 | 10 | Chlorid |
| 2256 | H₃C-CH₂-CH=CH-CH₂-CH=CH-CH₂-CH=CH-(CH₂)₈- | 1 | 1 | Bromid |
| 2257 | H₃C-CH₂-CH=CH-CH₂-CH=CH-CH₂-CH=CH-(CH₂)₈- | 2 | 1 | Bromid |
| 2258 | H₃C-CH₂-CH=CH-CH₂-CH=CH-CH₂-CH=CH-(CH₂)₈- | 3 | 1 | Bromid |
| 2259 | H₃C-CH₂-CH=CH-CH₂-CH=CH-CH₂-CH=CH-(CH₂)₈- | 4 | 1 | Bromid |
| 2260 | H₃C-CH₂-CH=CH-CH₂-CH=CH-CH₂-CH=CH-(CH₂)₈- | 5 | 1 | Bromid |
| 2261 | H₃C-CH₂-CH=CH-CH₂-CH=CH-CH₂-CH=CH-(CH₂)₈- | 6 | 1 | Bromid |
| 2262 | H₃C-CH₂-CH=CH-CH₂-CH=CH-CH₂-CH=CH-(CH₂)₈- | 7 | 1 | Bromid |
| 2263 | H₃C-CH₂-CH=CH-CH₂-CH=CH-CH₂-CH=CH-(CH₂)₈- | 8 | 1 | Bromid |
| 2264 | H₃C-CH₂-CH=CH-CH₂-CH=CH-CH₂-CH=CH-(CH₂)₈- | 9 | 1 | Bromid |
| 2265 | H₃C-CH₂-CH=CH-CH₂-CH=CH-CH₂-CH=CH-(CH₂)₈- | 10 | 1 | Bromid |
| 2266 | H₃C-CH₂-CH=CH-CH₂-CH=CH-CH₂-CH=CH-(CH₂)₈- | 2 | 2 | Bromid |
| 2267 | H₃C-CH₂-CH=CH-CH₂-CH=CH-CH₂-CH=CH-(CH₂)₈- | 3 | 2 | Bromid |
| 2268 | H₃C-CH₂-CH=CH-CH₂-CH=CH-CH₂-CH=CH-(CH₂)₈- | 4 | 2 | Bromid |
| 2269 | H₃C-CH₂-CH=CH-CH₂-CH=CH-CH₂-CH=CH-(CH₂)₈- | 5 | 2 | Bromid |
| 2270 | H₃C-CH₂-CH=CH-CH₂-CH=CH-CH₂-CH=CH-(CH₂)₈- | 6 | 2 | Bromid |
| 2271 | H₃C-CH₂-CH=CH-CH₂-CH=CH-CH₂-CH=CH-(CH₂)₈- | 7 | 2 | Bromid |
| 2272 | H₃C-CH₂-CH=CH-CH₂-CH=CH-CH₂-CH=CH-(CH₂)₈- | 8 | 2 | Bromid |
| 2273 | H₃C-CH₂-CH=CH-CH₂-CH=CH-CH₂-CH=CH-(CH₂)₈- | 9 | 2 | Bromid |
| 2274 | H₃C-CH₂-CH=CH-CH₂-CH=CH-CH₂-CH=CH-(CH₂)₈- | 10 | 2 | Bromid |
| 2275 | H₃C-CH₂-CH=CH-CH₂-CH=CH-CH₂-CH=CH-(CH₂)₈- | 3 | 3 | Bromid |
| 2276 | H₃C-CH₂-CH=CH-CH₂-CH=CH-CH₂-CH=CH-(CH₂)₈- | 4 | 3 | Bromid |
| 2277 | H₃C-CH₂-CH=CH-CH₂-CH=CH-CH₂-CH=CH-(CH₂)₈- | 5 | 3 | Bromid |
| 2278 | H₃C-CH₂-CH=CH-CH₂-CH=CH-CH₂-CH=CH-(CH₂)₈- | 6 | 3 | Bromid |
| 2279 | H₃C-CH₂-CH=CH-CH₂-CH=CH-CH₂-CH=CH-(CH₂)₈- | 7 | 3 | Bromid |
| 2280 | H₃C-CH₂-CH=CH-CH₂-CH=CH-CH₂-CH=CH-(CH₂)₈- | 8 | 3 | Bromid |
| 2281 | H₃C-CH₂-CH=CH-CH₂-CH=CH-CH₂-CH=CH-(CH₂)₈- | 9 | 3 | Bromid |
| 2282 | H₃C-CH₂-CH=CH-CH₂-CH=CH-CH₂-CH=CH-(CH₂)₈- | 10 | 3 | Bromid |
| 2283 | H₃C-CH₂-CH=CH-CH₂-CH=CH-CH₂-CH=CH-(CH₂)₈- | 4 | 4 | Bromid |
| 2284 | H₃C-CH₂-CH=CH-CH₂-CH=CH-CH₂-CH=CH-(CH₂)₈- | 5 | 4 | Bromid |
| 2285 | H₃C-CH₂-CH=CH-CH₂-CH=CH-CH₂-CH=CH-(CH₂)₈- | 6 | 4 | Bromid |
| 2286 | H₃C-CH₂-CH=CH-CH₂-CH=CH-CH₂-CH=CH-(CH₂)₈- | 7 | 4 | Bromid |
| 2287 | H₃C-CH₂-CH=CH-CH₂-CH=CH-CH₂-CH=CH-(CH₂)₈- | 8 | 4 | Bromid |
| 2288 | H₃C-CH₂-CH=CH-CH₂-CH=CH-CH₂-CH=CH-(CH₂)₈- | 9 | 4 | Bromid |
| 2289 | H₃C-CH₂-CH=CH-CH₂-CH=CH-CH₂-CH=CH-(CH₂)₈- | 10 | 4 | Bromid |
| 2290 | H₃C-CH₂-CH=CH-CH₂-CH=CH-CH₂-CH=CH-(CH₂)₈- | 5 | 5 | Bromid |
| 2291 | H₃C-CH₂-CH=CH-CH₂-CH=CH-CH₂-CH=CH-(CH₂)₈- | 6 | 5 | Bromid |
| 2292 | H₃C-CH₂-CH=CH-CH₂-CH=CH-CH₂-CH=CH-(CH₂)₈- | 7 | 5 | Bromid |
| 2293 | H₃C-CH₂-CH=CH-CH₂-CH=CH-CH₂-CH=CH-(CH₂)₈- | 8 | 5 | Bromid |
| 2294 | H₃C-CH₂-CH=CH-CH₂-CH=CH-CH₂-CH=CH-(CH₂)₈- | 9 | 5 | Bromid |
| 2295 | H₃C-CH₂-CH=CH-CH₂-CH=CH-CH₂-CH=CH-(CH₂)₈- | 10 | 5 | Bromid |
| 2296 | H₃C-CH₂-CH=CH-CH₂-CH=CH-CH₂-CH=CH-(CH₂)₈- | 6 | 6 | Bromid |
| 2297 | H₃C-CH₂-CH=CH-CH₂-CH=CH-CH₂-CH=CH-(CH₂)₈- | 7 | 6 | Bromid |
| 2298 | H₃C-CH₂-CH=CH-CH₂-CH=CH-CH₂-CH=CH-(CH₂)₈- | 8 | 6 | Bromid |
| 2299 | H₃C-CH₂-CH=CH-CH₂-CH=CH-CH₂-CH=CH-(CH₂)₈- | 9 | 6 | Bromid |
| 2300 | H₃C-CH₂-CH=CH-CH₂-CH=CH-CH₂-CH=CH-(CH₂)₈- | 10 | 6 | Bromid |
| 2301 | H₃C-CH₂-CH=CH-CH₂-CH=CH-CH₂-CH=CH-(CH₂)₈- | 7 | 7 | Bromid |
| 2302 | H₃C-CH₂-CH=CH-CH₂-CH=CH-CH₂-CH=CH-(CH₂)₈- | 8 | 7 | Bromid |
| 2303 | H₃C-CH₂-CH=CH-CH₂-CH=CH-CH₂-CH=CH-(CH₂)₈- | 9 | 7 | Bromid |
| 2304 | H₃C-CH₂-CH=CH-CH₂-CH=CH-CH₂-CH=CH-(CH₂)₈- | 10 | 7 | Bromid |
| 2305 | H₃C-CH₂-CH=CH-CH₂-CH=CH-CH₂-CH=CH-(CH₂)₈- | 8 | 8 | Bromid |
| 2306 | H₃C-CH₂-CH=CH-CH₂-CH=CH-CH₂-CH=CH-(CH₂)₈- | 9 | 8 | Bromid |
| 2307 | H₃C-CH₂-CH=CH-CH₂-CH=CH-CH₂-CH=CH-(CH₂)₈- | 10 | 8 | Bromid |
| 2308 | H₃C-CH₂-CH=CH-CH₂-CH=CH-CH₂-CH=CH-(CH₂)₈- | 9 | 9 | Bromid |
| 2309 | H₃C-CH₂-CH=CH-CH₂-CH=CH-CH₂-CH=CH-(CH₂)₈- | 10 | 9 | Bromid |
| 2310 | H₃C-CH₂-CH=CH-CH₂-CH=CH-CH₂-CH=CH-(CH₂)₈- | 10 | 10 | Bromid |
| 2311 | H₃C-CH₂-CH=CH-CH₂-CH=CH-CH₂-CH=CH-(CH₂)₈- | 1 | 1 | H₃C-OSO₃⁻ |
| 2312 | H₃C-CH₂-CH=CH-CH₂-CH=CH-CH₂-CH=CH-(CH₂)₈- | 2 | 1 | H₃C-OSO₃⁻ |
| 2313 | H₃C-CH₂-CH=CH-CH₂-CH=CH-CH₂-CH=CH-(CH₂)₈- | 3 | 1 | H₃C-OSO₃⁻ |
| 2314 | H₃C-CH₂-CH=CH-CH₂-CH=CH-CH₂-CH=CH-(CH₂)₈- | 4 | 1 | H₃C-OSO₃⁻ |
| 2315 | H₃C-CH₂-CH=CH-CH₂-CH=CH-CH₂-CH=CH-(CH₂)₈- | 5 | 1 | H₃C-OSO₃⁻ |
| 2316 | H₃C-CH₂-CH=CH-CH₂-CH=CH-CH₂-CH=CH-(CH₂)₈- | 6 | 1 | H₃C-OSO₃⁻ |
| 2317 | H₃C-CH₂-CH=CH-CH₂-CH=CH-CH₂-CH=CH-(CH₂)₈- | 7 | 1 | H₃C-OSO₃⁻ |
| 2318 | H₃C-CH₂-CH=CH-CH₂-CH=CH-CH₂-CH=CH-(CH₂)₈- | 8 | 1 | H₃C-OSO₃⁻ |
| 2319 | H₃C-CH₂-CH=CH-CH₂-CH=CH-CH₂-CH=CH-(CH₂)₈- | 9 | 1 | H₃C-OSO₃⁻ |
| 2320 | H₃C-CH₂-CH=CH-CH₂-CH=CH-CH₂-CH=CH-(CH₂)₈- | 10 | 1 | H₃C-OSO₃⁻ |
| 2321 | H₃C-CH₂-CH=CH-CH₂-CH=CH-CH₂-CH=CH-(CH₂)₈- | 2 | 2 | H₃C-OSO₃⁻ |
| 2322 | H₃C-CH₂-CH=CH-CH₂-CH=CH-CH₂-CH=CH-(CH₂)₈- | 3 | 2 | H₃C-OSO₃⁻ |
| 2323 | H₃C-CH₂-CH=CH-CH₂-CH=CH-CH₂-CH=CH-(CH₂)₈- | 4 | 2 | H₃C-OSO₃⁻ |
| 2324 | H₃C-CH₂-CH=CH-CH₂-CH=CH-CH₂-CH=CH-(CH₂)₈- | 5 | 2 | H₃C-OSO₃⁻ |
| 2325 | H₃C-CH₂-CH=CH-CH₂-CH=CH-CH₂-CH=CH-(CH₂)₈- | 6 | 2 | H₃C-OSO₃⁻ |
| 2326 | H₃C-CH₂-CH=CH-CH₂-CH=CH-CH₂-CH=CH-(CH₂)₈- | 7 | 2 | H₃C-OSO₃⁻ |
| 2327 | H₃C-CH₂-CH=CH-CH₂-CH=CH-CH₂-CH=CH-(CH₂)₈- | 8 | 2 | H₃C-OSO₃⁻ |
| 2328 | H₃C-CH₂-CH=CH-CH₂-CH=CH-CH₂-CH=CH-(CH₂)₈- | 9 | 2 | H₃C-OSO₃⁻ |
| 2329 | H₃C-CH₂-CH=CH-CH₂-CH=CH-CH₂-CH=CH-(CH₂)₈- | 10 | 2 | H₃C-OSO₃⁻ |
| 2330 | H₃C-CH₂-CH=CH-CH₂-CH=CH-CH₂-CH=CH-(CH₂)₈- | 3 | 3 | H₃C-OSO₃⁻ |
| 2331 | H₃C-CH₂-CH=CH-CH₂-CH=CH-CH₂-CH₌CH-(CH₂)₈- | 4 | 3 | H₃C-OSO₃⁻ |
| 2332 | H₃C-CH₂-CH=CH-CH₂-CH=CH-CH₂-CH=CH-(CH₂)₈- | 5 | 3 | H₃C-OSO₃⁻ |
| 2333 | H₃C-CH₂-CH=CH-CH₂-CH=CH-CH₂-CH=CH-(CH₂)₈- | 6 | 3 | H₃C-OSO₃⁻ |
| 2334 | H₃C-CH₂-CH=CH-CH₂-CH=CH-CH₂-CH=CH-(CH₂)₈- | 7 | 3 | H₃C-OSO₃⁻ |
| 2335 | H₃C-CH₂-CH=CH-CH₂-CH=CH-CH₂-CH=CH-(CH₂)₈- | 8 | 3 | H₃C-OSO₃⁻ |
| 2336 | H₃C-CH₂-CH=CH-CH₂-CH=CH-CH₂-CH=CH-(CH₂)₈- | 9 | 3 | H₃C-OSO₃⁻ |
| 2337 | H₃C-CH₂-CH=CH-CH₂-CH=CH-CH₂-CH=CH-(CH₂)₈- | 10 | 3 | H₃C-OSO₃⁻ |
| 2338 | H₃C-CH₂-CH=CH-CH₂-CH=CH-CH₂-CH=CH-(CH₂)₈- | 4 | 4 | H₃C-OSO₃⁻ |
| 2339 | H₃C-CH₂-CH=CH-CH₂-CH=CH-CH₂-CH=CH-(CH₂)₈- | 5 | 4 | H₃C-OSO₃⁻ |
| 2340 | H₃C-CH₂-CH=CH-CH₂-CH=CH-CH₂-CH=CH-(CH₂)₈- | 6 | 4 | H₃C-OSO₃⁻ |
| 2341 | H₃C-CH₂-CH=CH-CH₂-CH=CH-CH₂-CH=CH-(CH₂)₈- | 7 | 4 | H₃C-OSO₃⁻ |
| 2342 | H₃C-CH₂-CH=CH-CH₂-CH=CH-CH₂-CH=CH-(CH₂)₈- | 8 | 4 | H₃C-OSO₃⁻ |
| 2343 | H₃C-CH₂-CH=CH-CH₂-CH=CH-CH₂-CH=CH-(CH₂)₈- | 9 | 4 | H₃C-OSO₃⁻ |
| 2344 | H₃C-CH₂-CH=CH-CH₂-CH=CH-CH₂-CH=CH-(CH₂)₈- | 10 | 4 | H₃C-OSO₃⁻ |
| 2345 | H₃C-CH₂-CH=CH-CH₂-CH=CH-CH₂-CH=CH-(CH₂)₈- | 5 | 5 | H₃C-OSO₃⁻ |
| 2346 | H₃C-CH₂-CH=CH-CH₂-CH=CH-CH₂-CH=CH-(CH₂)₈- | 6 | 5 | H₃C-OSO₃⁻ |
| 2347 | H₃C-CH₂-CH=CH-CH₂-CH=CH-CH₂-CH=CH-(CH₂)₈- | 7 | 5 | H₃C-OSO₃⁻ |
| 2348 | H₃C-CH₂-CH=CH-CH₂-CH=CH-CH₂-CH=CH-(CH₂)₈- | 8 | 5 | H₃C-OSO₃⁻ |
| 2349 | H₃C-CH₂-CH=CH-CH₂-CH=CH-CH₂-CH=CH-(CH₂)₈- | 9 | 5 | H₃C-OSO₃⁻ |
| 2350 | H₃C-CH₂-CH=CH-CH₂-CH=CH-CH₂-CH=CH-(CH₂)₈- | 10 | 5 | H₃C-OSO₃⁻ |
| 2351 | H₃C-CH₂-CH=CH-CH₂-CH=CH-CH₂-CH=CH-(CH₂)₈- | 6 | 6 | H₃C-OSO₃ |
| 2352 | H₃C-CH₂-CH=CH-CH₂-CH=CH-CH₂-CH=CH-(CH₂)₈- | 7 | 6 | H₃C-OSO₃⁻ |
| 2353 | H₃C-CH₂-CH=CH-CH₂-CH=CH-CH₂-CH=CH-(CH₂)₈- | 8 | 6 | H₃C-OSO₃⁻ |
| 2354 | H₃C-CH₂-CH=CH-CH₂-CH=CH-CH₂-CH=CH-(CH₂)₈- | 9 | 6 | H₃C-OSO₃⁻ |
| 2355 | H₃C-CH₂-CH=CH-CH₂-CH=CH-CH₂-CH=CH-(CH₂)₈- | 10 | 6 | H₃C-OSO₃⁻ |
| 2356 | H₃C-CH₂-CH=CH-CH₂-CH=CH-CH₂-CH=CH-(CH₂)₈- | 7 | 7 | H₃C-OSO₃⁻ |
| 2357 | H₃C-CH₂-CH=CH-CH₂-CH=CH-CH₂-CH=CH-(CH₂)₈- | 8 | 7 | H₃C-OSO₃⁻ |
| 2358 | H₃C-CH₂-CH=CH-CH₂-CH=CH-CH₂-CH=CH-(CH₂)₈- | 9 | 7 | H₃C-OSO₃⁻ |
| 2359 | H₃C-CH₂-CH=CH-CH₂-CH=CH-CH₂-CH=CH-(CH₂)₈- | 10 | 7 | H₃C-OSO₃⁻ |
| 2360 | H₃C-CH₂-CH=CH-CH₂-CH=CH-CH₂-CH=CH-(CH₂)₈- | 8 | 8 | H₃C-OSO₃⁻ |
| 2361 | H₃C-CH₂-CH=CH-CH₂-CH=CH-CH₂-CH=CH-(CH₂)₈- | 9 | 8 | H₃C-OSO₃⁻ |
| 2362 | H₃C-CH₂-CH=CH-CH₂-CH=CH-CH₂-CH=CH-(CH₂)₈- | 10 | 8 | H₃C-OSO₃⁻ |
| 2363 | H₃C-CH₂-CH=CH-CH₂-CH=CH-CH₂-CH=CH-(CH₂)₈- | 9 | 9 | H₃C-OSO₃⁻ |
| 2364 | H₃C-CH₂-CH=CH-CH₂-CH=CH-CH₂-CH=CH-(CH₂)₈- | 10 | 9 | H₃C-OSO₃⁻ |
| 2365 | H₃C-CH₂-CH=CH-CH₂-CH=CH-CH₂-CH=CH-(CH₂)₈- | 10 | 10 | H₃C-OSO₃⁻ |
| 2366 | H₃C-CH₂-CH=CH-CH₂-CH=CH-CH₂-CH=CH-(CH₂)₈- | 1 | 1 | H₃C-CH₂-OSO₃⁻ |
| 2367 | H₃C-CH₂-CH=CH-CH₂-CH=CH-CH₂-CH=CH-(CH₂)₈- | 2 | 1 | H₃C-CH₂-OSO₃⁻ |
| 2368 | H₃C-CH₂-CH=CH-CH₂-CH=CH-CH₂-CH=CH-(CH₂)₈- | 3 | 1 | H₃C-CH₂-OSO₃⁻ |
| 2369 | H₃C-CH₂-CH=CH-CH₂-CH=CH-CH₂-CH=CH-(CH₂)₈- | 4 | 1 | H₃C-CH₂-OSO₃⁻ |
| 2370 | H₃C-CH₂-CH=CH-CH₂-CH=CH-CH₂-CH=CH-(CH₂)₈- | 5 | 1 | H₃C-CH₂-OSO₃⁻ |
| 2371 | H₃C-CH₂-CH=CH-CH₂-CH=CH-CH₂-CH=CH-(CH₂)₈- | 6 | 1 | H₃C-CH₂-OSO₃⁻ |
| 2372 | H₃C-CH₂-CH=CH-CH₂-CH=CH-CH₂-CH=CH-(CH₂)₈- | 7 | 1 | H₃C-CH₂-OSO₃⁻ |
| 2373 | H₃C-CH₂-CH=CH-CH₂-CH=CH-CH₂-CH=CH-(CH₂)₈- | 8 | 1 | H₃C-CH₂-OSO₃⁻ |
| 2374 | H₃C-CH₂-CH=CH-CH₂-CH=CH-CH₂-CH=CH-(CH₂)₈- | 9 | 1 | H₃C-CH₂-OSO₃⁻ |
| 2375 | H₃C-CH₂-CH=CH-CH₂-CH=CH-CH₂-CH=CH-(CH₂)₈- | 10 | 1 | H₃C-CH₂-OSO₃⁻ |
| 2376 | H₃C-CH₂-CH=CH-CH₂-CH=CH-CH₂-CH=CH-(CH₂)₈- | 2 | 2 | H₃C-CH₂-OSO₃⁻ |
| 2377 | H₃C-CH₂-CH=CH-CH₂-CH=CH-CH₂-CH=CH-(CH₂)₈- | 3 | 2 | H₃C-CH₂-OSO₃⁻ |
| 2378 | H₃C-CH₂-CH=CH-CH₂-CH=CH-CH₂-CH=CH-(CH₂)₈- | 4 | 2 | H₃C-CH₂-OSO₃⁻ |
| 2379 | H₃C-CH₂-CH=CH-CH₂-CH=CH-CH₂-CH=CH-(CH₂)₈- | 5 | 2 | H₃C-CH₂-OSO₃⁻ |
| 2380 | H₃C-CH₂-CH=CH-CH₂-CH=CH-CH₂-CH=CH-(CH₂)₈- | 6 | 2 | H₃C-CH₂-OSO₃ |
| 2381 | H₃C-CH₂-CH=CH-CH₂-CH=CH-CH₂-CH=CH-(CH₂)₈- | 7 | 2 | H₃C-CH₂-OSO₃⁻ |
| 2382 | H₃C-CH₂-CH=CH-CH₂-CH=CH-CH₂-CH=CH-(CH₂)₈- | 8 | 2 | H₃C-CH₂-OSO₃⁻ |
| 2383 | H₃C-CH₂-CH=CH-CH₂-CH=CH-CH₂-CH=CH-(CH₂)₈- | 9 | 2 | H₃C-CH₂-OSO₃⁻ |
| 2384 | H₃C-CH₂-CH=CH-CH₂-CH=CH-CH₂-CH=CH-(CH₂)₈- | 10 | 2 | H₃C-CH₂-OSO₃⁻ |
| 2385 | H₃C-CH₂-CH=CH-CH₂-CH=CH-CH₂-CH=CH-(CH₂)₈- | 3 | 3 | H₃C-CH₂-OSO₃⁻ |
| 2386 | H₃C-CH₂-CH=CH-CH₂-CH=CH-CH₂-CH=CH-(CH₂)₈- | 4 | 3 | H₃C-CH₂-OSO₃⁻ |
| 2387 | H₃C-CH₂-CH=CH-CH₂-CH=CH-CH₂-CH=CH-(CH₂)₈- | 5 | 3 | H₃C-CH₂-OSO₃⁻ |
| 2388 | H₃C-CH₂-CH=CH-CH₂-CH=CH-CH₂-CH=CH-(CH₂)₈- | 6 | 3 | H₃C-CH₂-OSO₃⁻ |
| 2389 | H₃C-CH₂-CH=CH-CH₂-CH=CH-CH₂-CH=CH-(CH₂)₈- | 7 | 3 | H₃C-CH₂-OSO₃⁻ |
| 2390 | H₃C-CH₂-CH=CH-CH₂-CH=CH-CH₂-CH=CH-(CH₂)₈- | 8 | 3 | H₃C-CH₂-OSO₃⁻ |
| 2391 | H₃C-CH₂-CH=CH-CH₂-CH=CH-CH₂-CH=CH-(CH₂)₈- | 9 | 3 | H₃C-CH₂-OSO₃⁻ |
| 2392 | H₃C-CH₂-CH=CH-CH₂-CH=CH-CH₂-CH=CH-(CH₂)₈- | 10 | 3 | H₃C-CH₂-OSO₃⁻ |
| 2393 | H₃C-CH₂-CH=CH-CH₂-CH=CH-CH₂-CH=CH-(CH₂)₈- | 4 | 4 | H₃C-CH₂-OSO₃⁻ |
| 2394 | H₃C-CH₂-CH=CH-CH₂-CH=CH-CH₂-CH=CH-(CH₂)₈- | 5 | 4 | H₃C-CH₂-OSO₃⁻ |
| 2395 | H₃C-CH₂-CH=CH-CH₂-CH=CH-CH₂-CH=CH-(CH₂)₈- | 6 | 4 | H₃C-CH₂-OSO₃⁻ |
| 2396 | H₃C-CH₂-CH=CH-CH₂-CH=CH-CH₂-CH=CH-(CH₂)₈- | 7 | 4 | H₃C-CH₂-OSO₃⁻ |
| 2397 | H₃C-CH₂-CH=CH-CH₂-CH=CH-CH₂-CH=CH-(CH₂)₈- | 8 | 4 | H₃C-CH₂-OSO₃⁻ |
| 2398 | H₃C-CH₂-CH=CH-CH₂-CH=CH-CH₂-CH=CH-(CH₂)₈- | 9 | 4 | H₃C-CH₂-OSO₃⁻ |
| 2399 | H₃C-CH₂-CH=CH-CH₂-CH=CH-CH₂-CH=CH-(CH₂)₈- | 10 | 4 | H₃C-CH₂-OSO₃⁻ |
| 2400 | H₃C-CH₂-CH=CH-CH₂-CH=CH-CH₂-CH=CH-(CH₂)₈- | 5 | 5 | H₃C-CH₂-OSO₃⁻ |
| 2401 | H₃C-CH₂-CH=CH-CH₂-CH=CH-CH₂-CH=CH-(CH₂)₈- | 6 | 5 | H₃C-CH₂-OSO₃⁻ |
| 2402 | H₃C-CH₂-CH=CH-CH₂-CH=CH-CH₂-CH=CH-(CH₂)₈- | 7 | 5 | H₃C-CH₂-OSO₃⁻ |
| 2403 | H₃C-CH₂-CH=CH-CH₂-CH=CH-CH₂-CH=CH-(CH₂)₈- | 8 | 5 | H₃C-CH₂-OSO₃⁻ |
| 2404 | H₃C-CH₂-CH=CH-CH₂-CH=CH-CH₂-CH=CH-(CH₂)₈- | 9 | 5 | H₃C-CH₂-OSO₃⁻ |
| 2405 | H₃C-CH₂-CH=CH-CH₂-CH=CH-CH₂-CH=CH-(CH₂)8- | 10 | 5 | H₃C-CH₂-OSO₃⁻ |
| 2406 | H₃C-CH₂-CH=CH-CH₂-CH=CH-CH₂-CH=CH-(CH₂)₈- | 6 | 6 | H₃C-CH₂-OSO₃⁻ |
| 2407 | H₃C-CH₂-CH=CH-CH₂-CH=CH-CH₂-CH=CH-(CH₂)₈- | 7 | 6 | H₃C-CH₂-OSO₃⁻ |
| 2408 | H₃C-CH₂-CH=CH-CH₂-CH=CH-CH₂-CH=CH-(CH₂)₈- | 8 | 6 | H₃C-CH₂-OSO₃⁻ |
| 2409 | H₃C-CH₂-CH=CH-CH₂-CH=CH-CH₂-CH=CH-(CH₂)₈- | 9 | 6 | H₃C-CH₂-OSO₃⁻ |
| 2410 | H₃C-CH₂-CH=CH-CH₂-CH=CH-CH₂-CH=CH-(CH₂)₈- | 10 | 6 | H₃C-CH₂-OSO₃⁻ |
| 2411 | H₃C-CH₂-CH=CH-CH₂-CH=CH-CH₂-CH=CH-(CH₂)₈- | 7 | 7 | H₃C-CH₂-OSO₃⁻ |
| 2412 | H₃C-CH₂-CH=CH-CH₂-CH=CH-CH₂-CH=CH-(CH₂)₈- | 8 | 7 | H₃C-CH₂-OSO₃⁻ |
| 2413 | H₃C-CH₂-CH=CH-CH₂-CH=CH-CH₂-CH=CH-(CH₂)₈- | 9 | 7 | H₃C-CH₂-OSO₃⁻ |
| 2414 | H₃C-CH₂-CH=CH-CH₂-CH=CH-CH₂-CH=CH-(CH₂)₈- | 10 | 7 | H₃C-CH₂-OSO₃⁻ |
| 2415 | H₃C-CH₂-CH=CH-CH₂-CH=CH-CH₂-CH=CH-(CH₂)₈- | 8 | 8 | H₃C-CH₂-OSO₃⁻ |
| 2416 | H₃C-CH₂-CH=CH-CH₂-CH=CH-CH₂-CH=CH-(CH₂)₈- | 9 | 8 | H₃C-CH₂-OSO₃⁻ |
| 2417 | H₃C-CH₂-CH=CH-CH₂-CH=CH-CH₂-CH=CH-(CH₂)₈- | 10 | 8 | H₃C-CH₂-OSO₃⁻ |
| 2418 | H₃C-CH₂-CH=CH-CH₂-CH=CH-CH₂-CH=CH-(CH₂)₈- | 9 | 9 | H₃C-CH₂-OSO₃⁻ |
| 2419 | H₃C-CH₂-CH=CH-CH₂-CH=CH-CH₂-CH=CH-(CH₂)₈- | 10 | 9 | H₃C-CH₂-OSO₃⁻ |
| 2420 | H₃C-CH₂-CH=CH-CH₂-CH=CH-CH₂-CH=CH-(CH₂)₈- | 10 | 10 | H₃C-CH₂-OSO₃⁻ |

Insbesondere bevorzugte erfindungsgemäße kosmetisches Mittel sind dadurch gekennzeichnet, daß die Zubereitung B mindestens eine ionische Flüssigkeit der Formel 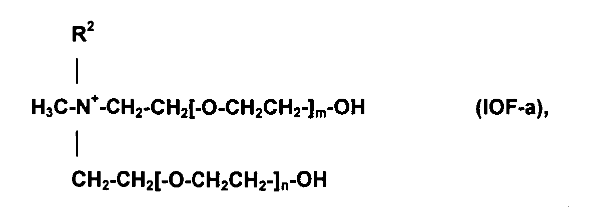 enthält, in der die Indices m und n unabhängig voneinander jeweils für Werte von 1 bis 7, vorzugsweise von 2, 3, 4, 5 stehen, das Gegenion ausgewählt ist aus Methosulfat, Ethosulfat und der Rest R² ein gesättigter oder ungesättigter langkettiger Fettrest, vorzugsweise ein C₈₋₁₈-Alkyl- oder Alkenylrest ist.

Zusätzlich zu Aniontensid(en), gegebenenfalls weiteren Tensiden und ionische(r/n) Flüssigkeit(en) können die erfindungsgemäßen Mittel weitere Inhaltsstoffe von Kosmetika enthalten.
Diese werden nachfolgend beschrieben.

In einer weiteren bevorzugten Ausführungsform können die erfindungsgemäßen Mittel Emulgatoren (F) enthalten. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Erfindungsgemäß verwendbare Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®} PGPH),
- Lineare und verzweigte Fettsäuren mit 8 bis 30 C - Atomen und deren Na-, K-, Ammonium-, Ca-, Mg- und Zn - Salze.

Die erfindungsgemäßen Mittel enthalten die Emulgatoren bevorzugt in Mengen von 0,1 - 25 Gew.%, insbesondere 0,5 - 15 Gew.-%, bezogen auf das gesamte Mittel.

Bevorzugt können die erfindungsgemäßen Zusammensetzungen mindestens einen nichtionogenen Emulgator mit einem HLB-Wert von 8 bis 18 enthalten. Nichtionogene Emulgatoren mit einem HLB-Wert von 10 - 15 können erfindungsgemäß besonders bevorzugt sein.

Als weiterhin vorteilhaft hat es sich gezeigt, wenn Polymere (G) in den erfindungsgemäßen Mitteln enthalten sind. In einer bevorzugten Ausführungsform werden den erfindungsgemäß verwendeten Mitteln daher Polymere zugesetzt, wobei sich sowohl kationische, anionische, amphotere als auch nichtionische Polymere als wirksam erwiesen haben.

Erfindungsgemäß einsetzbar sind vorzugsweise kationische bzw. amphotere Polymere. Unter kationischen bzw. amphoteren Polymeren sind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C1-4-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (G1-I), 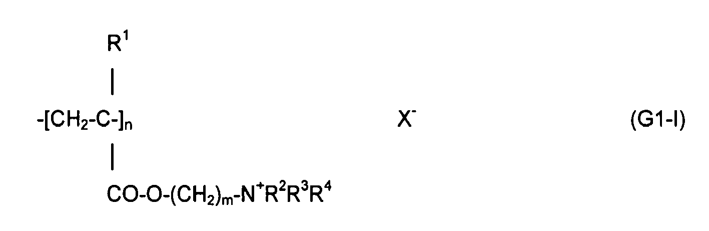 in der R¹= -H oder -CH₃ ist, R², R³ und R⁴ unabhängig voneinander ausgewählt sind aus C1-4-Alkyl-, -Alkenyl- oder -Hydroxyalkylgruppen, m = 1, 2, 3 oder 4, n eine natürliche Zahl und X' ein physiologisch verträgliches organisches oder anorganisches Anion ist, sowie Copolymere, bestehend im wesentlichen aus den in Formel (G1-I) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
- R¹ steht für eine Methylgruppe
- R², R³ und R⁴ stehen für Methylgruppen
- m hat den Wert 2.

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Solche Produkte sind beispielsweise unter den Bezeichnungen Rheocare^{®} CTH (Cosmetic Rheologies) und Synthalen^{®} CR (Ethnichem) im Handel erhältlich. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel (G1-1) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Weitere bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- kationische Alkylpolyglycoside gemäß der DE-PS 44 13 686,
- kationiserter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Weitere in den erfindungsgemäßen Mitteln einsetzbare kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel frei verfügbar sind.

Erfindungsgemäß bevorzugte kationische Polymere sind kationische Cellulose-Derivate und Chitosan und dessen Derivate, insbesondere die Handelsprodukte Polymer^{®}JR 400, Hydagen^{®} HCMF und Kytamer^{®} PC, kationische Guar-Derivate, kationische Honig-Derivate, insbesondere das Handelsprodukt Honeyquat^{®} 50, kationische Alkylpolyglycodside gemäß der DE-PS 44 13 686 und Polymere vom Typ Polyquaternium-37.

Weiterhin sind kationiserte Proteinhydrolysate zu den kationischen Polymeren zu zählen, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann. Die den erfindungsgemäßen kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder einer Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die erfindungsgemäßen kationischen Proteinhydrolysate und -derivate seien die unter den INCI - Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte genannt: Cocodimonium Hydroxypropyl Hydrolyzed Collagen, Cocodimopnium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Soy Protein, Cocodimonium Hydroxypropyl Hydrolyzed Wheat Protein, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed Keratin, Hydroxypropyltrimonium Hydrolyzed Rice Bran Protein, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Soy Protein, Quaternium-79 Hydrolyzed Wheat Protein.

Ganz besonders bevorzugt sind die kationischen Proteinhydrolysate und -derivate auf pflanzlicher Basis.

Zusätzlich zu kationischen Polymerisaten oder an ihrer Stelle können die erfindungsgemäßen Mittel auch amphotere Polymere enthalten. Diese weisen zusätzlich mindestens eine negativ geladene Gruppe im Molekül auf und werden auch als zwitterionische Polymere bezeichnet. Im Rahmen der vorliegenden Erfindung bevorzugt einsetzbare zwitterionische Polymerisate setzen sich im wesentlichen zusammen aus
A) Monomeren mit quartären Ammoniumgruppen der allgemeinen Formel (Z-I),

   R¹-CH=CR²-CO-Z-(CₙH₂ₙ)-N⁽⁺⁾R³R⁴R⁵ A⁽⁻⁾ (Z-I)

   In der R¹ und R² unabhängig voneinander stehen für Wasserstoff oder eine Methylgruppe und R³, R⁴ und R⁵ unabhängig voneinander für Alkylgruppen mit 1 bis 4 Kohlenstoff-Atomen, Z eine NH-Gruppe oder ein Sauerstoffatom, n eine ganze Zahl von 2 bis 5 und A⁽⁻⁾ das Anion einer organischen oder anorganischen Säure ist
   und
B) monomeren Carbonsäuren der allgemeinen Formel (Z-II),

   R⁶-CH=CR⁷-COOH (Z-II)

   in denen R⁶ und R⁷ unabhängig voneinander Wasserstoff oder Methylgruppen sind.

Geeignete Ausgangsmonomere sind z. B. Dimethylaminoethylacrylamid, Dimethylaminoethylmethacrylamid, Dimethylaminopropylacrylamid, Dimethylaminopropylmethacrylamid und Diethylaminoethylacrylamid, wenn Z eine NH-Gruppe bedeutet oder Dimethylaminoethylacrylat, Dimethylaminoethylmethacrylat und Diethylaminoethylacrylat, wenn Z ein Sauerstoffatom ist.

Die eine tertiäre Aminogruppe enthaltenden Monomeren werden dann in bekannter Weise quarterniert, wobei als Alkylierungsreagenzien Methylchlorid, Dimethylsulfat oder Diethylsulfat besonders geeignet sind. Die Quaternisierungsreaktion kann in wäßriger Lösung oder im Lösungsmittel erfolgen.

Vorteilhafterweise werden solche Monomere der Formel (Z-I) verwendet, die Derivate des Acrylamids oder Methacrylamids darstellen. Weiterhin bevorzugt sind solche Monomeren, die als Gegenionen Halogenid-, Methoxysulfat- oder Ethoxysulfat-Ionen enthalten. Ebenfalls bevorzugt sind solche Monomeren der Formel (Z-I), bei denen R³, R⁴ und R⁵ Methylgruppen sind.

Das Acrylamidopropyl-trimethylammoniumchlorid ist ein ganz besonders bevorzugtes Monomer der Formel (Z-I).

Als monomere Carbonsäuren der Formel (Z-II) eignen sich Acrylsäure, Methacrylsäure, Crotonsäure und 2-Methyl-crotonsäure. Bevorzugt werden Acryl- oder Methacrylsäure, insbesondere Acrylsäure, eingesetzt.

Die erfindungsgemäß einsetzbaren zwitterionischen Polymerisate werden aus Monomeren der Formeln (Z-I) und (Z-II) nach an sich bekannten Polymerisationsverfahren hergestellt. Die Polymerisation kann entweder in wäßriger oder wäßrig-alkoholischer Lösung erfolgen. Als Alkohole werden Alkohole mit 1 bis 4 Kohlenstoffatomen, vorzugsweise Isopropanol, verwendet, die gleichzeitig als Polymerisationsregler dienen. Der Monomerlösung können aber auch andere Komponenten als Regler zugesetzt werden, z. B. Ameisensäure oder Mercaptane, wie Thioethanol und Thioglykolsäure. Die Initiierung der Polymerisation erfolgt mit Hilfe von radikalbildenden Substanzen. Hierzu können Redoxsysteme und/oder thermisch zerfallende Radikalbildner vom Typ der Azoverbindungen, wie z. B. Azoisobuttersäurenitril, Azo-bis-(cyanopentansäure) oder Azo-bis-(amidinopropan)dihydrochlorid verwendet werden. Als Redoxsysteme eignen sich z. B. Kombinationen aus Wasserstoffperoxid, Kalium- oder Ammoniumperoxodisulfat sowie tertiäres Butylhydroperoxid mit Natriumsulfit, Natriumdithionit oder Hydroxylaminhydrochlorid als Reduktionskomponente.

Die Polymerisation kann isotherm oder unter adiabatischen Bedingungen durchgeführt werden, wobei in Abhängigkeit von den Konzentrationsverhältnissen durch die freiwerdende Polymerisationswärme der Temperaturbereich für den Ablauf der Reaktion zwischen 20 und 200 °C schwanken kann, und die Reaktion gegebenenfalls unter dem sich einstellenden Überdruck durchgeführt werden muß. Bevorzugterweise liegt die Reaktionstemperatur zwischen 20 und 100 °C.

Der pH-Wert während der Copolymerisation kann in einem weiten Bereich schwanken. Vorteilhafterweise wird bei niedrigen pH-Werten polymerisiert; möglich sind jedoch auch pH-Werte oberhalb des Neutralpunktes. Nach der Polymerisation wird mit einer wäßrigen Base, z. B. Natronlauge, Kalilauge oder Ammoniak, auf einen pH-Wert zwischen 5 und 10, vorzugsweise 6 bis 8, eingestellt. Nähere Angaben zum Polymerisationsverfahren können den Beispielen entnommen werden.

Als besonders wirksam haben sich solche Polymerisate erwiesen, bei denen die Monomeren der Formel (Z-I) gegenüber den Monomeren der Formel (Z-II) im Überschuß vorlagen. Es ist daher erfindungsgemäß bevorzugt, solche Polymerisate zu verwenden, die aus Monomeren der Formel (Z-I) und die Monomeren der Formel (Z-II) in einem Molverhältnis von 60:40 bis 95:5, insbesondere von 75:25 bis 95:5, bestehen.

Die kationischen bzw. amphoteren Polymere sind in den erfindungsgemäßen Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Erfindungsgemäß besonders bevorzugte kosmetische Mittel sind dadurch gekennzeichnet, daß sie - vorzugsweise die fließfähige Zubereitung B - zusätzlich kationische und/oder amphotere Polymere, vorzugsweise
- Poly(methacryloyloxyethyltrimethylammoniumchlorid) (INCI: Polyquaternium-37) und/oder;
- quaternisierte Cellulose-Derivate (INCI: Polyquaternium 10) und/oder
- kationische Alkylpolyglycoside und/oder
- kationiserter Honig und/oder
- kationische Guar-Derivate und/oder
- polymere Dimethyldiallylammoniumsalze und deren Copolymere mit Estern und Amiden von Acrylsäure und Methacrylsäure und/oder
- Copolymere des Vinylpyrrolidons mit quaternierten Derivaten des Dialkylaminoalkylacrylats und -methacrylats und/oder
- Vinylpyrrolidon-Vinylimidazoliummethochlorid-Copolymere und/oder
- quaternierter Polyvinylalkohol und/oder
- Polyquaternium 2 und/oder
- Polyquaternium 17 und/oder
- Polyquaternium 18 und/oder
- Polyquaternium 24 und/oder
- Polyquaternium 27
vorzugsweise in Mengen von 0,1 bis 5 Gew.-%, besonders bevorzugt von 0,2 bis 4 Gew.-% und insbesondere von 0,4 bis 2,5 Gew.-%, jeweils bezogen auf das Mittel, enthält.

Zusätzlich zu den kationischen Polymeren - oder auch an ihrer Stelle - können die erfindungsgemäßen Mittel weitere Polymere enthalten.

Bei den anionischen Polymeren (G2) handelt es sich um anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.

Als ganz besonders wirkungsvoll haben sich anionische Polymere erwiesen, die als alleiniges oder Co-Monomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann.

Besonders bevorzugt ist das Homopolymer der 2-Acrylamido-2-methylpropansulfonsäure, das beispielsweise unter der Bezeichnung Rheothik^{®}11-80 im Handel erhältlich ist.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.
Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylenbisacrylamid zum Einsatz kommen. Ein solches Polymer ist in dem Handelsprodukt Sepigel^{®}305 der Firma SEPPIC enthalten. Die Verwendung dieses Compounds, das neben der Polymerkomponente eine Kohlenwasserstoffmischung (C₁₃-C₁₄-lsoparaffin) und einen nichtionogenen Emulgator (Laureth-7) enthält, hat sich im Rahmen der erfindungsgemäßen Lehre als besonders vorteilhaft erwiesen.

Auch die unter der Bezeichnung Simulgel^{®}600 als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol^{®} im Handel erhältlich.

Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnung Stabileze^{®} QM im Handel erhältlich.

Weiterhin können als Polymere zur Steigerung der Wirkung des erfindungsgemäßen Wirkstoffkomplexes (A) amphotere Polymere (G3) verwendet werden. Unter dem Begriff amphotere Polymere werden sowohl solche Polymere, die im Molekül sowohl freie Aminogruppen als auch freie -COOH- oder SO₃H-Gruppen enthalten und zur Ausbildung innerer Salze befähigt sind, als auch zwitterionische Polymere, die im Molekül quartäre Ammoniumgruppen und -COO⁻- oder -SO₃⁻-Gruppen enthalten, und solche Polymere zusammengefaßt, die -COOH- oder SO₃H-Gruppen und quartäre Ammoniumgruppen enthalten.

Ein Beispiel für ein erfindungsgemäß einsetzbares Amphopolymer ist das unter der Bezeichnung Amphomer^{®} erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Bevorzugt eingesetzte amphotere Polymere sind solche Polymerisate, die sich im wesentlichen zusammensetzen aus
(a) Monomeren mit quartären Ammoniumgruppen der allgemeinen Formel (G3-I),

   R¹-CH=CR²-CO-Z-(CₙH₂ₙ)-N⁽⁺⁾R³R⁴R⁵ A⁽⁻⁾ (G3-I)

   in der R¹ und R² unabhängig voneinander stehen für Wasserstoff oder eine Methylgruppe und R³, R⁴ und R⁵ unabhängig voneinander für Alkylgruppen mit 1 bis 4 Kohlenstoffatomen, Z eine NH-Gruppe oder ein Sauerstoffatom, n eine ganze Zahl von 2 bis 5 und A⁽⁻⁾ das Anion einer organischen oder anorganischen Säure ist, und
(b) monomeren Carbonsäuren der allgemeinen Formel (G3-II),

   R⁶-CH=CR⁷-COOH (G3-II)

   in denen R⁶ und R⁷ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Ganz besonders bevorzugt sind solche Polymerisate, bei denen Monomere des Typs (a) eingesetzt werden, bei denen R³, R⁴ und R⁵ Methylgruppen sind, Z eine NH-Gruppe und A⁽⁻⁾ ein Halogenid-, Methoxysulfat- oder Ethoxysulfat-Ion ist; Acrylamidopropyl-trimethylammoniumchlorid ist ein besonders bevorzugtes Monomeres (a). Als Monomeres (b) für die genannten Polymerisate wird bevorzugt Acrylsäure verwendet.

Die erfindungsgemäßen Mittel können in einer weiteren Ausführungsform nichtionogene Polymere (G4) enthalten.

Geeignete nichtionogene Polymere sind beispielsweise:
- VinylpyrrolidonNinylester-Copolymere, wie sie beispielsweise unter dem Warenzeichen Luviskol^{®} (BASF) vertrieben werden. Luviskol^{®} VA 64 und Luviskol^{®} VA 73, jeweils Vinylpyrrolidon/Vinylacetat-Copolymere, sind ebenfalls bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal^{®} und Benecel^{®} (AQUALON) und Natrosol^{®}-Typen (Hercules) vertrieben werden.
- Stärke und deren Derivate, insbesondere Stärkeether, beispielsweise Structure^{®} XL (National Starch), eine multifunktionelle, salztolerante Stärke;
- Schellack
- Polyvinylpyrrolidone, wie sie beispielsweise unter der Bezeichnung Luviskol^{®} (BASF) vertrieben werden.
- Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200 °C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder Hydroxy-Gruppen enthalten.
- Glycosidisch substituierte Silicone.

Es ist erfindungsgemäß auch möglich, daß die verwendeten Zubereitungen mehrere, insbesondere zwei verschiedene Polymere gleicher Ladung und/oder jeweils ein ionisches und ein amphoteres und/oder nicht ionisches Polymer enthalten.

Die Polymere (G) sind in den erfindungsgemäßen Mitteln bevorzugt in Mengen von 0,05 bis 10 Gew.-%, bezogen auf das gesamte Mittel, enthalten. Mengen von 0,1 bis 5, insbesondere von 0,1 bis 3 Gew.-%, sind besonders bevorzugt.

Zusätzlich zu den genannten Stoffen können die erfindungsgemäßen Mittel weitere Pflegestoffe enthalten. Mit besonderem Vorzug sind dies beispielsweise Vitamine, Provitamine oder Vitaminvorstufen, so daß erfindungsgemäß bevorzugte Mittel dadurch gekennzeichnet sind, daß sie zusätzlich mindestens einen Stoff aus der Gruppe der Vitamine, Provitamine und Vitaminvorstufen sowie deren Derivate enthalten, wobei Vitamine, Pro-Vitamine und Vitaminvorstufen bevorzugt sind, die den Gruppen A, B, C, E, F und H zugeordnet werden. Diese werden weiter unten ausführlich beschrieben.

Zusätzlich kann es sich als vorteilhaft erweisen, wenn in den erfindungsgemäßen Mitteln Penetrationshilfsstoffe und/ oder Quellmittel (M) enthalten sind. Hierzu sind beispielsweise zu zählen Harnstoff und Harnstoffderivate, Guanidin und dessen Derivate, Arginin und dessen Derivate, Wasserglas, Imidazol und Dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol.

Vorteilhaft im Sinne der Erfindung können zusätzlich kurzkettige Carbonsäuren (N) den Wirkstoffkomplex (A) unterstützen. Unter kurzkettigen Carbonsäuren und deren Derivaten im Sinne der Erfindung werden Carbonsäuren verstanden, welche gesättigt oder ungesättigt und/oder geradkettig oder verzweigt oder cyclisch und/oder aromatisch und/oder heterocyclisch sein können und ein Molekulargewicht kleiner 750 aufweisen. Bevorzugt im Sinne der Erfindung können gesättigte oder ungesättigte geradkettige oder verzweigte Carbonsäuren mit einer Kettenlänge von 1 bis zu 16 C-Atomen in der Kette sein, ganz besonders bevorzugt sind solche mit einer Kettenlänge von 1 bis zu 12 C - Atomen in der Kette.

Die kurzkettigen Carbonsäuren im Sinne der Erfindung können ein, zwei, drei oder mehr Carboxygruppen aufweisen. Bevorzugt im Sinne der Erfindung sind Carbonsäuren mit mehreren Carboxygruppen, insbesondere Di- und Tricarbonsäuren. Die Carboxygruppen können ganz oder teilweise als Ester, Säureanhydrid, Lacton, Amid, Imidsäure, Lactam, Lactim, Dicarboximid, Carbohydrazid, Hydrazon, Hydroxam, Hydroxim, Amidin, Amidoxim, Nitril, Phosphon- oder Phosphatester vorliegen. Die erfindungsgemäßen Carbonsäuren können selbstverständlich entlang der Kohlenstoffkette oder des Ringgerüstes substituiert sein. Zu den Substituenten der erfindungsgemäßen Carbonsäuren sind beispielsweise zu zählen C1-C8-Alkyl-, C2-C8-Alkenyl-, Aryl-, Aralkyl- und Aralkenyl-, Hydroxymethyl-, C2-C8-Hydroxyalkyl-,C2-C8-Hydroxyalkenyl-, Aminomethyl-, C2-C8-Aminoalkyl-, Cyano-, Formyl-, Oxo-, Thioxo-, Hydroxy-, Mercapto-, Amino-, Carboxy- oder Iminogruppen. Bevorzugte Substituenten sind Cl-C8-Alkyl-, Hydroxymethyl-, Hydroxy-, Amino- und Carboxygruppen. Besonders bevorzugt sind Substituenten in □ -Stellung. Ganz besonders bevorzugte Substituenten sind Hydroxy-, Alkoxy- und Aminogruppen, wobei die Aminofunktion gegebenenfalls durch Alkyl-, Aryl-, Aralkyl- und/oder Alkenylreste weiter substituiert sein kann. Weiterhin sind ebenfalls bevorzugte Carbonsäurederivate die Phosphon- und Phosphatester.

Als Beispiele für erfindungsgemäße Carbonsäuren seien genannt Ameisensäure, Essigsäure, Propionsäure, Buttersäure, Isobuttersäure, Valeriansäure, Isovaleriansäure, Pivalinsäure, Oxalsäure, Malonsäure, Bernsteinsäure, Glutarsäure, Glycerinsäure, Glyoxylsäure, Adipinsäure, Pimelinsäure, Korksäure, Azelainsäure, Sebacinsäure, Propiolsäure, Crotonsäure, Isocrotonsäure, Elaidinsäure, Maleinsäure, Fumarsäure, Muconsäure, Citraconsäure, Mesaconsäure, Camphersäure, Benzoesäure, o,m,p-Phthalsäure, Naphthoesäure, Toluoylsäure, Hydratropasäure, Atropasäure, Zimtsäure, Isonicotinsäure, Nicotinsäure, Bicarbaminsäure, 4,4'-Dicyano-6,6'-binicotinsäure, 8-Carbamoyloctansäure, 1,2,4-Pentantricarbonsäure, 2-Pyrrolcarbonsäure, 1,2,4,6,7-Napthalinpentaessigsäure, Malonaldehydsäure, 4-Hydroxyphthalamidsäure, 1-Pyrazolcarbonsäure, Gallussäure oder Propantricarbonsäure, eine Dicarbonsäure ausgewählt aus der Gruppe, die gebildet wird durch Verbindungen der allgemeinen Formel (N-I), 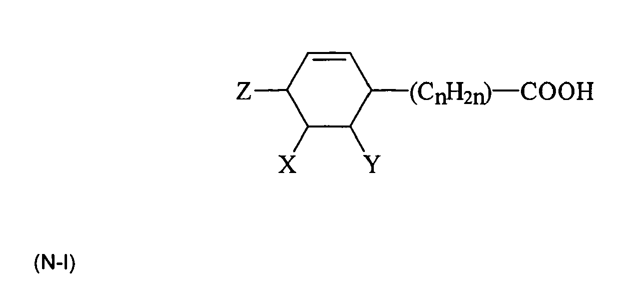 in der Z steht für eine lineare oder verzweigte Alkyl- oder Alkenylgruppe mit 4 bis 12 Kohlenstoffatomen, n für eine Zahl von 4 bis 12 sowie eine der beiden Gruppen X und Y für eine COOH-Gruppe und die andere für Wasserstoff oder einen Methyl- oder Ethylrest, Dicarbonsäuren der allgemeinen Formel (N-1), die zusätzlich noch 1 bis 3 Methyl- oder Ethylsubstituenten am Cyclohexenring tragen sowie Dicarbonsäuren, die aus den Dicarbonsäuren gemäß Formel (N-1) formal durch Anlagerung eines Moleküls Wasser an die Doppelbindung im Cyclohexenring entstehen.

Dicarbonsäuren der Formel (N-I) sind in der Literatur bekannt.

Die Dicarbonsäuren der Formel (N-I) können beispielsweise durch Umsetzung von mehrfach ungesättigten Dicarbonsäuren mit ungesättigten Monocarbonsäuren in Form einer Diels-Alder-Cyclisierung hergestellt werden. Üblicherweise wird man von einer mehrfach ungesättigten Fettsäure als Dicarbonsäurekomponente ausgehen. Bevorzugt ist die aus natürlichen Fetten und Ölen zugängliche Linolsäure. Als Monocarbonsäurekomponente sind insbesondere Acrylsäure, aber auch z.B. Methacrylsäure und Crotonsäure bevorzugt. Üblicherweise entstehen bei Reaktionen nach Diels-Alder Isomerengemische, bei denen eine Komponente im Überschuß vorliegt. Diese Isomerengemische können erfindungsgemäß ebenso wie die reinen Verbindungen eingesetzt werden.

Erfindungsgemäß einsetzbar neben den bevorzugten Dicarbonsäuren gemäß Formel (N-1) sind auch solche Dicarbonsäuren, die sich von den Verbindungen gemäß Formel (N-I) durch 1 bis 3 Methyl- oder Ethyl-Substituenten am Cyclohexylring unterscheiden oder aus diesen Verbindungen formal durch Anlagerung von einem Molekül Wasser an die Doppelbildung des Cyclohexenrings gebildet werden.

Als erfindungsgemäß besonders wirksam hat sich die Dicarbonsäure(-mischung) erwiesen, die durch Umsetzung von Linolsäure mit Acrylsäure entsteht. Es handelt sich dabei um eine Mischung aus 5- und 6-Carboxy-4-hexyl-2-cyclohexen-1-octansäure. Solche Verbindungen sind kommerziell unter den Bezeichnungen Westvaco Diacid^{®} 1550 und Westvaco Diacid^{®} 1595 (Hersteller: Westvaco) erhältlich.

Neben den zuvor beispielhaft aufgeführten erfindungsgemäßen kurzkettigen Carbonsäuren selbst können auch deren physiologisch verträgliche Salze erfindungsgemäß eingesetzt werden. Beispiele für solche Salze sind die Alkali-, Erdalkali-, Zinksalze sowie Ammoniumsalze, worunter im Rahmen der vorliegenden Anmeldung auch die Mono-, Di- und Trimethyl-, -ethyl- und - hydroxyethyl-Ammoniumsalze zu verstehen sind. Ganz besonders bevorzugt können im Rahmen der Erfindung jedoch mit alkalisch reagierenden Aminosäuren, wie beispielsweise Arginin, Lysin, Ornithin und Histidin, neutralisierte Säuren eingesetzt werden. Weiterhin kann es aus Formulierungsgründen bevorzugt sein, die Carbonsäure aus den wasserlöslichen Vertretern, insbesondere den wasserlöslichen Salzen, auszuwählen.

Weiterhin ist es erfindungsgemäß bevorzugt, Hydroxycarbonsäuren und hierbei wiederum insbesondere die Dihydroxy-, Trihydroxy- und Polyhydroxycarbonsäuren sowie die Dihydroxy-, Trihydroxy- und Polyhydroxy- di-, tri- und polycarbonsäuren gemeinsam mit dem Wirkstoff (A) einzusetzen. Hierbei hat sich gezeigt, daß neben den Hydroxycarbonsäuren auch die Hydroxycarbonsäureester sowie die Mischungen aus Hydroxycarbonsäuren und deren Estern als auch polymere Hydroxycarbonsäuren und deren Ester ganz besonders bevorzugt sein können. Bevorzugte Hydroxycarbonsäureester sind beispielsweise Vollester der Glycolsäure, Milchsäure, Äpfelsäure, Weinsäure oder Citronensäure. Weitere grundsätzlich geeigneten Hydroxycarbonsäureester sind Ester der β-Hydroxypropionsäure, der Tartronsäure, der D-Gluconsäure, der Zuckersäure, der Schleimsäure oder der Glucuronsäure. Als Alkoholkomponente dieser Ester eignen sich primäre, lineare oder verzweigte aliphatische Alkohole mit 8 - 22 C-Atomen, also z.B. Fettalkohole oder synthetische Fettalkohole. Dabei sind die Ester von C12-C15-Fettalkoholen besonders bevorzugt. Ester dieses Typs sind im Handel erhältlich, z.B. unter dem Warenzeichen Cosmacol^{®} der EniChem, Augusta Industriale. Besonders bevorzugte Polyhydroxypolycarbonsäuren sind Polymilchsäure und Polyweinsäure sowie deren Ester.

Zusätzlich zu Aniontensid(en) und der/den ionischen Flüssigkeit(en) können die erfindungsgemäßen Mittel weitere Inhaltstoffe enthalten, die üblicherweise in kosmetischen Zusammensetzungen enthalten sind. Die nachfolgend genannten Inhaltsstoffe sind insbesondere in Reinigungs- und Pflegemitteln für die Haut übliche Inhaltsstoffe.

Hier sind insbesondere erfindungsgemäße kosmetische Zusammensetzungen bevorzugt, die mindestens einen weiteren kosmetischen Wirkstoff, ausgewählt aus:
a) Monomeren, Oligomeren und Polymeren von Aminosäuren, N-C₂-C₂₄-Acylaminosäuren, den Estern und/oder den physiologisch verträglichen Metallsalzen dieser Substanzen,
b) DNA- oder RNA-Oligonucleotiden,
c) natürlichen Betainverbindungen,
d) Vitaminen, Provitaminen und Vitaminvorstufen der Gruppen A, B, C, E, H und K und den Estern der vorgenannten Substanzen,
e) α-Hydroxycarbonsäuren, α-Ketocarbonsäuren, β-Hydroxycarbonsäuren und deren Ester-, Lacton- oder Salzform,
f) Flavonoiden und Flavonoid-reichen Pflanzenextrakten,
g) Isoflavonoiden und Isoflavonoid-reichen Pflanzenextrakten,
h) Polyphenolen und Polyphenol-reichen Pflanzenextrakten,
i) Ubichinon und Ubichinol sowie deren Derivaten,
j) Silymarin,
k) Ectoin,
l) Repellentien,
m) anorganischen und organischen UV-Filtersubstanzen,
n) selbstbräunenden Wirkstoffen,
o) hautaufhellenden Wirkstoffen,
p) hautberuhigenden Wirkstoffen,
q) feuchtigkeitsspendenden Wirkstoffen,
r) sebumregulierenden Wirkstoffen,
s) mechanischen Exfoliationsmitteln,
t) antimikrobiellen Wirkstoffen,
u) desodorierenden Wirkstoffen,
v) schweißhemmenden Wirkstoffen,
w) anionischen, kationischen, zwitterionischen, ampholytischen und/oder nichtionischen Tensiden,
x) konditionierenden Wirkstoffen,
y) haarfestigenden oder Haarstyling-Wirkstoffen,
z) Wirkstoffen zur Vorbeugung der Zahnsteinbildung" aa) sowie Mischungen dieser Wirkstoffe a) - z)
enthalten.

In einer ersten bevorzugten Ausführungsform enthalten die erfindungsgemäßen Zusammensetzungen neben der bzw. den ionischen Flüssigkeit(en) mindestens ein Monomer, Oligomer oder Polymer von Aminosäuren, N-C₂-C₂₄-Acylaminosäuren und/oder den Estern und/oder den physiologisch verträglichen Metallsalzen dieser Substanzen.
Die Monomere der Aminosäuren und/oder der N-C₂-C₂₄-Acylaminosäuren sind ausgewählt aus Alanin, Arginin, Asparagin, Asparaginsäure, Canavanin, Citrullin, Cystein, Cystin, Desmosin, Glutamin, Glutaminsäure, Glycin, Histidin, Homophenylalanin, Hydroxylysin, Hydroxyprolin, Isodesmosin, Isoleucin, Leucin, Lysin, Methionin, Methylnorleucin, Ornithin, Phenylalanin, Prolin, Pyroglutaminsäure, Sarcosin, Serin, , Taurin, Threonin, Thyroxin, Tryptophan, Tyrosin, Valin, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat. Besonders bevorzugt sind Lysin, Serin, , Taurin, Zink- und Natriumpyroglutamat und Natriumlauroylglutamat.
Der C₂ - C₂₄-Acylrest, mit dem die genannten Aminosäuren an der Aminogruppe derivatisiert sind, ist ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl- , Cetoyl-, Palmitoyl-, Stearoyl-, Elaidoyl-, Arachidoyl- oder Behenoyl-Rest. Mischungen von C₈-C₁₈-Acylresten werden auch als Cocoyl-Rest bezeichnet und sind ebenfalls bevorzugte Substituenten.
Die physiologisch verträglichen Salze der erfindungsgemäß bevorzugten Wirkstoffe, die Säuregruppen enthalten und Salze bilden können, sind ausgewählt aus den Ammonium-, Alkalimetall-, Magnesium-, Calcium-, Aluminium-, Zink- und Mangan-Salzen. Bevorzugt sind die Natrium-, Kalium-, Magnesium-, Aluminium-, Zink- und Mangan-Salze.
Die Oligomere der Aminosäuren und/oder der N-C₂-C₂₄-Acylaminosäuren sind ausgewählt aus Di- , Tri-, Tetra-, Penta-, Hexa- oder Pentadecapeptiden, die acyliert und/oder verestert sein können. Erfindungsgemäß bevorzugte, gegebenenfalls acylierte und/oder veresterte Dipeptide sind Tyr-Arg (Dipeptide-1), Val-Trp (Dipeptide-2), Asn-Phe, Asp-Phe, N-Palmitoyl-β-Ala-His, N-Acetyl-Tyr-Arg-hexyldecylester (z. B. Calmosensine von Sederma), Carnosin (β-Ala-His) und N-Palmitoyl-Pro-Arg. Erfindungsgemäß bevorzugte, gegebenenfalls acylierte und/oder veresterte Tripeptide sind Lys-Pro-Val, Gly-His-Lys (Tripeptide-1, z. B. Omega-CH-Aktivator von GfN), N-Palmitoyl-Gly-His-Lys, Gly-Lys-His, Tyr-Tyr-Val, Tyr-Val-Tyr, Val-Tyr-Val (Tripeptide-2), Gly-His-Arg (Tripeptide-3), N-Myristoyl-Gly-His-Arg (z. B. Collasyn 314-GR von Therapeutic Peptide Inc.), Tripeptide-4 (z. B. ATPeptide, zu beziehen über IMPAG), His-Ala-Orn, Lys-Phe-Lys, N-Elaidoyl-Lys-Phe-Lys und N-Acetyl-Arg-Lys-Arg-NH₂. Erfindungsgemäß bevorzugte, gegebenenfalls acylierte und/oder veresterte Tetrapeptide sind Val-Val-Arg-Pro, Gly-Gln-Pro-Arg, Gly-Gln-Arg-Pro und N-Palmitoyl-Gly-Gln-Pro-Arg. Erfindungsgemäß bevorzugte, gegebenenfalls acylierte und/oder veresterte Pentapeptide sind Lys-Thr-Thr-Lys-Ser, N-Palmitoyl-Lys-Thr-Thr-Lys-Ser, N-Palmitoyl-Tyr-Gly-Gly-Phe-Met, Val-Val-Arg-Pro-Pro und N-Palmitoyl-Tyr-Gly-Gly-Phe-Leu. Erfindungsgemäß bevorzugte, gegebenenfalls acylierte und/oder veresterte Hexapeptide sind Palmitoyl-Val-Gly-Val-Ala-Pro-Gly (Biopeptide EL von Sederma), Ala-Arg-His-Leu-Phe-Trp (Hexapeptide-1), Acetyl Hexapeptide-1 (z. B. Modulene von Vincience), Acetyl Glutamyl Hexapeptide-1 (z. B. SNAP-7 von Centerchem), Hexapeptide-2 (z. B. Melanostatine-DM von Vincience), Ala-Asp-Leu-Lys-Pro-Thr (Hexapeptide-3, z. B. Peptide 02 von Vincience), Val-Val-Arg-Pro-Pro-Pro, Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Ala-Arg-His-Methylnorleucin-Homophenylalanin-Trp (Hexapeptide-7), Hexapeptide-8 (Z. B. Collasyn 6KS von TPI), Myristoyl Hexapeptide-8 (z. B. Collasyn Lipo-6KS von TPI), Hexapeptide-9 (z. B. Collaxyl von Vincience), Hexapeptide-10 (z. B. Collaxyl von Vincience) und Hexapeptide-11 (z. B. Peptamide-6 von Arch Personal Care). Ein erfindungsgemäß bevorzugtes Pentadecapeptid ist z. B. der Rohstoff Vinci 01 von Vincience (Pentadecapeptide-1).

Es kann erfindungsgemäß besonders bevorzugt sein, ein Gemisch aus mindestens zwei Oligopeptiden einzusetzen. Ein besonders bevorzugtes Gemisch ist die Kombination aus N-Palmitoyl-Gly-His-Lys (z.B. Biopeptide CL von Sederma) und N-Palmitoyl-Gly-Gln-Pro-Arg (z. B. in Eyeliss von Sederma). Eine vorgefertigte Mischung des Tripeptids Palmitoyl-Gly-His-Lys und des Tetrapeptids N-Palmitoyl-Gly-Gln-Pro-Arg ist unter dem Handelsnamen Matrixyl 3000, ebenfalls von Sederma, erhältlich und ist erfindungsgemäß ebenfalls besonders bevorzugt.

Die Polymere der Aminosäuren und/oder der N-C₂-C₂₄-Acylaminosäuren sind ausgewählt aus pflanzlichen und tierischen Proteinhydrolysaten und/oder Proteinen. Tierische Proteinhydrolysate sind z. B. Elastin-, Collagen-, Keratin-, Seiden- und Milcheiweiß-Proteinhydrolysate, die auch in Form von Salzen vorliegen können. Erfindungsgemäß bevorzugt sind pflanzliche Proteinhydrolysate, z. B. Soja-, Weizen-, Mandel-, Erbsen-, Kartoffel- und Reisproteinhydrolysate. Entsprechende Handelsprodukte sind z. B. DiaMin^{®} (Diamalt), Gluadin^{®} (Cognis), Lexein^{®} (Inolex) und Crotein^{®} (Croda). Besonders bevorzugt sind Sojaproteinhydrolysate, z. B. die Handelsprodukte Phytokine von Coletica oder Ridulisse C von Silab. Proteinhydrolysate können naturgemäß auch monomere Aminosäuren und Oligopeptide enthalten; ihre Zusammensetzung ist normalerweise nicht definiert.
Ebenfalls möglich ist der Einsatz von Acylderivaten der Proteinhydrolysate, z. B. in Form ihrer Fettsäure-Kondensationsprodukte. Entsprechende Handelsprodukte sind z. B. Lamepon^{®} (Cognis), Gluadin^{®} (Cognis), Lexein^{®} (Inolex), Crolastin^{®} oder Crotein^{®} (Croda).
Erfindungsgemäß einsetzbar sind auch kationisierte Proteinhydrolysate. Bevorzugt sind kationische Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Weiterhin können die kationischen Proteinhydrolysate auch noch weiter derivatisiert sein. Als typische Beispiele für erfindungsgemäß verwendete kationische Proteinhydrolysate und -derivate sind einige der unter den INCI- Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte aufgeführt: Cocodimonium Hydroxypropyl Hydrolyzed Collagen, Steardimonium Hydroxypropyl Hydrolyzed Collagen, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCI. Ganz besonders bevorzugt sind die kationischen Proteinhydrolysate und -derivate auf pflanzlicher Basis.

In einer weiteren bevorzugten Ausführungsform sind die Polymeren der Aminosäuren ausgewählt aus DNA-Reparaturenzymen.
Erfindungsgemäß bevorzugte DNA-Reparaturenzyme sind Photolyase und T4 Endonuclease V, letztere im weiteren mit "T4N5" abgekürzt. Diese beiden Enzyme sind im Stand der Technik bereits als sogenannte DNA-Reparatur-Enzyme bekannt. Unter DNA-Reparatur ist definitionsgemäß die Spaltung bzw. Entfernung von UV-induzierten Pyrimidindimeren aus der DNA zu verstehen.

Photolyase ist die Kurzbezeichnung für Desoxyribodipyrimidin-Photolyase bzw. DNA-Photolyase, ein Enzym mit der Klassifizierungsnummer EC 4.1.99.3. Eine besonders effiziente Photolyase stammt aus *Anacystis nidulans,* einem phototrophen marinen Mikroorganismus. Die Photolyase aus *A. nidulans* wird in technisch relevanten Mengen mittlerweile aus E. coli gewonnen. Photolyase ist zur Aktivierung auf Licht angewiesen.
Das Enzym T4 Endonuclease V wird vom *den*V-Gen der Bakteriophage T4 produziert und gehört zu den Phosphodiesterasen, die die Nucleinsäuren an der (5'-3')-Bindung hydrolytisch spalten. T4N5 ist auch ohne Lichteinfluss aktiv.
Erfindungsgemäß besonders bevorzugt ist der Einsatz von liposomenverkapselten DNA-Reparaturenzymen. Liposomenverkapselte Photolyase ist im Handel z. B. unter der Produktbezeichnung Photosome^{™}, liposomenverkapselte T4N5 z. B. unter der Bezeichnung Ultrasome^{™} von der Firma AGI Dermatics, USA, erhältlich.
In den erfindungsgemäßen Zusammensetzungen sind die Photosome^{™} oder Ultrasome^{™} in Mengen von 0,1 - 10 Gew.-%, bevorzugt 0,5 - 5,0 Gew.-% und besonders bevorzugt 1,0 - 4,0 Gew.-%, bezogen auf das gesamte Mittel, enthalten.

In den erfindungsgemäßen Zusammensetzungen sind die Monomere, Oligomere oder Polymere von Aminosäuren, N-C₂-C₂₄-Acylaminosäuren und/oder den Estern und/oder den physiologisch verträglichen Metallsalzen dieser Substanzen vorzugsweise in Mengen von 0,01 - 10 Gew.-%, bevorzugt 0,1 - 5 Gew.-% und besonders bevorzugt 0,1 - 3 Gew.-%, jeweils bezogen auf die gesamte Zusammensetzung, enthalten.

In einer weiteren bevorzugten Ausführungsform liegen die Monomeren, Oligomeren und Polymeren von Aminosäuren, N-C₂-C₂₄-Acylaminosäuren, den Estern und/oder den physiologisch verträglichen Metallsalzen dieser Substanzen in geträgerter Form vor, insbesondere aufgetragen auf feinteiligen, pulverförmigen Substraten wie Kieselgel, insbesondere Aerosil-Typen, Talkum, Microsponges, modifizierten Stärken und Stärkederivaten, kristalliner Cellulose, Cellulosepulvern, Lactoglobulinderivaten, Polymerpartikeln aus Nylon, Polyolefinen, Polycarbonaten, Polyurethanen, Polyacrylaten, (Meth)acrylat- oder (Meth)acrylat-Vinyliden-Copolymeren, die vernetzt sein können, Polyestern, Polyamiden, Polystyrolen, Teflon und Siliconen. Ein besonders bevorzugter Rohstoff dieser Art sind die Vegetal Filling Spheres von Coletica.

In einer weiteren bevorzugten Ausführungsform enthalten die erfindungsgemäßen Zusammensetzungen neben der bzw. den ionischen Flüssigkeit(en) mindestens ein DNA-Oligonucleotid oder ein RNA-Oligonucleotid.
Erfindungsgemäß werden unter einem Oligonucleotid Polymerisate aus 2 bis 20, bevorzugt 2 bis 10 Mononucleotiden verstanden, die ebenso wie bei Polynucleotiden und Nucleinsäuren durch Phosphorsäurediester-Brücken verknüpft sind. Die Nucleotide bestehen aus Nucleobasen (meist Pyrimidin- oder Purin-Derivaten), Pentosen (meist D-Ribofuranose oder 2-Desoxy-D-ribofuranose in β-N-glykosidischer Bindung an die Nucleobase) und Phosphorsäure. Die Mononucleotide sind zum Beispiel Adenosinphosphate, Cytidinphosphate, Guanosinphosphate, Uridinphosphate und Thymidinphosphate, insbesondere CMP (Cytidin-5'-monophosphat), UDP (Uridin-5'-diphosphat), ATP (Adenosin-5'-triphosphat) und GTP (Guanosin-5'-triphosphat).
Ein erfindungsgemäß besonders bevorzugtes Oligonucleotid ist das Thymidin-Dinucleotid.
In den erfindungsgemäßen Zusammensetzungen sind die DNA-Oligonucleotide oder RNA-Oligonucleotide vorzugsweise in Mengen von 0,0001 - 5 Gew.-%, bevorzugt 0,001 - 1,0 Gew.-% und besonders bevorzugt 0,01 - 0,5 Gew.-%, bezogen auf das gesamte Mittel, enthalten.

In einer weiteren bevorzugten Ausführungsform enthalten die erfindungsgemäßen Zusammensetzungen neben der bzw. den ionischen Flüssigkeit(en) mindestens eine natürliche Betainverbindung. Erfindungsgemäß eingesetzte natürliche Betainverbindungen sind natürlich vorkommende Verbindungen mit der Atomgruppierung R₃N⁺-CH₂-X-COO⁻ gemäß IUPAC-Regel C-816.1. Sogenannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Erfindungsgemäß bevorzugte Betainverbindungen sind Betain (Me₃N⁺-CH₂-COO⁻) und Carnitin (Me₃N⁺-CH₂-CHOH-CH₂-COO⁻), jeweils mit Me = Methyl.
Die Betainverbindungen sind in den erfindungsgemäßen Mitteln vorzugsweise in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.

In einer weiteren bevorzugten Ausführungsform enthalten die erfindungsgemäßen Zusammensetzungen neben der bzw. den ionischen Flüssigkeit(en) mindestens ein Vitamin, Provitamin oder eine als Vitaminvorstufe bezeichnete Verbindung aus den Vitamingruppen A, B, C, E, H und K und den Estern der vorgenannten Substanzen.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester, wie Retinylpalmitat und Retinylacetat in Betracht. Die erfindungsgemäßen Zubereitungen enthalten die Vitamin A-Komponente bevorzugt in Mengen von 0,05 - 1 Gew.-%, bezogen auf die gesamte Zusammensetzung.

Zur Vitamin B-Gruppe oder zu dem Vitamin B-Komplex gehören unter anderem
- Vitamin B₁, Trivialname Thiamin, chemische Bezeichnung 3-[(4'-Amino-2'-methyl-5'-pyrimidinyl)-methyl]-5-(2-hydroxyethyl)-4-methylthiazoliumchlorid. Bevorzugt wird Thiaminhydrochlorid in Mengen von 0,05 bis 1 Gew.-%, bezogen auf das gesamte Mittel, eingesetzt.
- Vitamin B₂, Trivialname Riboflavin, chemische Bezeichnung 7,8-Dimethyl-10-(1-D-ribityl)-benzo[g]pteridin-2,4(3*H*,10*H*)-dion. Bevorzugt werden Riboflavin oder seine Derivate in Mengen von 0,05 bis 1 Gew.-%, bezogen auf das gesamte Mittel, eingesetzt.
- Vitamin B₃. Unter dieser Bezeichnung werden die Verbindungen Nicotinsäure und Nicotinsäureamid (Niacinamid) geführt. Erfindungsgemäß bevorzugt ist das Nicotinsäureamid, das in den erfindungsgemäßen Mitteln bevorzugt in Mengen von 0,05 bis 1 Gew.-%, bezogen auf das gesamte Mittel, enthalten ist.
- Vitamin B₅ (Pantothensäure und Panthenol). Bevorzugt wird Panthenol eingesetzt. Erfindungsgemäß einsetzbare Derivate des Panthenols sind insbesondere die Ester und Ether des Panthenols sowie kationisch derivatisierte Panthenole. In einer weiteren bevorzugten Ausführungsform der Erfindung können an Stelle von sowie zusätzlich zu Pantothensäure oder Panthenol auch Derivate des 2-Furanon mit der allgemeinen Strukturformel (IV) eingesetzt werden.

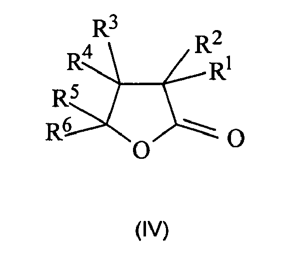

Bevorzugt sind die 2-Furanon-Derivate, in denen die Substituenten R¹ bis R⁶ unabhängig voneinander ein Wasserstoffatom, einen Hydroxylrest, einen Methyl-, Methoxy-, Aminomethyl- oder Hydroxymethylrest, einen gesättigten oder ein- oder zweifach ungesättigten, linearen oder verzweigten C₂-C₄ - Kohlenwasserstoffrest, einen gesättigten oder ein- oder zweifach ungesättigten, verzweigten oder linearen Mono-, Di- oder Trihydroxy-C₂-C₄ - Kohlenwasserstoffrest oder einen gesättigten oder ein- oder zweifach ungesättigten, verzweigten oder linearen Mono-, Di- oder Triamino-C₂-C₄ - Kohlenwasserstoffrest darstellen. Besonders bevorzugte Derivate sind die auch im Handel erhältlichen Substanzen Dihydro-3-hydroxy-4,4-dimethyl-2(3H)-furanon mit dem Trivialnamen Pantolacton (Merck), 4-Hydroxymethyl-γ-butyrolacton (Merck), 3,3-Dimethyl-2-hydroxy-γ-butyrolacton (Aldrich) und 2,5-Dihydro-5-methoxy-2-furanon (Merck), wobei ausdrücklich alle Stereoisomeren eingeschlossen sind. Das erfindungsgemäß außerordentlich bevorzugte 2-Furanon-Derivat ist Pantolacton (Dihydro-3-hydroxy-4,4-dimethyl-2(3H)-furanon), wobei in Formel (IV) R¹ für eine Hydroxylgruppe, R² für ein Wasserstoffatom, R³ und R⁴ für eine Methylgruppe und R⁵ und R⁶ für ein Wasserstoffatom stehen. Das Stereoisomer (R)-Pantolacton entsteht beim Abbau von Pantothensäure.
Die genannten Verbindungen des Vitamin B₅-Typs sowie die 2-Furanonderivate sind in den erfindungsgemäßen Mitteln vorzugsweise in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.
- Vitamin B₆, wobei man hierunter keine einheitliche Substanz, sondern die unter den Trivialnamen Pyridoxin, Pyridoxamin und Pyridoxal bekannten Derivate des 5-Hydroxymethyl-2-methylpyridin-3-ols versteht. Vitamin B₆ ist in den erfindungsgemäßen Mitteln bevorzugt in Mengen von 0,0001 bis 1,0 Gew.-%, insbesondere in Mengen von 0,001 bis 0,01 Gew.-%, enthalten.
- Vitamin B₇ (Biotin), auch als Vitamin H oder "Hautvitamin" bezeichnet. Bei Biotin handelt es sich um (3aS,4S, 6a*R*)-2-Oxohexahydrothienol[3,4-*d*]-imidazol-4-valeriansäure. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Vitamin C (Ascorbinsäure) wird bevorzugt in Mengen von 0,1 bis 3 Gew.-%, bezogen auf die gesamte Zusammensetzung, eingesetzt. Die Verwendung der Derivate Ascorbylpalmitat, -stearat, -dipalmitat, -acetat, Mg-Ascorbylphosphat, Na-Ascorbylphosphat, Natrium- und Magnesiumascorbat, Dinatriumascorbylphosphat und -sulfat, Kaliumascorbyltocopherylphosphat, Chitosanascorbat oder Ascorbylglucosid kann bevorzugt sein. Die Verwendung in Kombination mit Tocopherolen kann ebenfalls bevorzugt sein.
Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat, - succinat, -linoleat, -oleat, Tocophereth-5, Tocophereth-10, Tocophereth-12, Tocophereth-18, Tocophereth-50 und Tocophersolan. Tocopherol und seine Derivate sind bevorzugt in Mengen von 0,05 - 1 Gew.-%, bezogen auf die gesamte Zusammensetzung, enthalten.
Unter Vitamin F werden üblicherweise essentielle Fettsäuren, insbesondere Linolsäure, Linolensäure und Arachidonsäure, verstanden.
Vitamin H ist eine andere Bezeichnung für Biotin oder Vitamin B₇ (siehe oben).
Zu den fettlöslichen Vitaminen der Vitamin K-Gruppe, denen das Grundgerüst des 2-Methyl-1,4-naphthochinons zugrunde liegt, gehören Phyllochinon (Vitamin K₁), Farnochinon oder Menachinon-7 (Vitamin K2) und Menadion (Vitamin K₃). Vitamin K ist bevorzugt in Mengen von 0,0001 bis 1,0 Gew.-%, insbesondere 0,01 bis 0,5 Gew.-%, jeweils bezogen auf die gesamte Zusammensetzung, enthalten.
Vitamin A-palmitat (Retinylpalmitat), Panthenol, Pantolacton, Nicotinsäureamid, Pyridoxin, Pyridoxamin, Pyridoxal, Biotin, Ascorbylpalmitat, -acetat, Mg-Ascorbylphosphat, Na-Ascorbylphosphat, Natrium- und Magnesiumascorbat und die Tocopherolester, besonders Tocopherylacetat, sind erfindungsgemäß besonders bevorzugt.

In einer weiteren bevorzugten Ausführungsform enthalten die erfindungsgemäßen Zusammensetzungen neben der bzw. den ionischen Flüssigkeit(en) mindestens eine α-Hydroxycarbonsäure, α-Ketocarbonsäure oder β-Hydroxycarbonsäure oder deren Ester-, Lacton- oder Salzform. Erfindungsgemäß geeignete α-Hydroxycarbonsäuren oder α-Ketocarbonsäuren sind Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure. Besonders bevorzugte α-Hydroxycarbonsäuren sind Milchsäure, Citronensäure, Glycolsäure und Gluconsäure. Eine besonders bevorzugte β-Hydroxycarbonsäure ist Salicylsäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Die α-Hydroxycarbonsäuren, α-Ketocarbonsäuren oder β-Hydroxycarbonsäuren oder ihre Derivate sind vorzugsweise in Mengen von 0,1 - 10 Gew.-%, bevorzugt 0,5 - 5 Gew.-%, jeweils bezogen auf die gesamte Zusammensetzung, enthalten.

In einer weiteren bevorzugten Ausführungsform enthalten die erfindungsgemäßen Zusammensetzungen neben der bzw. den ionischen Flüssigkeit(en) mindestens ein Flavonoid oder mindestens einen Flavonoid-reichen Pflanzenextrakt.
Die erfindungsgemäß bevorzugten Flavonoide umfassen die Glycoside der Flavone, der Flavanone, der 3-Hydroxyflavone (Flavonole), der Aurone und der Isoflavone. Besonders bevorzugte Flavonoide sind ausgewählt aus Naringin (Aurantiin, Naringenin-7-rhamnoglucosid), α-Glucosylrutin, α-Glucosylmyricetin, α-Glucosylisoquercetin, α-Glucosylquercetin, Hesperidin (3',5,7-Trihydroxy-4'-methoxyflavanon-7-rhamnoglucosid, Hesperitin-7-O-rhamnoglucosid), Neohesperidin, Rutin (3,3',4',5,7-Pentahydroxyflavon-3-rhamnoglucosid, Quercetin-3-rhamnoglucosid), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)-flavon-3-(6-O-(6-deoxy-α-L-mannopyranosyl)-β-D-glucopyranosid)), Diosmin (3',4',7-Trihydroxy-5-methoxyflavanon-7-rhamnoglucosid), Eriodictin und Apigenin-7-glucosid (4',5,7-Trihydroxyflavon-7-glucosid).
Erfindungsgemäß außerordentlich bevorzugte Flavonoide sind α-Glucosylrutin, Naringin und Apigenin-7-glucosid.
Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phloricin und Neohesperidindihydrochalkon.

Erfindungsgemäß bevorzugt werden die Flavonoide in Mengen von 0,0001 bis 1 Gew.-%, bevorzugt 0,0005 bis 0,5 Gew.-% und besonders bevorzugt 0,001 bis 0,1 Gew.-%, jeweils bezogen auf die Flavonoidaktivsubstanz in der gesamten kosmetischen Zusammensetzung, eingesetzt.

In einer weiteren bevorzugten Ausführungsform enthalten die erfindungsgemäßen Zusammensetzungen neben der bzw. den ionischen Flüssigkeit(en) mindestens ein Isoflavonoid oder mindestens einen Isoflavonoid-reichen Pflanzenextrakt. Zu den Isoflavonoiden werden an dieser Stelle die Isoflavone und die Isoflavon-Glycoside gezählt.
Unter Isoflavonen sind im Sinne der vorliegenden Erfindung Stoffe zu verstehen, die Hydrierungs- , Oxidations- oder Substitutionsprodukte des 3-Phenyl-4H-1-benzopyrans darstellen, wobei eine Hydrierung in der 2,3-Stellung des Kohlenstoffgerüsts vorliegen kann, eine Oxidation unter Ausbildung einer Carbonylgruppe in der 4-Stellung vorliegen kann, und unter Substitution der Ersatz eines oder mehrerer Wasserstoffatome durch Hydroxy- oder Methoxy-Gruppen zu verstehen ist. Zu den erfindungsgemäß bevorzugten Isoflavonen zählen beispielsweise Daidzein, Genistein, Prunetin, Biochanin, Orobol, Santal, Pratensein, Irigenin, Glycitein, Biochanin A und Formononetin. Als Isoflavone besonders bevorzugt sind Daidzein, Genistein, Glycitein und Formononetin.
In den erfindungsgemäß bevorzugten Isoflavon-Glycosiden sind die Isoflavone über mindestens eine Hydroxygruppe mit mindestens einem Zucker glycosidisch verknüpft. Als Zucker kommen Mono- oder Oligosaccharide, insbesondere D-Glucose, D-Galactose, D-Glucuronsäure, D-Galacturonsäure, D-Xylose, D-Apiose, L-Rhamnose, L-Arabinose und Rutinose in Betracht. Erfindungsgemäß besonders bevorzugte Isoflavon-Glycoside sind Daidzin und Genistin.
Erfindungsgemäß weiterhin bevorzugt ist es, wenn die Isoflavone und/oder deren Glycoside als Bestandteile eines aus einer Pflanze gewonnenen Substanzgemisches, insbesondere eines pflanzlichen Extraktes, in den Zubereitungen enthalten sind. Solche pflanzlichen Substanzgemische können in dem Fachmann geläufiger Weise beispielsweise durch Auspressen oder Extrahieren aus Pflanzen wie Soja, insbesondere aus den Sojakeimen, Rotklee oder Kichererbsen gewonnen werden. Besonders bevorzugt werden in den erfindungsgemäßen Zubereitungen Isoflavone oder Isoflavon-Glycoside in Form von aus Soja gewonnenen Extrakten eingesetzt, wie sie beispielsweise unter der Produktbezeichnung Soy Protein Isolate SPI (Protein Technology International, St. Louis) oder Soy Phytochemicals Concentrate SPC (Archer Daniels Midland, Decatur) im Handel erhältlich sind. Ein weiterer besonders bevorzugter Isoflavonoidreicher Pflanzenextrakt ist Apfelkernextrakt, insbesondere das Handelsprodukt Ederline von Seporga. Ederline enthält Phytohormone, Isoflavonoide, Phytosterole, Triterpenoide, Tocopherole und natürliche Wachse.

Erfindungsgemäß werden die Isoflavonoide vorzugsweise in Mengen von 0,00001 bis 1 Gew.-%, bevorzugt 0,0005 bis 0,5 Gew.-% und besonders bevorzugt 0,001 bis 0,1 Gew.-%, jeweils bezogen auf die Isoflavonoidaktivsubstanz in der gesamten kosmetischen Zusammensetzung, eingesetzt.

In einer weiteren bevorzugten Ausführungsform enthalten die erfindungsgemäßen Zusammensetzungen neben der bzw. den ionischen Flüssigkeit(en) mindestens ein Polyphenol oder einen Polyphenol-reichen Pflanzenextrakt.
Unter Polyphenolen sind erfindungsgemäß aromatische Verbindungen zu verstehen, die mindestens zwei phenolische Hydroxy-Gruppen im Molekül enthalten. Hierzu zählen die drei Dihydroxybenzole Brenzcatechin, Resorcin und Hydrochinon, weiterhin Phloroglucin, Pyrogallol und Hexahydroxybenzol. In der Natur treten freie und veretherte Polyphenole beispielsweise in Blütenfarbstoffen (Anthocyanidine, Flavone), in Gerbstoffen (Catechine, Tannine), als Flechten- oder Farn-Inhaltsstoffe (Usninsäure, Acylpolyphenole), in Ligninen und als Gallussäure-Derivate auf. Bevorzugte Polyphenole sind Flavone, Catechine, Usninsäure, und als Tannine die Derivate der Gallussäure, Digallussäure und Digalloylgallussäure. Besonders bevorzugte Polyphenole sind die monomeren Catechine, das heißt die Derivate der Flavan-3-ole, und Leukoanthocyanidine, das heißt die Derivate der Leukoanthocyanidine, die bevorzugt in 5,7,3',4',5'-Stellung phenolische Hydroxygruppen tragen, bevorzugt Epicatechin und Epigallocatechin, sowie die daraus durch Selbstkondensation entstehenden Gerbstoffe. Solche Gerbstoffe werden bevorzugt nicht in isolierter Reinsubstanz, sondern als Extrakte gerbstoffreicher Pflanzenteile eingesetzt, z. B. Extrakte von Catechu, Quebracho, Eichenrinde und Pinienrinde sowie anderen Baumrinden, Blättern von Grünem Tee (camellia sinensis) und Mate. Ebenfalls besonders bevorzugt sind die Tannine.
Ein besonders bevorzugter Polyphenol-reicher kosmetischer Wirkstoff ist das Handelsprodukt Sepivinol R, ein Extrakt aus Rotwein, erhältlich von der Firma Seppic. Ein weiterer besonders bevorzugter Polyphenol-reicher kosmetischer Wirkstoff ist das Handelsprodukt Crodarom Chardonnay, ein Extrakt aus den Kernen der Chardonnay-Traube, erhältlich von der Firma Croda. Erfindungsgemäß werden die Polyphenole vorzugsweise in Mengen von 0,001 bis 10 Gew.-%, bevorzugt 0,005 bis 5 Gew.-% und besonders bevorzugt 0,01 bis 3 Gew.-%, jeweils bezogen auf die gesamte kosmetische Zusammensetzung, eingesetzt.

In einer weiteren bevorzugten Ausführungsform enthalten die erfindungsgemäßen Zusammensetzungen neben der bzw. den ionischen Flüssigkeit(en) mindestens ein Ubichinon oder ein Ubichinol oder deren Derivate. Ubichinole sind die reduzierte Form der Ubichinone. Die erfindungsgemäß bevorzugten Ubichinone weisen die Formel (II) auf: 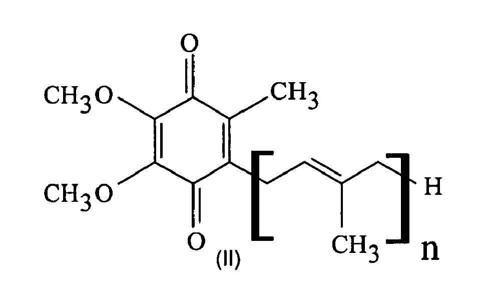 mit n = 6, 7, 8, 9 oder 10.
Besonders bevorzugt ist das Ubichinon der Formel (II) mit n = 10, auch bekannt als Coenzym Q10.
Erfindungsgemäß werden die Ubichinone, Ubichinole oder deren Derivate vorzugsweise in Mengen von 0,0001 bis 1 Gew.-%, bevorzugt 0,001 bis 0,5 Gew.-% und besonders bevorzugt 0,005 bis 0,1 Gew.-%, jeweils bezogen auf die gesamte Zusammensetzung, eingesetzt.

In einer weiteren bevorzugten Ausführungsform enthalten die erfindungsgemäßen Zusammensetzungen neben der bzw. den ionischen Flüssigkeit(en) Silymarin. Silymarin stellt erfindungsgemäß ein früher als einheitliche Substanz angesehenes Wirkstoff-Konzentrat aus den Früchten der Mariendistel (Silybum marianum) dar. Die Hauptbestandteile des Silymarins sind Silybin (Silymarin I), Silychristin (Silymarin II) und Silydianin, die zur Gruppe der Flavanolignane gehören.
Erfindungsgemäß wird Silymarin vorzugsweise in Mengen von 0,0001 bis 1 Gew.-%, bevorzugt 0,001 bis 0,5 Gew.-% und besonders bevorzugt 0,005 bis 0,1 Gew.-%, jeweils bezogen auf die gesamte Zusammensetzung, eingesetzt.

In einer weiteren bevorzugten Ausführungsform enthalten die erfindungsgemäßen Zusammensetzungen neben der bzw. den ionischen Flüssigkeit(en) Ectoin. Ectoin ist der Trivialname für 2-Methyl-1,4,5,6-tetrahydropyrimidin-4-carboxylat. Erfindungsgemäß ist Ectoin vorzugsweise in Mengen von 0,0001 bis 1 Gew.-%, bevorzugt 0,001 bis 0,5 Gew.-% und besonders bevorzugt 0,005 bis 0,01 Gew.-%, jeweils bezogen auf die gesamte Zusammensetzung, enthalten.

In einer weiteren bevorzugten Ausführungsform enthalten die erfindungsgemäßen Zusammensetzungen neben der bzw. den ionischen Flüssigkeit(en) mindestens ein Repellent.
Von den heute in Insektenabwehrmitteln ca. 15 häufig eingesetzten Wirkstoffen wird das N,N-Diethyl-3-methylbenzamid (DEET) als bestes Allround-Repellent bezeichnet. Es wirkt abwehrend gegen Stechmücken, Bremsen, Sandfliegen, Zecken, Stechfliegen, Milben, Flöhe und Wanzen,
wobei die Wirkungsdauer - wie bei allen Repellent-Wirkstoffen - unterschiedlich lang gegenüber den verschiedenen Spezies ist. Handelsübliche DEET-Präparate beispielsweise sind ca. 6 bis 8 Stunden gegen Mücken wirksam, jedoch nur ca. 2 bis 4 Stunden gegen Zecken. Ein weiterer gebräuchlicher Repellent Wirkstoff ist der 3-(N-n-Butyl-N-acetyl-amino)propionsäureethylester (auch als Repellent 3535 bezeichnet). Repellent 3535 ist gegen Stechmücken (Aedes aegypti, Anopheles albimanus), Tsetsefliegen (Glossinae) und Bremsen (Tabanidae) wirksam. Ferner gebräuchlich ist Dimethylphthalat (Palatinol M, DMP), das gegen Stechmücken (insbesondere Aedes- und Anopheles-Arten), Läuse, Zecken und Milben wirksam ist, allerdings vorwiegend in Kombination mit weiteren Repellent-Wirkstoffen eingesetzt wird.

In einer weiteren bevorzugten Ausführungsform enthalten die erfindungsgemäßen Zusammensetzungen neben der bzw. den ionischen Flüssigkeit(en) mindestens eine anorganische und/oder mindestens eine organische UV-Filtersubstanz.
Bei den UV-Filtersubstanzen handelt es sich um bei Raumtemperatur flüssig oder kristallin vorliegende Substanzen, die in der Lage sind, ultraviolette Strahlen zu absorbieren und die aufgenommene Energie in Form längerwelliger Strahlung, z. B. Wärme wieder abzugeben. Man unterscheidet UVA-Filter und UVB-Filter. Die UVA- und UVB-Filter können sowohl einzeln als auch in Mischungen eingesetzt werden. Der Einsatz von Filtermischungen ist erfindungsgemäß bevorzugt.
Die erfindungsgemäß verwendeten organischen UV-Filter sind ausgewählt aus den Derivaten von Dibenzoylmethan, Zimtsäureestern, Diphenylacrylsäureestern, Benzophenon, Campher, p-Aminobenzoesäureestern, o-Aminobenzoesäureestern, Salicylsäureestern, Benzimidazolen, symmetrisch oder unsymmetrisch substituierten 1,3,5-Triazinen, monomeren und oligomeren 4,4-Diarylbutadiencarbonsäureestern und -carbonsäureamiden, Ketotricyclo(5.2.1.0)decan, Benzalmalonsäureestern, Benzoxazol sowie beliebigen Mischungen der genannten Komponenten. Die organischen UV-Filter können öllöslich oder wasserlöslich sein. Die Benzoxazol-Derivate liegen vorteilhaft in gelöster Form in den erfindungsgemäßen kosmetischen Zubereitungen vor. Es kann ggf. aber auch von Vorteil sein, wenn die Benzoxazol-Derivate in pigmentärer, d. h. ungelöster Form - beispielsweise in Partikelgrößen von 10 nm bis zu 300 nm - vorliegen. Erfindungsgemäß besonders bevorzugte öllösliche UV-Filter sind 1-(4-tert.-Butylphenyl)-3-(4'-methoxyphenyl)propan-1,3-dion (Parsol^{®} 1789), 1-Phenyl-3-(4'-isopropylphenyl)-propan-1,3-dion, 3-(4'-Methylbenzyliden)-D,L-campher, 4-(Dimethylamino)-benzoesäure-2-ethylhexylester, 4-(Dimethylamino)benzoesäure-2-octylester, 4-(Dimethylamino)-benzoesäureamylester, 4-Methoxyzimtsäure-2-ethylhexylester, 4-Methoxyzimtsäurepropylester, 4-Methoxyzimtsäureisopentylester, 2-Cyano-3,3-phenylzimtsäure-2-ethylhexylester (Octocrylene), Salicylsäure-2-ethylhexylester, Salicylsäure-4-isopropylbenzylester, Salicylsäurehomomenthylester (3,3,5-Trimethyl-cyclohexylsalicylat), 2-Hydroxy-4-methoxybenzophenon, 2-Hydroxy-4-methoxy-4'-methylbenzophenon, 2,2'-Dihydroxy-4-methoxybenzophenon, 2-(4'-Diethylamino-2'-hydroxybenzoyl)-benzoesäurehexylester (auch: Aminobenzophenon, unter der Bezeichnung Uvinul A Plus bei der Firma BASF erhältlich), 4-Methoxybenzmalonsäuredi-2-ethylhexylester, an Polymere gebundene UV-Filter, z. B. das 3-(4-(2,2-Bis-Ethoxycarbonylvinyl)-phenoxy)propenyl)-methoxysiloxan/ Dimethylsiloxan-Copolymer mit der INCI-Bezeichnung Dimethicodiethylbenzal malonate (CAS-Nr. 207574-74-1, Parsol^{®} SLX), Triazinderivate, wie z. B. 2,4-Bis-{[4-(2-Ethylhexyloxy)-2-hydroxy]-phenyl}-6-(4-methoxyphenyl)-1,3,5-triazin (INCI: Bis-Ethylhexyloxyphenol Methoxyphenyl Triazin, unter dem Namen Tinosorb S bei CIBA erhältlich), Dioctylbutylamidotriazon (INCI: Diethylhexyl Butamido Triazone, unter dem Namen Uvasorb^{®} HEB bei Sigma 3V erhältlich), 2,4,6-Trianilino-(p-carbo-2'-ethyl-1'-hexyloxy)-1,3,5-triazin (Ethylhexyl Triazone, Uvinul^{®} T 150), 2-[4,6-Bis(2,4-dimethylphenyl)-1,3,5-triazin-2-yl]-5-(octyloxy)phenol (CAS Nr.: 2725-22-6), 2,4-bis-[5-1(di-methylpropyl)benzoxazol-2-yl-(4-phenyl)-imino]-6-(2-ethylhexyl)-imino-1,3,5-triazin (CAS Nr. 288254-16-0, Uvasorb^{®} K2A von 3V Sigma), die Benzotriazolderivate 2,2'-Methylen-bis-(6-(2H-benzotriazol-2-yl)-4-(1,1,3,3-tetramethylbutyl)-phenol) [Tinosorb M (Ciba)], 2,2'-Methyl-bis-[6(2H-benzotriazol-2-yl)-4-(methyl)phenol] (MIXXIM BB/200 der Firma Fairmount Chemical), 2-(2'-Hydroxy-3',5'-di-t-amylphenyl)benzotriazol (CAS- Nr.: 025973-551), 2-(2'-Hydroxy-5'-octylphenyl)-benzotriazol (CAS-Nr. 003147-75-9), 2-(2'-Hydroxy-5'-methylphenyl)benzotriazol (CAS-Nr. 2440-22-4), 2-(2H-benzotriazol-2-yl)-4-methyl-6-[2-methyl-3-[1,3,3,3-tetramethyl-1-((trimethylsilyl)oxy]disiloxanyl)propyl]-phenol (CAS-Nr.: 155633-54-8) mit der INCI-Bezeichnung Drometrizole Trisiloxane, 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin oder auch Aniso Triazin, erhältlich als Tinosorb^{®} S von CIBA), 2,4-Bis-{[4-(3-sulfonato)-2-hydroxypropyloxy)-2-hydroxy]-phenyl}-6-(4-methoxyphenyl)-1,3,5-triazin-Natriumsalz, 2,4-Bis-{[4-(3-(2-propyloxy)-2-hydroxy-propyloxy)-2-hydroxy]-phenyl}-6-(4-methoxyphenyl)-1,3,5-triazin, 2,4-Bis-{(4-(2-ethyl-hexyloxy)-2-hydroxy]-phenyl}-6-[4-(2-methoxyethylcarboxyl)-phenylamino]-1,3,5-triazin, 2,4-Bis-{[4-(3-(2- propyloxy)-2-hydroxy-propyloxy)-2-hydroxy]-phenyl}-6-[4-(ethylcarboxyl)-phenylamino]-1,3,5-triazin, 2,4-Bis-{[4-(2-ethylhexyloxy)-2-hydroxy]-phenyl}-6-(1-methyl-pyrrol-2-yl)-1,3,5-triazin, 2,4-Bis- {[4-tris(trimethylsiloxy-silylpropyloxy)-2-hydroxy]-phenyl}-6-(4-methoxyphenyl)-1,3,5-triazin, 2,4-Bis-{[4-(2-methylpropenyloxy)-2-hydroxy]-phenyl}-6-(4-methoxyphenyl)-1,3,5-triazin, 2,4-Bis-{[4-(1',1',1',3',5',5',5'-Heptamethylsiloxy-2-methyl-propyloxy)-2-hydroxy]-phenyl}-6-(4-methoxyphenyl)-1,3,5-triazin sowie Mischungen der genannten Komponenten.

Bevorzugte wasserlösliche UV-Filter sind 2-Phenylbenzimidazol-5-sulfonsäure, Phenylen-1,4-bis-(2-benzimidazyl)-3,3'-5,5'-tetrasulfonsäure und deren Alkali-, Erdalkali-, Ammonium-, Alkylammonium-, Alkanolammonium- und Glucammoniumsalze, insbesondere die Sulfonsäure selbst mit der INCI Bezeichnung Phenylbenzimidazole Sulfonic Acid (CAS.-Nr. 27503-81-7), die beispielsweise unter dem Handelsnamen Eusolex 232 bei Merck oder unter Neo Heliopan Hydro bei Symrise erhältlich ist, und das Phenylen-1,4-bis-(2-benzimidazyl)-3,3'-5,5'-tetrasulfonsäure-bis-natriumsalz mit der INCI-Bezeichnung Disodium Phenyl Dibenzimidazol Tetrasulfonate (CAS-Nr.: 180898-37-7), das beispielsweise unter dem Handelsnamen Neo Heliopan AP bei Symrise erhältlich ist, Sulfonsäurederivate von Benzophenonen, vorzugsweise 2-Hydroxy-4-methoxybenzophenon-5-sulfonsäure und ihre Salze, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)-sulfonsäure und deren Salze mit der INCI-Bezeichnung Terephthalydene Dicampher Sulfonic Acid (CAS.-Nr.: 90457-82-2, als Mexoryl SX von der Firma Chimex erhältlich).

Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4'methoxyphenyl)propan-1,3-dion (z. B. Parsol^{®} 1789) in verschiedenen UV-B-Filtern herstellen. Die erfindungsgemäßen Zusammensetzungen enthalten daher in einer weiteren bevorzugten Ausführungsform 1-(4-tert.-Butylphenyl)-3-(4'-methoxyphenyl)propan-1,3-dion in Kombination mit mindestens einem UV-B-Filter, ausgewählt aus 4-Methoxyzimtsäure-2-ethylhexylester, 2-Cyano-3,3-phenylzimtsäure-2-ethylhexylester, Salicylsäure-2-ethylhexylester und 3,3,5-Trimethyl-cyclohexylsalicylat. In diesen Kombinationen liegt das Gewichtsverhältnis von UV-B-Filter zu 1-(4-tert.-Butylphenyl)-3-(4'methoxyphenyl)propan-1,3-dion zwischen 1:1 und 10:1, bevorzugt zwischen 2:1 und 8:1, das molare Verhältnis liegt entsprechend zwischen 0,3 und 3,8, bevorzugt zwischen 0,7 und 3,0.

Bei den erfindungsgemäß bevorzugten anorganischen Lichtschutzpigmenten handelt es sich um feindisperse oder kolloiddisperse Metalloxide und Metallsalze, beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen, so genannte Nanopigmente. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z. B. Titandioxid T 805 (Degussa) oder Eusolex^{®} T2000 (Merck). Als hydrophobe Coatingmittel kommen dabei vor allem Silicone und dabei speziell Trialkoxyoctylsilane oder Simethicone in Frage. Besonders bevorzugt sind Titandioxid und Zinkoxid.

Erfindungsgemäß sind die organischen UV-Filtersubstanzen vorzugsweise in Mengen von 0,1 - 30 Gew.-%, bevorzugt 0,5 - 20 Gew.-%, besonders bevorzugt 1,0 - 15 Gew.-% und außerordentlich bevorzugt 3,0 - 10 Gew.-%, jeweils bezogen auf die gesamte Zusammensetzung, enthalten.
Erfindungsgemäß sind die anorganischen UV-Filtersubstanzen vorzugsweise in Mengen von 0,1 - 15 Gew.-%, bevorzugt 0,5 - 10 Gew.-%, besonders bevorzugt 1,0 - 5 Gew.-% und außerordentlich bevorzugt 2,0 - 4,0 Gew.-%, jeweils bezogen auf die gesamte Zusammensetzung, enthalten.

In einer weiteren bevorzugten Ausführungsform enthalten die erfindungsgemäßen Zusammensetzungen neben der bzw. den ionischen Flüssigkeit(en) mindestens einen selbstbräunenden Wirkstoff. Prinzipiell sind hierbei hinsichtlich der Wirkungsweise ein physikalischer und ein chemischer Mechanismus der künstlichen Hautbräunung zu unterscheiden. Walnußschalen-Extrakte und Henna (Wirkstoffe Hydrojuglon, Juglon, Lawson) sowie Tanninsäure und das synthetische Alloxan gehen physikalische Bindungen mit der Hautoberfläche ein, während Dihydroxyaceton und Erythrulose, die hauptsächlich verwendet werden, oderα,β-ungesättigte Aldehyde als aktivierte Carbonyl-Verbindungen mit den Aminosäuren der Haut im Sinne einer Maillard-Reaktion zu gefärbten Produkten abreagieren. *trans-trans*-Mucondialdehyd liefert eine der natürlichen Hautbräunung sehr ähnliche Farbe, wird aber aufgrund toxikologischer Überlegungen selten eingesetzt.

Die erfindungsgemäß bevorzugten Bräunungswirkstoffe werden nachstehend beschrieben.

Als Bräunungswirkstoff können die erfindungsgemäßen Zusammensetzungen Dihydroxyaceton enthalten. Dihydroxyaceton (Abkürzung: DHA, 1,3-Dihydroxypropan-2-on, Ketotriose, INCl-Bez.: Dihydroxyacetone, EINECS 2024945) läßt sich durch die Formel

HO-CH₂-C(O)-CH₂-OH

beschreiben. In der Kosmetik-Industrie wird Dihydroxyaceton wird zur Herst. Von Hautbräunungsmitteln verwendet, meist in Kombination mit hautpflegenden und UV-Schutzbestandteilen. Dem Pigmentierungsmechanismus liegt die Reaktion einer aktivierten Carbonyl-Gruppe mit den Aminosäuren der Haut im Sinne einer Maillrad-Reaktion zugrunde.

Erfindungsgemäß bevorzugte kosmetische Zusammensetzungen sind dadurch gekennzeichnet, daß sie zusätzlich Dihydroxyaceton, vorzugsweise in Mengen von 0,5 bis 10,0 Gew.-%, besonders bevorzugt von 0,75 bis 7,5 Gew.-% und insbesondere von 1,0 bis 5,0 Gew.-%, jeweils bezogen auf die gesamte Zusammensetzung, enthalten.

Als weiteren Bräunungswirkstoff können die erfindungsgemäßen Zusammensetzungen auch Erythrulose enthalten. Erythrulose (1,2,3,4-Trihydroxybutan-2-on) ist ein chiraler Ketozucker, der sich von der Aldose Erythrose ableitet. Er wird in kosmetischen Mitteln aufgrund seiner selbstbräunenden Eigenschaften verwendet. Erythrulose wird aus 1,2,3,4-Butantetrol biotechnologisch durch das Bakterium *Gluconobacter oxydans* hergestellt und für diese Zwecke biotechnologisch gewonnen.

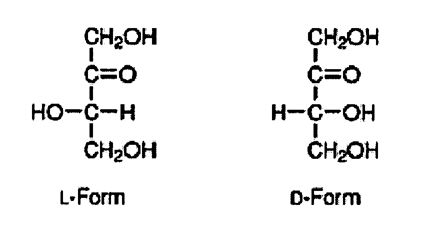

Erythrulose reagiert mit freien primären oder sekundären Amino-Gruppen in der sogenannten Maillrad-Reaktion. Findet diese Reaktion mit den Aminosäuren des Keratins in der Haut statt, so führt dies zur Bildung bräunlicher Polymere, der Melanoide. Hierdurch erscheint die Haut gebräunt. Im Vergleich zu Dihydroxyaceton erfolgt die Maillard-Reaktion mit Erythrulose langsamer. Die Hautbräunung entsteht später, wird aber gleichzeitig gleichmäßiger als beim Dihydroxyaceton, da sich Erythrulose gleichmäßiger im Stratum corneum verteilen kann.

Erfindungsgemäß bevorzugte kosmetische Zusammensetzungen sind dadurch gekennzeichnet, daß sie zusätzlich Erythrulose, vorzugsweise in Mengen von 0,1 bis 7,0 Gew.-%, besonders bevorzugt von 0,25 bis 5,0 Gew.-% und insbesondere von 0,5 bis 2,5 Gew.-%, jeweils bezogen auf die gesamte Zusammensetzung, enthalten.

Erfindungsgemäß sind die selbstbräunenden Wirkstoffe vorzugsweise in Mengen von 0,1 - 15 Gew.-%, bevorzugt 0,5 - 10 Gew.-%, besonders bevorzugt 1,0 - 5 Gew.-% und außerordentlich bevorzugt 2,0 - 4,0 Gew.-%, jeweils bezogen auf die gesamte Zusammensetzung, enthalten.

In einer weiteren bevorzugten Ausführungsform enthalten die erfindungsgemäßen Zusammensetzungen neben der bzw. den ionischen Flüssigkeit(en) mindestens einen hautaufhellenden Wirkstoff. Erfindungsgemäß bevorzugte hautaufhellende Wirkstoffe sind ausgewählt aus Ascorbinsäure, den Estern der Ascorbinsäure mit Phosphorsäure und/ oder organischen C₂-C₂₀-Carbonsäuren sowie deren Alkali- und Erdalkalimetallsalzen, Kojisäure, Hydrochinon, Arbutin, Maulbeerbaumextrakt und Süßholzextrakt sowie Mischungen hiervon. Sowohl als Einzelsubstanz wie auch in Mischung bevorzugt sind die Ascorbinsäurederivate sowie Kojisäure bevorzugt.

Besonders bevorzugt sind Natriumascorbylphosphat, Magnesiumascorbylphosphat, Ascorbylmonopalmitat, Ascorbyldipalmitat, Ascorbylmonostearat, Ascorbyldistearat, Ascorbylmonoethylhexanoat, Ascorbyldiethylhexanoat, Ascorbylmonooctanoat, Ascorbyldioctanoat, Ascorbylmonoisostearat und Ascorbyldiisostearat. Die erfindungsgemäß außerordentlich bevorzugten Ascorbinsäurederivate sind Natriumascorbylphosphat und Magnesiumascorbylphosphat.
Die hautaufhellenden Wirkstoffe sind vorzugsweise in einer Menge von 0,05 bis 5 Gew.-%, bevorzugt von 0,1 - 2 Gew-%, jeweils bezogen auf die gesamte Zusammensetzung, enthalten.

In einer weiteren bevorzugten Ausführungsform enthalten die erfindungsgemäßen Zusammensetzungen neben der bzw. den ionischen Flüssigkeit(en) mindestens einen hautberuhigenden Wirkstoff. Erfindungsgemäß bevorzugte hautberuhigende Wirkstoffe sind ausgewählt aus Allantoin, α-Bisabolol und α-Liponsäure.
Die hautberuhigenden Wirkstoffe sind vorzugsweise in Mengen von 0,001 bis 5 Gew.-%, bevorzugt 0,01 bis 2 Gew.-% und besonders bevorzugt 0,1 bis 1 Gew.-%, jeweils bezogen auf die gesamte Zusammensetzung, enthalten.

In einer weiteren bevorzugten Ausführungsform enthalten die erfindungsgemäßen Zusammensetzungen neben der bzw. den ionischen Flüssigkeit(en) mindestens einen feuchtigkeitsspendenden Wirkstoff. Erfindungsgemäß bevorzugte feuchtigkeitsspendende Wirkstoffe sind ausgewählt aus Desoxyzuckern, besonders bevorzugt Rhamnose und Fucose, Polysacchariden, die mindestens einen Desoxyzucker-Baustein enthalten, besonders bevorzugt das Handelsprodukt Fucogel^{®}, Harnstoff, (2-Hydroxyethyl)harnstoff, Glycosaminoglycanen, besonders bevorzugt Hyaluronsäure, Dextran, Dextransulfat, Chondroitin-4-sulfat und Chondroitin-6-sulfat.
Die feuchtigkeitsspendenden Wirkstoffe sind vorzugsweise in Mengen von 0,001 bis 5 Gew.-%, bevorzugt 0,01 bis 2 Gew.-% und besonders bevorzugt 0,1 bis 1 Gew.-%, jeweils bezogen auf die gesamte Zusammensetzung, enthalten.

In einer weiteren bevorzugten Ausführungsform enthalten die erfindungsgemäßen Zusammensetzungen neben der bzw. den ionischen Flüssigkeit(en) mindestens einen sebumregulierenden Wirkstoff. Erfindungsgemäß bevorzugte sebumregulierende Wirkstoffe sind ausgewählt aus Azelainsäure, Sebacinsäure, 10-Hydroxydecansäure, 1,10-Decandiol, die als erfindungsgemäß besonders bevorzugte Dreierkombination in dem Handelsprodukt Acnacidol PG von Vincience eingesetzt werden, weiterhin aus dem Handelsprodukt Azeloglicina (Potassium Azeloyl Diglycinate) von Sinerga, Extrakten aus Spiraea Ulmaria, wie sie z. B. im Produkt Seboregul der Firma Silab enthalten sind, weiterhin aus wasser- und öllöslichen Extrakten aus Hamamelis, Klettenwurzel und Brennessel, Zimtbaumextract (z. B. Sepicontrol^{®} A5 von der Firma Seppic), Chrysanthemenextrakt (z. B. Laricyl^{®} von Laboratoires Sérobiologiques) und den Wirkstoffmischungen Asebiol^{®} BT 2 (Laboratoires Sérobiologiques, INCI: Aqua, Hydrolyzed Yeast Protein, Pyridoxine, Niacinamide, Glycerin, Panthenol, , Allantoin, Biotin) und Antifettfaktor^{®} COS-218/2-A (Cosmetochem, INCI: Aqua, Cetyl-PCA, PEG-8 Isolauryl Thioether, PCA, Cetyl Alcohol).
Die sebumregulierenden Wirkstoffe sind vorzugsweise in Mengen von 0,001 bis 5 Gew.-%, bevorzugt 0,01 bis 2 Gew.-% und besonders bevorzugt 0,1 bis 1 Gew.-%, jeweils bezogen auf die gesamte Zusammensetzung, enthalten.

In einer weiteren bevorzugten Ausführungsform enthalten die erfindungsgemäßen Zusammensetzungen neben der bzw. den ionischen Flüssigkeit(en) mindestens ein mechanisches Exfoliationsmittel bzw. Peelingkörper. Das kosmetische Hautpeeling zur porentiefen Reinigung der Haut kann vom Anwender gelegentlich als unangenehm empfunden werden. Eine Kombination mit Wirkstoffen, die die β-Endorphinausschüttung stimulieren, kann nicht nur das Anwendungserlebnis angenehmer gestalten, sondern auch die Hautverträglichkeit verbessern.
Erfindungsgemäß bevorzugte mechanische Exfoliationsmittel bzw. Peelingkörper sind ausgewählt aus gemahlenen Pflanzenteilen wie Mandelkleie oder Weizenkleie, kristalliner Cellulose, gehärtetem Jojobaöl (Jojobabeads), Polymerkügelchen, bevorzugt aus Polyethylen oder Polyamid-11, mit mittleren Durchmessern von 90 - 600 µm und aus wirkstoffhaltigen Mikro- oder Millikapseln, die petrochemische Polymere (z. B. aus Polyamid wie Nylon-11) und/oder Biopolymere wie Gelatine, Pektin, pflanzlichen Gummen, Alginaten und Carrageenan enthalten. Bevorzugt als Peelingsubstanzen eingesetzt werden Mandelkleie, Weizenkleie, gehärtetes Jojobaöl und Polymerkügelchen, insbesondere Polyethylenkügelchen. Ebenfalls bevorzugt sind wirkstoffhaltige Mikro- oder Millikapseln. Die handelsüblichen Kapseln liegen häufig als wässrige Polymer-Dispersion vor, beispielsweise die besonders bevorzugten Millicapsules^{®} der Firma Lipotec SA (INCI-Bezeichnung: Aqua, Tocopheryl Acetate, Glycerine, Carbomer, Sebacic Acid, Agar, Green Colourant, Alginic Acid).

In einer weiteren bevorzugten Ausführungsform enthalten die erfindungsgemäßen Zusammensetzungen neben der bzw. den ionischen Flüssigkeit(en) mindestens einen desodorierenden und/oder einen schweißhemmenden Wirkstoff. Deodorantien und Antitranspirantien werden überwiegend im Bereich der Achselhöhlen angewendet. Die Haut im Unterarmbereich ist dünner und damit empfindlicher als die Gesichtshaut, daher kann die Applikation von Deodorantien und Antitranspirantien, insbesondere bei regelmäßiger Anwendung, als unangenehm empfunden werden oder sogar zu Hautirritationen führen. Eine Kombination mit Wirkstoffen, die die β-Endorphinausschüttung stimulieren, kann nicht nur das Anwendungserlebnis und das Hautgefühl angenehmer gestalten, sondern auch die Hautverträglichkeit verbessern.

Erfindungsgemäß bevorzugte desodorierende Wirkstoffe sind ausgewählt aus Geruchsabsorbern, desodorierend wirkenden lonenaustauschern, keimhemmenden Mitteln, präbiotisch wirksamen Komponenten sowie Enzyminhibitoren oder, besonders bevorzugt, Kombinationen der genannten Wirkstoffe.

Als Geruchsabsorber dienen Silicate, erfindungsgemäß besonders bevorzugt vor allem Schichtsilicate, insbesondere Montmorillonit, Kaolinit, Ilit, Beidellit, Nontronit, Saponit, Hectorit, Bentonit, Smectit und Talkum, weiterhin Zeolithe, Zinkricinoleat, Cyclodextrine, bestimmte Metalloxide, wie z. B. Aluminiumoxid, sowie Chlorophyll. Sie werden vorzugsweise in einer Menge von 0,1 - 10 Gew.-%, vorzugsweise 0,5 - 7 Gew.-% und insbesondere 1 - 5 Gew.-%, jeweils bezogen auf die Gesamtzusammensetzung, eingesetzt.
Die als desodorierende Wirkstoffe offenbarten keimhemmenden oder antimikrobiellen Wirkstoffe können erfindungsgemäß nicht nur in Deodorantzusammensetzungen, sondern auch in antimikrobiell wirkenden kosmetischen Zusammensetzungen oder zur Konservierung kosmetischer Zusammensetzungen eingesetzt werden. Als keimhemmende oder antimikrobielle Wirkstoffe erfindungsgemäß bevorzugt sind insbesondere Organohalogenverbindungen sowie - halogenide, quartäre Ammoniumverbindungen, eine Reihe von Pflanzenextrakten und Zinkverbindungen. Hierzu zählen u. a. Triclosan, Chlorhexidin und Chlorhexidingluconat, 3,4,4'-Trichlorcarbanilid, Bromchlorophen, Dichlorophen, Chlorothymol, Chloroxylenol, Hexachlorophen, Dichloro-m-xylenol, Dequaliniumchlorid, Domiphenbromid, Ammoniumphenolsulfonat, Benzalkoniumhalogenide, Benzalkoniumcetylphosphat, Benzalkoniumsaccharinate, Benzethoniumchlorid, Cetylpyridiniumchlorid, Laurylpyridiniumchlorid, Laurylisoquinoliniumbromid, Methylbenzedoniumchlorid. Besonders bevorzugt sind Phenol, Phenoxyethanol, Dinatriumdihydroxyethylsulfosuccinylundecylenat, Natriumbicarbonat, Zinklactat, Natriumphenolsulfonat und Zinkphenolsulfonat, Ketoglutarsäure, Terpenalkohole wie z. B. Farnesol, Chlorophyllin-Kupfer-Komplexe, α-Monoalkylglycerinether mit einem verzweigten oder linearen gesättigten oder ungesättigten, gegebenenfalls hydroxylierten C₆ - C₂₂-Alkylrest, besonders bevorzugt α-(2-Ethylhexyl)glycerinether, im Handel erhältlich als Sensiva^{®} SC 50 (ex Schülke & Mayr), Carbonsäureester des Mono-, Di- und Triglycerins (z. B. Glycerinmonolaurat, Diglycerinmonocaprinat), Lantibiotika sowie Pflanzenextrakte (z. B. grüner Tee und Bestandteile des Lindenblütenöls).
Weitere bevorzugte Deodorant-Wirkstoffe sind ausgewählt aus sogenannten präbiotisch wirksamen Komponenten, worunter erfindungsgemäß solche Komponenten zu verstehen sind, die nur oder zumindest überwiegend die geruchsbildenden Keime der Hautmikroflora hemmen, nicht aber die erwünschten, das heißt, die nicht-geruchsbildenden Keime. Explizit sind hier die Wirkstoffe, die in der DE 10333245 und der DE 10 2004 011 968 als präbiotisch wirksam offenbart sind, mit einbezogen.

Weitere bevorzugte Deodorant-Wirkstoffe sind ausgewählt aus den keimhemmend wirkenden Parfümölen und den Deosafe-Parfümölen, die von der Firma Symrise, vormals Haarmann und Reimer, erhältlich sind.
Zu den Enzyminhibitoren gehören Stoffe, die die für die Schweißzersetzung verantwortlichen Enzyme, insbesondere die Arylsulfatase, β-Glucuronidase, Aminoacylase, die esterspaltenden Lipasen und die Lipoxigenase, hemmen, z. B. Trialkylcitronensäureester, insbesondere Triethylcitrat, oder Zinkglycinat.

Die Menge der Deodorant-Wirkstoffe in den erfindungsgemäßen Zusammensetzungen beträgt vorzugsweise 0,1 - 10 Gew.-%, vorzugsweise 0,2 - 7 Gew.-%, insbesondere 0,3 - 5 Gew.-% und außerordentlich bevorzugt 0,4 - 1,0 Gew.-%, bezogen auf das Gesamtgewicht der Zusammensetzung.
Erfindungsgemäß geeignete schweißhemmende Wirkstoffe sind ausgewählt aus den wasserlöslichen adstringierenden anorganischen und organischen Salzen des Aluminiums, Zirkoniums und Zinks bzw. beliebigen Mischungen dieser Salze. Bevorzugte Antitranspirant-Wirkstoffe sind ausgewählt aus den Aluminiumchlorhydraten, zum Beispiel Aluminiumsesquichlorhydrat, Aluminiumchlorhydrex-Propylenglykol (PG) oder -Polyethylenglykol (PEG), Aluminiumsesquichlorhydrex-PG oder -PEG, Aluminium-PG-dichlorhydrex oder Aluminium-PEGdichlorhydrex, Aluminiumhydroxid, weiterhin ausgewählt aus den Aluminiumzirconiumchlorhydraten, wie Aluminiumzirconiumtrichlorhydrat, Aluminiumzirconiumtetrachlorhydrat, Aluminiumzirconiumpentachlorhydrat, Aluminiumzirconiumoctachlorhydrat, den Aluminium-Zirkonium-Chlorohydrat-Glycin-Komplexen wie Aluminiumzirconiumtrichlorhydrexglycin, Aluminiumzirconiumtetrachlorhydrexglycin, Aluminiumzirconiumpentachlorhydrexglycin, Aluminiumzirconiumoctachlorhydrexglycin, Kaliumaluminiumsulfat (KAI(SO₄)₂ · 12 H₂O, Alaun), Aluminiumundecylenoylkollagenaminosäure, Natriumaluminiumlactat + Aluminiumsulfat, Natriumaluminiumchlorhydroxylactat, Aluminiumbromhydrat, Aluminiumchlorid, den Komplexen von Zink- und Natriumsalzen, den Komplexe von Lanthan und Cer, den Aluminiumsalzen von Lipoaminosäuren, Aluminiumsulfat, Aluminiumlactat, Aluminiumchlorhydroxyallantoinat, Natrium-Aluminium-Chlorhydroxylactat, Zinkchlorid, Zinksulfocarbolat, Zinksulfat und Zirkoniumchlorohydrat. Erfindungsgemäß wird unter Wasserlöslichkeit eine Löslichkeit von wenigstens 5 Gew.-% bei 20 °C verstanden, das heißt, dass Mengen von wenigstens 5 g des Antitranspirant-Wirkstoffes in 95 g Wasser bei 20 °C löslich sind. Die Antitranspirant-Wirkstoffe können als wässrige Lösungen eingesetzt werden. Sie sind in den erfindungsgemäßen Zusammensetzungen in einer Menge von 3 - 25 Gew.-%, vorzugsweise 5 - 22 Gew.-% und insbesondere 10 - 20 Gew.-% enthalten, bezogen auf die Menge der Aktivsubstanz in der Gesamtzusammensetzung. In einer bevorzugten Ausführungsform enthält die Zusammensetzung ein adstringierendes Aluminiumsalz, insbesondere Aluminiumchlorohydrat, das beispielsweise pulverförmig als Micro Dry^{®} Ultrafine von Reheis, in Form einer wässrigen Lösung als Locron^{®} L von Clariant, als Chlorhydrol^{®} sowie in aktivierter Form als Reach^{®} 501 von Reheis vertrieben wird. Unter der Bezeichnung Reach^{®} 301 wird ein Aluminiumsesquichlorohydrat von Reheis angeboten. Auch die Verwendung von Aluminium-Zirkonium-Tetrachlorohydrex-Glycin-Komplexen, die beispielsweise von Reheis unter der Bezeichnung Rezal^{®} 36G im Handel sind, kann erfindungsgemäß besonders bevorzugt sein.

In einer weiteren bevorzugten Ausführungsform enthalten die erfindungsgemäßen Zusammensetzungen neben der bzw. den ionischen Flüssigkeit(en) mindestens ein anionisches, kationisches, amphoteres und/oder nichtionisches Tensid. Tenside sind ein essentieller Bestandteil vieler kosmetischer Zusammensetzungen, insbesondere von Mitteln zur Haut- und Haarreinigung, zur Zahnreinigung, Haut- und Haarkonditionierung, weiteren Haarbehandlungsmitteln wie Haarstyling-, Dauerwell-, Haarglättungs- und Blondiermitteln und Haarcolorationen, desweiteren Rasier- und Depilationsmitteln und Unterarmprodukten.

In einer weiteren bevorzugten Ausführungsform enthalten die erfindungsgemäßen Zusammensetzungen neben der bzw. den ionischen Flüssigkeit(en) mindestens einen konditionierenden Wirkstoff. Unter konditionierenden Wirkstoffen sind erfindungsgemäß solche Substanzen zu verstehen, die auf keratinische Materialien, insbesondere auf das Haar und/oder die Haut, aufziehen und die physikalischen und sensorischen Eigenschaften verbessern. Konditionierungsmittel glätten die aufgeraute und/oder geschädigte Cuticula des Haares oder die oberste Schicht der Haut, verbessern die Kämmbarkeit, den Griff, die Weichheit und das Volumen des Haares und machen die Haut weich und geschmeidig.
Erfindungsgemäß bevorzugte konditionierende Wirkstoffe sind ausgewählt aus Fettstoffen, insbesondere pflanzlichen Ölen, wie Sonnenblumenöl, Olivenöl, Sojaöl, Rapsöl, Mandelöl, Jojobaöl, Orangenöl, Weizenkeimöl, Pfirsichkernöl und den flüssigen Anteilen des Kokosöls, Lanolin und seinen Derivaten, flüssigen Paraffinölen, Isoparaffinölen und synthetischen Kohlenwasserstoffen, Di-n-alkylethern mit insgesamt 12 bis 36 C-Atomen, z. B. Di-n-octylether und n-Hexyl-n-octylether, Fettsäuren, besonders linearen und/oder verzweigten, gesättigten und/oder ungesättigten C₈₋₃₀-Fettsäuren, Fettalkoholen, besonders gesättigten, ein- oder mehrfach ungesättigten, verzweigten oder unverzweigten Fettalkoholen mit 4 - 30 Kohlenstoffatomen, die mit 1 - 75, bevorzugt 5 - 20 Ethylenoxid-Einheiten ethoxyliert und/oder mit 3 - 30, bevorzugt 9 - 14 Propylenoxid-Einheiten propoxyliert sein können, Esterölen, das heißt Estern von C₆₋₃₀-Fettsäuren mit C₂₋₃₀-Fettalkoholen, Hydroxycarbonsäurealkylestern, Dicarbonsäureestern wie Di-n-butyladipat sowie Diolestern wie Ethylenglykoldioleat oder Propylenglykoldi(2-ethylhexanoat), symmetrischen, unsymmetrischen oder cyclischen Estern der Kohlensäure mit Fettalkoholen, z. B. Glycerincarbonat oder Dicaprylylcarbonat (Cetiol^{®} CC), Mono,- Di- und Trifettsäureestern von gesättigten und/oder ungesättigten linearen und/oder verzweigten Fettsäuren mit Glycerin, die mit 1-10, bevorzugt 7-9 Ethylenoxid-Einheiten ethoxyliert sein können, z. B. PEG-7 Glyceryl Cocoate, Wachsen, insbesondere Insektenwachsen, Pflanzenwachsen, Fruchtwachsen, Ozokerit, Mikrowachsen, Ceresin, Paraffinwachsen, Triglyceriden gesättigter und gegebenenfalls hydroxylierter C₁₆₋₃₀-Fettsäuren, z. B. gehärteten Triglyceridfetten, Phospholipiden, beispielsweise Sojalecithin, Ei-Lecithin und Kephalinen, Siliconverbindungen, ausgewählt aus Decamethylcyclopentasiloxan, Dodecamethylcyclohexasiloxan und Siliconpolymeren, die gewünschtenfalls quervernetzt sein können, z. B. Polydialkylsiloxanen, Polyalkylarylsiloxanen, ethoxylierten und/oder propoxylierten Polydialkylsiloxanen mit der früheren INCI-Bezeichnung Dimethicone Copolyol, sowie Polydialkylsiloxanen, die Amin- und/oder Hydroxy-Gruppen enthalten, bevorzugt Substanzen mit den INCI-Bezeichnungen Dimethiconol, Amodimethicone oder Trimethylsilylamodimethicone.
Die Einsatzmenge der Fettstoffe beträgt für Hautbehandlungsmittel vorzugsweise 0,1 - 50 Gew.%, bevorzugt 0,1 - 20 Gew.% und besonders bevorzugt 0,1 - 15 Gew.%, jeweils bezogen auf das gesamte Hautbehandlungsmittel. Für Haarbehandlungsmittel beträgt die Einsatzmenge der Fettstoffe vorzugsweise 0,01 - 10 Gew.%, bevorzugt 0,1 - 8 Gew.%, besonders bevorzugt 0,25 - 7,5 Gew.% und am bevorzugtesten 0,5 - 5 Gew.%, jeweils bezogen auf das gesamte Haarbehandlungsmittel.

Weitere erfindungsgemäß bevorzugte konditionierende Wirkstoffe sind ausgewählt aus quaternierten Aminen wie Methyl-1-alkylamidoethyl-2-alkylimidazolinium-methosulfat, Gluconamidopropyl-dimethyl-2-hydroxyethyl-ammoniumchlorid (Quaternium-22), PPG-9 diethylmonium chloride, Quaternium-26, Quaternium-33, Quaternium-60 und Quaternium-70. Weitere erfindungsgemäß bevorzugte konditionierende Wirkstoffe sind ausgewählt aus quaternierten Polymeren wie Polyquaternium-4, Polyquaternium-7, Polyquaternium-8, Polyquaternium-10, Polyquaternium-11, Polyquaternium-23 und Polyquaternium-24. Die Einsatzmenge der quaternierten Amine und/oder Polymere beträgt 0,05 - 5 Gew.%, bevorzugt 0,1 - 4 Gew.% und besonders bevorzugt 0,1 - 1,0 Gew.%, jeweils bezogen auf die gesamte Zusammensetzung.

Keratinische Fasern, insbesondere menschliche Haare, werden heutzutage einer Vielzahl von Behandlungen unterzogen. Dabei spielen die Behandlungen, die zu einer temporären Formgebung der Haare dienen, eine wichtige Rolle. Temporäre Formgebungen, die einen guten Halt ergeben sollen, ohne das gesunde Aussehen der Haare, wie zum Beispiel deren Glanz, zu beeinträchtigen, können beispielsweise durch Haarsprays, Haarwachse, Haargele, Fönwellen etc. erzielt werden. Als temporär formgebende Komponente sind in diesen Produkten üblicherweise synthetische Polymere enthalten. Sprayzubereitungen, die ein gelöstes oder dispergiertes Polymer enthalten, können mittels Treibgasen oder durch einen Pumpmechanismus auf das Haar aufgebracht werden.
Die festigenden Wirkstoffe, bei denen es sich in der Regel um polymere Verbindungen handelt, können auch in übliche Haarreinigungs- oder -konditioniermittel eingearbeitet werden. In vielen Fällen ist es aber vorteilhaft, sie in Form spezieller Mittel wie Haarfestiger, Haargelen, Haarwachsen oder Haarsprays anzuwenden.
Geeignete synthetische, filmbildende, haarfestigende Polymere sind Homo- oder Copolymere, die aus mindestens einem der folgenden Monomere aufgebaut sind: Vinylpyrrolidon, Vinylcaprolactam, Vinylester wie z.B. Vinylacetat, Vinylalkohol, Acrylamid, Methacrylamid, Alkyl- und Dialkylacrylamid, Alkyl- und Dialkylmethacrylamid, Alkylacrylat, Alkylmethacrylat, Propylenglykol oder Ethylenglykol, wobei die Alkylgruppen dieser Monomere vorzugsweise C1- bis C7-Alkylgruppen, besonders bevorzugt C1- bis C3-Alkylgruppen sind. Insbesondere geeignet sind z.B. die weiter oben genannten Polymere, insbesondere Homopolymere des Vinylcaprolactams, des Vinylpyrrolidons oder des N-Vinylformamids. Weitere geeignete synthetische filmbildende, haarfestigende Polymere sind z.B. Copolymerisate aus Vinylpyrrolidon und Vinylacetat, Terpolymere aus Vinylpyrrolidon, Vinylacetat und Vinylpropionat, Polyacrylamide, die beispielsweise unter den Handelsbezeichnungen Akypomine^{®} P 191 von der Firma CHEM-Y oder Sepigel^{®} 305 von der Firma Seppic vertrieben werden; Polyvinylalkohole, die beispielsweise unter den Handelsbezeichnungen Elvanol^{®} von Du Pont oder Vinol^{®} 523/540 von der Firma Air Products vertrieben werden sowie Polyethylenglykol/ Polypropylenglykol-Copolymere, die beispielsweise, unter den Handelsbezeichnungen Ucon^{®} der Union Carbide vertrieben werden. Besonders bevorzugt sind Polyvinylpyrrolidon und Polyvinylpyrrolidon/Vinylacetat-Copolymere.

Festigende Polymere tragen zum Halt und/oder zum Aufbau des Haarvolumens, der Haarfülle der Gesamtfrisur bei. Diese sogenannten festigenden Polymere sind gleichzeitig auch filmbildende Polymere und daher generell typische Substanzen für Haarbehandlungsmittel wie Haarfestiger, Haarschäume, Haarwachse, Haarsprays. Die Filmbildung kann dabei durchaus punktuell sein und nur einige Fasern miteinander verbinden.

Vorteilhafterweise liegen die erfindungsgemäßen hautkosmetischen Mittel in Form einer flüssigen, fließfähigen oder festen Öl-in-Wasser-Emulsion, Wasser-in-Öl-Emulsion, Mehrfach-Emulsion, insbesondere einer Öl-in-Wasser-in-Öl- oder Wasser-in-Öl-in-Wasser-Emulsion, Makroemulsion, Miniemulsion, Mikroemulsion, PIT-Emulsion, Nanoemulsion, Pickering-Emulsion, Hydrodispersion, eines Hydrogels, eines Lipogels, einer ein- oder mehrphasigen Lösung, eines Schaumes, eines Puders oder einer Mischung mit mindestens einem als medizinischen Klebstoff geeigneten Polymer vor. Die Mittel können auch in wasserfreier Form, wie beispielsweise einem Öl oder einem Balsam, dargereicht werden. Hierbei kann der Träger ein pflanzliches oder tierisches Öl, ein Mineralöl, ein synthetisches Öl oder eine Mischung solcher Öle sein.
In einer besonderen Ausführungsform der erfindungsgemäßen Mittel liegen die Mittel als Mikroemulsion vor. Unter Mikroemulsionen werden im Rahmen der Erfindung neben den thermodynamisch stabilen Mikroemulsionen auch die sogenannten "PIT"-Emulsionen verstanden. Bei diesen Emulsionen handelt es sich um Systeme mit den 3 Komponenten Wasser, Öl und Emulgator, die bei Raumtemperatur als ÖI-in-Wasser-Emulsion vorliegen. Beim Erwärmen dieser Systeme bilden sich in einem bestimmten Temperaturbereich (als Phaseninversiontemperatur oder "PIT" bezeichnet) Mikroemulsionen aus, die sich bei weiterer Erwärmung in Wasser-in-Öl-Emulsionen umwandeln. Beim anschließenden Abkühlen werden wieder O/W-Emulsionen gebildet, die aber auch bei Raumtemperatur als Mikroemulsionen oder als sehr feinteilige Emulsionen mit einem mittleren Teilchendurchmesser unter 400 nm und insbesondere von etwa 100-300 nm, vorliegen. Erfindungsgemäß können solche Mikro- oder "PIT"-Emulsionen bevorzugt sein, die einen mittleren Teilchendurchmesser von etwa 200 nm aufweisen.
In der Ausführungsform als Emulsion oder als tensidische Lösung, z. B. als Reinigungsmittel, enthalten die erfindungsgemäßen Zusammensetzungen mindestens eine oberflächenaktive Substanz als Emulgator oder Dispergiermittel. Geeignete Emulgatoren sind beispielsweise Anlagerungsprodukte von 4 bis 30 Mol Ethylenoxid und/oder 0 bis 5 Mol Propylenoxid an lineare C₈-C₂₂-Fettalkohole, an C₁₂-C₂₂-Fettsäuren und an C₈-C₁₅-Alkylphenole, C₁₂-C₂₂-Fettsäuremono- und -diester von Anlagerungsprodukten von 1 bis 30 Mol Ethylenoxid an C₃-C₆-Polyole, insbesondere an Glycerin, Ethylenoxid- und Polyglycerin-Anlagerungsprodukte an Methylglucosid-Fettsäureester, Fettsäurealkanolamide und Fettsäureglucamide, C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind, Gemische aus Alkyl-(oligo)-glucosiden und Fettalkoholen, z. B. das im Handel erhältliche Produkt Montanov^{®}68, Anlagerungsprodukte von 5 bis 60 Mol Ethylenoxid an Rizinusöl und gehärtetes Rizinusöl, Partialester von Polyolen mit 3-6 Kohlenstoffatomen mit gesättigten C₈-C₂₂-Fettsäuren, Sterole (Sterine), insbesondere Cholesterol, Lanosterol, Beta-Sitosterol, Stigmasterol, Campesterol und Ergosterol sowie Mykosterole, Phospholipide, vor allem Glucose-Phospolipide, Fettsäureester von Zuckern und Zuckeralkoholen wie Sorbit, Polyglycerine und Polyglycerinderivate, bevorzugt Polyglyceryl-2-dipolyhydroxystearat (Handelsprodukt Dehymuls^{®} PGPH) und Polyglyceryl-3-diisostearat (Handelsprodukt Lameform^{®} TGI) sowie lineare und verzweigte C₈-C₃₀-Fettsäuren und deren Na-, K-, Ammonium-, Ca-, Mg- und Zn - Salze.
Die erfindungsgemäßen Mittel enthalten die Emulgatoren bevorzugt in Mengen von 0,1 bis 25 Gew.-%, insbesondere 0,5 - 15 Gew.-%, bezogen auf das gesamte Mittel.
In einer besonders bevorzugten Ausführungsform ist mindestens ein nichtionischer Emulgator mit einem HLB-Wert von 8 und darunter enthalten. Derart geeignete Emulgatoren sind beispielsweise Verbindungen der allgemeinen Formel R¹ - O - R², in der R¹ eine primäre lineare Alkyl-, Alkenyl- oder Acylgruppe mit 20 - 30 C-Atomen und R² Wasserstoff, eine Gruppe mit der Formel -(CₙH₂ₙO)ₓ-H mit x = 1 oder 2 und n = 2 - 4 oder eine Polyhydroxyalkylgruppe mit 4 - 6 C-Atomen und 2 - 5 Hydroxylgruppen ist. Weitere bevorzugt geeignete Emulgatoren mit einem HLB-Wert von 8 und darunter sind die Anlagerungsprodukte von 1 oder 2 Mol Ethylenoxid oder Propylenoxid an Behenylalkohol, Erucylalkohol, Arachidylalkohol oder auch an Behensäure oder Erucasäure. Bevorzugt eignen sich auch die Monoester von C₁₆-C₃₀-Fettsäuren mit Polyolen wie z. B. Pentaerythrit, Trimethylolpropan, Diglycerin, Sorbit, Glucose oder Methylglucose. Beispiele für solche Produkte sind z. B. Sorbitanmonobehenat oder Pentaerythrit-monoerucat.
Weitere geeignete Zusatzstoffe sind Verdickungsmittel, z. B. natürliche und synthetische Tone und Schichtsilikate wie Bentonit, Hectorit, Montmorillonit oder Laponite^{®}, oder anionische Polymere aus Acrylsäure, Methacrylsäure, Crotonsäure, Maleinsäureanhydrid und 2-Acrylamido-2-methylpropansulfonsäure, wobei die sauren Gruppen ganz oder teilweise als Natrium-, Kalium-, Ammonium-, Mono- oder Triethanolammonium-Salz vorliegen können und wobei mindestens ein nichtionisches Monomer enthalten sein kann. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Diese Copolymere können auch vernetzt vorliegen. Geeignete Handelsprodukte sind Sepigel^{®}305, Simulgel^{®}600, Simulgel^{®} NS und Simulgel^{®} EG der Firma SEPPIC. Weitere besonders bevorzugte anionische Homo- und Copolymere sind unvernetzte und vernetzte Polyacrylsäuren. Solche Verbindungen sind zum Beispiel die Handelsprodukte Carbopol^{®}. Ein besonders bevorzugtes anionisches Copolymer enthält als Monomer zu 80 - 98 % eine ungesättigte, gewünschtenfalls substituierte C₃₋₆-Carbonsäure oder ihr Anhydrid sowie zu 2 - 20 % gewünschtenfalls substituierte Acrylsäureester von gesättigten C₁₀₋₃₀-Carbonsäuren, wobei das Copolymer mit den vorgenannten Vernetzungsagentien vernetzt sein kann. Entsprechende Handelsprodukte sind Pemulen^{®} und die Carbopol^{®}-Typen 954, 980, 1342 und ETD 2020 (ex B.F. Goodrich).
Geeignete nichtionische Polymere sind beispielsweise Polyvinylalkohole, die teilverseift sein können, z. B. die Handelsprodukte Mowiol^{®} sowie VinylpyrrolidonNinylester-Copolymere und Polyvinylpyrrolidone, die z. B. unter dem Warenzeichen Luviskol® (BASF) vertrieben werden.
Weitere geeignete Zusatzstoffe sind Antioxidantien, Konservierungsmittel, Lösungsmittel wie Ethanol, Isopropanol, Ethylenglykol, Propylenglykol, Propylenglykolmonoethylether, Glycerin und Diethylenglykol, Adsorbentien und Füllstoffe, wie Talkum und Veegum^{®}, Parfümöle, Pigmente sowie Farbstoffe zum Anfärben des Mittels, Substanzen zur Einstellung des pH-Wertes, Komplexbildner wie EDTA, NTA, β-Alanindiessigsäure und Phosphonsäuren, Antischuppenwirkstoffe wie Piroctone Olamine (Octopirox), Zinc Omadine und Climbazol, Perlglanzmittel wie Ethylenglykolmono- und -distearat, Trübungsmittel und Treibmittel wie Propan-Butan-Gemische, Pentan, Isopentan, Isobutan, N₂O, Dimethylether, CO₂ und Luft.

Erfindungsgemäß besonders bevorzugte kosmetische Zusammensetzungen enthalten zusätzlich Silikon(e). Hier sind besonders bevorzugte erfindungsgemäße kosmetische Zusammensetzungen dadurch gekennzeichnet, daß sie zusätzlich Silikon(e), vorzugsweise in Mengen von 0,1 bis 10 Gew.%, vorzugsweise von 0,25 bis 7 Gew.-% und insbesondere von 0,5 bis 5 Gew.-%, jeweils bezogen auf das gesamte Mittel, enthalten.

Insbesondere bevorzugt sind erfindungsgemäße kosmetische Zusammensetzungen, die mindestens ein Silicon enthalten, das ausgewählt ist unter:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopoylmeren vom Typ (A-B)ₙ mit n > 3;
(iv) gepfropften Siliconpolymeren mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
(v) gepfropften Siliconpolymeren mit Polysiloxan- Grundgerüst, auf das nicht siliconhaltige, organische Monomere gepfropft wurden, die eine Polysiloxan-Hauptkette aufweisen, auf die in derKette sowie gegebenenfalls an mindestens einem ihrer Enden mindestens ein organisches Makromer gepfropft wurde, das kein Silicon enthält;
(vi) oder deren Gemischen.

Besonders bevorzugte erfindungsgemäße kosmetische Zusammensetzungen sind dadurch gekennzeichnet, daß sie mindestens ein Silikon der Formel (Si-I),

**(CH₃)₃Si-[O-Si(CH₃)₂]ₓ-O-Si(CH₃)₃** **(Si-I)**,

enthalten, in der x für eine Zahl von 0 bis 100, vorzugsweise von 0 bis 50, weiter bevorzugt von 0 bis 20 und insbesondere 0 bis 10, steht.

Die erfindungsgemäß bevorzugten kosmetischen Zusammensetzungen enthalten ein Silikon der vorstehenden Formel (Si-I). Diese Silikone werden nach der INCI-Nomenklatur als DIMETHICONE bezeichnet. Es werden im Rahmen der vorliegenden Erfindung als Silicon der Formel (Si-I), vorzugsweise die Verbindungen:
(CH₃)₃Si-O-Si(CH₃)₃
(CH₃)₃Si-O-(CH₃)₂Si-O-Si(CH₃)₃
(CH₃)₃Si-[O-(CH₃)₂Si]₂-O-Si(CH₃)₃
(CH₃)₃Si-[O-(CH₃)₂Si]₃-O-Si(CH₃)₃
(CH₃)₃Si-[O-(CH₃)₂Si]₄-O-Si(CH₃)₃
(CH₃)₃Si-[O-(CH₃)₂Si]₅-O-Si(CH₃)₃
(CH₃)₃Si-[O-(CH₃)₂Si]₆-O-Si(CH₃)₃
(CH₃)₃Si-[O-(CH₃)₂Si]₇-O-Si(CH₃)₃
(CH₃)₃Si-[O-(CH₃)₂Si]₈-O-Si(CH₃)₃
(CH₃)₃Si-[O-(CH₃)₂Si]₉-O-Si(CH₃)₃
(CH₃)₃Si-[O-(CH₃)₂Si]₁₀-O-Si(CH₃)₃
(CH₃)₃Si-[O-(CH₃)₂Si]₁₁-O-Si(CH₃)₃
(CH₃)₃Si-[O-(CH₃)₂Si]₁₂-O-Si(CH₃)₃
(CH₃)₃Si-[O-(CH₃)₂Si]₁₃-O-Si(CH₃)₃
(CH₃)₃Si-[O-(CH₃)₂Si]₁₄-O-Si(CH₃)₃
(CH₃)₃Si-[O-(CH₃)₂Si]₁₅-O-Si(CH₃)₃
(CH₃)₃Si-[O-(CH₃)₂Si]₁₆-O-Si(CH₃)₃
(CH₃)₃Si-[O-(CH₃)₂Si]₁₇-O-Si(CH₃)₃
(CH₃)₃Si-[O-(CH₃)₂Si]₁₈-O-Si(CH₃)₃
(CH₃)₃Si-[O-(CH₃)₂Si]₁₉-O-Si(CH₃)₃
(CH₃)₃Si-[O-(CH₃)₂Si]₂₀-O-Si(CH₃)₃
eingesetzt, wobei (CH₃)₃Si-O-Si(CH₃)₃, (CH₃)₃Si-O-(CH₃)₂Si-O-Si(CH₃)₃ und/oder (CH₃)₃Si-[O-(CH₃)₂Si]₂-O-Si(CH₃)₃ besonders bevorzugt sind.

Selbstverständlich können auch Mischungen der o.g. Silikone in den erfindungsgemäßen Mitteln enthalten sein.

Bevorzugte erfindungsgemäß einsetzbare Silikone weisen bei 20°C Viskositäten von 0,2 bis 2 mm²s⁻¹ auf, wobei Silikone mit Viskositäten von 0,5 bis 1 mm²s⁻¹ besonders bevorzugt sind.

Besonders bevorzugte erfindungsgemäße Mittel enthalten ein oder mehrere aminofunktionelle Silicone. Solche Silicone können z.B. durch die Formel

M(RₐQ_{b}SiO_{(4-a-b)/2)x}(R_{c}SiO_{(4-c)/2)y}M

beschrieben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel -R¹HZ ist, worin R¹ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silicon-Endgruppe ist, wie sie im Stande der Technik bekannt ist, vorzugsweise Trimethylsiloxy. Nicht einschränkende Beispiele der durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von R¹ schließen Methylen, Ethylen, Propylen, Hexamethylen, Decamethylen, -CH₂CH(CH₃)CH₂-, Phenylen, Naphthylen, -CH₂CH₂SCH₂CH₂-, -CH₂CH₂OCH₂-, -OCH₂CH₂-, -OCH₂ CH₂CH₂-, -CH₂CH(CH₃)C(O)OCH₂-, -(CH₂)₃ CC(O)OCH₂CH₂- , -C₆H₄C₆H₄-, -C₆H₄CH₂C₆H₄-; und -(CH₂)₃C(O)SCH₂CH₂- ein.

Z ist ein organischer, aminofunktioneller Rest, enthaltend mindestens eine funktionelle Aminogruppe. Eine mögliche Formel für Z ist NH(CH₂)_{z}NH₂, worin z 1 oder mehr ist. Eine andere mögliche Formel für Z ist -NH(CH₂)_{z}(CH₂)_{zz}NH, worin sowohl z als auch zz unabhängig 1 oder mehr sind, wobei diese Struktur Diamino-Ringstrukturen umfaßt, wie Piperazinyl. Z ist am bevorzugtesten ein -NHCH₂CH ₂NH₂-Rest. Eine andere mögliche Formel für Z ist - N(CH₂)_{z}(CH₂)_{zz}NX₂ oder -NX₂, worin jedes X von X₂ unabhängig ausgewählt ist aus der Gruppe bestehend aus Wasserstoff und Alkylgruppen mit 1 bis 12 Kohlenstoffatomen, und zz 0 ist.

Q ist am bevorzugtesten ein polarer, aminofunktioneller Rest der Formel - CH₂CH₂CH₂NHCH₂CH₂NH₂. In den Formeln nimmt "a" Werte im Bereich von etwa 0 bis etwa 2 an, "b" nimmt Werte im Bereich von etwa 2 bis etwa 3 an, "a" + "b" ist kleiner als oder gleich 3, und "c" ist eine Zahl im Bereich von etwa 1 bis etwa 3. Das molare Verhältnis der RₐQ_{b} SiO_{(4-a-b)/2}-Einheiten zu den R_{c}SiO_{(4-c)/2}-Einheiten liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silicone der obigen Formel eingesetzt, dann können die verschiedenen variablen Substituenten in der obigen Formel bei den verschiedenen Siliconkomponenten, die in der Siliconmischung vorhanden sind, verschieden sein.

Bevorzugte erfindungsgemäße kosmetische Zusammensetzungen enthalten ein aminofunktionelles Silikon der Formel (Si-II)

**R'ₐG₃₋ₐ-Si(OSiG₂)ₙ-(OSiG_{b}R'_{2-b})ₘ-O-SiG₃₋ₐ-R'ₐ** **(Si-II),**

worin bedeutet:
- G ist-H, eine Phenylgruppe, -OH, -O-CH₃, -CH₃, -O-CH₂CH₃, -CH₂CH₃, -O-CH₂CH₂CH₃,-CH₂CH₂CH₃, -O-CH(CH₃)₂, -CH(CH₃)₂, -O-CH₂CH₂CH₂CH₃, - CH₂CH₂CH₂CH₃, -O-CH₂CH(CH₃)₂, -CH₂CH(CH₃)₂, -O-CH(CH₃)CH₂CH₃, - CH(CH₃)CH₂CH₃, -O-C(CH₃)₃, -C(CH₃)₃ ;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus
   o -Q-N(R")-CH₂-CH₂-N(R")₂
   o -Q-N(R")₂
   o -Q-N⁺(R")₃A⁻
   o -Q-N⁺H(R")₂ A⁻
   o -Q-N⁺H₂(R")A⁻
   o -Q-N(R")-CH₂-CH₂-N⁺R"H₂A⁻,
   wobei jedes Q für eine chemische Bindung, -CH₂-, -CH₂-CH₂-, -CH₂CH₂CH₂-, -C(CH₃)₂-, - CH₂CH₂CH₂CH₂-, -CH₂C(CH₃)₂-, -CH(CH₃)CH₂CH₂- steht,
   R" für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃, -CH(CH₃)₂, -CH₂CH₂CH₂H₃, - CH₂CH(CH₃)₂, -CH(CH₃)CH₂CH₃, -C(CH₃)₃, steht und A ein Anion repräsentiert, welches vorzugsweise ausgewählt ist aus Chlorid, Bromid, Iodid oder Methosulfat.

Besonders bevorzugte erfindungsgemäße kosmetischen Zusammensetzungen sind dadurch gekennzeichnet, daß sie mindestens es ein aminofunktionelles Silikon der Formel (Si-Ila) 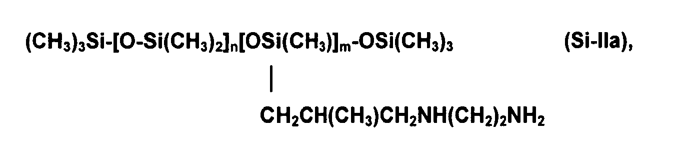 enthalten, worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.
Diese Silicone werden nach der INCI-Deklaration als Trimethylsilylamodimethicone bezeichnet.

Besonders bevorzugt sind auch erfindungsgemäße kosmetische Zusammensetzungen, die mindestens ein aminofunktionelles Silikon der Formel (Si-IIb) 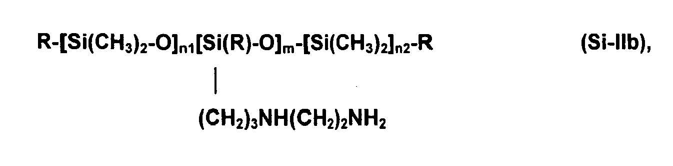 enthalten, worin R für -OH, -O-CH₃ oder eine -CH₃-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Amodimethicone bezeichnet.

Unabhängig davon, welche aminofunktionellen Silicone eingesetzt werden, sind erfindungsgemäße kosmetische Zusammensetzungen bevorzugt, die ein aminofunktionelles Silikon enthalten dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silicons. Sie kann durch Titration ermittelt und auch in der Einheit mg KOH/g angegeben werden.
Erfindungsgemäß bevorzugte kosmetische Zusammensetzungen sind dadurch gekennzeichnet, daß sie, bezogen auf ihr Gewicht, 0,01 bis 10 Gew.%, vorzugsweise 0,1 bis 8 Gew.%, besonders bevorzugt 0,25 bis 7,5 Gew.% und insbesondere 0,5 bis 5 Gew.% aminofunktionelle(s) Silikon(e) enthalten.

Auch die nach INCI als CYCLOMETHICONE bezeichneten cyclischen Dimethicone sind erfindungsgemäß mit Vorzug einsetzbar. Hier sind erfindungsgemäße kosmetische Zusammensetzungen bevorzugt, die mindestens ein Silikon der Formel III 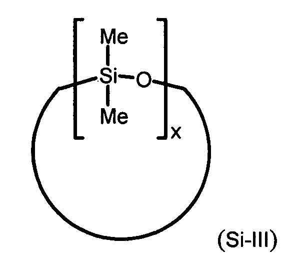 enthalten, in der x für eine Zahl von 0 bis 200, vorzugsweise von 0 bis 10, weiter bevorzugt von 0 bis 7 und insbesondere 0, 1, 2, 3, 4, 5 oder 6, steht.

Die vorstehend beschriebenen Silicone weisen ein Rückgrat auf, welches aus -Si-O-Si-Einheiten aufgebaut ist. Selbstverständlich können diese Si-O-Si-Einheiten auch durch Kohlenstoffketten unterbrochen sein. Entsprechende Moleküle sind durch Kettenverlängerungsreaktionen zugänglich und kommen vorzugsweise in Form von Silikon-in-Wasser-Emulsionen zum Einsatz.

Die erfindungsgemäß einsetzbaren Silikon-in-Wasser Emulsionen können nach bekannten Verfahren hergestellt werden, wie sie beispielsweise in US 5,998,537 und EP 0 874 017 A1 offenbart sind.

Zusammenfassend umfaßt dieses Herstellungsverfahren die emulgierende Mischung von Komponenten, deren eine mindestens ein Polysiloxane enthält, deren andere mindestens ein Organosilikonmaterial enthält, das mit dem Polysiloxane in einer Kettenverlängerungsreaktion reagiert, wobei mindestens ein ein Metallion-enthaltender Katalysator für die Kettenverlängerungsreaktion, mindestens ein Tensid und Wasser zugegen sind.

Kettenverlängerungsreaktionen mit Polysiloxanen sind bekannt und können beispielsweise die Hydrosilylierungsreaktion umfassen, in der eine Si-H Gruppe mit einer aliphatisch ungesättigten Gruppe in Gegenwart eines Platin/Rhodium-Katalysators unter Bildung von Polysiloxanes mit einigen Si-(C)ₚ-Si Bindungen (p = 1-6) reagiert, wobei die Polysiloxane auch als Polysiloxane-Polysilalkylene-Copolymere bezeichnet werden.

Die Kettenverlängerungsreaktion kann auch die Reaktion einer Si-OH Gruppe (beispielsweise eines Hydroxy-terminierten Polysiloxans) mit einer Alkoxygruppe (beispielsweise Alkoxysilanen, Silikaten oder Alkoxysiloxanen) in Gegenwart eines metallhaltigen Katalysators unter Bildung von Polysiloxanen umfassen.

Die Polysiloxane, die in der Kettenverlängerungsreaktion eingesetzt werden, umfassen ein substantiell lineares Polymer der folgenden Struktur:

R-Si(R₂)-[-O-Si(R₂)-]ₙ-O-SiR₃

In dieser Struktur steht jedes R unabhängig voneinander für einen Kohlenwasserstoffrest mit bis zu 20 Kohlenstoffatomen, vorzugsweise mit 1 bis 6 C-Atomen, wie beispielsweise einer Alkylgruppe (beispielsweise Methyl, Ethyl, Propyl oder Butyl), eine Arylgruppe (beispielsweise Phenyl), oder die für die Kettenverlängerungsreaktion benötigte Gruppe ("reaktive Gruppe", beispielsweise Si-gebundene H-Atome, aliphatisch ungesättigte Gruppen wie Vinyl, Allyl oder Hexenyl, Hydroxy, Alkoxy wie Methoxy, Ethoxy oder Propoxy, Alkoxy-Alkoxy, Acetoxy, Amino usw.), mit der Maßgabe, daß durchschnittlich ein bis zwei reaktive Gruppen pro Polymer vorliegen, n ist eine positive Zahl > 1. Vorzugsweise ist eine Mehrzahl der reaktiven Gruppen, besonders bevorzugt > 90%, und insbesondere > 98% der reaktiven Gruppen, an den endständigen Si-Atomen im Siloxan gebunden. Vorzugsweise steht n für Zahlen, die Polysiloxane beschreiben, welche Viskositäten zwischen 1 und 1.000.000 mm²/s besitzen, besonders bevorzugt Viskositäten zwischen 1.000 und 100.000 mm²/s.

Die Polysiloxane können zu einem geringen Grad verzweigt sein (beispielsweise < 2 Mol-% der Siloxaneinheiten), vzw sind die Polymere aber substantiell linear, besonders bevorzugt vollständig linear. Zudem können die Substituenten R ihrerseits substituiert sein, beispielsweise mit N-haltigen Gruppen (beispielsweise Aminogruppen), Epoxygruppen, S-haltige Gruppen, Si-haltige Gruppen, O-haltige Gruppen usw.. Vorzugsweise sind mindestens 80% der Reste R Alkylrste, besonders bevorzugt Methylgruppen.

Das Organosilikonmaterial, das mit dem Polysiloxan in der Kettenverlängerungsreaktion reagiert, kann entweder ein zweites Polysiloxan sein, oder ein Molekül, das als Kettenverlängerer agiert. Wenn das Organosilikonmaterial ein Polysiloxan ist, hat es die vorstehend erwähnte generelle Struktur. In diesen Fällen besitzt ein Polysiloxan in der Reaktion (mindestens) eine reaktive Gruppe, und ein zweites Polysiloxan besitzt (mindestens) eine zweite reaktive Gruppe, die mit der ersten reagiert.

Falls das Organosilikonmaterial ein Kettenverlängerungs-Agens umfaßt, kann dies ein Material sein wie beispielsweise ein Silan, ein Siloxan (beispielsweise Disiloxane oder Trisiloxan) oder ein Silazan. So kann beispielsweise eine Zusammensetzung, die ein Polysiloxan gemäß der vorstehend beschriebenen generellen Struktur umfaßt, welches mindestens eine Si-OH Gruppe aufweist, kettenverlängert werden, indem mit einem Alkoxysilan (beispielsweise einem Dialkoxysilan oder Trialkoxysilan) in Gegenwart von Zinn- oder Titan-haltigen Katalysatoren reagiert wird.

Die metallhaltigen Katalysatoren in der Kettenverlängerungsreaktion sind meist spezifisch für eine bestimmte Reaktion. Solche Katalysatoren sind im Stand der Technik bekannt und enthalten beispielsweise Metalle wie Platin, Rhodium, Zinn, Titan, Kupfer, Blei, etc.. In einer bevorzugten Kettenverlängerungsreaktion wird ein Polysiloxan mit mindestens einer aliphatisch ungesättigten Gruppe, vorzugsweise einer Endgruppe, mit einem Organosilikonmaterial in Gegenwart eines Hydrosilylierungskatalysators zur Reaktion gebracht, das ein Siloxan oder Polysiloxan mit mindestens einer (vorzugsweise endständigen) Si-H Gruppe ist. Das Polysiloxan besitzt mindestens eine aliphatisch ungesättigte Gruppe und genügt der allgemeinen oben angegeben Formel, in der R und n wie vorstehend definiert sind, wobei im Durchschnitt zwischen 1 und 2 Gruppen R eine aliphatisch ungesättigte Gruppe pro Polymer besitzen. Repräsentative aliphatisch ungesättigte Gruppen sind beispielsweise Vinyl, Allyl, Hexenyl und Cyclohexenyl oder eine Gruppe R²CH=CHR³, in der R² für eine divalente aliphatische an das Silicium gebundene Kette und R³ für ein Wasserstoffatom oder eine Alkylgruppe steht. Das Organosilikonmaterial mit mindestens einer Si-H Gruppe hat vorzugsweise die oben genannte Struktur, in der R und n wie vorstehend definiert sind und wobei im Durchschnitt zwischen 1 und 2 Gruppen R ein Wasserstoff bedeuten und n 0 oder eine positive ganze Zahl ist

Dieses Material kann ein Polymer oder ein niedermolekulares Material wie ein Siloxan sein (beispielsweise ein Disiloxane oder ein Trisiloxan).

Das Polysiloxan mit mindestens einer aliphatisch ungesättigten Gruppe und das Organosilikonmaterial mit mindestens einer Si-H Gruppe reagieren in Gegenwart eines Hydrosilylierungskatalysators. Solche Katalysatoren sind aus dem Stand der Technik bekannt und umfassen beispielsweise Platin- und Rhodium-enthaltende Materialien. Die Katalysatoren können jede bekannte Form annehmen, beispielsweise auf Trägermaterialien (wie beispielsweise Silica Gel oder Aktivkohle) aufgebrachtes Platin oder Rhodium oder andere geeignete Compounds wie Platinchlorid, Salze von Platin- oder Chloroplatinsäuren. Ein wegen der guten Dispergierbarkeit in Organosilikonsystemen und der geringen Farbveränderungen bevorzugter Katalysator ist Chloroplatinsäure entweder als kommerziell verfügbares Hexahydrat oder in wasserfreier Form.

Bei einer weiteren bevorzugten Kettenerweiterungsreaktion wird ein Polysiloxan mit mindestens einer Si-OH Gruppe, vorzugsweise einer Endgruppe, mit einem Organosilikonmaterial zu Reaktion gebracht, das mindestens eine Alkoxygruppe besitzt, vorzugsweise ein Siloxan mit mindestens einer Si-OR Gruppe oder ein Alkoxysilan mit mindestens zwei Alkoxygruppen. Hierbei wird als Katalysator wieder ein metallhaltiger Katalysator eingesetzt.

Für die Reaktion einer Si-OH Gruppe mit einer Si-OR Gruppe existieren viele literaturbekannte Katalysatoren, beispielsweise Organometallverbindungen wie Organozinnsalze, Titanate oder Titanchelate bzw. -komplexe. Beispiele umfassen Zinn-octoat, Dibutylzinn-dilaurat, Dibutylzinndiacetat, Dimethyltinn-dineodecanoat, Dibutylzinn-dimethoxid, Isobutylzinn-triceroat, Dimethylzinn-dibutyrat, Dimethylzinn-dineodecanoat, Triethylzinn-tartrat, Zinnoleat, Zinnnaphthenat, Zinnbutyrat, Zinnacetat, Zinnbenzoat, Zinnsebacat, Zinnsuccinat, Tetrabutyltitanat, Tetraisopropyltitante, Tetraphenyltitant, Tetraoctadecyltitanat, Titan-naphthanat, Ethyltriethanolamin-Titanat, Titani-diisopropyl-diethyl-acetoacetat, Titan-diisopropoxy-diacetylacetonat und Titani-tetra-Alkoxide, bei denen das Alkoxid Butoxy oder Propoxy ist.

Die Silikon-in-Wasser-Emulsionen enthaltene darüber hinaus vorzugsweise mindestens ein Tensid.

Erfindungsgemäß ebenfalls bevorzugte kosmetische Zusammensetzungen sind dadurch gekennzeichnet, daß sie mindestens ein Silikon der Formel (Si-IV)

**R₃Si-[O-SiR₂]ₓ-(CH₂)ₙ-[O-SiR₂]_{y}-O-SiR₃** **(Si-IV),**

enthalten, in der R für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃, -CH(CH₃)₂, -CH₂CH₂CH₂H₃, -CH₂CH(CH₃)₂, -CH(CH₃)CH₂CH₃, -C(CH₃)₃, steht, x bzw. y für eine Zahl von 0 bis 200, vorzugsweise von 0 bis 10, weiter bevorzugt von 0 bis 7 und insbesondere 0, 1, 2, 3, 4, 5 oder 6, stehen, und n für eine Zahl von 0 bis 10, bevorzugt von 1 bis 8 und insbesondere für 2, 3, 4, 5, 6 steht.

Mit Vorzug sind die Silikone wasserlöslich. Erfindungsgemäß bevorzugte kosmetische oder dermatologische Zubereitungen sind dadurch gekennzeichnet, daß sie zusätzlich ein wasserlösliches Silikon enthalten.

Die erfindungsgemäßen Zusammensetzungen können als Cremes, Lotionen, Gele usw. formuliert und auf die zu behandelnden Köperpartien, d.h. die Haut und/oder das Haar, aufgetragen werden. Im Hinblick auf die mögliche Anwendung auf dem Haar können die erfindungsgemäßen Mittel beispielsweise als Shampoos, Lotionen, Gele, Cremes usw. formuliert werden. In diesem Falle ist es möglich, den Zusammensetzungen Farbstoffe hinzuzufügen, um sie beispielsweise als Färbeshampoos oder Haarfärbemittel mit Zusatznutzen zu formulieren. Solche erfindungsgemäßen Produkte werden nachstehend als "Färbemittel" bezeichnet.

Hinsichtlich der in den erfindungsgemäßen Färbemitteln einsetzbaren Farbstoffe oder Farbstoffvorprodukte unterliegt die vorliegende Erfindung keinerlei Einschränkungen. Die erfindungsgemäßen Färbemittel können als weitere Farbstoffvorprodukte
- Oxidationsfarbstoffvorprodukte vom Entwickler- und/oder Kuppler-Typ, und
- Vorstufen naturanaloger Farbstoffe, wie Indol- und Indolin-Derivate,
sowie Mischungen von Vertretern dieser Gruppen enthalten.

Erfindungsgemäß bevorzugte kosmetische Mittel sind dadurch gekennzeichnet, daß sie mindestens ein Oxidationsfarbstoffvorprodukt vom Entwicklertyp und/oder Kupplertyp enthalten, wobei bevorzugte Entwicklerkomponenten ausgewählt sind aus p-Phenylendiamin, p-Toluylendiamin, , N,N-Bis-(2-hydroxyethyl)amino-p-phenylendiamin, 1,3-Bis-[(2-hydroxyethyl-4'-aminophenyl)amino]-propan-2-ol, 1,10-Bis-(2',5'- diaminophenyl)-1,4,7,10-tetraoxadecan, 4-Aminophenol, 4- Amino-3-methylphenol, Bis-(5- amino-2-hydroxyphenyl)methan, 2,4,5,6-Tetraaminopyrimidin, 2-Hydroxy-4,5,6- triaminopyrimidin, 4,5-Diamino-1-(2-hydroxyethyl)-pyrazol und bevorzugte Kupplerkomponenten ausgewählt sind aus Resorcin, 2-Methylresorcin, 5-Methyl- resorcin, 2,5-Dimethylresorcin, 4-Chlorresorcin, Resorcinmonomethylether, 5-Aminophenol, 5-Amino-2-methylphenol, 5-(2-Hydroxyethyl)amino-2-methylphenol, 3- Amino-4-chlor-2-methylphenol, 3-Amino-2-chlor-6-methylphenol, 3-Amino-2,4-Dichlorphenol, 2,4-Diaminophenoxyethanol, 2-Amino-4-(2'-hydroxyethyl)amino-anisolsulfat, 1,3-Bis-(2,4-diaminophenoxy)propan, 2-Amino-3-hydroxypyridin, 2-Methylamino-3-amino-6-methoxypyridin, 2,6-Dihydroxy-3,4-dimethylpyridin, 3,5-Diamino-2,6-dimethoxypyridin, 1-Naphthol, 2-Methyl-1-naphthol, 1,5-Dihydroxynaphthalin, 2,7-Dihydroxynaphthalin, 1-Phenyl-3-methylpyrazol-5-on, 2,6-Bis-[(2'-hydroxyethyl)amino]-toluol, 4-Hydroxyindol, 6-Hydroxyindol, 6-Hydroxybenzomorpholin.

In einer ersten bevorzugten Ausführungsform enthält das Färbemittel weiterhin mindestens eine Entwicklerkomponente. Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterozyklische Hydrazone, 4-Aminopyrazolderivate sowie 2,4,5,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1) 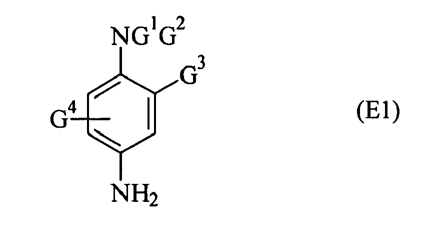 wobei
- G¹ steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁-bis C₄)-alkylrest, einen 4'-Aminophenylrest oder einen C₁- bis C₄-Alkylrest, der mit einer stickstoffhaltigen Gruppe, einem Phenyl- oder einem 4'-Aminophenylrest substituiert ist;
- G² steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁-bis C₄)-alkylrest oder einen C₁- bis C₄-Alkylrest, der mit einer stickstoffhaltigen Gruppe substituiert ist;
- G³ steht für ein Wasserstoffatom, ein Halogenatom, wie ein Chlor-, Brom-, lod- oder Fluoratom, einen C₁- bis C₄-Alkylrest, einen C₁- bis C₄-Monohydroxyalkylrest, einen C₂- bis C₄-Polyhydroxyalkylrest, einen C₁- bis C₄-Hydroxyalkoxyrest, einen C₁- bis C₄-Acetylaminoalkoxyrest, einen C₁- bis C₄- Mesylaminoalkoxyrest oder einen C₁- bis C₄-Carbamoylaminoalkoxyrest;
- G⁴ steht für ein Wasserstoffatom, ein Halogenatom oder einen C₁- bis C₄-Alkylrest oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden.

Beispiele für die als Substituenten in den erfindungsgemäß verwendeten Verbindungen genannten C₁- bis C₄-Alkylreste sind die Gruppen Methyl, Ethyl, Propyl, Isopropyl und Butyl. Ethyl und Methyl sind bevorzugte Alkylreste. Erfindungsgemäß bevorzugte C₁- bis C₄-Alkoxyreste sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (E1) sind insbesondere die Aminogruppen, C₁- bis C₄-Monoalkylaminogruppen, C₁- bis C₄-Dialkylaminogruppen, C₁- bis C₄-Trialkylammoniumgruppen, C₁- bis C₄-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)amino-2-methylanilin, 4-N,N-Bis-(β-hydroxyethyl)amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbemitteln gemäß der Erfindung verwendet werden können, kann man insbesondere die Verbindungen nennen, die der folgenden Formel (E2) entsprechen, sowie ihre physiologisch verträglichen Salze: 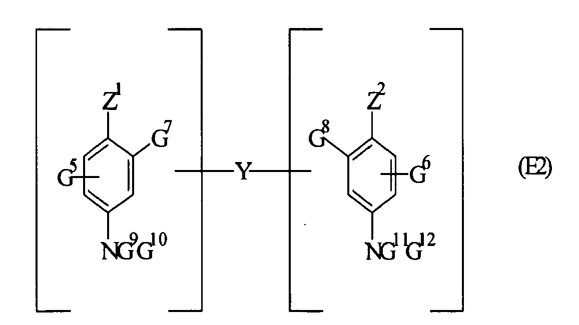 wobei:
- Z¹ und Z² stehen unabhängig voneinander für einen Hydroxyl- oder NH₂-Rest, der gegebenenfalls durch einen C₁- bis C₄-Alkylrest, durch einen C₁- bis C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁- bis C₈-Alkoxyreste substituiert sein kann, oder eine direkte Bindung,
- G⁵ und G⁶ stehen unabhängig voneinander für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen C₁- bis C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁- bis C₄-Alkylrest,
mit den Maßgaben, dass
- die Verbindungen der Formel (E2) nur eine Verbrückung Y pro Molekül enthalten und
- die Verbindungen der Formel (E2) mindestens eine Aminogruppe enthalten, die mindestens ein Wasserstoffatom trägt.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'- bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (E2) sind N,N'-Bis-(β-hydroxyethyl)-N,N'-bis-(4'-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 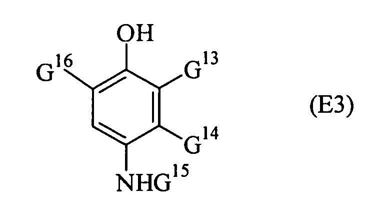 wobei:
- G¹³ steht für ein Wasserstoffatom, ein Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest, einen Hydroxy-(C₁- bis C₄)-alkylaminorest, einen C₁- bis C₄-Hydroxyalkoxyrest, einen C₁- bis C₄-Hydroxyalkyl-(C₁-bis C₄)-aminoalkylrest oder einen (Di-C₁- bis C₄-Alkylamino)-(C₁- bis C₄)-alkylrest, und
- G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest oder einen C₁- bis C₄-Cyanoalkylrest,
- G¹⁵ steht für Wasserstoff, einen C₁- bis C₄-Alkylrest, einen C₁- bis C₄-Monohydroxyalkylrest, einen C₂- bis C₄-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterozyklischen Entwicklerkomponenten, wie beispielsweise den Pyridin-, Pyrimidin-, Pyrazol-, Pyrazol-PyrimidinDerivaten und ihren physiologisch verträglichen Salzen.

Bevorzugte Pyridin-Derivate sind insbesondere die Verbindungen 2,5-Diamino-pyridin, 2-(4'-Methoxyphenyl)amino-3-amino-pyridin, 2,3-Diamino-6-methoxy-pyridin, 2-(β-Methoxyethyl)amino-3-amino-6-methoxy-pyridin und 3,4-Diamino-pyridin.

Bevorzugte Pyrimidin-Derivate sind insbesondere 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(□-aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)amino-1-methylpyrazol.

Bevorzugte Pyrazol-Pyrimidin-Derivate sind insbesondere die Derivate des Pyrazol-[1,5-a]-pyrimidin der folgenden Formel (E4) und dessen tautomeren Formen, sofern ein tautomeres Gleichgewicht besteht: 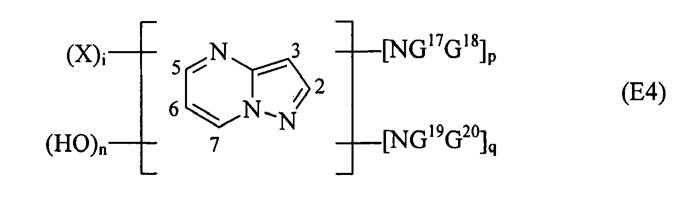 wobei:
- G¹⁷, G¹⁸, G¹⁹ und G²⁰ unabhängig voneinander stehen für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest einen (C₁- bis C₄)-Alkoxy-(C₁- bis C₄)-alkylrest, einen C₁- bis C₄-Aminoalkylrest, der gegebenenfalls durch ein Acetyl-Ureid- oder einen Sulfonyl-Rest geschützt sein kann, einen (C₁- bis C₄)-Alkylamino-(C₁- bis C₄)-alkylrest, einen Di-[(C₁- bis C₄)-alkyl]-(C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄)-[Hydroxyalkyl]-(C₁- bis C₄)-aminoalkylrest,
- die X-Reste stehen unabhängig voneinander für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest, einen C₁- bis C₄-Aminoalkylrest, einen (C₁- bis C₄)-Alkylamino-(C₁- bis C₄)-alkylrest, einen Di-[(C₁- bis C₄)alkyl]- (C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄-hydroxyalkyl)aminoalkylrest, einen Aminorest, einen C₁- bis C₄-Alkyl- oder Di-(C₁- bis C₄-hydroxyalkyl)aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
mit der Maßgabe, dass
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (E4) verwendeten Substituenten sind erfindungsgemäß analog zu den obigen Ausführungen definiert.

Wenn das Pyrazol-[1,5-a]-pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird:

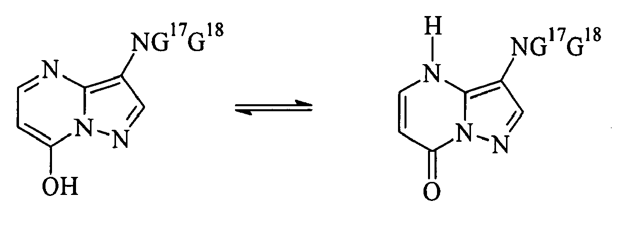

Unter den Pyrazol-[1,5-a]-pyrimidinen der obenstehenden Formel (E4) kann man insbesondere nennen:
- Pyrazol-[1,5-a]-pyrimidin-3,7-diamin;
- 2,5-Dimethyl-pyrazol-[1,5-a]-pyrimidin-3,7-diamin;
- Pyrazol-[1,5-a]-pyrimidin-3,5-diamin;
- 2,7-Dimethyl-pyrazol-[1,5-a]-pyrimidin-3,5-diamin;
- 3-Aminopyrazol-[1,5-a]-pyrimidin-7-ol;
- 3-Aminopyrazol-[1,5-a]-pyrimidin-5-ol;
- 2-(3-Aminopyrazol-[1,5-a]-pyrimidin-7-ylamino)-ethanol;
- 2-(7-Aminopyrazol-[1,5-a]-pyrimidin-3-ylamino)-ethanol;
- 2-[(3-Aminopyrazol-[1,5-a]-pyrimidin-7-yl)-(2-hydroxy-ethyl)amino]-ethanol;
- 2-[(7-Aminopyrazol-[1,5-a]-pyrimidin-3-yl)-(2-hydroxy-ethyl)amino]-ethanol;
- 5,6-Dimethylpyrazol-[1,5-a]-pyrimidin-3,7-diamin;
- 2,6-Dimethylpyrazol-[1,5-a]-pyrimidin-3,7-diamin;
- 3-Amino-7-dimethylamino-2,5-dimethylpyrazol-[1,5-a]-pyrimidin;
sowie ihre physiologisch verträglichen Salze und ihre tautomeren Formen, wenn ein tautomers Gleichgewicht vorhanden ist.

Die Pyrazol-[1,5-a]-pyrimidine der obenstehenden Formel (E4) können wie in der Literatur beschrieben durch Zyklisierung ausgehend von einem Aminopyrazol oder von Hydrazin hergestellt werden.

In einer weiteren bevorzugten Ausführungsform enthalten die erfindungsgemäßen Färbemittel mindestens eine Kupplerkomponente.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Erfindungsgemäß bevorzugte Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol und 1-Amino-3-bis-(2'-hydroxyethyl)aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)amino-3,4-methylendioxybenzol.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer zweiten bevorzugten Ausführungsform enthalten die Färbemittel mindestens ein Indol- und/oder Indolinderivat.

Besonders gut als Vorstufen naturanaloger Haarfarbstoffe geeignet sind Derivate des 5,6-Dihydroxyindolins der Formel (NA-la), 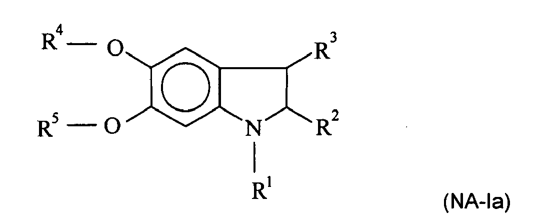 in der unabhängig voneinander
- R¹ steht für Wasserstoff, eine C₁-C₄-Alkylgruppe oder eine C₁-C₄-Hydroxy-alkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (NA-Ib), 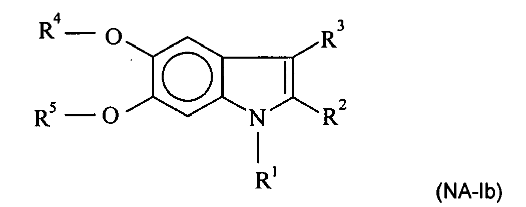 in der unabhängig voneinander
- R¹ steht für Wasserstoff, eine C₁-C₄-Alkylgruppe oder eine C₁-C₄-Hydroxyalkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den im Rahmen der erfindungsgemäßen Verwendung eingesetzten Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

In einer weiteren Ausführungsform kann es erfindungsgemäß bevorzugt sein, das Indolin- oder Indolderivat in Haarfärbemitteln in Kombination mit mindestens einer Aminosäure oder einem Oligopeptid einzusetzen. Die Aminosäure ist vorteilhafterweise eine α-Aminosäure; ganz besonders bevorzugte α-Aminosäuren sind Arginin, Ornithin, Lysin, Serin und Histidin, insbesondere Arginin.

Bevorzugte erfindungsgemäße Mittel sind dadurch gekennzeichnet, daß sie mindestens einen Farbstoffvorläufer aus den Gruppen der aromatischen und heteroaromatischen Diamine, Aminophenole, Naphthole, Polyphenole CH-aciden Kupplerkomponenten und ihrer Derivate in Mengen von 0,01 bis 25 Gew.%, vorzugsweise von 0,5 bis 10 Gew.%, insbesondere von 1 bis 5 Gew.-%, jeweils bezogen auf das gesamte Mittel, enthalten.

Zusätzlich oder anstelle von Farbstoffvorprodukten können die erfindungsgemäßen Färbemittel zur Nuancierung oder zur Färbung einen oder mehrere direktziehende Farbstoffe enthalten. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)amino-2-nitrobenzol, 3-Nitro-4-(β-hydroxyethyl)aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Entsprechende erfindungsgemäße kosmetische Mittel, die mindestens einen direktziehenden Farbstoff enthalten, der ausgewählt ist aus Nitrophenylendiaminen, Nitroaminophenolen, Azofarbstoffen, Anthrachinonen oder Indophenolen, vorzugsweise aus der Gruppe der unter den internationalen Bezeichnungen bzw. Handelsnamen bekannten Farbstoffe HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2-Hydroxyethyl)amino-4,6-dinitrophenol, 1-(2'-Hydroxyethyl)amino-4-methyl-2-nitrobenzol, 1-Amino-4-(2-hydroxyethyl)-amino-5-chlor-2-nitrobenzol, 4-Amino-3-nitrophenol, 1-(2-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol, sind bevorzugte Ausführungsformen der vorliegenden Erfindung.

Unter den vorstehend genannten Farbstoffen sind einige Vertreter besonders bevorzugt, weshalb weiter bevorzugte erfindungsgemäße Mittel, die dadurch gekennzeichnet sind, daß sie mindestens einen Direktzieher, ausgewählt aus Basic Blue 7, Basic Blue 99, Basic Violet 14, Basic Brown 16, Basic Brown 17, Basic Orange 31, Basic Red 46, Basic Red 51, Basic Red 76, Basic Yellow 57, Basic Yellow 87, Acid Black 1, Acid Blue 7, Acid Violet 43, Acid Red 23, Acid Red 52, Acid Orange 7, Acid Yellow 1, Acid Yellow 10, Acid Yellow 36, Food Green 3, Pigment Red 57-1, Disperse Black 9, Disperse Blue 1, Disperse Blue 3, Disperse Violet 1, Disperse Violet 4, HC Orange 1, HC Red 1, HC Red 3, HC Red 13, HC Yellow 2, HC Yellow 4, Na-Pikramat, 1,4-Bis-(2'-hydroxyethyl)amino-2-nitro- p-phenylendiamin, HC Yellow 5, HC Blue 2, HC Blue 12, 4-Amino-3-nitrophenol, HC Yellow 6, HC Yellow 12, 2-Nitro-1-(2'hydroxyethyl)amino-4-methylbenzol, 2-Nitro-4-amino-diphenylamin-2-carbonsäure, 2-Amino-6-chlor-4-nitrophenol, HC Red BN; 6-Nitro-1,2,3,4-tetranitrochinoxalin, o-Nitro-p-phenylendiamin, p-Nitro-m-phenylendiamin, HC Red B 54, HC Red 10, HC Red 11, HC Red 13, 2-(2'-Hydroxyethyl)amino-1-hydroxy-4,6-dinitrobenzol, 4-Ethylamino-3-nitrobenzoesäure, 2-Chlor-6-ethylamino-4-nitrophenol, 2-Hydroxy-1,4-napthochinon, 1-Propen-(4-amino-2-nitrophenyl)amin, Isatin, N-Methylylisatin, HC Violet 1, HC Violet 2, 4-Nitrophenyl-aminoethylharnstoff in Mengen von 0,01 bis 25 Gew.%, vorzugsweise von 0,5 bis 10 Gew.%, insbesondere von 1 bis 5 Gew.-%, jeweils bezogen auf das gesamte Mittel, enthalten, bevorzugt sind.

Ferner können die erfindungsgemäßen Mittel einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterozyklus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

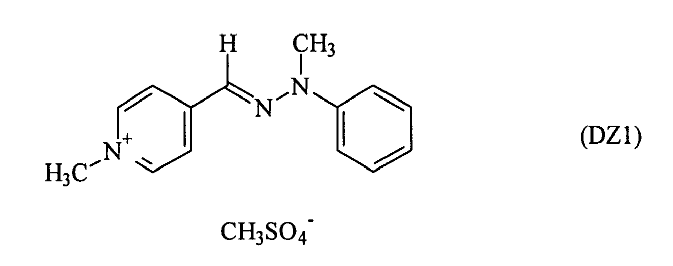

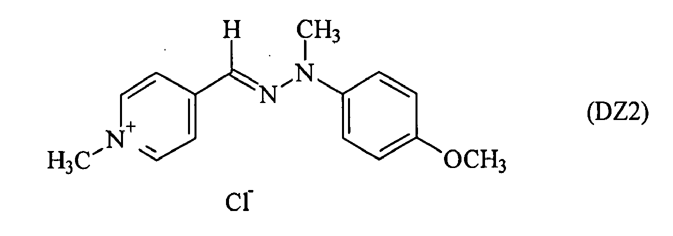

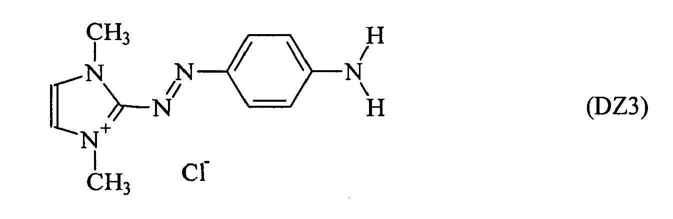

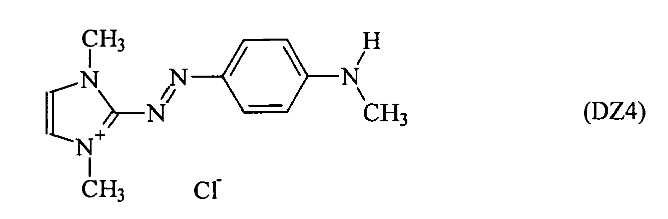

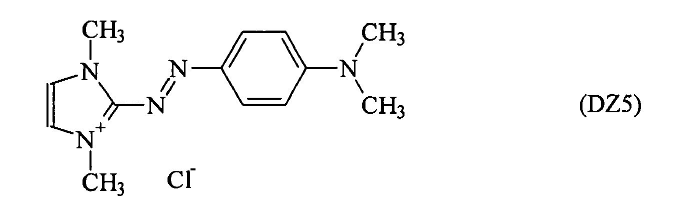

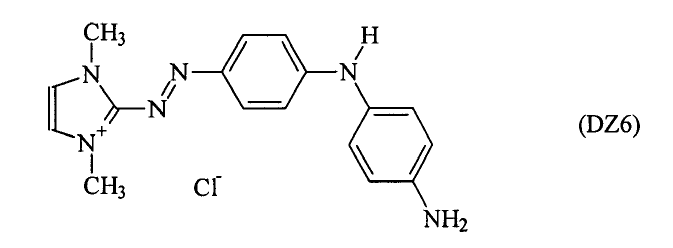

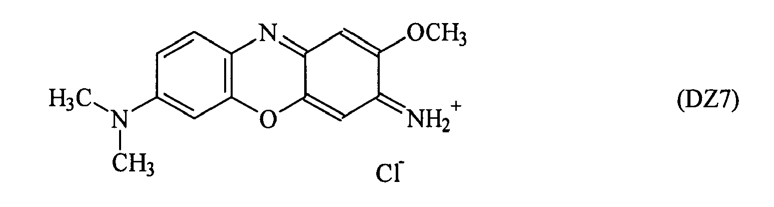

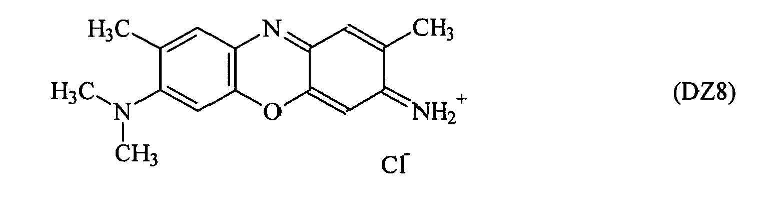

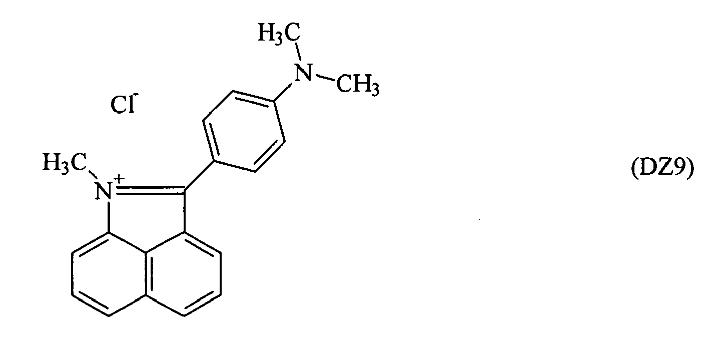

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

In einer weiteren Ausführungsform können erfindungsgemäße Färbemittel auch so formuliert werden, daß die farbgebenden Komponenten durch Reaktion von
A Verbindungen, die eine reaktive Carbonylgruppe enthalten mit
B Verbindungen, ausgewählt aus (a) CH-aciden Verbindungen, (b) Verbindungen mit primärer oder sekundärer Aminogruppe oder Hydroxygruppe, ausgewählt aus primären oder sekundären aromatischen Aminen, stickstoffhaltigen heterozyklischen Verbindungen und aromatischen Hydroxyverbindungen, (c) Aminosäuren, (d) aus 2 bis 9 Aminosäuren aufgebauten Oligopeptiden
entstehen.

Verbindungen mit einer reaktiven Carbonylgruppe (im Folgenden auch reaktive Carbonylverbindungen) besitzen mindestens eine Carbonylgruppe als reaktive Gruppe, welche mit Verbindungen wie (a) CH-aciden Verbindungen, (b) Verbindungen mit primärer oder sekundärer Aminogruppe oder Hydroxygruppe, ausgewählt aus primären oder sekundären aromatischen Aminen, stickstoffhaltigen heterozyklischen Verbindungen und aromatischen Hydroxyverbindungen, (c) Aminosäuren, (d) aus 2 bis 9 Aminosäuren aufgebauten Oligopeptiden unter Ausbildung einer beide Komponenten verknüpfenden chemischen Bindung reagiert. Ferner sind erfindungsgemäß auch solche Verbindungen als reaktive Carbonylverbindung umfaßt, in denen die reaktive Carbonylgruppe derart derivatisiert bzw. maskiert ist, daß die Reaktivität des Kohlenstoffatoms der derivatisierten bzw. maskierten Carbonylgruppe gegenüber dem Reaktionspartner (a) bis (d) stets vorhanden ist. Diese Derivate sind bevorzugt Kondensationsverbindungen von reaktiven Carbonylverbindungen mit
a) Aminen und deren Derivate unter Bildung von Iminen oder Oximen als Kondensationsverbindung
b) von Alkoholen unter Bildung von Acetalen oder Ketalen als Kondensationsverbindung.

Solche erfindungsgemäßen Mittel lassen sich formulieren, indem man zusätzlich zu der Zubereitung A (die Aniontensid(e) enthält) und der Zubereitung B (die ionische Flüssigkeit(en) enthält) eine dritte Zubereitung bereitstellt, welche eine der genannten Reaktionskomponenten enthält. Die andere Reaktionskomponente kann in die Zubereitung A und/oder die Zubereitung B inkorporiert werden. Die Vermischung der drei Zubereitungen unmittelbar vor der Anwendung ergibt dann das Färbemittel.

Demnach sind einerseits kosmetische Mittel, bei denen die Zubereitung A und/oder die Zubereitung B mindestens eine C,H-acide Verbindung enthalten und dem Mittel unmittelbar vor der Anwendung eine weitere Zubereitung C zugemischt wird, die mindestens eine reaktive Carbonylverbindung enthält und andererseits kosmetische Mittel, bei denen die Zubereitung A und/oder die Zubereitung B mindestens eine reaktive Carbonylverbindung enthalten und dem Mittel unmittelbar vor der Anwendung eine weitere Zubereitung C zugemischt wird, die mindestens eine C,H-acide Verbindung enthält, bevorzugte Ausführungsformen der vorliegenden Erfindung.

Die Komponente A wird bevorzugt ausgewählt aus der Gruppe, die gebildet wird aus Acetophenon, Propiophenon, 2-Hydroxyacetophenon, 3-Hydroxyacetophenon, 4-Hydroxyacetophenon, 2-Hydroxypropiophenon, 3-Hydroxypropiophenon, 4-Hydroxypropiophenon, 2-Hydroxybutyrophenon, 3-Hydroxybutyrophenon, 4-Hydroxybutyrophenon, 2,4-Dihydroxyacetophenon, 2,5-Dihydroxyacetophenon, 2,6-Dihydroxyacetophenon, 2,3,4-Trihydroxyacetophenon, 3,4,5-Trihydroxyacetophenon, 2,4,6-Trihydroxyacetophenon, 2,4,6-Trimethoxyacetophenon, 3,4,5-Trimethoxyacetophenon, 3,4,5-Trimethoxy-acetophenon-diethylketal, 4-Hydroxy-3-methoxy-acetophenon, 3,5-Dimethoxy-4-hydroxyacetophenon, 4-Aminoacetophenon, 4-Dimethylaminoacetophenon, 4-Morpholinoacetophenon, 4-Piperidinoacetophenon, 4-Imidazolinoacetophenon, 2-Hydroxy-5-brom-acetophenon, 4-Hydroxy-3-nitroacetophenon, Acetophenon-2-carbonsäure, Acetophenon-4-carbonsäure, Benzophenon, 4-Hydroxybenzophenon, 2-Aminobenzophenon, 4,4'-Dihydroxybenzophenon, 2,4-Dihydroxybenzophenon, 2,4,4'-Trihydroxybenzophenon, 2,3,4-Trihydroxybenzophenon, 2-Hydroxy-1-acetonaphthon, 1-Hydroxy-2-acetonaphthon, Chromon, Chromon-2-carbonsäure, Flavon, 3-Hydroxyflavon, 3,5,7-Trihydroxyflavon, 4',5,7-Trihydroxyflavon, 5,6,7-Trihydroxyflavon, Quercetin, 1-Indanon, 9-Fluorenon, 3-Hydroxyfluorenon, Anthron, 1,8-Dihydroxyanthron, Vanillin, Coniferylaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxy-benzaldehyd, 4-Hydroxy-2,6-dimethoxy-benzaldehyd, 4-Hydroxy-2-methyl-benzaldehyd, 4-Hydroxy-3-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 4-Hydroxy-3,5-dimethoxy-benzaldehyd, 4-Hydroxy-3,5-dimethylbenzaldehyd, 3,5-Diethoxy-4-hydroxy-benzaldehyd, 2,6-Diethoxy-4-hydroxy-benzaldehyd, 3-Hydroxy-4-methoxy-benzaldehyd, 2-Hydroxy-4-methoxy-benzaldehyd, 2-Ethoxy-4-hydroxybenzaldehyd, 3-Ethoxy-4-hydroxy-benzaldehyd, 4-Ethoxy-2-hydroxy-benzaldehyd, 4-Ethoxy-3-hydroxy-benzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,5-Dihydroxybenzaldehyd, 2,6-Dihydroxybenzaldehyd, 3,4-Dihydroxybenzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 4-Hydroxy-2-methoxybenzaldehyd, 4-Dimethylaminobenzaldehyd, 4-Diethylaminobenzaldehyd, 4-Dimethylamino-2-hydroxybenzaldehyd, 4-Diethylamino-2-hydroxybenzaldehyd, 4-Pyrrolidinobenzaldehyd, 4-Morpholinobenzaldehyd, 2-Morpholinobenzaldehyd, 4-Piperidinobenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Hydroxy-1-naphthaldehyd, 4-Dimethylamino-1-naphthaldehyd, 2-Methoxy-zimtaldehyd, 4-Methoxy-zimtaldehyd, 4-Hydroxy-3-methoxy-zimtaldehyd, 3,5-Dimethoxy-4-hydroxy-zimtaldehyd, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylaminozimtaldehyd, 4-Dibutylamino-benzaldehyd, 4-Diphenylamino-benzaldehyd, 4-Dimethylamino-2-methoxybenzaldehyd, 4-(1-lmidazolyl)-benzaldehyd, Piperonal, 2,3,6,7-Tetrahydro-1H,5H-benzo[ij]chinolizin-9-carboxaldehyd, 2,3,6,7-Tetrahydro-8-hydroxy-1H,5H-benzo[ij]chinolizin-9-carboxaldehyd, N-Ethylcarbazol-3-aldehyd, 2-Formylmethylen-1,3,3-trimethylindolin (Fischers Aldehyd oder Tribasen Aldehyd),
2-Indolaldehyd, 3-Indolaldehyd, 1-Methylindol-3-aldehyd, 2-Methylindol-3-aldehyd, 1-Acetylindol-3-aldehyd, 3-Acetylindol, 1-Methyl-3-acetylindol, 2-(1',3',3'-Trimethyl-2-indolinyliden)-acetaldehyd, 1-Methylpyrrol-2-aldehyd, 1-Methyl-2-acetylpyrrol, 4-Pyridinaldehyd, 2-Pyridinaldehyd, 3-Pyridinaldehyd, 4-Acetylpyridin, 2-Acetylpyridin, 3-Acetylpyridin, Pyridoxal, Chinolin-3-aldehyd, Chinolin-4-aldehyd, Antipyrin-4-aldehyd, Furfural, 5-Nitrofurfural, 2-Thenoyl-trifluor-aceton, Chromon-3-aldehyd, 3-(5'-Nitro-2'-furyl)-acrolein, 3-(2'-Furyl)-acrolein und Imidazol-2-aldehyd, 1,3-Diacetylbenzol, 1,4-Diacetylbenzol, 1,3,5-Triacetylbenzol, 2-Benzoyl-acetophenon, 2-(4'-Methoxybenzoyl)-acetophenon, 2-(2'-Furoyl)-acetophenon, 2-(2'-Pyridoyl)-acetophenon und 2-(3'-Pyridoyl)-acetophenon,
Benzylidenaceton, 4-Hydroxybenzylidenaceton, 2-Hydroxybenzylidenaceton, 4-Methoxybenzylidenaceton, 4-Hydroxy-3-methoxybenzylidenaceton, 4-Dimethylaminobenzylidenaceton, 3,4-Methylendioxybenzylidenaceton, 4-Pyrrolidinobenzylidenaceton, 4-Piperidinobenzylidenaceton, 4-Morpholinobenzylidenaceton, 4-Diethylaminobenzylidenaceton, 3-Benzyliden-2,4-pentandion, 3-(4'-Hydroxybenzyliden)-2,4-pentandion, 3-(4'-Dimethylaminobenzyliden)-2,4-pentandion, 2-Benzylidencyclohexanon, 2-(4'-Hydroxybenzyliden)-cyclohexanon, 2-(4'-Dimethylaminobenzyliden)-cyclohexanon, 2-Benzyliden-1,3-cyclohexandion, 2-(4'-Hydroxybenzyliden)-1,3-cyclohexandion, 3-(4'-Dimethylaminobenzyliden)-1,3-cyclohexandion, 2-Benzyliden-5,5-dimethyl-1,3-cyclohexandion, 2-(4'-Hydroxybenzyliden)-5,5-dimethyl-1,3-cyclohexandion, 2-(4'-Hydroxy-3-methoxybenzyliden)-5,5-dimethyl-1,3-cyclohexandion, 2-(4'-Dimethylaminobenzyliden)-5,5-dimethyl-1,3-cyclohexandion, 2-Benzylidencyclopentanon, 2'-(4-Hydroxybenzyliden)-cyclopentanon, 2-(4'-Dimethylaminobenzyliden)-cyclopentanon,
5-(4-Dimethylaminophenyl)penta-2,4-dienal, 5-(4-Diethylaminophenyl)penta-2,4-dienal, 5-(4-Methoxyphenyl)penta-2,4-dienal, 5-(3,4-Dimethoxyphenyl)penta-2,4-dienal, 5-(2,4-Dimethoxyphenyl)penta-2,4-dienal, 5-(4-Piperidinophenyl)penta-2,4-dienal, 5-(4-Morpholinophenyl)penta-2,4-dienal, 5-(4-Pyrrolidinophenyl)penta-2,4-dienal,
6-(4-Dimethylaminophenyl)hexa-3,5-dien-2-on, 6-(4-Diethylaminophenyl)hexa-3,5-dien-2-on, 6-(4-Methoxyphenyl)hexa-3,5-dien-2-on, 6-(3,4-Dimethoxyphenyl)hexa-3,5-dien-2-on, 6-(2,4-Dimethoxyphenyl)hexa-3,5-dien-2-on, 6-(4-Piperidinophenyl)hexa-3,5-dien-2-on, 6-(4-Morpholinophenyl)-hexa-3,5-dien-2-on, 6-(4-Pyrrolidinophenyl)hexa-3,5-dien-2-on,
5-(4-Dimethylamino-1-naphthyl)penta-3,5-dienal, 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 4-Hydroxy-3-nitrobenzaldehyd, 5-Hydroxy-2-nitrobenzaldehyd, 2-Hydroxy-5-nitrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Dinitrobenzaldehyd, 2,6-Dinitrobenzaldehyd, 2-Hydroxy-3-methoxy-5-nitrobenzaldehyd, 4,5-Dimethoxy-2-nitrobenzaldehyd, 6-Nitropiperonal, 2-Nitropiperonal, 5-Nitrovanillin, 2,5-Dinitrosalicylaldehyd, 5-Brom-3-nitrosalicylaldehyd, 3-Nitro-4-formylbenzolsulfonsäure, 4-Nitro-1-naphthaldehyd, 2-Nitrozimtaldehyd, 3-Nitrozimtaldehyd, 4-Nitrozimtaldehyd, 9-Methyl-3-carbazolaldehyd, 9-Ethyl-3-carbazolaldehyd, 3-Acetylcarbazol, 3,6-Diacetyl-9-ethylcarbazol, 3-Acetyl-9-methylcarbazol, 1,4-Dimethyl-3-carbazolaldehyd, 1,4,9-Trimethyl-3-carbazolaldehyd,
4-Formyl-1-methylpyridinium-, 2-Formyl-1-methylpyridinium-, 4-Formyl-1-ethylpyridinium-, 2-Formyl-1-ethylpyridinium-, 4-Formyl-1-benzylpyridinium-, 2-Formyl-1-benzylpyridinium-, 4-Formyl-1,2-dimethylpyridinium-, 4-Formyl-1,3-dimethylpyridinium-, 4-Formyl-1-methylchinolinium-, 2-Formyl-1-methylchinolinium-, 4-Acetyl-1-methylpyridinium-, 2-Acetyl-1-methylpyridinium-, 4-Acetyl-1-methylchinolinium-, 5-Formyl-1-methylchinolinium-, 6-Formyl-1-methylchinolinium-, 7-Formyl-1-methylchinolinium-, 8-Formyl-1-methylchinolinium, 5-Formyl-1-ethylchinolinium-, 6-Formyl-1-ethylchinolinium-, 7-Formyl-1-ethylchinolinium-, 8-Formyl-1-ethylchinolinium, 5-Formyl-1-benzylchinolinium-, 6-Formyl-1-benzylchinolinium-, 7-Formyl-1-benzylchinolinium-, 8-Formyl-1-benzylchinolinium, 5-Formyl-1-allylchinolinium-, 6-Formyl-1-allylchinolinium-, 7-Formyl-1-allylchinolinium- und 8-Formyl-1-allylchinolinium-, 5-Acetyl-1-methylchinolinium-, 6-Acetyl-1-methylchinolinium-, 7-Acetyl-1-methylchinolinium-, 8-Acetyl-1-methylchinolinium, 5-Acetyl-1-ethylchinolinium-, 6-Acetyl-1-ethylchinolinium-, 7-Acetyl-1-ethylchinolinium-, 8-Acetyl-1-ethylchinolinium, 5-Acetyl-1-benzylchinolinium-, 6-Acetyl-1-benzylchinolinium-, 7-Acetyl-1-benzylchinolinium-, 8-Acetyl-1-benzylchinolinium, 5-Acetyl-1-allylchinolinium-, 6-Acetyl-1-allylchinolinium-, 7-Acetyl-1-allylchinolinium- und 8-Acetyl-1-allylchinolinium, 9-Formyl-10-methyl-acridinium-, 4-(2'-Formylvinyl)-1-methylpyridinium-, 1,3-Dimethyl-2-(4'-formylphenyl)-benzimidazolium-, 1,3-Dimethyl-2-(4'-formylphenyl)-imidazolium-, 2-(4'-Formylphenyl)-3-methylbenzothiazolium-, 2-(4'-Acetylphenyl)-3-methylbenzothiazolium-, 2-(4'-Formylphenyl)-3-methylbenzoxazolium-, 2-(5'-Formyl-2'-furyl)-3-methylbenzothiazolium-, 2-(5'-Formyl-2'-furyl)-3-methylbenzothiazolium-, 2-(5'-Formyl-2'-thienyl)-3-methylbenzothiazolium-, 2-(3'-Formylphenyl)-3-methylbenzothiazolium-, 2-(4'-Formyl-1-naphthyl)-3-methylbenzothiazolium-, 5-Chlor-2-(4'-formylphenyl)-3-methylbenzothiazolium-, 2-(4'-Formylphenyl)-3,5-dimethylbenzothiazoliumbenzolsulfonat, -p-toluolsulfonat, -methansulfonat, -perchlorat, -sulfat, -chlorid, -bromid, -iodid, - tetrachlorozinkat, -methylsulfat-, trifluormethansulfonat, -tetrafluoroborat,
Isatin, 1-Methyl-isatin, 1-Allyl-isatin, 1-Hydroxymethyl-isatin, 5-Chlor-isatin, 5-Methoxy-isatin, 5-Nitroisatin, 6-Nitro-isatin, 5-Sulfo-isatin, 5-Carboxy-isatin, Chinisatin, 1-Methylchinisatin, sowie beliebigen Gemischen der voranstehenden Verbindungen.

Als CH-acide werden im allgemeinen solche Verbindungen angesehen, die ein an ein aliphatisches Kohlenstoffatom gebundenes Wasserstoffatom tragen, wobei aufgrund von Elektronen-ziehenden Substituenten eine Aktivierung der entsprechenden Kohlenstoff-Wasserstoff-Bindung bewirkt wird. Unter CH-acide Verbindungen fallen erfindungsgemäß auch Enamine, die durch alkalische Behandlung von quaternierten N-Heterozyklen mit einer in Konjugation zum quartären Stickstoff stehenden CH-aciden Alkylgruppe entstehen.

Die CH-aciden Verbindungen der Komponente B sind bevorzugt ausgewählt aus der Gruppe bestehend aus 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, 1,3,3-Trimethyl-2-methylenindolin (Fischersche Base), 2,3-Dimethyl-benzothiazoliumiodid, 2,3-Dimethyl-benzothiazolium-p-toluolsulfonat, 2,3-Dimethyl-naphtho[1,2-d]thiazolium-p-toluolsulfonat, 3-Ethyl-2-methyl-naphtho[1,2-d]thiazolium-p-toluolsulfonat, Rhodanin, Rhodanin-3-essigsäure, 1,4-Dimethylchinolinium-iodid, 1,2-Dimethylchinolinium-iodid, Barbitursäure, Thiobarbitursäure, 1,3-Dimethylthiobarbitursäure, 1,3-Diethylthiobarbitursäure, 1,3-Diethylbarbitursäure, Oxindol, 3-Indoxylacetat, 2-Cumaranon, 5-Hydroxy-2-cumaranon, 6-Hydroxy-2-cumaranon, 3-Methyl-1-phenylpyrazolin-5-on, Indan-1,2-dion, Indan-1,3-dion, Indan-1-on, Benzoylacetonitril, 3-Dicyanmethylenindan-1-on, 2-Amino-4-imino-1,3-thiazolin-hydrochlorid, 5,5-Dimethylcyclohexan-1,3-dion, 2H-1,4-Benzoxazin-4H-3-on, 3-Ethyl-2-methyl-benzoxazoliumiodid, 3-Ethyl-2-methyl-benzothiazoliumiodid, 1-Ethyl-4-methyl-chinoliniumiodid, 1-Ethyl-2-methylchinoliniumiodid, 1,2,3-Trimethylchinoxaliniumiodid, 3-Ethyl-2-methyl-benzoxazolium-p-toluolsulfonat, 3-Ethyl-2-methyl-benzothiazolium-p-toluolsulfonat, 1-Ethyl-4-methyl-chinolinium-p-toluolsulfonat, 1-Ethyl-2-methylchinolinium-p-toluolsulfonat, und 1,2,3-Trimethylchinoxalinium-p-toluolsulfonat.

Eine oxidative Färbung der Fasern kann in Gegenwart von Oxidationsfarbstoffvorprodukten grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Dieser Aufhelleffekt kann unabhängig von der Färbemethode gewünscht sein. Die Gegenwart von Oxidationsfarbstoffvorprdukten ist demnach keine zwingende Voraussetzung für einen Einsatz von Oxidationsmitteln in den erfindungsgemäß verwendeten Mitteln. Als Oxidationsmittel kommen Persulfate, Chlorite und insbesondere Wasserstoffperoxid oder dessen Anlagerungsprodukte an Harnstoff, Melamin sowie Natriumborat in Frage.

Die erfindungsgemäßen Färbemittel können demnach zusätzlich eine Peroxoverbindung enthalten. Die Auswahl dieser Peroxoverbindung unterliegt prinzipiell keinen Beschränkungen; übliche, dem Fachmann bekannte Peroxoverbindungen sind beispielsweise Ammoniumperoxidisulfat, Kaliumperoxidisulfat, Natriumperoxidisulfat, Ammoniumpersulfat, Kaliumpersulfat, Natriumpersulfat, Kaliumperoxidiphosphat, Percarbonate wie Magnesiumpercarbonat, Peroxide wie Bariumperoxid sowie Perborate, Harnstoffperoxid und Melaminperoxid. Unter diesen Peroxoverbindungen, die auch in Kombination eingesetzt werden können, sind erfindungsgemäß die anorganischen Verbindungen bevorzugt. Besonders bevorzugt sind die Peroxidisulfate, insbesondere Kombinationen aus mindestens zwei Peroxidisulfaten. Erfindungsgemäß bevorzugte Mittel sind dadurch gekennzeichnet, dass sie zusätzlich ein Peroxodisulfat enthalten.

Die Peroxoverbindungen sind in den erfindungsgemäßen Mitteln bevorzugt in Mengen von 2-80 Gew.-%, insbesondere in Mengen von 5-50 Gew.-% enthalten.

Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z.B. Metallionen, Iodide, Chinone oder bestimmte Enzyme.

Geeignete Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Zusätzlich können die erfindungsgemäß verwendeten Mittel weitere Inhaltsstoffe enthalten. Ein Einsatz bestimmter Metallionen oder -komplexe kann beispielsweise bevorzugt sein, um intensive Färbungen zu erhalten. Hier sind erfindungsgemäß verwendete Mittel bevorzugt, die zusätzlich Cu-, Fe-, Mn-, Ru-lonen oder Komplexe dieser Ionen enthalten.

Bevorzugte erfindungsgemäße Haarfärbe- und - aufhellungsmittel enthalten 0,0001 bis 2,5 Gew.-%, vorzugsweise 0,001 bis 1 Gew.-%, bezogen auf die Gesamtzusammensetzung des Mittels, mindestens einer Verbindung aus der Gruppe Kupferchlorid (CuCl₂), Kupfersulfat (CuSO₄), Eisen(II)sulfat, Mangan(II)sulfat, Mangan(II)chlorid, Kobalt(II)chlorid, Cersulfat, Cerchlorid, Vanadiumsulfat, Kaliumjodid, Natriumjodid, Lithiumchlorid, Kaliumdichromat, Magnesiumacetat, Calciumchlorid, Calciumnitrat, Bariumnitrat, Mangandioxid (MnO₂) und/oder Hydrochinon.

Als weiteren Bestandteil können die erfindungsgemäßen Zusammensetzungen mindestens eine Ammoniumverbindung aus der Gruppe Ammoniumchlorid, Ammoniumcarbonat, Ammoniumbicarbonat, Ammoniumsulfat und/oder Ammoniumcarbamat in einer Menge von 0,5 bis 10, vorzugsweise 1 bis 5 Gew.-%, bezogen auf die Gesamtzusammensetzung des Mittels enthalten.

Bei einer Anwendung von Oxidationsmitteln wird das eigentliche Färbemittel zweckmäßigerweise unmittelbar vor der Anwendung durch Mischung der Zubereitung des Oxidationsmittels mit der Zubereitung, enthaltend die Verbindungen der Formel I und gegebenenfalls Farbstoffvorprodukte, hergestellt. Das dabei entstehende gebrauchsfertige Haarfärbepräparat sollte bevorzugt einen pH-Wert im Bereich von 6 bis 12 aufweisen.

Insbesondere bei schwer färbbarem Haar kann ein erfindungsgemäßes Mittel gegebenenfalls mit zusätzlichen Farbstoffvorprodukten aber auch ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Ein weiterer Gegenstand der vorliegenden Erfindung ist ein Verfahren zur Behandlung keratinischer Fasern, insbesondere menschlicher Haare, bei dem ein erfindungsgemäßes kosmetisches Mittel auf die Fasern aufgetragen und nach einer Einwirkzeit von 1 bis 20 Minuten wieder ausgespült wird.

Bezüglich bevorzugter erfindungsgemäßer Verfahren gilt mutatis mutandis das zu den erfindungsgemäßen Mitteln weiter oben Ausgeführte.

Die erfindungsgemäßen Mittel zeichnen sich durch eine Reihe von Vorteilen aus. Sie sind lagerstabil, lassen sich leicht anwenden und erzeugen bei der Anwendung ein angenehmes, nicht klebriges, nicht-schmierendes und nicht-fettiges Hautgefühl.

Zubereitungen zur Haarbehandlung, insbesondere zur Haarfärbung und/oder Aufhellung lassen sich ebenfalls leicht applizieren und fallen durch vorteilhafte sensorische Eigenschaften und verringerten Geruch auf. Allen erfindungsgemäßen kosmetischen Zusammensetzungen zur Haarbehandlung (Shampoos, Haarkuren, Frisiergele, Färbemittel, Blondiermittel usw.) gemeinsam ist die Eigenschaft, dem Haar verbesserte Eigenschaften zu verleihen. So werden insbesondere Frisurenhalt und Frisierbarkeit und Kämmbarkeit und Griff verbessert.

Besonders vorteilhaft ist, daß die erfindungsgemäßen Mittel bei Vereinigung der Zubereitungen A und B ihre Viskosität steigern, so daß aus zwei relativ dünnflüssigen Formulierungen ein höherviskoses Mittel bereitet werden kann. Dies vereint die Vorteile einer guten Restentleerbarkeit der einzelnen Zubereitungen aus ihren Behältnissen und einer guten Durchmischbarkeit der beiden Zubereitungen mit einer besser applizierbaren Mischung, die weniger tropft und sich vom Anwender leicht und ohne die Probleme dünnflüssiger Mittel anwenden läßt. Zusätzlich führt die Mischung aus Aniontensid(en) und ionischen(r/n) Flüssigkeit(en) zu einem stabileren, sehr feinporigen Schaum, der ebenfalls als vorteilhaft empfunden wird und sich durch eine hohe Schaumstabilität und eine cremiges Gefühl auszeichnet.

Ein weiterer Gegenstand der vorliegenden Erfindung ist daher die Verwendung von ionischen Flüssigkeiten zur Viskositätssteigerung aniontensidhaltiger Mittel.

Auch bezüglich bevorzugter erfindungsgemäßer Verwendungen gilt mutatis mutandis das zu den erfindungsgemäßen Mitteln weiter oben Ausgeführte.

Die folgenden Beispiele sollen den Gegenstand der vorliegenden Erfindung näher erläutern, ohne ihn zu beschränken. Anstell der in den Beispielen genannten ionischen Flüssigkeiten können jeweils auch andere ionische Flüssigkeiten eingesetzt werden, vorzugsweise einer der bevorzugten, in der vorstehenden Tabelle genannten ionischen Flüssigkeiten 1 bis 2420 oder Mischungen aus diesen.

### Beispiele:

Es wurden die folgenden erfindungsgemäßen Zusammensetzungen hergestellt (Angaben in Gew.-%):

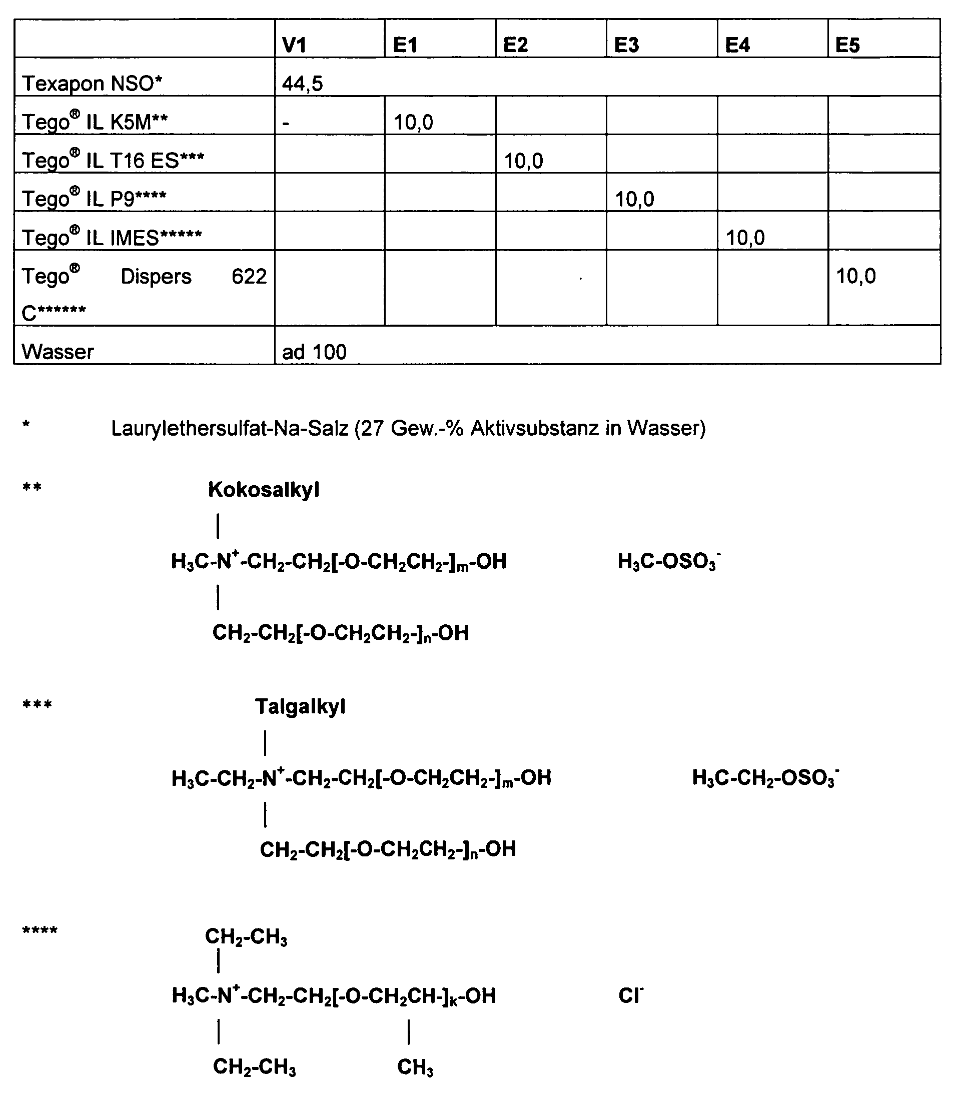

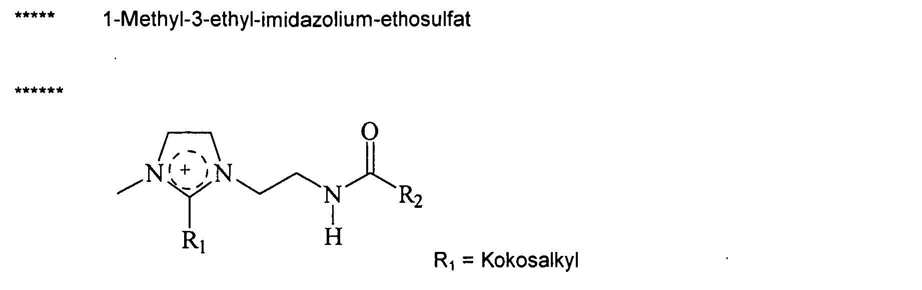

Viskosität und Schaumverhalten der einzelnen erfindungsgemäßen Rezepturen E1 bis E5 wurden gegen die Vergleichsrezeptur V1 verglichen:

| | **Viskosität** | **Schaumverhalten** |
|---|---|---|
| E1 | starke Verdickung | deutlich höhere Schaumentwicklung Schaum deutlich stabiler und feinporiger |
| E2 | geringfügige Verdickung | höhere Schaumentwicklung Schaum stabiler und feinporiger |
| E3 | geringfügige Verdickung | geringfügig höhere Schaumentwicklung Schaum feinporiger |
| E4 | geringfügige Verdickung | geringfügig höhere Schaumentwicklung Schaum feinporiger |
| E5 | geringfügige Verdickung | geringfügig höhere Schaumentwicklung Schaum feinporiger |

## Patentansprüche

1. Kosmetisches Mittel, welches unmittelbar vor der Anwendung durch Vermischen einer fließfähigen Zubereitung A und einer fließfähigen Zubereitung B erhalten wird, **dadurch gekennzeichnet, daß**
a) die Zubereitung A mindestens ein Aniontensid und
b) die Zubereitung B mindestens eine ionische Flüssigkeit enthält.

2. Kosmetisches Mittel nach Anspruch 1, **dadurch gekennzeichnet, daß** die Zubereitung A als Aniontensid(e) Alkylsulfat(e) und/oder Alkylethersulfat(e), vorzugsweise C₈₋₂₂-Alkylsulfat(e) und/oder C₈₋₂₂-Alkylethersulfat(e) mit 2 bis 20 Ethylenoxideinheiten, besonders bevorzugt C₁₀₋₁₈-Alkylsulfat(e) und/oder C₁₀₋₁₈-Alkylethersulfat(e) mit 3 bis 10 Ethylenoxideinheiten und insbesondere C₁₂₋₁₆-Alkylsulfat(e) und/oder C₁₂₋₁₆-Alkylethersulfat(e) mit 4 bis 8 Ethylenoxideinheiten, vorzugsweise in Mengen von 0,5 bis 60 Gew.-%, besonders bevorzugt von 1 bis 50 Gew.-% und insbesondere von 5 bis 40 Gew.-%, jeweils bezogen auf das Gewicht der Zubereitung A, enthält.

3. Kosmetisches Mittel nach einem der Ansprüche 1 oder 2, **dadurch gekennzeichnet, daß** die Zubereitung B bezogen auf ihr Gewicht 0,1 bis 20 Gew.-%, vorzugsweise 0,5 bis 15 Gew.-% und insbesondere 1 bis 12 Gew.-% ionische Flüssigkeit(en) enthält.

4. Kosmetisches Mittel nach einem der Ansprüche 1 bis 3, **dadurch gekennzeichnet, daß** die Zubereitung A zusätzlich Co-Tenside, vorzugsweise Aniontenside aus den Gruppen der Seifen, Ethercarbonsäuren, carboxylierten Alkylglucoside, Acylglutamate, Acylphosphate, Sulfosuccinate, Acyllactylate, Acylsarcosinate, Acyltaurate, Acylisethionate und/oder Amphotenside, zwitterionische Tenside, Niotenside, beispielsweise Alkylglucoside, Aminoxide, Ethanolamide, enthält.

5. Kosmetisches Mittel nach einem der Ansprüche 1 bis 4, **dadurch gekennzeichnet, daß** die Zubereitung B mindestens eine ionische Flüssigkeit enthält, die ausgewählt ist aus 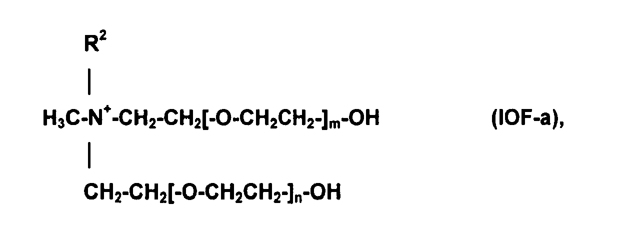 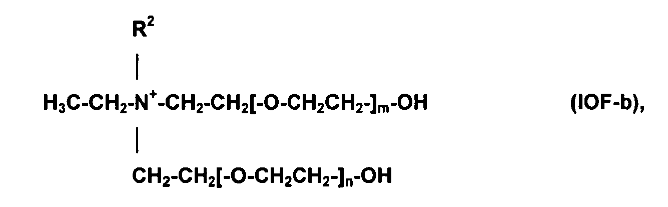 in denen die Indices m und n unabhängig voneinander jeweils für Werte von 1 bis 15, vorzugsweise von 1, 2, 3, 4, 5, 6, 7, 8, 9, 10, stehen, das Gegenion ausgewählt ist aus Chlorid, Bromid, Methosulfat, Ethosulfat und der Rest R² ein gesättigter oder ungesättigter langkettiger Fettrest ist.

6. Kosmetisches Mittel nach Anspruch 5, **dadurch gekennzeichnet, daß** die Zubereitung B mindestens eine ionische Flüssigkeit der Formel 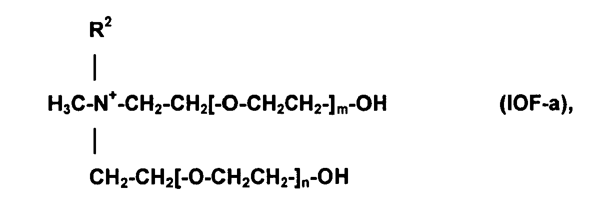 enthält, in der die Indices m und n unabhängig voneinander jeweils für Werte von 1 bis 7, vorzugsweise von 2, 3, 4, 5 stehen, das Gegenion ausgewählt ist aus Methosulfat, Ethosulfat und der Rest R² ein gesättigter oder ungesättigter langkettiger Fettrest, vorzugsweise ein C₈₋₁₈-Alkyl- oder Alkenylrest ist.

7. Kosmetisches Mittel nach einem der Ansprüche 1 bis 6, **dadurch gekennzeichnet, daß** es
- vorzugsweise die fließfähige Zubereitung B - zusätzlich kationische und/oder amphotere Polymere, vorzugsweise
- Poly(methacryloyloxyethyltrimethylammoniumchlorid) (INCI: Polyquaternium-37) und/oder;
- quaternisierte Cellulose-Derivate (INCI: Polyquaternium 10) und/oder
- kationische Alkylpolyglycoside und/oder
- kationiserter Honig und/oder
- kationische Guar-Derivate und/oder
- polymere Dimethyldiallylammoniumsalze und deren Copolymere mit Estern und Amiden von Acrylsäure und Methacrylsäure und/oder
- Copolymere des Vinylpyrrolidons mit quaternierten Derivaten des Dialkylaminoalkylacrylats und -methacrylats und/oder
- Vinylpyrrolidon-Vinylimidazoliummethochlorid-Copolymere und/oder
- quaternierter Polyvinylalkohol und/oder
- Polyquaternium 2 und/oder
- Polyquaternium 17 und/oder
- Polyquaternium 18 und/oder
- Polyquaternium 24 und/oder
- Polyquaternium 27
vorzugsweise in Mengen von 0,1 bis 5 Gew.-%, besonders bevorzugt von 0,2 bis 4 Gew.-% und insbesondere von 0,4 bis 2,5 Gew.-%, jeweils bezogen auf das Mittel, enthält.

8. Kosmetisches Mittel nach einem der Ansprüche 1 bis 7, **dadurch gekennzeichnet, daß** es mindestens ein Oxidationsfarbstoffvorprodukt vom Entwicklertyp und/oder Kupplertyp enthält, wobei bevorzugte Entwicklerkomponenten ausgewählt sind aus p-Phenylendiamin, p-Toluylendiamin, , N,N-Bis-(2-hydroxyethyl)amino-p-phenylendiamin, 1,3-Bis-[(2-hydroxyethyl-4'-aminophenyl)amino]-propan-2-ol, 1,10-Bis-(2',5'-diaminophenyl)-1,4,7,10-tetraoxadecan, 4-Aminophenol, 4- Amino-3-methylphenol, Bis-(5- amino-2-hydroxyphenyl)methan, 2,4,5,6- Tetraaminopyrimidin, 2-Hydroxy-4,5,6-triaminopyrimidin, 4,5-Diamino-1-(2-hydroxyethyl)-pyrazol und bevorzugte Kupplerkomponenten ausgewählt sind aus Resorcin, 2-Methylresorcin, 5-Methyl-resorcin, 2,5-Dimethylresorcin, 4-Chlorresorcin, Resorcinmonomethylether, 5-Aminophenol, 5-Amino-2-methylphenol, 5-(2-Hydroxyethyl)amino-2-methylphenol, 3- Amino-4-chlor-2-methylphenol, 3-Amino-2-chlor-6-methylphenol, 3-Amino-2,4-Dichlorphenol, 2,4-Diaminophenoxyethanol, 2-Amino-4-(2'-hydroxyethyl)amino-anisolsulfat, 1,3-Bis-(2,4-diaminophenoxy)propan, 2-Amino-3-hydroxypyridin, 2-Methylamino-3-amino-6-methoxypyridin, 2,6-Dihydroxy-3,4-dimethylpyridin, 3,5-Diamino-2,6-dimethoxypyridin, 1-Naphthol, 2-Methyl-1-naphthol, 1,5-Dihydroxynaphthalin, 2,7-Dihydroxynaphthalin, 1-Phenyl-3-methylpyrazol-5-on, 2,6-Bis-[(2'-hydroxyethyl)amino]-toluol, 4-Hydroxyindol, 6-Hydroxyindol, 6-Hydroxybenzomorpholin.

9. Kosmetisches Mittel nach einem der Ansprüche 1 bis 8, **dadurch gekennzeichnet, daß** es mindestens einen direktziehenden Farbstoff enthält, der ausgewählt ist aus Nitrophenylendiaminen, Nitroaminophenolen, Azofarbstoffen, Anthrachinonen oder Indophenolen, vorzugsweise aus der Gruppe der unter den internationalen Bezeichnungen bzw. Handelsnamen bekannten Farbstoffe HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2-Hydroxyethyl)amino-4,6-dinitrophenol, 1-(2'-Hydroxyethyl)amino-4-methyl-2-nitrobenzol, 1-Amino-4-(2-hydroxyethyl)-amino-5-chlor-2-nitrobenzol, 4-Amino-3-nitrophenol, 1-(2-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

10. Kosmetisches Mittel nach einem der Ansprüche 1 bis 9, **dadurch gekennzeichnet, daß** die Zubereitung A und/oder die Zubereitung B mindestens eine C,H-acide Verbindung enthalten und dem Mittel unmittelbar vor der Anwendung eine weitere Zubereitung C zugemischt wird, die mindestens eine reaktive Carbonylverbindung enthält.

11. Kosmetisches Mittel nach einem der Ansprüche 1 bis 9, **dadurch gekennzeichnet, daß** die Zubereitung A und/oder die Zubereitung B mindestens eine reaktive Carbonylverbindung enthalten und dem Mittel unmittelbar vor der Anwendung eine weitere Zubereitung C zugemischt wird, die mindestens eine C,H-acide Verbindung enthält.

12. Verfahren zur Behandlung keratinischer Fasern, insbesondere menschlicher Haare, **dadurch gekennzeichnet, daß** ein kosmetisches Mittel nach einem der Ansprüche 1 bis 9 auf die Fasern aufgetragen und nach einer Einwirkzeit von 1 bis 20 Minuten wieder ausgespült wird.

13. Verwendung von ionischen Flüssigkeiten zur Viskositätssteigerung aniontensidhaltiger Mittel.
